# EUROPEAN PATENT SPECIFICATION

(11) **EP 1 252 300 B1**
(45) Date of publication and mention of the grant of the patent: **19.01.2011**
(21) Application number: 01905377.6
(22) Date of filing: 02.02.2001
(51) Int. Cl.: C12N 9/64

(54) **NUCLEIC ACID MOLECULES ENCODING TRANSMEMBRANE SERINE PROTEASES, THE ENCODED PROTEINS AND METHODS BASED THEREON**
NUKLEINSÄUREN, WELCHE FÜR TRAMSMEMBRAN SERIN PROTEASEN KODIEREN, DIE KODIERTEN PROTEINE UND DARAUF BASIERENDE METHODEN
MOLECULES D'ACIDES NUCLEIQUES CODANT POUR DES PROTEASES A SERINE TRANSMEMBRANAIRES, PROTEINES CODEES ET PROCEDES ASSOCIES

(30) Priority: 03.02.2000 US 179982 P; 18.02.2000 US 183542 P; 22.06.2000 US 213124 P; 26.07.2000 US 220970 P; 08.09.2000 US 657986; 22.09.2000 US 234840 P
(43) Date of publication of application: 30.10.2002
(73) Proprietor: DENDREON CORPORATION, Seattle, Washington 98121 (US)
(72) Inventor: MADISON, Edwin, L., San Diego, CA 92121 (US); ONG, Edgar, O., San Diego, CA 92122 (US); YEH, Jiunn-chern, San Diego, CA 92126 (US)
(74) Representative: Baldock, Sharon Claire
(86) International application number: PCT/US2001/003471
(87) International publication number: WO 2001/057194

(56) References cited:
- WO-A-00/52044
- WO-A-01/04141
- WO-A-99/36550
- WO-A-99/42120
- LIN C-Y ET AL: "Molecular cloning of cDNA for Matriptase, a matrix-degrading serine protease with trypsin-like activity" JOURNAL OF BIOLOGICAL CHEMISTRY,US,AMERICAN SOCIETY OF BIOLOGICAL CHEMISTS, BALTIMORE, MD, vol. 274, no. 26, 25 June 1999 (1999-06-25), pages 8231-18236, XP002139963 ISSN: 0021-9258 cited in the application
- DATABASE EMBL [Online] Accession Number W22987, 8 October 1997 (1997-10-08) SUNTORY LDT: "Human Serine Protease 67" XP002169836 & PATENT ABSTRACTS OF JAPAN vol. 1997, no. 10, 31 October 1997 (1997-10-31) & JP 09 149790 A (SUNTORY LTD), 10 June 1997 (1997-06-10)
- DATABASE EMBL [Online] Accession Number AAY41710, 7 December 1999 (1999-12-07) GENENTECH INC.: "Human PRO618 protein sequence" XP002175683 & WO 99 46281 A (GENENTECH INC.) 16 September 1999 (1999-09-16)
- DATABASE EMBL [Online] Accession Number AAZ34033, 7 December 1999 (1999-12-07) GENENTECH INC: "Human PRO618 nucleotide sequence" XP002175684 & WO 99 46281 A (GENENTECH INC.) 16 September 1999 (1999-09-16)
- DATABASE EMBL [Online] Accession Number AAZ33949, 7 December 1999 (1999-12-07) GENENTECH INC.: "Human PRO382 nucloetide sequence" XP002175685 & WO 99 46281 A (GENENTECH INC.) 16 September 1999 (1999-09-16)
- DATABASE EMBL [Online] Accession Number AAY41694, 7 December 1999 (1999-12-07) GENENTECH INC.: "Human PRO382 protein sequence" XP002175687 & WO 99 46281 A (GENENTECH INC.) 16 September 1999 (1999-09-16)
- DATABASE EMBL [Online] Accession Number AI469095, 17 March 1999 (1999-03-17) NCI-CGAP: "tm06c09.x1 Homo sapiens cDNA clone IMAGE:2155792" XP002175686
- TAKEUCHI TOSHIHIKO ET AL: "Reverse biochemistry: Use of macromolecular protease inhibitors to dissect complex biological processes and identify a membrane-type serine protease in epithelial cancer and normal tissue." PROCEEDINGS OF THE NATIONAL ACADEMY OF SCIENCES OF THE UNITED STATES, vol. 96, no. 20, 28 September 1999 (1999-09-28), pages 11054-11061, XP002169835 ISSN: 0027-8424 cited in the application
- VALLIE LA E R ET AL: "CLONING AND FUNCTIONAL EXPRESSION OF A CDNA ENCODING THE CATALYTIC SUBUNIT OF BOVINE ENTEROKINASE" JOURNAL OF BIOLOGICAL CHEMISTRY, AMERICAN SOCIETY OF BIOLOGICAL CHEMISTS, BALTIMORE, MD, US, vol. 268, no. 31, 5 November 1993 (1993-11-05), pages 23311-23317, XP000999230 ISSN: 0021-9258
- LU DESHUN ET AL: "Bovine proenteropeptidase is activated by trypsin, and the specificity of enteropeptidase depends on the heavy chain." JOURNAL OF BIOLOGICAL CHEMISTRY, vol. 272, no. 50, 12 December 1997 (1997-12-12), pages 31293-31300, XP000999222 ISSN: 0021-9258
- TAKEUCHI TOSHIHIKO ET AL: "Cellular localization of membrane-type serine protease 1 and identification of protease-activated receptor-2 and single-chain urokinase-type plasminogen activator as substrates." JOURNAL OF BIOLOGICAL CHEMISTRY, vol. 275, no. 34, 25 August 2000 (2000-08-25), pages 26333-26342, XP001000977 ISSN: 0021-9258 cited in the application
- LEE SHEAU-LING ET AL: "Activation of hepatocyte growth factor and urokinase/plasminogen activator by matriptase, an epithelial membrane serine protease." JOURNAL OF BIOLOGICAL CHEMISTRY, vol. 275, no. 47, 24 November 2000 (2000-11-24), pages 36720-36725, XP000999224 ISSN: 0021-9258
- VU T.-K. ET AL: 'Identification and cloning of the membrane-associated serine protease, hepsin, from mouse preimplantation embryos' THE JOURNAL OF BIOLOGICAL CHEMISTRY vol. 272, no. 50, 12 December 1997, pages 31315 - 31320
- YAN W. ET AL: 'Corin, a mosaic transmembrane serine protease encoded by a novel cDNA from human heart' THE JOURNAL OF BIOLOGICAL CHEMISTRY vol. 274, no. 21, 21 May 1999, pages 14926 - 14935
- HOOPER J.D. ET AL: 'Localization of the mosaic transmembrane serine protease corin to heart myocytes' EUR J BIOCHEM vol. 267, 2000, pages 6931 - 9637
- HOOPER J.D. ET AL: 'Type II transmembrane serine proteases' THE JOURNAL OF BIOLOGICAL CHEMISTRY vol. 276, no. 2, 12 January 2001, pages 857 - 860

### SEQUENCE LISTING

<110> Edwin L. Madison
   Edgar O. Ong
   Jiunn-Chern Yeh
   Corvas International, Inc.
<120> NUCLEIC ACID MOLECULES ENCODING TRANSMEMBRANE SERINE PROTEASES, THE ENCODED PROTEINS AND METHODS BASED THEREON
<130> 24745-1607
<140> 09/000,000
   <141> 2001-02-01
<150> 60/213,124
   <151> 2000-06-22
<150> 60/234,840
   <151> 2000-06-22
<150> 60/179,982
   <151> 2000-02-03
<150> 60/183,542
   <151> 2000-02-18
<150> 09/657,968
   <151> 2000-02-08
<160> 72
<170> FastSEQ for Windows Version 4.0
<210> 1
   <211> 3147
   <212> DNA
   <213> Homo Sapien
<220>
   <223> Nucleotide encoding MTSP1
<221> CDS
   <222> (23)...(2589)
<300>
   <301> O'Brien, T.J. and Tanimoto, H.
   <308> GenBank AR081724
   <310> US Pat 5972616
   <311> 1998-02-20
   <312> 1999-10-26
<400> 1
   tcaagagcgg cctcggggta cc atg ggg agc gat cgg gcc cgc aag ggc gga      52
   ggg ggc ccg aag gac ttc ggc gcg gga ctc aag tac aac tcc cgg cac      100
   gag aaa gtg aat ggc ttg gag gaa ggc gtg gag ttc ctg cca gtc aac      148
   aac gtc aag aag gtg gaa aag cat ggc ccg ggg cgc tgg gtg gtg ctg      196
   gca gcc gtg ctg atc ggc ctc ctc ttg gtc ttg ctg ggg atc ggc ttc      244
   ctg gtg tgg cat ttg cag tac cgg gac gtg cgt gtc cag aag gtc ttc      292
   aat ggc tac atg agg atc aca aat gag aat ttt gtg gat gcc tac gag      340
   aac tcc aac tcc act gag ttt gta agc ctg gcc agc aag gtg aag gac      388
   gcg ctg aag ctg ctg tac agc gga gtc cca ttc ctg ggc ccc tac cac      436
   aag gag tcg gct gtg acg gcc ttc agc gag ggc agc gtc atc gcc tac      484
   tac tgg tct gag ttc agc atc ccg cag cac ctg gtg gag gag gcc gag      532
   cgc gtc atg gcc gag gag cgc gta gtc atg ctg ccc ccg cgg gcg cgc      580
   tcc ctg aag tcc ttt gtg gtc acc tca gtg gtg gct ttc ccc acg gac      628
   tcc aaa aca gta cag agg acc cag gac aac agc tgc agc ttt ggc ctg      676
   cac gcc cgc ggt gtg gag ctg atg cgc ttc acc acg ccc ggc ttc cct      724
   gac agc ccc tac ccc gct cat gcc cgc tgc cag tgg gcc ctg cgg ggg      772
   gac gcc gac tca gtg ctg agc ctc acc ttc cgc agc ttt gac ctt gcg      820
   tcc tgc gac gag cgc ggc agc gac ctg gtg acg gtg tac aac acc ctg      868
   agc ccc atg gag ccc cac gcc ctg gtg cag ttg tgt ggc acc tac cct       916
   ccc tcc tac aac ctg acc ttc cac tcc tcc cag aac gtc ctg ctc atc       964
   aca ctg ata acc aac act gag cgg cgg cat ccc ggc ttt gag gcc acc      1012
   ttc ttc cag ctg cct agg atg agc agc tgt gga ggc cgc tta cgt aaa      1060
   gcc cag ggg aca ttc aac agc ccc tac tac cca ggc cac tac cca ccc      1108
   aac att gac tgc aca tgg aac att gag gtg ccc aac aac cag cat gtg      1156
   aag gtg agc ttc aaa ttc ttc tac ctg ctg gag ccc ggc gtg cct gcg      1204
   ggc acc tgc ccc aag gac tac gtg gag atc aat ggg gag aaa tac tgc      1252
   gga gag agg tcc cag ttc gtc gtc acc agc aac agc aac aag atc aca      1300
   gtt cgc ttc cac tca gat cag tcc tac acc gac acc ggc ttc tta gct      1348
   gaa tac ctc tcc tac gac tcc agt gac cca tgc ccg ggg cag ttc acg      1396
   tgc cgc acg ggg cgg tgt atc cgg aag gag ctg cgc tgt gat ggc tgg      1444
   gcc gac tgc acc gac cac agc gat gag ctc aac tgc agt tgc gac gcc      1492
   ggc cac cag ttc acg tgc aag aac aag ttc tgc aag ccc ctc ttc tgg      1540
   gtc tgc gac agt gtg aac gac tgc gga gac aac agc gac gag cag ggg      1588
   tgc agt tgt ccg gcc cag acc ttc agg tgt tcc aat ggg aag tgc ctc      1636
   tcg aaa agc cag cag tgc aat ggg aag gac gac tgt ggg gac ggg tcc      1684
   gac gag gcc tcc tgc ccc aag gtg aac gtc gtc act tgt acc aaa cac      1732
   acc tac cgc tgc ctc aat ggg ctc tgc ttg agc aag ggc aac cct gag      1780
   tgt gac ggg aag gag gac tgt agc gac ggc tca gat gag aag gac tgc      1828
   gac tgt ggg ctg cgg tca ttc acg aga cag gct cgt gtt gtt ggg ggc      1876
   acg gat gcg gat gag ggc gag tgg ccc tgg cag gta agc ctg cat gct      1924
   ctg ggc cag ggc cac atc tgc ggt gct tcc ctc atc tct ccc aac tgg      1972
   ctg gtc tct gcc gca cac tgc tac atc gat gac aga gga ttc agg tac      2020
   tca gac ccc acg cag tgg acg gcc ttc ctg ggc ttg cac gac cag agc      2068
   cag cgc agc gcc cct ggg gtg cag gag cgc agg ctc aag cgc atc atc      2116
   tcc cac ccc ttc ttc aat gac ttc acc ttc gac tat gac atc gcg ctg      2164
   ctg gag ctg gag aaa ccg gca gag tac agc tcc atg gtg cgg ccc atc      2212
   tgc ctg ccg gac gcc tcc cat gtc ttc cct gcc ggc aag gcc atc tgg      2260
   gtc acg ggc tgg gga cac acc cag tat gga ggc act ggc gcg ctg atc      2308
   ctg caa aag ggt gag atc cgc gtc atc aac cag acc acc tgc gag aac      2356
   ctc ctg ccg cag cag atc acg ccg cgc atg atg tgc gtg ggc ttc ctc       2404
   agc ggc ggc gtg gac tcc tgc cag ggt gat tcc ggg gga ccc ctg tcc       2452
   agc gtg gag gcg gat ggg cgg atc ttc cag gcc ggt gtg gtg agc tgg       2500
   gga gac ggc tgc gct cag agg aac aag cca ggc gtg tac aca agg ctc       2548
   cct ctg ttt cgg gac tgg atc aaa gag aac act ggg gta ta ggggccgggg     2599
   ccacccaaat gtgtacacct gcggggccac ccatcgtcca ccccagtgtg cacgcctgca     2659
   ggctggagac tggaccgctg actgcaccag cgcccccaga acatacactg tgaactcaat     2719
   ctccagggct ccaaatctgc ctagaaaacc tctcgcttcc tcagcctcca aagtggagct     2779
   gggaggtaga aggggaggac actggtggtt ctactgaccc aactgggggc aaaggtttga     2839
   agacacagcc tcccccgcca gccccaagct gggccgaggc gcgtttgtgt atatctgcct     2899
   cccctgtctg taaggagcag cgggaacgga gcttcggagc ctcctcagtg aaggtggtgg     2959
   ggctgccgga tctgggctgt ggggcccttg ggccacgctc ttgaggaagc ccaggctcgg     3019
   aggaccctgg aaaacagacg ggtctgagac tgaaattgtt ttaccagctc ccagggtgga     3079
   cttcagtgtg tgtatttgtg taaatgggta aaacaattta tttcttttta aaaaaaaaaa     3139
   aaaaaaaa                                                              3147
<210> 2
   <211> 855
   <212> PRT
   <213> Homo Sapien
<400> 2
<210> 3
   <211> 2137
   <212> DNA
   <213> Homo Sapien
<220>
   <221> CDS
   <222> (261)...(1574)
   <223> DNA sequence encoding a transmembrane serine protease (MTSP3) protein
<400> 3
   ccatcctaat acgactcact atagggctcg agcggccgcc cgggcaggtc agagagaggc     60
   agcagcttgc tcagcggaca aggatgctgg gcgtgaggga ccaaggcctg ccctgcactc    120
   gggcctcctc cagccagtgc tgaccaggga cttctgacct gctggccagc caggacctgt    180
   gtggggaggc cctcctgctg ccttggggtg acaatctcag ctccaggcta cagggagacc    240
   gggaggatca cagagccagc atg tta cag gat cct gac agt gat caa cct ctg    293
                         Met Leu Gln Asp Pro Asp Ser Asp Gln Pro Leu
                           1               5                  10
   aac agc ctc gat gtc aaa ccc ctg cgc aaa ccc cgt atc ccc atg gag      341
   Asn Ser Leu Asp Val Lys Pro Leu Arg Lys Pro Arg Ile Pro Met Glu
               15                  20                  25
   acc ttc aga aag gtg ggg atc ccc atc atc ata gca cta ctg agc ctg      389
   Thr Phe Arg Lys Val Gly Ile Pro Ile Ile Ile Ala Leu Leu Ser Leu
           30                  35                  40
   gcg agt atc atc att gtg gtt gtc ctc atc aag gtg att ctg gat aaa      437
   Ala Ser Ile Ile Ile Val Val Val Leu Ile Lys Val Ile Leu Asp Lys
       45                  50                  55
   tac tac ttc ctc tgc ggg cag cct ctc cac ttc atc ccg agg aag cag      485
   Tyr Tyr Phe Leu Cys Gly Gln Pro Leu His Phe Ile Pro Arg Lys Gln
    60                  65                  70                  75
   ctg tgt gac gga gag ctg gac tgt ccc ttg ggg gag gac gag gag cac      533
   Leu Cys Asp Gly Glu Leu Asp Cys Pro Leu Gly Glu Asp Glu Glu His
                       80                                     85                                   90
   tgt gtc aag agc ttc ccc gaa ggg cct gca gtg gca gtc cgc ctc tcc          581
   Cys Val Lys Ser Phe Pro Glu Gly Pro Ala Val Ala Val Arg Leu Ser
                   95                  100                 105
   aag gac cga tcc aca ctg cag gtg ctg gac tcg gcc aca ggg aac tgg          629
   Lys Asp Arg Ser Thr Leu Gln Val Leu Asp Ser Ala Thr Gly Asn Trp
           110                 115                 120
   ttc tct gcc tgt ttc gac aac ttc aca gaa gct ctc gct gag aca gcc          677
   Phe Ser Ala Cys Phe Asp Asn Phe Thr Glu Ala Leu Ala Glu Thr Ala
       125                 130                 135
   tgt agg cag atg ggc tac agc agc aaa ccc acc ttc aga gct gtg gag          725
   Cys Arg Gln Met Gly Tyr Ser Ser Lys Pro Thr Phe Arg Ala Val Glu
   140                 145                 150                 155
   att ggc cca gac cag gat ctg gat gtt gtt gaa atc aca gaa aac agc          773
   Ile Gly Pro Asp Gln Asp Leu Asp Val Val Glu Ile Thr Glu Asn Ser
                   160                 165                 170
   cag gag ctt cgc atg cgg aac tca agt ggg ccc tgt ctc tca ggc tcc          821
   Gln Glu Leu Arg Met Arg Asn Ser Ser Gly Pro Cys Leu Ser Gly Ser
               175                 180                 185
   ctg gtc tcc ctg cac tgt ctt gcc tgt ggg aag agc ctg aag acc ccc          869
   Leu Val Ser Leu His Cys Leu Ala Cys Gly Lys Ser Leu Lys Thr Pro
           190                 195                 200
   cgt gtg gtg ggt ggg gag gag gcc tct gtg gat tct tgg cct tgg cag          917
   Arg Val Val Gly Gly Glu Glu Ala Ser Val Asp Ser Trp Pro Trp Gln
       205                 210                 215
   gtc agc atc cag tac gac ata cag cac gtc tgt gga ggg agc atc ctg          965
   Val Ser Ile Gln Tyr Asp Ile Gln His Val Cys Gly Gly Ser Ile Leu
   220                 225                 230                 235
   gac ccc cac tgg gtc ctc acg gca gcc cac tgc ttc agg aaa cat acc         1013
   Asp Pro His Trp Val Leu Thr Ala Ala His Cys Phe Arg Lys His Thr
                   240                 245                 250
   gat gtg ttc aac tgg aag gtg cgg gca ggc tca gac aaa ctg ggc agc         1061
   Asp Val Phe Asn Trp Lys Val Arg Ala Gly Ser Asp Lys Leu Gly Ser
               255                 260                 265
   ttc cca tcc ctg gct gtg gcc aag atc atc atc att gaa ttc aac ccc         1109
   Phe Pro Ser Leu Ala Val Ala Lys Ile Ile Ile Ile Glu Phe Asn Pro
           270                 275                 280
   atg tac ccc aaa gac aat gac atc gcc ctc atg aag ctg cag ttc cca         1157
   Met Tyr Pro Lys Asp Asn Asp Ile Ala Leu Met Lys Leu Gln Phe Pro
       285                 290                 295
   ctc act ttc tca ggc aca gtc agg ctc atc tgt ctg ccc ttc ttt gat         1205
   Leu Thr Phe Ser Gly Thr Val Arg Leu Ile Cys Leu Pro Phe Phe Asp
   300                 305                 310                 315
   gag gag ctc act cca gcc acc cca ctc tgg atc att gga tgg ggc ttt         1253
   Glu Glu Leu Thr Pro Ala Thr Pro Leu Trp Ile Ile Gly Trp Gly Phe
                   320                 325                 330
   acg aag cag aat gga ggg aag atg tct gac ata ctg ctg cag gcg tca      1301
   Thr Lys Gln Asn Gly Gly Lys Met Ser Asp Ile Leu Leu Gln Ala Ser
               335                 340                 345
   gtc cag gtc att gac agc aca cgg tgc aat gca gac gat gcg tac cag      1349
   Val Gln Val Ile Asp Ser Thr Arg Cys Asn Ala Asp Asp Ala Tyr Gln
           350                 355                 360
   ggg gaa gtc acc gag aag atg atg tgt gca ggc atc ccg gaa ggg ggt      1397
   Gly Glu Val Thr Glu Lys Met Met Cys Ala Gly Ile Pro Glu Gly Gly
       365                 370                 375
   gtg gac acc tgc cag ggt gac agt ggt ggg ccc ctg atg tac caa tct      1445
   Val Asp Thr Cys Gln Gly Asp Ser Gly Gly Pro Leu Met Tyr Gln Ser
   380                 385                 390                 395
   gac cag tgg cat gtg gtg ggc atc gtt agc tgg ggc tat ggc tgc ggg      1493
   Asp Gln Trp His Val Val Gly Ile Val Ser Trp Gly Tyr Gly Cys Gly
                   400                 405                 410
   ggc ccg agc acc cca gga gta tac acc aag gtc tca gcc tat ctc aac      1541
   Gly Pro Ser Thr Pro Gly Val Tyr Thr Lys Val Ser Ala Tyr Leu Asn
               415                 420                 425
   tgg atc tac aat gtc tgg aag gct gag ctg taa tgctgctgcc cctttgcagt    1594
   Trp Ile Tyr Asn Val Trp Lys Ala Glu Leu  *
           430                 435
   gctgggagcc gcttccttcc tgccctgccc acctggggat cccccaaagt cagacacaga    1654
   gcaagagtcc ccttgggtac acccctctgc ccacagcctc agcatttctt ggagcagcaa    1714
   agggcctcaa ttcctgtaag agaccctcgc agcccagagg cgcccagagg aagtcagcag    1774
   ccctagctcg gccacacttg gtgctcccag catcccaggg agagacacag cccactgaac    1834
   aaggtctcag gggtattgct aagccaagaa ggaactttcc cacactactg aatggaagca     1894
   ggctgtcttg taaaagccca gatcactgtg ggctggagag gagaaggaaa gggtctgcgc    1954
   cagccctgtc cgtcttcacc catccccaag cctactagag caagaaacca gttgtaatat    2014
   aaaatgcact gccctactgt tggtatgact accgttacct actgttgtca ttgttattac    2074
   agctatggcc actattatta aagagctgtg taacaaaaaa aaaaaaaaaa aaaaaaaaaa    2134
   aaa                                                                   2137
<210> 4
   <211> 437
   <212> PRT
   <213> Homo Sapien
<400> 4
   Met Leu Gln Asp Pro Asp Ser Asp Gln Pro Leu Asn Ser Leu Asp Val
    1              5                   10                  15
   Lys Pro Leu Arg Lys Pro Arg Ile Pro Met Glu Thr Phe Arg Lys Val
               20                  25                  30
   Gly Ile Pro Ile Ile Ile Ala Leu Leu Ser Leu Ala Ser Ile Ile Ile
           35                  40                  45
   Val Val Val Leu Ile Lys Val Ile Leu Asp Lys Tyr Tyr Phe Leu Cys
       50                  55                  60
   Gly Gln Pro Leu His Phe Ile Pro Arg Lys Gln Leu Cys Asp Gly Glu
   65                  70                  75                  80
   Leu Asp Cys Pro Leu Gly Glu Asp Glu Glu His Cys Val Lys Ser Phe
                   85                  90                  95
   Pro Glu Gly Pro Ala Val Ala Val Arg Leu Ser Lys Asp Arg Ser Thr
               100                 105                 110
   Leu Gln Val Leu Asp Ser Ala Thr Gly Asn Trp Phe Ser Ala Cys Phe
           115                 120                 125
   Asp Asn Phe Thr Glu Ala Leu Ala Glu Thr Ala Cys Arg Gln Met Gly
       130                 135                 140
   Tyr Ser Ser Lys Pro Thr Phe Arg Ala Val Glu Ile Gly Pro Asp Gln
   145                 150                 155                 160
   Asp Leu Asp Val Val Glu Ile Thr Glu Asn Ser Gln Glu Leu Arg Met
                   165                 170                 175
   Arg Asn Ser Ser Gly Pro Cys Leu Ser Gly Ser Leu Val Ser Leu His
               180                 185                 190
   Cys Leu Ala Cys Gly Lys Ser Leu Lys Thr Pro Arg Val Val Gly Gly
           195                 200                 205
   Glu Glu Ala Ser Val Asp Ser Trp Pro Trp Gln Val Ser Ile Gln Tyr
       210                 215                 220
   Asp Ile Gln His Val Cys Gly Gly Ser Ile Leu Asp Pro His Trp Val
   225                 230                 235                 240
   Leu Thr Ala Ala His Cys Phe Arg Lys His Thr Asp Val Phe Asn Trp
                   245                 250                 255
   Lys Val Arg Ala Gly Ser Asp Lys Leu Gly Ser Phe Pro Ser Leu Ala
               260                 265                 270
   Val Ala Lys Ile Ile Ile Ile Glu Phe Asn Pro Met Tyr Pro Lys Asp
           275                 280                 285
   Asn Asp Ile Ala Leu Met Lys Leu Gln Phe Pro Leu Thr Phe Ser Gly
       290                 295                 300
   Thr Val Arg Leu Ile Cys Leu Pro Phe Phe Asp Glu Glu Leu Thr Pro
   305                 310                 315                 320
   Ala Thr Pro Leu Trp Ile Ile Gly Trp Gly Phe Thr Lys Gln Asn Gly
                   325                 330                 335
   Gly Lys Met Ser Asp Ile Leu Leu Gln Ala Ser Val Gln Val Ile Asp
               340                 345                 350
   Ser Thr Arg Cys Asn Ala Asp Asp Ala Tyr Gln Gly Glu Val Thr Glu
           355                 360                 365
   Lys Met Met Cys Ala Gly Ile Pro Glu Gly Gly Val Asp Thr Cys Gln
       370                 375                 380
   Gly Asp Ser Gly Gly Pro Leu Met Tyr Gln Ser Asp Gln Trp His Val
   385                 390                 395                 400
   Val Gly Ile Val Ser Trp Gly Tyr Gly Cys Gly Gly Pro Ser Thr Pro
                   405                 410                 415
   Gly Val Tyr Thr Lys Val Ser Ala Tyr Leu Asn Trp Ile Tyr Asn Val
               420                 425                 430
   Trp Lys Ala Glu Leu
           435
<210> 5
   <211> 708
   <212> DNA
   <213> Homo Sapien
<220>
   <221> CDS
   <222> (1)...(708)
   <223> MTSP4 protease domain cDNA
<400> 5
   att gtt ggt gga gct gtg tcc tcc gag ggt gag tgg cca tgg cag gcc 48
   Ile Val Gly Gly Ala Val Ser Ser Glu Gly Glu Trp Pro Trp Gln Ala
   1 5 10 15
   agc ctc cag gtt cgg ggt cga cac atc tgt ggg ggg gcc ctc atc gct 96
   Ser Leu Gln Val Arg Gly Arg His Ile Cys Gly Gly Ala Leu Ile Ala
   20 25 30
   gac cgc tgg gtg ata aca gct gcc cac tgc ttc cag gag gac agc atg 144
   Asp Arg Trp Val Ile Thr Ala Ala His Cys Phe Gln Glu Asp Ser Met
   35 40 45
   gcc tcc acg gtg ctg tgg acc gtg ttc ctg ggc aag gtg tgg cag aac 192
   Ala Ser Thr Val Leu Trp Thr Val Phe Leu Gly Lys Val Trp Gln Asn
   50 55 60
   tcg cgc tgg cct gga gag gtg tcc ttc aag gtg agc cgc ctg ctc ctg 240
   Ser Arg Trp Pro Gly Glu Val Ser Phe Lys Val Ser Arg Leu Leu Leu
   65 70 75 80
   cac ccg tac cac gaa gag gac agc cat gac tac gac gtg gcg ctg ctg 288
   His Pro Tyr His Glu Glu Asp Ser His Asp Tyr Asp Val Ala Leu Leu
   85 90 95
   cag ctc gac cac ccg gtg gtg cgc tcg gcc gcc gtg cgc ccc gtc tgc 336
   Gln Leu Asp His Pro Val Val Arg Ser Ala Ala Val Arg Pro Val Cys
   100 105 110
   ctg ccc gcg cgc tcc cac ttc ttc gag ccc ggc ctg cac tgc tgg att 384
   Leu Pro Ala Arg Ser His Phe Phe Glu Pro Gly Leu His Cys Trp Ile
   115 120 125
   acg ggc tgg ggc gcc ttg cgc gag ggc ggc ccc atc agc aac gct ctg 432
   Thr Gly Trp Gly Ala Leu Arg Glu Gly Gly Pro Ile Ser Asn Ala Leu
   130 135 140
   cag aaa gtg gat gtg cag ttg atc cca cag gac ctg tgc agc gag gtc 480
   Gln Lys Val Asp Val Gln Leu Ile Pro Gln Asp Leu Cys Ser Glu Val
   145 150 155 160
   tat cgc tac cag gtg acg cca cgc atg ctg tgt gcc ggc tac cgc aag 528
   Tyr Arg Tyr Gln Val Thr Pro Arg Met Leu Cys Ala Gly Tyr Arg Lys
   165 170 175
   ggc aag aag gat gcc tgt cag ggt gac tca ggt ggt ccg ctg gtg tgc 576
   Gly Lys Lys Asp Ala Cys Gln Gly Asp Ser Gly Gly Pro Leu Val Cys
   180 185 190
   aag gca ctc agt ggc cgc tgg ttc ctg gcg ggg ctg gtc agc tgg ggc 624
   Lys Ala Leu Ser Gly Arg Trp Phe Leu Ala Gly Leu Val Ser Trp Gly
   195 200 205
   ctg ggc tgt ggc cgg cct aac tac ttc ggc gtc tac acc cgc atc aca 672
   Leu Gly Cys Gly Arg Pro Asn Tyr Phe Gly Val Tyr Thr Arg Ile Thr
   210 215 220
   ggt gtg atc agc tgg atc cag caa gtg gtg acc tga 708
   Gly Val Ile Ser Trp Ile Gln Gln Val Val Thr *
   225 230 235
<210> 6
   <211> 235
   <212> PRT
   <213> Homo Sapien
<400> 6
   Ile Val Gly Gly Ala Val Ser Ser Glu Gly Glu Trp Pro Trp Gln Ala
    1               5                  10                  15
   Ser Leu Gln Val Arg Gly Arg His Ile Cys Gly Gly Ala Leu Ile Ala
               20                  25                  30
   Asp Arg Trp Val Ile Thr Ala Ala His Cys Phe Gln Glu Asp Ser Met
           35                  40                  45
   Ala Ser Thr Val Leu Trp Thr Val Phe Leu Gly Lys Val Trp Gln Asn
       50                  55                  60
   Ser Arg Trp Pro Gly Glu Val Ser Phe Lys Val Ser Arg Leu Leu Leu
   65                  70                  75                  80
   His Pro Tyr His Glu Glu Asp Ser His Asp Tyr Asp Val Ala Leu Leu
                   85                  90                  95
   Gln Leu Asp His Pro Val Val Arg Ser Ala Ala Val Arg Pro Val Cys
               100                 105                 110
   Leu Pro Ala Arg Ser His Phe Phe Glu Pro Gly Leu His Cys Trp Ile
           115                 120                 125
   Thr Gly Trp Gly Ala Leu Arg Glu Gly Gly Pro Ile Ser Asn Ala Leu
       130                 135                 140
   Gln Lys Val Asp Val Gln Leu Ile Pro Gln Asp Leu Cys Ser Glu Val
   145                 150                 155                 160
   Tyr Arg Tyr Gln Val Thr Pro Arg Met Leu Cys Ala Gly Tyr Arg Lys
                   165                 170                 175
   Gly Lys Lys Asp Ala Cys Gln Gly Asp Ser Gly Gly Pro Leu Val Cys
               180                 185                 190
   Lys Ala Leu Ser Gly Arg Trp Phe Leu Ala Gly Leu Val Ser Trp Gly
           195                 200                 205
   Leu Gly Cys Gly Arg Pro Asn Tyr Phe Gly Val Tyr Thr Arg Ile Thr
       210                 215                 220
   Gly Val Ile Ser Trp Ile Gln Gln Val Val Thr
   225                 230                 235
<210> 7
   <211> 3104
   <212> DNA
   <213> Homo Sapien
<220>
   <221> CDS
   <222> (33)...(2441)
   <223> cDNA encoding :MTSP4-L (long form) splice variant
<400> 7
   tcatcggcca gagggtgatc agtgagcaga ag atg ccc gtg gcc gag gcc ccc       53
                                       Met Pro Val Ala Glu Ala Pro
                                       1               5
   cag gtg gct ggc ggg cag ggg gac gga ggt gat ggc gag gaa gcg gag      101
   Gln Val Ala Gly Gly Gln Gly Asp Gly Gly Asp Gly Glu Glu Ala Glu
           10                  15                  20
   ccg gag ggg atg ttc aag gcc tgt gag gac tcc aag aga aaa gcc cgg      149
   Pro Glu Gly Met Phe Lys Ala Cys Glu Asp Ser Lys Arg Lys Ala Arg
       25                  30                  35
   ggc tac ctc cgc ctg gtg ccc ctg ttt gtg ctg ctg gcc ctg ctc gtg      197
   Gly Tyr Leu Arg Leu Val Pro Leu Phe Val Leu Leu Ala Leu Leu Val
   40                  45                  50                  55
   ctg gct tcg gcg ggg gtg cta ctc tgg tat ttc cta ggg tac aag gcg      245
   Leu Ala Ser Ala Gly Val Leu Leu Trp Tyr Phe Leu Gly Tyr Lys Ala
                   60                  65                  70
   gag gtg atg gtc agc cag gtg tac tca ggc agt ctg cgt gta ctc aat      293
   Glu Val Met Val Ser Gln Val Tyr Ser Gly Ser Leu Arg Val Leu Asn
               75                  80                  85
   cgc cac ttc tcc cag gat ctt acc cgc cgg gaa tct agt gcc ttc cgc      341
   Arg His Phe Ser Gln Asp Leu Thr Arg Arg Glu Ser Ser Ala Phe Arg
           90                  95                  100
   agt gaa acc gcc aaa gcc cag aag atg ctc aag gag ctc atc acc agc      389
   Ser Glu Thr Ala Lys Ala Gln Lys Met Leu Lys Glu Leu Ile Thr Ser
       105                 110                 115
   acc cgc ctg gga act tac tac aac tcc agc tcc gtc tat tcc ttt ggg      437
   Thr Arg Leu Gly Thr Tyr Tyr Asn Ser Ser Ser Val Tyr Ser Phe Gly
   120                 125                 130                 135
   gag gga ccc ctc acc tgc ttc ttc tgg ttc att ctc caa atc ccc gag      485
   Glu Gly Pro Leu Thr Cys Phe Phe Trp Phe Ile Leu Gln Ile Pro Glu
                   140                 145                 150
   cac cgc cgg ctg atg ctg agc ccc gag gtg gtg cag gca ctg ctg gtg      533
   His Arg Arg Leu Met Leu Ser Pro Glu Val Val Gln Ala Leu Leu Val
               155                 160                 165
   gag gag ctg ctg tcc aca gtc aac agc tcg gct gcc gtc ccc tac agg      581
   Glu Glu Leu Leu Ser Thr Val Asn Ser Ser Ala Ala Val Pro Tyr Arg
           170                 175                 180
   gcc gag tac gaa gtg gac ccc gag ggc cta gtg atc ctg gaa gcc agt      629
   Ala Glu Tyr Glu Val Asp Pro Glu Gly Leu Val Ile Leu Glu Ala Ser
       185                 190                 195
   gtg aaa gac ata gct gca ttg aat tcc acg ctg ggt tgt tac cgc tac      677
   Val Lys Asp Ile Ala Ala Leu Asn Ser Thr Leu Gly Cys Tyr Arg Tyr
   200                 205                 210                 215
   agc tac gtg ggc cag ggc cag gtc ctc cgg ctg aag ggg cct gac cac      725
   Ser Tyr Val Gly Gln Gly Gln Val Leu Arg Leu Lys Gly Pro Asp His
                   220                 225                 230
   ctg gcc tcc agc tgc ctg tgg cac ctg cag ggc ccc aag gac ctc atg      773
   Leu Ala Ser Ser Cys Leu Trp His Leu Gln Gly Pro Lys Asp Leu Met
               235                 240                 245
   ctc aaa ctc cgg ctg gag tgg acg ctg gca gag tgc cgg gac cga ctg      821
   Leu Lys Leu Arg Leu Glu Trp Thr Leu Ala Glu Cys Arg Asp Arg Leu
           250                 255                 260
   gcc atg tat gac gtg gcc ggg ccc ctg gag aag agg ctc atc acc tcg      869
   Ala Met Tyr Asp Val Ala Gly Pro Leu Glu Lys Arg Leu Ile Thr Ser
       265                 270                 275
   gtg tac ggc tgc agc cgc cag gag ccc gtg gtg gag gtt ctg gcg tcg      917
   Val Tyr Gly Cys Ser Arg Gln Glu Pro Val Val Glu Val Leu Ala Ser
   280                 285                 290                 295
   ggg gcc atc atg gcg gtc gtc tgg aag aag ggc ctg cac agc tac tac      965
   Gly Ala Ile Met Ala Val Val Trp Lys Lys Gly Leu His Ser Tyr Tyr
                   300                 305                 310
   gac ccc ttc gtg ctc tcc gtg cag ccg gtg gtc ttc cag gcc tgt gaa     1013
   Asp Pro Phe Val Leu Ser Val Gln Pro Val Val Phe Gln Ala Cys Glu
               315                 320                 325
   gtg aac ctg acg ctg gac aac agg ctc gac tcc cag ggc gtc ctc agc     1061
   Val Asn Leu Thr Leu Asp Asn Arg Leu Asp Ser Gln Gly Val Leu Ser
           330                 335                 340
   acc ccg tac ttc ccc agc tac tac tcg ccc caa acc cac tgc tcc tgg 1109
   Thr Pro Tyr Phe Pro Ser Tyr Tyr Ser Pro Gln Thr His Cys Ser Trp
   345 350 355
   cac ctc acg gtg ccc tct ctg gac tac ggc ttg gcc ctc tgg ttt gat 1157
   His Leu Thr Val Pro Ser Leu Asp Tyr Gly Leu Ala Leu Trp Phe Asp
   360 365 370 375
   gcc tat gca ctg agg agg cag aag tat gat ttg ccg tgc acc cag ggc 1205
   Ala Tyr Ala Leu Arg Arg Gln Lys Tyr Asp Leu Pro Cys Thr Gln Gly
   380 385 390
   cag tgg acg atc cag aac agg agg ctg tgt ggc ttg cgc atc ctg cag 1253
   Gln Trp Thr Ile Gln Asn Arg Arg Leu Cys Gly Leu Arg Ile Leu Gln
   395 400 405
   ccc tac gcc gag agg atc ccc gtg gtg gcc acg gcc ggg atc acc atc 1301
   Pro Tyr Ala Glu Arg Ile Pro Val Val Ala Thr Ala Gly Ile Thr Ile
   410 415 420
   aac ttc acc tcc cag atc tcc ctc acc ggg ccc ggt gtg cgg gtg cac 1349
   Asn Phe Thr Ser Gln Ile Ser Leu Thr Gly Pro Gly Val Arg Val His
   425 430 435
   tat ggc ttg tac aac cag tcg gac ccc tgc cct gga gag ttc ctc tgt 1397
   Tyr Gly Leu Tyr Asn Gln Ser Asp Pro Cys Pro Gly Glu Phe Leu Cys
   440 445 450 455
   tct gtg aat gga ctc tgt gtc cct gcc tgt gat ggg gtc aag gac tgc 1445
   Ser Val Asn Gly Leu Cys Val Pro Ala Cys Asp Gly Val Lys Asp Cys
   460 465 470
   ccc aac ggc ctg gat gag aga aac tgc gtt tgc aga gcc aca ttc cag 1493
   Pro Asn Gly Leu Asp Glu Arg Asn Cys Val Cys Arg Ala Thr Phe Gln
   475 480 485
   tgc aaa gag gac agc aca tgc atc tca ctg ccc aag gtc tgt gat ggg 1541
   Cys Lys Glu Asp Ser Thr Cys Ile Ser Leu Pro Lys Val Cys Asp Gly
   490 495 500
   cag cct gat tgt ctc aac ggc agc gac gaa gag cag tgc cag gaa ggg 1589
   Gln Pro Asp Cys Leu Asn Gly Ser Asp Glu Glu Gln Cys Gln Glu Gly
   505 510 515
   gtg cca tgt ggg aca ttc acc ttc cag tgt gag gac cgg agc tgc gtg 1637
   Val Pro Cys Gly Thr Phe Thr Phe Gln Cys Glu Asp Arg Ser Cys Val
   520 525 530 535
   aag aag ccc aac ccg cag tgt gat ggg cgg ccc gac tgc agg gac ggc 1685
   Lys Lys Pro Asn Pro Gln Cys Asp Gly Arg Pro Asp Cys Arg Asp Gly
   540 545 550
   tcg gat gag gag cac tgt gaa tgt ggc ctc cag ggc ccc tcc agc cgc 1733
   Ser Asp Glu Glu His Cys Glu Cys Gly Leu Gln Gly Pro Ser Ser Arg
   555 560 565
   att gtt ggt gga gct gtg tcc tcc gag ggt gag tgg cca tgg cag gcc 1781
   Ile Val Gly Gly Ala Val Ser Ser Glu Gly Glu Trp Pro Trp Gln Ala
   570 575 580
   agc ctc cag gtt cgg ggt cga cac atc tgt ggg ggg gcc ctc atc gct 1829
   Ser Leu Gln Val Arg Gly Arg His Ile Cys Gly Gly Ala Leu Ile Ala
           585                     590                     595
   gac cgc tgg gtg ata aca gct gcc cac tgc ttc cag gag gac agc atg      1877
   Asp Arg Trp Val Ile Thr Ala Ala His Cys Phe Gln Glu Asp Ser Met
   600                 605                 610                 615
   gcc tcc acg gtg ctg tgg acc gtg ttc ctg ggc aag gtg tgg cag aac      1925
   Ala Ser Thr Val Leu Trp Thr Val Phe Leu Gly Lys Val Trp Gln Asn
                   620                 625                 630
   tcg cgc tgg cct gga gag gtg tcc ttc aag gtg agc cgc ctg ctc ctg      1973
   Ser Arg Trp Pro Gly Glu Val Ser Phe Lys Val Ser Arg Leu Leu Leu
               635                 640                 645
   cac ccg tac cac gaa gag gac agc cat gac tac gac gtg gcg ctg ctg      2021
   His Pro Tyr His Glu Glu Asp Ser His Asp Tyr Asp Val Ala Leu Leu
           650                 655                 660
   cag ctc gac cac ccg gtg gtg cgc tcg gcc gcc gtg cgc ccc gtc tgc      2069
   Gln Leu Asp His Pro Val Val Arg Ser Ala Ala Val Arg Pro Val Cys
       665                 670                 675
   ctg ccc gcg cgc tcc cac ttc ttc gag ccc ggc ctg cac tgc tgg att      2117
   Leu Pro Ala Arg Ser His Phe Phe Glu Pro Gly Leu His Cys Trp Ile
   680                 685                 690                 695
   acg ggc tgg ggc gcc ttg cgc gag ggc ggc ccc atc agc aac gct ctg      2165
   Thr Gly Trp Gly Ala Leu Arg Glu Gly Gly Pro Ile Ser Asn Ala Leu
                   700                 705                 710
   cag aaa gtg gat gtg cag ttg atc cca cag gac ctg tgc agc gag gtc      2213
   Gln Lys Val Asp Val Gln Leu Ile Pro Gln Asp Leu Cys Ser Glu Val
               715                 720                 725
   tat cgc tac cag gtg acg cca cgc atg ctg tgt gcc ggc tac cgc aag      2261
   Tyr Arg Tyr Gln Val Thr Pro Arg Met Leu Cys Ala Gly Tyr Arg Lys
           730                 735                 740
   ggc aag aag gat gcc tgt cag ggt gac tca ggt ggt ccg ctg gtg tgc      2309
   Gly Lys Lys Asp Ala Cys Gln Gly Asp Ser Gly Gly Pro Leu Val Cys
       745                 750                 755
   aag gca ctc agt ggc cgc tgg ttc ctg gcg ggg ctg gtc agc tgg ggc      2357
   Lys Ala Leu Ser Gly Arg Trp Phe Leu Ala Gly Leu Val Ser Trp Gly
   760                 765                 770                 775
   ctg ggc tgt ggc cgg cct aac tac ttc ggc gtc tac acc cgc atc aca      2405
   Leu Gly Cys Gly Arg Pro Asn Tyr Phe Gly Val Tyr Thr Arg Ile Thr
                   780                 785                 790
   ggt gtg atc agc tgg atc cag caa gtg gtg acc tga ggaactgccc           2451
   Gly Val Ile Ser Trp Ile Gln Gln Val Val Thr  *
               795                 800
   ccctgcaaag cagggcccac ctcctggact cagagagccc agggcaactg ccaagcaggg    2511
   ggacaagtat tctggcgggg ggtgggggag agagcaggcc ctgtggtggc aggaggggca    2571
   tcttgtttcg tccctgatgt ctgtccagta tggcaggagg atgagaagtg ccagcagttg    2631
   ggggtcaaga cgtcccttga ggacccaggc ccacacccag cccttttgcc tcccaattct    2691
   ctctcctccg tccccttcct ccactgctgc ctaatgcaag gcagtggctc agcagcaaga    2751
   atgctggttc tacatcccga ggagtgtctg aggtgcgccc cactctgtac agaggctgtt    2811
   tgggcagcct tgcctccaga gagcagattc cagcttcgga agcccctggt ctaacttggg    2871
   atctgggaat ggaaggtgct cccatcggag gggaccctca gagccctgga gactgccagg    2931
   tgggcctgct gccactgtaa gccaaaaggt ggggaagtcc tgactccagg gtccttgccc    2991
   cacccctgcc tgccacctgg gccctcacag cccagaccct cactgggagg tgagctcagc    3051
   tgccctttgg aataaagctg cctgatgcaa aaaaaaaaaa aaaaaaaaaa aaa           3104
<210> 8
   <211> 802
   <212> PRT
   <213> Homo Sapien
<400> 8
   Met Pro Val Ala Glu Ala Pro Gln Val Ala Gly Gly Gln Gly Asp Gly
    1               5                  10                  15
   Gly Asp Gly Glu Glu Ala Glu Pro Glu Gly Met Phe Lys Ala Cys Glu
               20                  25                  30
   Asp Ser Lys Arg Lys Ala Arg Gly Tyr Leu Arg Leu Val Pro Leu Phe
           35                  40                  45
   Val Leu Leu Ala Leu Leu Val Leu Ala Ser Ala Gly Val Leu Leu Trp
       50                  55                  60
   Tyr Phe Leu Gly Tyr Lys Ala Glu Val Met Val Ser Gln Val Tyr Ser
   65                  70                  75                  80
   Gly Ser Leu Arg Val Leu Asn Arg His Phe Ser Gln Asp Leu Thr Arg
                   85                  90                  95
   Arg Glu Ser Ser Ala Phe Arg Ser Glu Thr Ala Lys Ala Gln Lys Met
               100                 105                 110
   Leu Lys Glu Leu Ile Thr Ser Thr Arg Leu Gly Thr Tyr Tyr Asn Ser
           115                 120                 125
   Ser Ser Val Tyr Ser Phe Gly Glu Gly Pro Leu Thr Cys Phe Phe Trp
       130                 135                 140
   Phe Ile Leu Gln Ile Pro Glu His Arg Arg Leu Met Leu Ser Pro Glu
   145                 150                 155                 160
   Val Val Gln Ala Leu Leu Val Glu Glu Leu Leu Ser Thr Val Asn Ser
                   165                 170                 175
   Ser Ala Ala Val Pro Tyr Arg Ala Glu Tyr Glu Val Asp Pro Glu Gly
               180                 185                 190
   Leu Val Ile Leu Glu Ala Ser Val Lys Asp Ile Ala Ala Leu Asn Ser
           195                 200                 205
   Thr Leu Gly Cys Tyr Arg Tyr Ser Tyr Val Gly Gln Gly Gln Val Leu
       210                 215                 220
   Arg Leu Lys Gly Pro Asp His Leu Ala Ser Ser Cys Leu Trp His Leu
   225                 230                 235                 240
   Gln Gly Pro Lys Asp Leu Met Leu Lys Leu Arg Leu Glu Trp Thr Leu
                   245                 250                 255
   Ala Glu Cys Arg Asp Arg Leu Ala Met Tyr Asp Val Ala Gly Pro Leu
               260                 265                 270
   Glu Lys Arg Leu Ile Thr Ser Val Tyr Gly Cys Ser Arg Gln Glu Pro
           275                 280                 285
   Val Val Glu Val Leu Ala Ser Gly Ala Ile Met Ala Val Val Trp Lys
       290                 295                 300
   Lys Gly Leu His Ser Tyr Tyr Asp Pro Phe Val Leu Ser Val Gln Pro
   305                 310                 315                 320
   Val Val Phe Gln Ala Cys Glu Val Asn Leu Thr Leu Asp Asn Arg Leu
                   325                 330                 335
   Asp Ser Gln Gly Val Leu Ser Thr Pro Tyr Phe Pro Ser Tyr Tyr Ser
               340                 345                 350
   Pro Gln Thr His Cys Ser Trp His Leu Thr Val Pro Ser Leu Asp Tyr
           355                 360                 365
   Gly Leu Ala Leu Trp Phe Asp Ala Tyr Ala Leu Arg Arg Gln Lys Tyr
       370                 375                 380
   Asp Leu Pro Cys Thr Gln Gly Gln Trp Thr Ile Gln Asn Arg Arg Leu
   385                 390                 395                 400
   Cys Gly Leu Arg Ile Leu Gln Pro Tyr Ala Glu Arg Ile Pro Val Val
                   405                 410                 415
   Ala Thr Ala Gly Ile Thr Ile Asn Phe Thr Ser Gln Ile Ser Leu Thr
               420                 425                 430
   Gly Pro Gly Val Arg Val His Tyr Gly Leu Tyr Asn Gln Ser Asp Pro
           435                 440                 445
   Cys Pro Gly Glu Phe Leu Cys Ser Val Asn Gly Leu Cys Val Pro Ala
       450                 455                 460
   Cys Asp Gly Val Lys Asp Cys Pro Asn Gly Leu Asp Glu Arg Asn Cys
   465                 470                 475                 480
   Val Cys Arg Ala Thr Phe Gln Cys Lys Glu Asp Ser Thr Cys Ile Ser
                   485                 490                 495
   Leu Pro Lys Val Cys Asp Gly Gln Pro Asp Cys Leu Asn Gly Ser Asp
               500                 505                 510
   Glu Glu Gln Cys Gln Glu Gly Val Pro Cys Gly Thr Phe Thr Phe Gln
           515                 520                 525
   Cys Glu Asp Arg Ser Cys Val Lys Lys Pro Asn Pro Gln Cys Asp Gly
       530                 535                 540
   Arg Pro Asp Cys Arg Asp Gly Ser Asp Glu Glu His Cys Glu Cys Gly
   545                 550                 555                 560
   Leu Gln Gly Pro Ser Ser Arg Ile Val Gly Gly Ala Val Ser Ser Glu
                   565                 570                 575
   Gly Glu Trp Pro Trp Gln Ala Ser Leu Gln Val Arg Gly Arg His Ile
               580                 585                 590
   Cys Gly Gly Ala Leu Ile Ala Asp Arg Trp Val Ile Thr Ala Ala His
           595                 600                 605
   Cys Phe Gln Glu Asp Ser Met Ala Ser Thr Val Leu Trp Thr Val Phe
       610                 615                 620
   Leu Gly Lys Val Trp Gln Asn Ser Arg Trp Pro Gly Glu Val Ser Phe
   625                 630                 635                 640
   Lys Val Ser Arg Leu Leu Leu His Pro Tyr His Glu Glu Asp Ser His
                   645                 650                 655
   Asp Tyr Asp Val Ala Leu Leu Gln Leu Asp His Pro Val Val Arg Ser
               660                 665                 670
   Ala Ala Val Arg Pro Val Cys Leu Pro Ala Arg Ser His Phe Phe Glu
           675                 680                 685
   Pro Gly Leu His Cys Trp Ile Thr Gly Trp Gly Ala Leu Arg Glu Gly
       690                 695                 700
   Gly Pro Ile Ser Asn Ala Leu Gln Lys Val Asp Val Gln Leu Ile Pro
   705                 710                 715                 720
   Gln Asp Leu Cys Ser Glu Val Tyr Arg Tyr Gln Val Thr Pro Arg Met
                   725                 730                 735
   Leu Cys Ala Gly Tyr Arg Lys Gly Lys Lys Asp Ala Cys Gln Gly Asp
               740                 745                 750
   Ser Gly Gly Pro Leu Val Cys Lys Ala Leu Ser Gly Arg Trp Phe Leu
           755                 760                 765
   Ala Gly Leu Val Ser Trp Gly Leu Gly Cys Gly Arg Pro Asn Tyr Phe
       770                 775                 780
   Gly Val Tyr Thr Arg Ile Thr Gly Val Ile Ser Trp Ile Gln Gln Val
   785                 790                 795                 800
   Val Thr
<210> 9
   <211> 2672
   <212> DNA
   <213> Homo Sapien
<220>
   <221> CDS
   <222> (33)...(2009)
   <223> cDNA encoding: MTSP4-S (short form) splice variant
<400> 9
   tcatcggcca gagggtgatc agtgagcaga ag atg ccc gtg gcc gag gcc ccc        53
                                       Met Pro Val Ala Glu Ala Pro
                                       1               5
   cag gtg gct ggc ggg cag ggg gac gga ggt gat ggc gag gaa gcg gag       101
   Gln Val Ala Gly Gly Gln Gly Asp Gly Gly Asp Gly Glu Glu Ala Glu
           10                  15                  20
   ccg gag ggg atg ttc aag gcc tgt gag gac tcc aag aga aaa gcc cgg       149
   Pro Glu Gly Met Phe Lys Ala Cys Glu Asp Ser Lys Arg Lys Ala Arg
       25                  30                  35
   ggc tac ctc cgc ctg gtg ccc ctg ttt gtg ctg ctg gcc ctg ctc gtg       197
   Gly Tyr Leu Arg Leu Val Pro Leu Phe Val Leu Leu Ala Leu Leu Val
   40                  45                  50                  55
   ctg gct tcg gcg ggg gtg cta ctc tgg tat ttc cta ggg tac aag gcg       245
   Leu Ala Ser Ala Gly Val Leu Leu Trp Tyr Phe Leu Gly Tyr Lys Ala
                   60                  65                  70
   gag gtg atg gtc agc cag gtg tac tca ggc agt ctg cgt gta ctc aat       293
   Glu Val Met Val Ser Gln Val Tyr Ser Gly Ser Leu Arg Val Leu Asn
               75                  80                  85
   cgc cac ttc tcc cag gat ctt acc cgc cgg gaa tct agt gcc ttc cgc       341
   Arg His Phe Ser Gln Asp Leu Thr Arg Arg Glu Ser Ser Ala Phe Arg
           90                  95                  100
   agt gaa acc gcc aaa gcc cag aag atg ctc aag gag ctc atc acc agc       389
   Ser Glu Thr Ala Lys Ala Gln Lys Met Leu Lys Glu Leu Ile Thr Ser
       105                 110                 115
   acc cgc ctg gga act tac tac aac tcc agc tcc gtc tat tcc ttt ggg       437
   Thr Arg Leu Gly Thr Tyr Tyr Asn Ser Ser Ser Val Tyr Ser Phe Gly
   120                 125                 130                 135
   gtg tac ggc tgc agc cgc cag gag ccc gtg gtg gag gtt ctg gcg tcg       485
   Val Tyr Gly Cys Ser Arg Gln Glu Pro Val Val Glu Val Leu Ala Ser
                   140                 145                 150
   ggg gcc atc atg gcg gtc gtc tgg aag aag ggc ctg cac agc tac tac       533
   Gly Ala Ile Met Ala Val Val Trp Lys Lys Gly Leu His Ser Tyr Tyr
               155                 160                 165
   gac ccc ttc gtg ctc tcc gtg cag ccg gtg gtc ttc cag gcc tgt gaa       581
   Asp Pro Phe Val Leu Ser Val Gln Pro Val Val Phe Gln Ala Cys Glu
           170                 175                 180
   gtg aac ctg acg ctg gac aac agg ctc gac tcc cag ggc gtc ctc agc       629
   Val Asn Leu Thr Leu Asp Asn Arg Leu Asp Ser Gln Gly Val Leu Ser
       185                 190                 195
   acc ccg tac ttc ccc agc tac tac tcg ccc caa acc cac tgc tcc tgg       677
   Thr Pro Tyr Phe Pro Ser Tyr Tyr Ser Pro Gln Thr His Cys Ser Trp
   200                 205                 210                 215
   cac ctc acg gtg ccc tct ctg gac tac ggc ttg gcc ctc tgg ttt gat       725
   His Leu Thr Val Pro Ser Leu Asp Tyr Gly Leu Ala Leu Trp Phe Asp
                   220                 225                 230
   gcc tat gca ctg agg agg cag aag tat gat ttg ccg tgc acc cag ggc       773
   Ala Tyr Ala Leu Arg Arg Gln Lys Tyr Asp Leu Pro Cys Thr Gln Gly
                   235                     240                     245
   cag tgg acg atc cag aac agg agg ctg tgt ggc ttg cgc atc ctg cag        821
   Gln Trp Thr Ile Gln Asn Arg Arg Leu Cys Gly Leu Arg Ile Leu Gln
           250                     255                     260
   ccc tac gcc gag agg atc ccc gtg gtg gcc acg gcc ggg atc acc atc        869
   Pro Tyr Ala Glu Arg Ile Pro Val Val Ala Thr Ala Gly Ile Thr Ile
       265                     270                     275
   aac ttc acc tcc cag atc tcc ctc acc ggg ccc ggt gtg cgg gtg cac        917
   Asn Phe Thr Ser Gln Ile Ser Leu Thr Gly Pro Gly Val Arg Val His
   280                     285                     290                     295
   tat ggc ttg tac aac cag tcg gac ccc tgc cct gga gag ttc ctc tgt        965
   Tyr Gly Leu Tyr Asn Gln Ser Asp Pro Cys Pro Gly Glu Phe Leu Cys
                   300                     305                     310
   tct gtg aat gga ctc tgt gtc cct gcc tgt gat ggg gtc aag gac tgc       1013
   Ser Val Asn Gly Leu Cys Val Pro Ala Cys Asp Gly Val Lys Asp Cys
               315                     320                     325
   ccc aac ggc ctg gat gag aga aac tgc gtt tgc aga gcc aca ttc cag       1061
   Pro Asn Gly Leu Asp Glu Arg Asn Cys Val Cys Arg Ala Thr Phe Gln
           330                     335                     340
   tgc aaa gag gac agc aca tgc atc tca ctg ccc aag gtc tgt gat ggg       1109
   Cys Lys Glu Asp Ser Thr Cys Ile Ser Leu Pro Lys Val Cys Asp Gly
       345                     350                     355
   cag cct gat tgt ctc aac ggc agc gac gaa gag cag tgc cag gaa ggg       1157
   Gln Pro Asp Cys Leu Asn Gly Ser Asp Glu Glu Gln Cys Gln Glu Gly
   360                     365                     370                     375
   gtg cca tgt ggg aca ttc acc ttc cag tgt gag gac cgg agc tgc gtg       1205
   Val Pro Cys Gly Thr Phe Thr Phe Gln Cys Glu Asp Arg Ser Cys Val
                   380                     385                     390
   aag aag ccc aac ccg cag tgt gat ggg cgg ccc gac tgc agg gac ggc       1253
   Lys Lys Pro Asn Pro Gln Cys Asp Gly Arg Pro Asp Cys Arg Asp Gly
               395                     400                     405
   tcg gat gag gag cac tgt gaa tgt ggc ctc cag ggc ccc tcc agc cgc       1301
   Ser Asp Glu Glu His Cys Glu Cys Gly Leu Gln Gly Pro Ser Ser Arg
           410                     415                     420
   att gtt ggt gga gct gtg tcc tcc gag ggt gag tgg cca tgg cag gcc       1349
   Ile Val Gly Gly Ala Val Ser Ser Glu Gly Glu Trp Pro Trp Gln Ala
       425                     430                     435
   agc ctc cag gtt cgg ggt cga cac atc tgt ggg ggg gcc ctc atc gct       1397
   Ser Leu Gln Val Arg Gly Arg His Ile Cys Gly Gly Ala Leu Ile Ala
   440                     445                     450                     455
   gac cgc tgg gtg ata aca gct gcc cac tgc ttc cag gag gac agc atg       1445
   Asp Arg Trp Val Ile Thr Ala Ala His Cys Phe Gln Glu Asp Ser Met
                   460                     465                     470
   gcc tcc acg gtg ctg tgg acc gtg ttc ctg ggc aag gtg tgg cag aac       1493
   Ala Ser Thr Val Leu Trp Thr Val Phe Leu Gly Lys Val Trp Gln Asn
               475                     480                     485
   tcg cgc tgg cct gga gag gtg tcc ttc aag gtg agc cgc ctg ctc ctg      1541
   Ser Arg Trp Pro Gly Glu Val Ser Phe Lys Val Ser Arg Leu Leu Leu
           490                 495                 500
   cac ccg tac cac gaa gag gac agc cat gac tac gac gtg gcg ctg ctg      1589
   His Pro Tyr His Glu Glu Asp Ser His Asp Tyr Asp Val Ala Leu Leu
       505                 510                 515
   cag ctc gac cac ccg gtg gtg cgc tcg gcc gcc gtg cgc ccc gtc tgc      1637
   Gln Leu Asp His Pro Val Val Arg Ser Ala Ala Val Arg Pro Val Cys
   520                 525                 530                 535
   ctg ccc gcg cgc tcc cac ttc ttc gag ccc ggc ctg cac tgc tgg att      1685
   Leu Pro Ala Arg Ser His Phe Phe Glu Pro Gly Leu His Cys Trp Ile
                   540                 545                 550
   acg ggc tgg ggc gcc ttg cgc gag ggc ggc ccc atc agc aac gct ctg      1733
   Thr Gly Trp Gly Ala Leu Arg Glu Gly Gly Pro Ile Ser Asn Ala Leu
               555                 560                 565
   cag aaa gtg gat gtg cag ttg atc cca cag gac ctg tgc agc gag gtc      1781
   Gln Lys Val Asp Val Gln Leu Ile Pro Gln Asp Leu Cys Ser Glu Val
           570                 575                 580
   tat cgc tac cag gtg acg cca cgc atg ctg tgt gcc ggc tac cgc aag      1829
   Tyr Arg Tyr Gln Val Thr Pro Arg Met Leu Cys Ala Gly Tyr Arg Lys
       585                 590                 595
   ggc aag aag gat gcc tgt cag ggt gac tca ggt ggt ccg ctg gtg tgc      1877
   Gly Lys Lys Asp Ala Cys Gln Gly Asp Ser Gly Gly Pro Leu Val Cys
   600                 605                 610                 615
   aag gca ctc agt ggc cgc tgg ttc ctg gcg ggg ctg gtc agc tgg ggc      1925
   Lys Ala Leu Ser Gly Arg Trp Phe Leu Ala Gly Leu Val Ser Trp Gly
                   620                 625                 630
   ctg ggc tgt ggc cgg cct aac tac ttc ggc gtc tac acc cgc atc aca      1973
   Leu Gly Cys Gly Arg Pro Asn Tyr Phe Gly Val Tyr Thr Arg Ile Thr
               635                 640                 645
   ggt gtg atc agc tgg atc cag caa gtg gtg acc tga ggaactgccc           2019
   Gly Val Ile Ser Trp Ile Gln Gln Val Val Thr  *
           650                 655
   ccctgcaaag cagggcccac ctcctggact cagagagccc agggcaactg ccaagcaggg    2079
   ggacaagtat tctggcgggg ggtgggggag agagcaggcc ctgtggtggc aggaggggca    2139
   tcttgtttcg tccctgatgt ctgtccagta tggcaggagg atgagaagtg ccagcagttg    2199
   ggggtcaaga cgtcccttga ggacccaggc ccacacccag cccttttgcc  tcccaattct    2259
   ctctcctccg tccccttcct ccactgctgc ctaatgcaag gcagtggctc agcagcaaga    2319
   atgctggttc tacatcccga ggagtgtctg aggtgcgccc cactctgtac agaggctgtt    2379
   tgggcagcct tgcctccaga gagcagattc cagcttcgga agcccctggt ctaacttggg    2439
   atctgggaat ggaaggtgct cccatcggag gggaccctca gagccctgga gactgccagg    2499
   tgggcctgct gccactgtaa gccaaaaggt ggggaagtcc tgactccagg gtccttgccc    2559
   cacccctgcc tgccacctgg gccctcacag cccagaccct cactgggagg tgagctcagc    2619
   tgccctttgg aataaagctg cctgatgcaa aaaaaaaaaa aaaaaaaaaa aaa           2672
<210> 10
   <211> 658
   <212> PRT
   <213> Homo Sapien
<400> 10
   Met Pro Val Ala Glu Ala Pro Gln Val Ala Gly Gly Gln Gly Asp Gly
    1               5                  10                  15
   Gly Asp Gly Glu Glu Ala Glu Pro Glu Gly Met Phe Lys Ala Cys Glu
               20                  25                  30
   Asp Ser Lys Arg Lys Ala Arg Gly Tyr Leu Arg Leu Val Pro Leu Phe
           35                  40                  45
   Val Leu Leu Ala Leu Leu Val Leu Ala Ser Ala Gly Val Leu Leu Trp
       50                  55                  60
   Tyr Phe Leu Gly Tyr Lys Ala Glu Val Met Val Ser Gln Val Tyr Ser
   65                  70                  75                  80
   Gly Ser Leu Arg Val Leu Asn Arg His Phe Ser Gln Asp Leu Thr Arg
                   85                  90                  95
   Arg Glu Ser Ser Ala Phe Arg Ser Glu Thr Ala Lys Ala Gln Lys Met
               100                 105                 110
   Leu Lys Glu Leu Ile Thr Ser Thr Arg Leu Gly Thr Tyr Tyr Asn Ser
           115                 120                 125
   Ser Ser Val Tyr Ser Phe Gly Val Tyr Gly Cys Ser Arg Gln Glu Pro
       130                 135                 140
   Val Val Glu Val Leu Ala Ser Gly Ala Ile Met Ala Val Val Trp Lys
   145                 150                 155                 160
   Lys Gly Leu His Ser Tyr Tyr Asp Pro Phe Val Leu Ser Val Gln Pro
                   165                 170                 175
   Val Val Phe Gln Ala Cys Glu Val Asn Leu Thr Leu Asp Asn Arg Leu
               180                 185                 190
   Asp Ser Gln Gly Val Leu Ser Thr Pro Tyr Phe Pro Ser Tyr Tyr Ser
           195                 200                 205
   Pro Gln Thr His Cys Ser Trp His Leu Thr Val Pro Ser Leu Asp Tyr
       210                 215                 220
   Gly Leu Ala Leu Trp Phe Asp Ala Tyr Ala Leu Arg Arg Gln Lys Tyr
   225                 230                 235                 240
   Asp Leu Pro Cys Thr Gln Gly Gln Trp Thr Ile Gln Asn Arg Arg Leu
                   245                 250                 255
   Cys Gly Leu Arg Ile Leu Gln Pro Tyr Ala Glu Arg Ile Pro Val Val
               260                 265                 270
   Ala Thr Ala Gly Ile Thr Ile Asn Phe Thr Ser Gln Ile Ser Leu Thr
           275                 280                 285
   Gly Pro Gly Val Arg Val His Tyr Gly Leu Tyr Asn Gln Ser Asp Pro
       290                 295                 300
   Cys Pro Gly Glu Phe Leu Cys Ser Val Asn Gly Leu Cys Val Pro Ala
   305                 310                 315                 320
   Cys Asp Gly Val Lys Asp Cys Pro Asn Gly Leu Asp Glu Arg Asn Cys
                   325                 330                 335
   Val Cys Arg Ala Thr Phe Gln Cys Lys Glu Asp Ser Thr Cys Ile Ser
               340                 345                 350
   Leu Pro Lys Val Cys Asp Gly Gln Pro Asp Cys Leu Asn Gly Ser Asp
           355                 360                 365
   Glu Glu Gln Cys Gln Glu Gly Val Pro Cys Gly Thr Phe Thr Phe Gln
       370                 375                 380
   Cys Glu Asp Arg Ser Cys Val Lys Lys Pro Asn Pro Gln Cys Asp Gly
   385                 390                 395                 400
   Arg Pro Asp Cys Arg Asp Gly Ser Asp Glu Glu His Cys Glu Cys Gly
                   405                 410                 415
   Leu Gln Gly Pro Ser Ser Arg Ile Val Gly Gly Ala Val Ser Ser Glu
               420                 425                 430
   Gly Glu Trp Pro Trp Gln Ala Ser Leu Gln Val Arg Gly Arg His Ile
           435                 440                 445
   Cys Gly Gly Ala Leu Ile Ala Asp Arg Trp Val Ile Thr Ala Ala His
       450                 455                 460
   Cys Phe Gln Glu Asp Ser Met Ala Ser Thr Val Leu Trp Thr Val Phe
   465                 470                 475                 480
   Leu Gly Lys Val Trp Gln Asn Ser Arg Trp Pro Gly Glu Val Ser Phe
                   485                 490                 495
   Lys Val Ser Arg Leu Leu Leu His Pro Tyr His Glu Glu Asp Ser His
               500                 505                 510
   Asp Tyr Asp Val Ala Leu Leu Gln Leu Asp His Pro Val Val Arg Ser
           515                 520                 525
   Ala Ala Val Arg Pro Val Cys Leu Pro Ala Arg Ser His Phe Phe Glu
       530                 535                 540
   Pro Gly Leu His Cys Trp Ile Thr Gly Trp Gly Ala Leu Arg Glu Gly
   545                 550                 555                 560
   Gly Pro Ile Ser Asn Ala Leu Gln Lys Val Asp Val Gln Leu Ile Pro
                   565                 570                 575
   Gln Asp Leu Cys Ser Glu Val Tyr Arg Tyr Gln Val Thr Pro Arg Met
               580                 585                 590
   Leu Cys Ala Gly Tyr Arg Lys Gly Lys Lys Asp Ala Cys Gln Gly Asp
           595                 600                 605
   Ser Gly Gly Pro Leu Val Cys Lys Ala Leu Ser Gly Arg Trp Phe Leu
       610                 615                 620
   Ala Gly Leu Val Ser Trp Gly Leu Gly Cys Gly Arg Pro Asn Tyr Phe
   625                 630                 635                 640
   Gly Val Tyr Thr Arg Ile Thr Gly Val Ile Ser Trp Ile Gln Gln Val
                   645                 650                 655
   Val Thr
<210> 11
   <211> 1656
   <212> DNA
   <213> Homo Sapien
<220>
   <221> CDS
   <222> (268)...(1629)
   <223> DNA sequence encoding a transmembrane serine protease (MTSP-6) protein
<400> 11
   cgcccgggca ggtcagtaac actgtggcct actatctctt ccgtggtgcc atctacattt    60
   ttgggactcg ggaattatga ctgtttttgg ttaatcgata ctgaatgcgc tttgtgtgga   120
   ctgtcgaatt tcaaagattt accgtatgac caagatgcac ctgatgctac aagtataaat   180
   aggggaacaa atgctttctg ttcttcctcg gctaaggagg tagaggtgga ggcggagccg   240
   gatgtcagag gtcctgaaat agtcacc atg ggg gaa aat gat ccg cct gct gtt   294
                                 Met Gly Glu Asn Asp Pro Pro Ala Val
                                  1               5
   gaa gcc ccc ttc tca ttc cga tcg ctt ttt ggc ctt gat gat ttg aaa   342
   Glu Ala Pro Phe Ser Phe Arg Ser Leu Phe Gly Leu Asp Asp Leu Lys
    10                  15                  20                  25
   ata agt cct gtt gca cca gat gca gat gct gtt gct gca cag atc ctg   390
   Ile Ser Pro Val Ala Pro Asp Ala Asp Ala Val Ala Ala Gln Ile Leu
                    30                  35                  40
   tca ctg ctg cca ttg aag ttt ttt cca atc atc gtc att ggg atc att   438
   Ser Leu Leu Pro Leu Lys Phe Phe Pro Ile Ile Val Ile Gly Ile Ile
                45                  50                  55
   gca ttg ata tta gca ctg gcc att ggt ctg ggc atc cac ttc gac tgc   486
   Ala Leu Ile Leu Ala Leu Ala Ile Gly Leu Gly Ile His Phe Asp Cys
            60                  65                  70
   tca ggg aag tac aga tgt cgc tca tcc ttt aag tgt atc gag ctg ata   534
   Ser Gly Lys Tyr Arg Cys Arg Ser Ser Phe Lys Cys Ile Glu Leu Ile
        75                  80                  85
   gct cga tgt gac gga gtc tcg gat tgc aaa gac ggg gag gac gag tac      582
   Ala Arg Cys Asp Gly Val Ser Asp Cys Lys Asp Gly Glu Asp Glu Tyr
    90                 95                 100                 105
   cgc tgt gtc cgg gtg ggt ggt cag aat gcc gtg ctc cag gtg ttc aca      630
   Arg Cys Val Arg Val Gly Gly Gln Asn Ala Val Leu Gln Val Phe Thr
                   110                 115                 120
   gct gct tcg tgg aag acc atg tgc tcc gat gac tgg aag ggt cac tac      678
   Ala Ala Ser Trp Lys Thr Met Cys Ser Asp Asp Trp Lys Gly His Tyr
               125                 130                 135
   gca aat gtt gcc tgt gcc caa ctg ggt ttc cca agc tat gta agt tca      726
   Ala Asn Val Ala Cys Ala Gln Leu Gly Phe Pro Ser Tyr Val Ser Ser
           140                 145                 150
   gat aac ctc aga gtg agc tcg cta gag ggg cag ttc cgg gag gag ttt      774
   Asp Asn Leu Arg Val Ser Ser Leu Glu Gly Gln Phe Arg Glu Glu Phe
       155                 160                 165
   gtg tcc atc gat cac ctc ttg cca gat gac aag gtg act gca tta cac      822
   Val Ser Ile Asp His Leu Leu Pro Asp Asp Lys Val Thr Ala Leu His
   170                 175                 180                 185
   cac tca gta tat gtg agg gag gga tgt gcc tct ggc cac gtg gtt acc      870
   His Ser Val Tyr Val Arg Glu Gly Cys Ala Ser Gly His Val Val Thr
                   190                 195                 200
   ttg cag tgc aca gcc tgt ggt cat aga agg ggc tac agc tca cgc atc      918
   Leu Gln Cys Thr Ala Cys Gly His Arg Arg Gly Tyr Ser Ser Arg Ile
               205                 210                 215
   gtg ggt gga aac atg tcc ttg ctc tcg cag tgg ccc tgg cag gcc agc      966
   Val Gly Gly Asn Met Ser Leu Leu Ser Gln Trp Pro Trp Gln Ala Ser
           220                 225                 230
   ctt cag ttc cag ggc tac cac ctg tgc ggg ggc tct gtc atc acg ccc     1014
   Leu Gln Phe Gln Gly Tyr His Leu Cys Gly Gly Ser Val Ile Thr Pro
       235                 240                 245
   ctg tgg atc atc act gct gca cac tgt gtt tat gac ttg tac ctc ccc     1062
   Leu Trp Ile Ile Thr Ala Ala His Cys Val Tyr Asp Leu Tyr Leu Pro
   250                 255                 260                 265
   aag tca tgg acc atc cag gtg ggt cta gtt tcc ctg ttg gac aat cca     1110
   Lys Ser Trp Thr Ile Gln Val Gly Leu Val Ser Leu Leu Asp Asn Pro
                   270                 275                 280
   gcc cca tcc cac ttg gtg gag aag att gtc tac cac agc aag tac aag     1158
   Ala Pro Ser His Leu Val Glu Lys Ile Val Tyr His Ser Lys Tyr Lys
               285                 290                 295
   cca aag agg ctg ggc aat gac atc gcc ctt atg aag ctg gcc ggg cca     1206
   Pro Lys Arg Leu Gly Asn Asp Ile Ala Leu Met Lys Leu Ala Gly Pro
           300                 305                 310
   ctc acg ttc aat gaa atg atc cag cct gtg tgc ctg ccc aac tct gaa     1254
   Leu Thr Phe Asn Glu Met Ile Gln Pro Val Cys Leu Pro Asn Ser Glu
       315                 320                 325
   gag aac ttc ccc gat gga aaa gtg tgc tgg acg tca gga tgg ggg gcc     1302
   Glu Asn Phe Pro Asp Gly Lys Val Cys Trp Thr Ser Gly Trp Gly Ala
   330                 335                 340                 345
   aca gag gat gga ggt gac gcc tcc cct gtc ctg aac cac gcg gcc gtc      1350
   Thr Glu Asp Gly Gly Asp Ala Ser Pro Val Leu Asn His Ala Ala Val
                   350                 355                 360
   cct ttg att tcc aac aag atc tgc aac cac agg gac gtg tac ggt ggc      1398
   Pro Leu Ile Ser Asn Lys Ile Cys Asn His Arg Asp Val Tyr Gly Gly
               365                 370                 375
   atc atc tcc ccc tcc atg ctc tgc gcg ggc tac ctg acg ggt ggc gtg      1446
   Ile Ile Ser Pro Ser Met Leu Cys Ala Gly Tyr Leu Thr Gly Gly Val
           380                 385                 390
   gac agc tgc cag ggg gac agc ggg ggg ccc ctg gtg tgt caa gag agg      1494
   Asp Ser Cys Gln Gly Asp Ser Gly Gly Pro Leu Val Cys Gln Glu Arg
       395                 400                 405
   agg ctg tgg aag tta gtg gga gcg acc agc ttt ggc atc ggc tgc gca      1542
   Arg Leu Trp Lys Leu Val Gly Ala Thr Ser Phe Gly Ile Gly Cys Ala
   410                 415                 420                 425
   gag gtg aac aag cct ggg gtg tac acc cgt gtc acc tcc ttc ctg gac      1590
   Glu Val Asn Lys Pro Gly Val Tyr Thr Arg Val Thr Ser Phe Leu Asp
                   430                 435                 440
   tgg atc cac gag cag atg gag aga gac cta aaa acc tga agaggaaggg       1639
   Trp Ile His Glu Gln Met Glu Arg Asp Leu Lys Thr  *
               445                 450
   gataagtagc cacctga                                                   1656
<210> 12
   <211> 453
   <212> PRT
   <213> Homo Sapien
<400> 12
   Met Gly Glu Asn Asp Pro Pro Ala Val Glu Ala Pro Phe Ser Phe Arg
    1               5                  10                  15
   Ser Leu Phe Gly Leu Asp Asp Leu Lys Ile Ser Pro Val Ala Pro Asp
               20                  25                  30
   Ala Asp Ala Val Ala Ala Gln Ile Leu Ser Leu Leu Pro Leu Lys Phe
           35                  40                  45
   Phe Pro Ile Ile Val Ile Gly Ile Ile Ala Leu Ile Leu Ala Leu Ala
       50                  55                  60
   Ile Gly Leu Gly Ile His Phe Asp Cys Ser Gly Lys Tyr Arg Cys Arg
   65                  70                  75                  80
   Ser Ser Phe Lys Cys Ile Glu Leu Ile Ala Arg Cys Asp Gly Val Ser
                   85                  90                  95
   Asp Cys Lys Asp Gly Glu Asp Glu Tyr Arg Cys Val Arg Val Gly Gly
               100                 105                 110
   Gln Asn Ala Val Leu Gln Val Phe Thr Ala Ala Ser Trp Lys Thr Met
           115                 120                 125
   Cys Ser Asp Asp Trp Lys Gly His Tyr Ala Asn Val Ala Cys Ala Gln
       130                 135                 140
   Leu Gly Phe Pro Ser Tyr Val Ser Ser Asp Asn Leu Arg Val Ser Ser
   145                 150                 155                 160
   Leu Glu Gly Gln Phe Arg Glu Glu Phe Val Ser Ile Asp His Leu Leu
                   165                 170                 175
   Pro Asp Asp Lys Val Thr Ala Leu His His Ser Val Tyr Val Arg Glu
               180                     185                     190
   Gly Cys Ala Ser Gly His Val Val Thr Leu Gln Cys Thr Ala Cys Gly
           195                 200                 205
   His Arg Arg Gly Tyr Ser Ser Arg Ile Val Gly Gly Asn Met Ser Leu
       210                 215                 220
   Leu Ser Gln Trp Pro Trp Gln Ala Ser Leu Gln Phe Gln Gly Tyr His
   225                 230                 235                     240
   Leu Cys Gly Gly Ser Val Ile Thr Pro Leu Trp Ile Ile Thr Ala Ala
                   245                 250                     255
   His Cys Val Tyr Asp Leu Tyr Leu Pro Lys Ser Trp Thr Ile Gln Val
               260                 265                 270
   Gly Leu Val Ser Leu Leu Asp Asn Pro Ala Pro Ser His Leu Val Glu
           275                 280                 285
   Lys Ile Val Tyr His Ser Lys Tyr Lys Pro Lys Arg Leu Gly Asn Asp
       290                 295                 300
   Ile Ala Leu Met Lys Leu Ala Gly Pro Leu Thr Phe Asn Glu Met Ile
   305                 310                 315                     320
   Gln Pro Val Cys Leu Pro Asn Ser Glu Glu Asn Phe Pro Asp Gly Lys
                   325                 330                 335
   Val Cys Trp Thr Ser Gly Trp Gly Ala Thr Glu Asp Gly Gly Asp Ala
               340                 345                 350
   Ser Pro Val Leu Asn His Ala Ala Val Pro Leu Ile Ser Asn Lys Ile
           355                 360                 365
   Cys Asn His Arg Asp Val Tyr Gly Gly Ile Ile Ser Pro Ser Met Leu
       370                 375                 380
   Cys Ala Gly Tyr Leu Thr Gly Gly Val Asp Ser Cys Gln Gly Asp Ser
   385                 390                 395                     400
   Gly Gly Pro Leu Val Cys Gln Glu Arg Arg Leu Trp Lys Leu Val Gly
                   405                 410                 415
   Ala Thr Ser Phe Gly Ile Gly Cys Ala Glu Val Asn Lys Pro Gly Val
               420                 425                 430
   Tyr Thr Arg Val Thr Ser Phe Leu Asp Trp Ile His Glu Gln Met Glu
           435                 440                 445
   Arg Asp Leu Lys Thr
       450
<210> 13
   <211> 23
   <212> DNA
   <213> Artificial Sequence
<220>
   <223> Oligonucleotide Primer
<221> misc feature
   <222> (0)...(0)
   <223> N= Inosine
<400> 13
   tggrtnvtnw sngcnrcnca ytg 23
<210> 14
   <211> 30
   <212> DNA
   <213> Artificial Sequence
<220>
   <223> Oligonucleotide Primer
<221> misc feature
   <222> (0)...(0)
   <223> N= Inosine
<400> 14
   nggnccnccn swrtcnccyt nrcanghrtc 30
<210> 15
   <211> 30
   <212> DNA
   <213> Artificial Sequence
<220>
   <223> Oligonucleotide primer
<400> 15
   tcaccgagaa gatgatgtgt gcaggcatcc 30
<210> 16
   <211> 29
   <212> DNA
   <213> Artificial Sequence
<220>
   <223> Oligonucleotide primer
<400> 16
   gggacagggg ctgtaaggca gggaatgag 29
<210> 17
   <211> 25
   <212> DNA
   <213> Artificial Sequence
<220>
   <223> Oligonucleotide primer
<400> 17
   cccgcagcca tagccccagc taacg 25
<210> 18
   <211> 27
   <212> DNA
   <213> Aritificial Sequence
<400> 18
   gcagacgatg cgtaccaggg ggaagtc 27
<210> 19
   <211> 39
   <212> DNA
   <213> Artificial Sequence
<220>
   <223> Oligonucleotide primer
<400> 19
   ctcgagaaaa gagtggtggg tggggaggag gcctctgtg 39
<210> 20
   <211> 30
   <212> DNA
   <213> Artificial Sequence
<220>
   <223> Oligonucleotide primer
<400> 20
   gcggccgcat tacagctcag ccttccagac 30
<210> 21
   <211> 27
   <212> DNA
   <213> Artificial Sequence
<220>
   <223> Oligonucleotide Primer
<400> 21
   cctccacggt gctgtggacc gtgttcc 27
<210> 22
   <211> 24
   <212> DNA
   <213> Artificial Sequence
<220>
   <223> Oligonucleotide Primer
<400> 22
   cctcgcgcaa ggcgccccag cccg 24
<210> 23
   <211> 32
   <212> DNA
   <213> Artificial Sequence
<220>
   <223> Oligonucleotide Primer
<400> 23
   gcgtggcgtc acctggtagc gatagacctc gc 32
<210> 24
   <211> 27
   <212> DNA
   <213> Artificial Sequence
<220>
   <223> Oligonucleotide Primer
<400> 24
   cctccacggt gctgtggacc gtgttcc 27
<210> 25
   <211> 24
   <212> DNA
   <213> Artificial Sequence
<220>
   <223> Oligonucleotide Primer
<400> 25
   cctcgcgcaa ggcgccccag cccg 24
<210> 26
   <211> 26
   <212> DNA
   <213> Artificial Sequence
<220>
   <223> Oligonucleotide Primer
<400> 26
   tcatcggcca gagggtgatc agtgag 26
<210> 27
   <211> 28
   <212> DNA
   <213> Artificial Sequence
<220>
   <223> Oligonucleotide Primer
<400> 27
   cctcctcagt gcataggcat caaaccag 28
<210> 28
   <211> 42
   <212> DNA
   <213> Artificial Sequence
<220>
   <223> Oligonucleotide Primer
<400> 28
   tctctcgaga aaagaattgt tggtggagct gtgtcctccg ag 42
<210> 29
   <211> 31
   <212> DNA
   <213> Artificial Sequence
<220>
   <223> Oligonucleotide Primer
<400> 29
   aggtgggcct tgctttgcag gggggcagtt c 31
<210> 30
   <211> 26
   <212> DNA
   <213> Artificial Sequence
<220>
   <223> Oligonucleotide Primer
<400> 30
   tcacgcatcg tgggtggaac atgtcc 26
<210> 31
   <211> 26
   <212> DNA
   <213> Artificial Sequence
<220>
   <223> Oligonucleotide Primer
<400> 31
   acccacctcc atctgctcgt ggatcc 26
<210> 32
   <211> 27
   <212> DNA
   <213> Artificial Sequence
<220>
   <223> Oligonucleotide primer
<400> 32
   ccacagcctc ctctcttgac acaccag 27
<210> 33
   <211> 26
   <212> DNA
   <213> Artificial Sequence
<220>
   <223> Oligonucleotide primer
<400> 33
   acgcccctgt ggatcatcac tgctgc 26
<210> 34
   <211> 27
   <212> DNA
   <213> Artificial Sequence
<220>
   <223> Oligonucleotide primer
<400> 34
   tccctccctc acatatactg agtggtg 27
<210> 35
   <211> 26
   <212> DNA
   <213> Artificial Sequence
<220>
   <223> Oligonucleotide primer
<400> 35
   cgactgctca gggaagtcag atgtcg 26
<210> 36
   <211> 36
   <212> DNA
   <213> Artificial Sequence
<220>
   <223> Oligonucleotide primer
<400> 36
   gcggccgcac tataccccag tgttctcttt gatcca 36
<210> 37
   <211> 27
   <212> DNA
   <213> Artificial Sequence
<220>
   <223> Oligonucleotide primer
<400> 37
   ctggtgtgtc aagagaggag gctgtgg 27
<210> 38
   <211> 28
   <212> DNA
   <213> Artificial Sequence
<220>
   <223> Oligonucleotide primer
<400> 38
   actcaggtgg ctacttatcc ccttcctc 28
<210> 39
   <211> 42
   <212> DNA
   <213> Artificial Sequence
<220>
   <223> Oligonucleotide Primer
<400> 39
   tctctcgaga aaagagtggt gggtggggag gaggcctctg tg 42
<210> 40
   <211> 34
   <212> DNA
   <213> Artificial Sequence
<220>
   <223> Oligonucleotide Primer
<400> 40
   attcgcggcc gcattacagc tcagccttcc agac 34
<210> 41
   <211> 42
   <212> DNA
   <213> Artificial Sequence
<220>
   <223> Oligonucleotide Primer
<400> 41
   tctctcgaga aaagaattgt tggtggagct gtgtcctccg ag 42
<210> 42
   <211> 37
   <212> DNA
   <213> Artificial Sequence
<220>
   <223> Oligonucleotide Primer
<400> 42
   attcgcggcc gctcaggtca ccacttgctg gatccag 37
<210> 43
   <211> 36
   <212> DNA
   <213> Artificial Sequence
<220>
   <223> Oligonucleotide Primer
<400> 43
   ctcgagaaac gcatcgtggg tggaaacatg tccttg 36
<210> 44
   <211> 28
   <212> DNA
   <213> Artificial Sequence
<220>
   <223> Oligonucleotide Primer
<400> 44
   actcaggtgg ctacttatcc ccttcctc 28
<210> 45
   <211> 9276
   <212> DNA
   <213> Pichia pastoris
<400> 45
   agatctaaca tccaaagacg aaaggttgaa tgaaaccttt ttgccatccg acatccacag     60
   gtccattctc acacataagt gccaaacgca acaggagggg atacactagc agcagaccgt    120
   tgcaaacgca ggacctccac tcctcttctc ctcaacaccc acttttgcca tcgaaaaacc    180
   agcccagtta ttgggcttga ttggagctcg ctcattccaa ttccttctat taggctacta    240
   acaccatgac tttattagcc tgtctatcct ggcccccctg gcgaggttca tgtttgttta   300
   tttccgaatg caacaagctc cgcattacac ccgaacatca ctccagatga gggctttctg    360
   agtgtggggt caaatagttt catgttcccc aaatggccca aaactgacag tttaaacgct    420
   gtcttggaac ctaatatgac aaaagcgtga tctcatccaa gatgaactaa gtttggttcg    480
   ttgaaatgct aacggccagt tggtcaaaaa gaaacttcca aaagtcgcca taccgtttgt    540
   cttgtttggt attgattgac gaatgctcaa aaataatctc attaatgctt agcgcagtct    600
   ctctatcgct tctgaacccc ggtgcacctg tgccgaaacg caaatgggga aacacccgct    660
   ttttggatga ttatgcattg tctccacatt gtatgcttcc aagattctgg tgggaatact    720
   gctgatagcc taacgttcat gatcaaaatt taactgttct aacccctact tgacagcaat    780
   atataaacag aaggaagctg ccctgtctta aacctttttt tttatcatca ttattagctt    840
   actttcataa ttgcgactgg ttccaattga caagcttttg attttaacga cttttaacga    900
   caacttgaga agatcaaaaa acaactaatt attcgaagga tccaaacgat gagatttcct    960
   tcaattttta ctgcagtttt attcgcagca tcctccgcat tagctgctcc agtcaacact   1020
   acaacagaag atgaaacggc acaaattccg gctgaagctg tcatcggtta ctcagattta   1080
   gaaggggatt tcgatgttgc tgttttgcca ttttccaaca gcacaaataa cgggttattg   1140
   tttataaata ctactattgc cagcattgct gctaaagaag aaggggtatc tctcgagaaa   1200
   agagaggctg aagcttacgt agaattccct agggcggccg cgaattaatt cgccttagac   1260
   atgactgttc ctcagttcaa gttgggcact tacgagaaga ccggtcttgc tagattctaa   1320
   tcaagaggat gtcagaatgc catttgcctg agagatgcag gcttcatttt tgatactttt   1380
   ttatttgtaa cctatatagt ataggatttt ttttgtcatt ttgtttcttc tcgtacgagc   1440
   ttgctcctga tcagcctatc tcgcagctga tgaatatctt gtggtagggg tttgggaaaa   1500
   tcattcgagt ttgatgtttt tcttggtatt tcccactcct cttcagagta cagaagatta   1560
   agtgagaagt tcgtttgtgc aagcttatcg ataagcttta atgcggtagt ttatcacagt   1620
   taaattgcta acgcagtcag gcaccgtgta tgaaatctaa caatgcgctc atcgtcatcc   1680
   tcggcaccgt caccctggat gctgtaggca taggcttggt tatgccggta ctgccgggcc   1740
   tcttgcggga tatcgtccat tccgacagca tcgccagtca ctatggcgtg ctgctagcgc   1800
   tatatgcgtt gatgcaattt ctatgcgcac ccgttctcgg agcactgtcc gaccgctttg   1860
   gccgccgccc agtcctgctc gcttcgctac ttggagccac tatcgactac gcgatcatgg   1920
   cgaccacacc cgtcctgtgg atctatcgaa tctaaatgta agttaaaatc tctaaataat   1980
   taaataagtc ccagtttctc catacgaacc ttaacagcat tgcggtgagc atctagacct   2040
   tcaacagcag ccagatccat cactgcttgg ccaatatgtt tcagtccctc aggagttacg   2100
   tcttgtgaag tgatgaactt ctggaaggtt gcagtgttaa ctccgctgta ttgacgggca   2160
   tatccgtacg ttggcaaagt gtggttggta ccggaggagt aatctccaca actctctgga   2220
   gagtaggcac caacaaacac agatccagcg tgttgtactt gatcaacata agaagaagca   2280
   ttctcgattt gcaggatcaa gtgttcagga gcgtactgat tggacatttc caaagcctgc   2340
   tcgtaggttg caaccgatag ggttgtagag tgtgcaatac acttgcgtac aatttcaacc 2400
   cttggcaact gcacagcttg gttgtgaaca gcatcttcaa ttctggcaag ctccttgtct 2460
   gtcatatcga cagccaacag aatcacctgg gaatcaatac catgttcagc ttgagacaga 2520
   aggtctgagg caacgaaatc tggatcagcg tatttatcag caataactag aacttcagaa 2580
   ggcccagcag gcatgtcaat actacacagg gctgatgtgt cattttgaac catcatcttg 2640
   gcagcagtaa cgaactggtt tcctggacca aatattttgt cacacttagg aacagtttct 2700
   gttccgtaag ccatagcagc tactgcctgg gcgcctcctg ctagcacgat acacttagca 2760
   ccaaccttgt gggcaacgta gatgacttct ggggtaaggg taccatcctt cttaggtgga 2820
   gatgcaaaaa caatttcttt gcaaccagca actttggcag gaacacccag catcagggaa 2880
   gtggaaggca gaattgcggt tccaccagga atatagaggc caactttctc aataggtctt 2940
   gcaaaacgag agcagactac accagggcaa gtctcaactt gcaacgtctc cgttagttga 3000
   gcttcatgga atttcctgac gttatctata gagagatcaa tggctctctt aacgttatct 3060
   ggcaattgca taagttcctc tgggaaagga gcttctaaca caggtgtctt caaagcgact 3120
   ccatcaaact tggcagttag ttctaaaagg gctttgtcac cattttgacg aacattgtcg 3180
   acaattggtt tgactaattc cataatctgt tccgttttct ggataggacg acgaagggca 3240
   tcttcaattt cttgtgagga ggccttagaa acgtcaattt tgcacaattc aatacgacct 3300
   tcagaaggga cttctttagg tttggattct tctttaggtt gttccttggt gtatcctggc 3360
   ttggcatctc ctttccttct agtgaccttt agggacttca tatccaggtt tctctccacc 3420
   tcgtccaacg tcacaccgta cttggcacat ctaactaatg caaaataaaa taagtcagca 3480
   cattcccagg ctatatcttc cttggattta gcttctgcaa gttcatcagc ttcctcccta 3540
   attttagcgt tcaacaaaac ttcgtcgtca aataaccgtt tggtataaga accttctgga 3600
   gcattgctct tacgatccca caaggtggct tccatggctc taagaccctt tgattggcca 3660
   aaacaggaag tgcgttccaa gtgacagaaa ccaacacctg tttgttcaac cacaaatttc 3720
   aagcagtctc catcacaatc caattcgata cccagcaact tttgagttgc tccagatgta 3780
   gcacctttat accacaaacc gtgacgacga gattggtaga ctccagtttg tgtccttata 3840
   gcctccggaa tagacttttt ggacgagtac accaggccca acgagtaatt agaagagtca 3900
   gccaccaaag tagtgaatag accatcgggg cggtcagtag tcaaagacgc caacaaaatt 3960
   tcactgacag ggaacttttt gacatcttca gaaagttcgt attcagtagt caattgccga 4020
   gcatcaataa tggggattat accagaagca acagtggaag tcacatctac caactttgcg 4080
   gtctcagaaa aagcataaac agttctacta ccgccattag tgaaactttt caaatcgccc 4140
   agtggagaag aaaaaggcac agcgatacta gcattagcgg gcaaggatgc aactttatca 4200
   accagggtcc tatagataac cctagcgcct gggatcatcc tttggacaac tctttctgcc 4260
   aaatctaggt ccaaaatcac ttcattgata ccattattgt acaacttgag caagttgtcg 4320
   atcagctcct caaattggtc ctctgtaacg gatgactcaa cttgcacatt aacttgaagc 4380
   tcagtcgatt gagtgaactt gatcaggttg tgcagctggt cagcagcata gggaaacacg 4440
   gcttttccta ccaaactcaa ggaattatca aactctgcaa cacttgcgta tgcaggtagc 4500
   aagggaaatg tcatacttga agtcggacag tgagtgtagt cttgagaaat tctgaagccg 4560
   tatttttatt atcagtgagt cagtcatcag gagatcctct acgccggacg catcgtggcc 4620
   gacctgcagg gggggggggg gcgctgaggt ctgcctcgtg aagaaggtgt tgctgactca 4680
   taccaggcct gaatcgcccc atcatccagc cagaaagtga gggagccacg gttgatgaga 4740
   gctttgttgt aggtggacca gttggtgatt ttgaactttt gctttgccac ggaacggtct 4800
   gcgttgtcgg gaagatgcgt gatctgatcc ttcaactcag caaaagttcg atttattcaa 4860
   caaagccgcc gtcccgtcaa gtcagcgtaa tgctctgcca gtgttacaac caattaacca 4920
   attctgatta gaaaaactca tcgagcatca aatgaaactg caatttattc atatcaggat 4980
   tatcaatacc atatttttga aaaagccgtt tctgtaatga aggagaaaac tcaccgaggc 5040
   agttccatag gatggcaaga tcctggtatc ggtctgcgat tccgactcgt ccaacatcaa 5100
   tacaacctat taatttcccc tcgtcaaaaa taaggttatc aagtgagaaa tcaccatgag 5160
   tgacgactga atccggtgag aatggcaaaa gcttatgcat ttctttccag acttgttcaa 5220
   caggccagcc attacgctcg tcatcaaaat cactcgcatc aaccaaaccg ttattcattc 5280
   gtgattgcgc ctgagcgaga cgaaatacgc gatcgctgtt aaaaggacaa ttacaaacag 5340
   gaatcgaatg caaccggcgc aggaacactg ccagcgcatc aacaatattt tcacctgaat 5400
   caggatattc ttctaatacc tggaatgctg ttttcccggg gatcgcagtg gtgagtaacc 5460
   atgcatcatc aggagtacgg ataaaatgct tgatggtcgg aagaggcata aattccgtca 5520
   gccagtttag tctgaccatc tcatctgtaa catcattggc aacgctacct ttgccatgtt 5580
   tcagaaacaa ctctggcgca tcgggcttcc catacaatcg atagattgtc gcacctgatt 5640
   gcccgacatt atcgcgagcc catttatacc catataaatc agcatccatg ttggaattta 5700
   atcgcggcct cgagcaagac gtttcccgtt gaatatggct cataacaccc cttgtattac 5760
   tgtttatgta agcagacagt tttattgttc atgatgatat atttttatct tgtgcaatgt 5820
   aacatcagag attttgagac acaacgtggc tttccccccc ccccctgcag gtcggcatca 5880
   ccggcgccac aggtgcggtt gctggcgcct atatcgccga catcaccgat ggggaagatc 5940
   gggctcgcca cttcgggctc atgagcgctt gtttcggcgt gggtatggtg gcaggccccg 6000
   tggccggggg actgttgggc gccatctcct tgcatgcacc attccttgcg gcggcggtgc 6060
   tcaacggcct caacctacta ctgggctgct tcctaatgca ggagtcgcat aagggagagc 6120
   gtcgagtatc tatgattgga agtatgggaa tggtgatacc cgcattcttc agtgtcttga 6180
   ggtctcctat cagattatgc ccaactaaag caaccggagg aggagatttc atggtaaatt 6240
   tctctgactt ttggtcatca gtagactcga actgtgagac tatctcggtt atgacagcag 6300
   aaatgtcctt cttggagaca gtaaatgaag tcccaccaat aaagaaatcc ttgttatcag 6360
   gaacaaactt cttgtttcga actttttcgg tgccttgaac tataaaatgt agagtggata 6420
   tgtcgggtag gaatggagcg ggcaaatgct taccttctgg accttcaaga ggtatgtagg 6480
   gtttgtagat actgatgcca acttcagtga caacgttgct atttcgttca aaccattccg 6540
   aatccagaga aatcaaagtt gtttgtctac tattgatcca agccagtgcg gtcttgaaac 6600
   tgacaatagt gtgctcgtgt tttgaggtca tctttgtatg aataaatcta gtctttgatc 6660
   taaataatct tgacgagcca aggcgataaa tacccaaatc taaaactctt ttaaaacgtt 6720
   aaaaggacaa gtatgtctgc ctgtattaaa ccccaaatca gctcgtagtc tgatcctcat 6780
   caacttgagg ggcactatct tgttttagag aaatttgcgg agatgcgata tcgagaaaaa 6840
   ggtacgctga ttttaaacgt gaaatttatc tcaagatctc tgcctcgcgc gtttcggtga 6900
   tgacggtgaa aacctctgac acatgcagct cccggagacg gtcacagctt gtctgtaagc 6960
   ggatgccggg agcagacaag cccgtcaggg cgcgtcagcg ggtgttggcg ggtgtcgggg 7020
   cgcagccatg acccagtcac gtagcgatag cggagtgtat actggcttaa ctatgcggca 7080
   tcagagcaga ttgtactgag agtgcaccat atgcggtgtg aaataccgca cagatgcgta 7140
   aggagaaaat accgcatcag gcgctcttcc gcttcctcgc tcactgactc gctgcgctcg 7200
   gtcgttcggc tgcggcgagc ggtatcagct cactcaaagg cggtaatacg gttatccaca 7260
   gaatcagggg ataacgcagg aaagaacatg tgagcaaaag gccagcaaaa ggccaggaac 7320
   cgtaaaaagg ccgcgttgct ggcgtttttc cataggctcc gcccccctga cgagcatcac 7380
   aaaaatcgac gctcaagtca gaggtggcga aacccgacag gactataaag ataccaggcg 7440
   tttccccctg gaagctccct cgtgcgctct cctgttccga ccctgccgct taccggatac 7500
   ctgtccgcct ttctcccttc gggaagcgtg gcgctttctc aatgctcacg ctgtaggtat 7560
   ctcagttcgg tgtaggtcgt tcgctccaag ctgggctgtg tgcacgaacc ccccgttcag 7620
   cccgaccgct gcgccttatc cggtaactat cgtcttgagt ccaacccggt aagacacgac 7680
   ttatcgccac tggcagcagc cactggtaac aggattagca gagcgaggta tgtaggcggt 7740
   gctacagagt tcttgaagtg gtggcctaac tacggctaca ctagaaggac agtatttggt 7800
   atctgcgctc tgctgaagcc agttaccttc ggaaaaagag ttggtagctc ttgatccggc 7860
   aaacaaacca ccgctggtag cggtggtttt tttgtttgca agcagcagat tacgcgcaga 7920
   aaaaaaggat ctcaagaaga tcctttgatc ttttctacgg ggtctgacgc tcagtggaac 7980
   gaaaactcac gttaagggat tttggtcatg agattatcaa aaaggatctt cacctagatc 8040
   cttttaaatt aaaaatgaag ttttaaatca atctaaagta tatatgagta aacttggtct 8100
   gacagttacc aatgcttaat cagtgaggca cctatctcag cgatctgtct atttcgttca 8160
   tccatagttg cctgactccc cgtcgtgtag ataactacga tacgggaggg cttaccatct 8220
   ggccccagtg ctgcaatgat accgcgagac ccacgctcac cggctccaga tttatcagca 8280
   ataaaccagc cagccggaag ggccgagcgc agaagtggtc ctgcaacttt atccgcctcc 8340
   atccagtcta ttaattgttg ccgggaagct agagtaagta gttcgccagt taatagtttg 8400
   cgcaacgttg ttgccattgc tgcaggcatc gtggtgtcac gctcgtcgtt tggtatggct 8460
   tcattcagct ccggttccca acgatcaagg cgagttacat gatcccccat gttgtgcaaa 8520
   aaagcggtta gctccttcgg tcctccgatc gttgtcagaa gtaagttggc cgcagtgtta 8580
   tcactcatgg ttatggcagc actgcataat tctcttactg tcatgccatc cgtaagatgc 8640
   ttttctgtga ctggtgagta ctcaaccaag tcattctgag aatagtgtat gcggcgaccg 8700
   agttgctctt gcccggcgtc aacacgggat aataccgcgc cacatagcag aactttaaaa 8760
   gtgctcatca ttggaaaacg ttcttcgggg cgaaaactct caaggatctt accgctgttg 8820
   agatccagtt cgatgtaacc cactcgtgca cccaactgat cttcagcatc ttttactttc 8880
   accagcgttt ctgggtgagc aaaaacagga aggcaaaatg ccgcaaaaaa gggaataagg 8940
   gcgacacgga aatgttgaat actcatactc ttcctttttc aatattattg aagcatttat 9000
   cagggttatt gtctcatgag cggatacata tttgaatgta tttagaaaaa taaacaaata 9060
   ggggttccgc gcacatttcc ccgaaaagtg ccacctgacg tctaagaaac cattattatc 9120
   atgacattaa cctataaaaa taggcgtatc acgaggccct ttcgtcttca agaattaatt 9180
   ctcatgtttg acagcttatc atcgataagc tgactcatgt tggtattgtg aaatagacgc 9240
   agatcgggaa cactgaaaaa taacagttat tattcg 9276
<210> 46
   <211> 3908
   <212> DNA
   <213> Escherichia coli
<400> 46
   agcgcccaat acgcaaaccg cctctccccg cgcgttggcc gattcattaa tgcagctggc 60
   acgacaggtt tcccgactgg aaagcgggca gtgagcgcaa cgcaattaat gtgagttagc      120
   tcactcatta ggcaccccag gctttacact ttatgcttcc ggctcgtatg ttgtgtggaa      180
   ttgtgagcgg ataacaattt cacacaggaa acagctatga ccatgattac gccaagcttg      240
   gtaccgagct cggatccact agtaacggcc gccagtgtgc tggaattcgc ccttaagggc      300
   gaattctgca gatatccatc acactggcgg ccgctcgagc atgcatctag agggcccaat      360
   tcgccctata gtgagtcgta ttacaattca ctggccgtcg ttttacaacg tcgtgactgg      420
   gaaaaccctg gcgttaccca acttaatcgc cttgcagcac atcccccttt cgccagctgg      480
   cgtaatagcg aagaggcccg caccgatcgc ccttcccaac agttgcgcag cctgaatggc      540
   gaatgggacg cgccctgtag cggcgcatta agcgcggcgg gtgtggtggt tacgcgcagc      600
   gtgaccgcta cacttgccag cgccctagcg cccgctcctt tcgctttctt cccttccttt      660
   ctcgccacgt tcgccggctt tccccgtcaa gctctaaatc gggggctccc tttagggttc      720
   cgatttagag ctttacggca cctcgaccgc aaaaaacttg atttgggtga tggttcacgt      780
   agtgggccat cgccctgata gacggttttt cgccctttga cgttggagtc cacgttcttt      840
   aatagtggac tcttgttcca aactggaaca acactcaacc ctatcgcggt ctattctttt      900
   gatttataag ggattttgcc gatttcggcc tattggttaa aaaatgagct gatttaacaa      960
   attcagggcg caagggctgc taaaggaacc ggaacacgta gaaagccagt ccgcagaaac     1020
   ggtgctgacc ccggatgaat gtcagctact gggctatctg gacaagggaa aacgcaagcg     1080
   caaagagaaa gcaggtagct tgcagtgggc ttacatggcg atagctagac tgggcggttt     1140
   tatggacagc aagcgaaccg gaattgccag ctggggcgcc ctctggtaag gttgggaagc     1200
   cctgcaaagt aaactggatg gctttcttgc cgccaaggat ctgatggcgc aggggatcaa     1260
   gatctgatca agagacagga tgaggatcgt ttcgcatgat tgaacaagat ggattgcacg     1320
   caggttctcc ggccgcttgg gtggagaggc tattcggcta tgactgggca caacagacaa     1380
   tcggctgctc tgatgccgcc gtgttccggc tgtcagcgca ggggcgcccg gttctttttg     1440
   tcaagaccga cctgtccggt gccctgaatg aactgcagga cgaggcagcg cggctatcgt     1500
   ggctggccac gacgggcgtt ccttgcgcag ctgtgctcga cgttgtcact gaagcgggaa     1560
   gggactggct gctattgggc gaagtgccgg ggcaggatct cctgtcatct cgccttgctc     1620
   ctgccgagaa agtatccatc atggctgatg caatgcggcg gctgcatacg cttgatccgg     1680
   ctacctgccc attcgaccac caagcgaaac atcgcatcga gcgagcacgt actcggatgg     1740
   aagccggtct tgtcgatcag gatgatctgg acgaagagca tcaggggctc gcgccagccg     1800
   aactgttcgc caggctcaag gcgcgcatgc ccgacggcga ggatctcgtc gtgatccatg     1860
   gcgatgcctg cttgccgaat atcatggtgg aaaatggccg cttttctgga ttcaacgact     1920
   gtggccggct gggtgtggcg gaccgctatc aggacatagc gttggatacc cgtgatattg     1980
   ctgaagagct tggcggcgaa tgggctgacc gcttcctcgt gctttacggt atcgccgctc     2040
   ccgattcgca gcgcatcgcc ttctatcgcc ttcttgacga gttcttctga attgaaaaag     2100
   gaagagtatg agtattcaac atttccgtgt cgcccttatt cccttttttg cggcattttg     2160
   ccttcctgtt tttgctcacc cagaaacgct ggtgaaagta aaagatgctg aagatcagtt     2220
   gggtgcacga gtgggttaca tcgaactgga tctcaacagc ggtaagatcc ttgagagttt     2280
   tcgccccgaa gaacgttttc caatgatgag cacttttaaa gttctgctat gtcatacact     2340
   attatcccgt attgacgccg ggcaagagca actcggtcgc cgggcgcggt attctcagaa     2400
   tgacttggtt gagtactcac cagtcacaga aaagcatctt acggatggca tgacagtaag     2460
   agaattatgc agtgctgcca taaccatgag tgataacact gcggccaact tacttctgac     2520
   aacgatcgga ggaccgaagg agctaaccgc ttttttgcac aacatggggg atcatgtaac     2580
   tcgccttgat cgttgggaac cggagctgaa tgaagccata ccaaacgacg agagtgacac     2640
   cacgatgcct gtagcaatgc caacaacgtt gcgcaaacta ttaactggcg aactacttac     2700
   tctagcttcc cggcaacaat taatagactg gatggaggcg gataaagttg caggaccact     2760
   tctgcgctcg gcccttccgg ctggctggtt tattgctgat aaatctggag ccggtgagcg     2820
   tgggtctcgc ggtatcattg cagcactggg gccagatggt aagccctccc gtatcgtagt     2880
   tatctacacg acggggagtc aggcaactat ggatgaacga aatagacaga tcgctgagat     2940
   aggtgcctca ctgattaagc attggtaact gtcagaccaa gtttactcat atatacttta     3000
   gattgattta aaacttcatt tttaatttaa aaggatctag gtgaagatcc tttttgataa     3060
   tctcatgacc aaaatccctt aacgtgagtt ttcgttccac tgagcgtcag accccgtaga     3120
   aaagatcaaa ggatcttctt gagatccttt ttttctgcgc gtaatctgct gcttgcaaac     3180
   aaaaaaacca ccgctaccag cggtggtttg tttgccggat caagagctac caactctttt     3240
   tccgaaggta actggcttca gcagagcgca gataccaaat actgtccttc tagtgtagcc     3300
   gtagttaggc caccacttca agaactctgt agcaccgcct acatacctcg ctctgctaat     3360
   cctgttacca gtggctgctg ccagtggcga taagtcgtgt cttaccgggt tggactcaag     3420
   acgatagtta ccggataagg cgcagcggtc gggctgaacg gggggttcgt gcacacagcc     3480
   cagcttggag cgaacgacct acaccgaact gagataccta cagcgtgagc attgagaaag     3540
   cgccacgctt cccgaaggga gaaaggcgga caggtatccg gtaagcggca gggtcggaac     3600
   aggagagcgc acgagggagc ttccaggggg aaacgcctgg tatctttata gtcctgtcgg     3660
   gtttcgccac ctctgacttg agcgtcgatt tttgtgatgc tcgtcagggg ggcggagcct     3720
   atggaaaaac gccagcaacg cggccttttt acggttcctg gccttttgct ggccttttgc     3780
   tcacatgttc tttcctgcgt tatcccctga ttctgtggat aaccgtatta ccgcctttga    3840
   gtgagctgat accgctcgcc gcagccgaac gaccgagcgc agcgagtcag tgagcgagga    3900
   agcggaag                                                             3908
<210> 47
   <211> 46
   <212> DNA
   <213> Artificial Sequence
<220>
   <223> Oligonucleotide primer
<400> 47
   ggaattccat atgccgcgct ttaaagtggt gggtggggag gaggcc 46
<210> 48
   <211> 32
   <212> DNA
   <213> Artificial Sequence
<220>
   <223> Oligonucleotide primer
<400> 48
   cgcgataccc gttacagctc agccttccag ac 32
<210> 49
   <211> 3147
   <212> DNA
   <213> Homo Sapien
<220>
   <221> CDS
   <222> (1865)...(2590)
   <223> Nucleic acid sequence of protease domain of MTSP1
<400> 49
   tcaagagcgg cctcggggta ccatggggag cgatcgggcc cgcaagggcg gagggggccc     60
   gaaggacttc ggcgcgggac tcaagtacaa ctcccggcac gagaaagtga atggcttgga    120
   ggaaggcgtg gagttcctgc cagtcaacaa cgtcaagaag gtggaaaagc atggcccggg    180
   gcgctgggtg gtgctggcag ccgtgctgat cggcctcctc ttggtcttgc tggggatcgg    240
   cttcctggtg tggcatttgc agtaccggga cgtgcgtgtc cagaaggtct tcaatggcta    300
   catgaggatc acaaatgaga attttgtgga tgcctacgag aactccaact ccactgagtt    360
   tgtaagcctg gccagcaagg tgaaggacgc gctgaagctg ctgtacagcg gagtcccatt    420
   cctgggcccc taccacaagg agtcggctgt gacggccttc agcgagggca gcgtcatcgc    480
   ctactactgg tctgagttca gcatcccgca gcacctggtg gaggaggccg agcgcgtcat    540
   ggccgaggag cgcgtagtca tgctgccccc gcgggcgcgc tccctgaagt cctttgtggt    600
   cacctcagtg gtggctttcc ccacggactc caaaacagta cagaggaccc aggacaacag    660
   ctgcagcttt ggcctgcacg cccgcggtgt ggagctgatg cgcttcacca cgcccggctt    720
   ccctgacagc ccctaccccg ctcatgcccg ctgccagtgg gccctgcggg gggacgccga    780
   ctcagtgctg agcctcacct tccgcagctt tgaccttgcg tcctgcgacg agcgcggcag    840
   cgacctggtg acggtgtaca acaccctgag ccccatggag ccccacgccc tggtgcagtt    900
   gtgtggcacc taccctccct cctacaacct gaccttccac tcctcccaga acgtcctgct    960
   catcacactg ataaccaaca ctgagcggcg gcatcccggc tttgaggcca ccttcttcca   1020
   gctgcctagg atgagcagct gtggaggccg cttacgtaaa gcccagggga cattcaacag   1080
   cccctactac ccaggccact acccacccaa cattgactgc acatggaaca ttgaggtgcc   1140
   caacaaccag catgtgaagg tgagcttcaa attcttctac ctgctggagc ccggcgtgcc   1200
   tgcgggcacc tgccccaagg actacgtgga gatcaatggg gagaaatact gcggagagag   1260
   gtcccagttc gtcgtcacca gcaacagcaa caagatcaca gttcgcttcc actcagatca   1320
   gtcctacacc gacaccggct tcttagctga atacctctcc tacgactcca gtgacccatg   1380
   cccggggcag ttcacgtgcc gcacggggcg gtgtatccgg aaggagctgc gctgtgatgg   1440
   ctgggccgac tgcaccgacc acagcgatga gctcaactgc agttgcgacg ccggccacca   1500
   gttcacgtgc aagaacaagt tctgcaagcc cctcttctgg gtctgcgaca gtgtgaacga   1560
   ctgcggagac aacagcgacg agcaggggtg cagttgtccg gcccagacct tcaggtgttc   1620
   caatgggaag tgcctctcga aaagccagca gtgcaatggg aaggacgact gtggggacgg   1680
   gtccgacgag gcctcctgcc ccaaggtgaa cgtcgtcact tgtaccaaac acacctaccg   1740
   ctgcctcaat gggctctgct tgagcaaggg caaccctgag tgtgacggga aggaggactg   1800
   tagcgacggc tcagatgaga aggactgcga ctgtgggctg cggtcattca cgagacaggc   1860
   tcgt gtt gtt ggg ggc acg gat gcg gat gag ggc gag tgg ccc tgg cag    1909
   gta agc ctg cat gct ctg ggc cag ggc cac atc tgc ggt gct tcc ctc     1957
   atc tct ccc aac tgg ctg gtc tct gcc gca cac tgc tac atc gat gac     2005
   aga gga ttc agg tac tca gac ccc acg cag tgg acg gcc ttc ctg ggc     2053
   ttg cac gac cag agc cag cgc agc gcc cct ggg gtg cag gag cgc agg     2101
   ctc aag cgc atc atc tcc cac ccc ttc ttc aat gac ttc acc ttc gac     2149
   tat gac atc gcg ctg ctg gag ctg gag aaa ccg gca gag tac agc tcc     2197
   atg gtg cgg ccc atc tgc ctg ccg gac gcc tcc cat gtc ttc cct gcc     2245
   ggc aag gcc atc tgg gtc acg ggc tgg gga cac acc cag tat gga ggc     2293
   act ggc gcg ctg atc ctg caa aag ggt gag atc cgc gtc atc aac cag     2341
   acc acc tgc gag aac ctc ctg ccg cag cag atc acg ccg cgc atg atg     2389
   tgc gtg ggc ttc ctc agc ggc ggc gtg gac tcc tgc cag ggt gat tcc     2437
   ggg gga ccc ctg tcc agc gtg gag gcg gat ggg cgg atc ttc cag gcc     2485
   ggt gtg gtg agc tgg gga gac ggc tgc gct cag agg aac aag cca ggc     2533
   gtg tac aca agg ctc cct ctg ttt cgg gac tgg atc aaa gag aac act     2581
   ggg gta tag gggccggggc cacccaaatg tgtacacctg cggggccacc             2630
   catcgtccac cccagtgtgc acgcctgcag gctggagact ggaccgctga ctgcaccagc   2690
   gcccccagaa catacactgt gaactcaatc tccagggctc caaatctgcc tagaaaacct   2750
   ctcgcttcct cagcctccaa agtggagctg ggaggtagaa ggggaggaca ctggtggttc   2810
   tactgaccca actgggggca aaggtttgaa gacacagcct cccccgccag ccccaagctg   2870
   ggccgaggcg cgtttgtgta tatctgcctc ccctgtctgt aaggagcagc gggaacggag   2930
   cttcggagcc tcctcagtga aggtggtggg gctgccggat ctgggctgtg gggcccttgg   2990
   gccacgctct tgaggaagcc caggctcgga ggaccctgga aaacagacgg gtctgagact   3050
   gaaattgttt taccagctcc cagggtggac ttcagtgtgt gtatttgtgt aaatgggtaa   3110
   aacaatttat ttctttttaa aaaaaaaaaa aaaaaaa                            3147
<210> 50
   <211> 241
   <212> PRT
   <213> Homo Sapien
<400> 50
<210> 51
   <211> 46
   <212> DNA
   <213> Artificial Sequence
<220>
   <223> Oligonucleoide Primer
<400> 51
   tctctcgaga aaagagtggt gggtgggtgg ggaggaggcc tctgtg 46
<210> 52
   <211> 43
   <212> DNA
   <213> Aritificial sequence
<400> 52
   gctcctcatc aaagaagggc agagagatgg gcctgactgt gcc 43
<210> 53
   <211> 34
   <212> DNA
   <213> Artificial Sequence
<220>
   <223> Oligonucleoide Primer
<400> 53
   attcgcggcc gcattacagc tcagccttcc agac 34
<210> 54
   <211> 43
   <212> DNA
   <213> Artificial Sequence
<220>
   <223> Oligonucleoide Primer
<400> 54
   ggcacagtca ggcccatctc tctgcccttc tttgatgagg agc 43
<210> 55
   <211> 28
   <212> DNA
   <213> Artificial Sequence
<220>
   <223> Oligonucleoide Primer
<400> 55
   caccccttct tcaatgactt caccttcg 28
<210> 56
   <211> 18
   <212> DNA
   <213> Artificial Sequence
<220>
   <223> Oligonucleoide Primer
<400> 56
   tacctctcct acgactcc 18
<210> 57
   <211> 25
   <212> DNA
   <213> Artificial Sequence
<220>
   <223> Oligonucleoide Primer
<400> 57
   gaggttctcg caggtggtct ggttg 25
<210> 58
   <211> 39
   <212> DNA
   <213> Artificial Sequence
<220>
   <223> Oligonucleoide Primer
<400> 58
   ctcgagaaaa gagttgttgg gggcacggat gcggatgag 39
<210> 59
   <211> 11
   <212> PRT
   <213> Homo Sapien
<400> 59
   Phe Glu Val Phe Ser Gln Ser Ser Ser Leu Gly
    1               5                   10
<210> 60
   <211> 32
   <212> PRT
   <213> Homo Sapien
<400> 60
   Glu Ile Val Ala Pro Arg Glu Arg Ala Asp Arg Arg Gly Arg Lys Leu
    1               5                  10                  15
   Leu Cys Trp Arg Lys Pro Thr Lys Met Lys Gly Pro Arg Pro Ser His
               20                  25                  30
<210> 61
   <211> 4933
   <212> DNA
   <213> Homo Sapien
<220>
   <221> CDS
   <222> (94)...(3222)
   <223> Nucleotide sequence encoding corin
<300>
   <308> GenBank AF133845
   <309> 1999-05-24
<400> 61
   aaatcatccg tagtgcctcc ccgggggaca cgtagaggag agaaaagcga ccaagataaa      60
   agtggacaga agaataagcg agacttttta tcc atg aaa cag tct cct gcc ctc      114
   gct ccg gaa gag cgc tac cgc aga gcc ggg tcc cca aag ccg gtc ttg      162
   aga gct gat gac aat aac atg ggc aat ggc tgc tct cag aag ctg gcg      210
   act gct aac ctc ctc cgg ttc cta ttg ctg gtc ctg att cca tgt atc      258
   tgt gct ctc gtt ctc ttg ctg gtg atc ctg ctt tcc tat gtt gga aca      306
   tta caa aag gtc tat ttt aaa tca aat ggg agt gaa cct ttg gtc act      354
   gat ggt gaa atc caa ggg tcc gat gtt att ctt aca aat aca att tat      402
   aac cag agc act gtg gtg tct act gca cat ccc gac caa cac gtt cca      450
   gcc tgg act acg gat gct tct ctc cca ggg gac caa agt cac agg aat      498
   aca agt gcc tgt atg aac atc acc cac agc cag tgt cag atg ctg ccc      546
   tac cac gcc acg ctg aca cct ctc ctc tca gtt gtc aga aac atg gaa      594
   atg gaa aag ttc ctc aag ttt ttc aca tat ctc cat cgc ctc agt tgc      642
   tat caa cat atc atg ctg ttt ggc tgt acc ctc gcc ttc cct gag tgc      690
   atc att gat ggc gat gac agt cat gga ctc ctg ccc tgt agg tcc ttc      738
   tgt gag gct gca aaa gaa ggc tgt gaa tca gtc ctg ggg atg gtg aat      786
   tac tcc tgg ccg gat ttc ctc aga tgc tcc cag ttt aga aac caa act      834
   gaa agc agc aat gtc agc aga att tgc ttc tca cct cag cag gaa aac      882
   gga aag caa ttg ctc tgt gga agg ggt gag aac ttt ctg tgt gcc agt      930
   gga atc tgc atc ccc ggg aaa ctg caa tgt aat ggc tac aac gac tgt      978
   gac gac tgg agt gac gag gct cat tgc aac tgc agc gag aat ctg ttt     1026
   cac tgt cac aca ggc aag tgc ctt aat tac agc ctt gtg tgt gat gga     1074
   tat gat gac tgt ggg gat ttg agt gat gag caa aac tgt gat tgc aat     1122
   ccc aca aca gag cat cgc tgc ggg gac ggg cgc tgc atc gcc atg gag     1170
   tgg gtg tgt gat ggt gac cac gac tgt gtg gat aag tcc gac gag gtc     1218
   aac tgc tcc tgt cac agc cag ggt ctg gtg gaa tgc aga aat gga caa     1266
   tgt atc ccc agc acg ttt caa tgt gat ggt gac gag gac tgc aag gat     1314
   ggg agt gat gag gag aac tgc agc gtc att cag act tca tgt caa gaa     1362
   gga gac caa aga tgc ctc tac aat ccc tgc ctt gat tca tgt ggt ggt     1410
   agc tct ctc tgt gac ccg aac aac agt ctg aat aac tgt agt caa tgt     1458
   gaa cca att aca ttg gaa ctc tgc atg aat ttg ccc tac aac agt aca     1506
   agt tat cca aat tat ttt ggc cac agg act caa aag gaa gca tcc atc     1554
   agc tgg gag tct tct ctt ttc cct gca ctt gtt caa acc aac tgt tat     1602
   aaa tac ctc atg ttc ttt tct tgc acc att ttg gta cca aaa tgt gat     1650
   gtg aat aca ggc gag cgt atc cct cct tgc agg gca ttg tgt gaa cac     1698
   tct aaa gaa cgc tgt gag tct gtt ctt ggg att gtg ggc cta cag tgg      1746
   cct gaa gac aca gat tgc agt caa ttt cca gag gaa aat tca gac aat      1794
   caa acc tgc ctg atg cct gat gaa tat gtg gaa gaa tgc tca cct agt      1842
   cat ttc aag tgc cgc tca gga cag tgt gtt ctg gct tcc aga aga tgt      1890
   gat ggc cag gcc gac tgt gac gat gac agt gat gag gaa aac tgt ggt      1938
   tgt aaa gag aga gat ctt tgg gaa tgt cca tcc aat aaa caa tgt ttg      1986
   aag cac aca gtg atc tgc gat ggg ttc cca gac tgc cct gat tac atg      2034
   gac gag aaa aac tgc tca ttt tgc caa gat gat gag ctg gaa tgt gca      2082
   aac cat gcg tgt gtg tca cgt gac ctg tgg tgt gat ggt gaa gcc gac      2130
   tgc tca gac agt tca gat gaa tgg gac tgt gtg acc ctc tct ata aat      2178
   gtg aac tcc tct tcc ttt ctg atg gtt cac aga gct gcc aca gaa cac      2226
   cat gtg tgt gca gat ggc tgg cag gag ata ttg agt cag ctg gcc tgc      2274
   aag cag atg ggt tta gga gaa cca tct gtg acc aaa ttg ata cag gaa      2322
   cag gag aaa gag ccg cgg tgg ctg aca tta cac tcc aac tgg gag agc      2370
   ctc aat ggg acc act tta cat gaa ctt cta gta aat ggg cag tct tgt      2418
   gag agc aga agt aaa att tct ctt ctg tgt act aaa caa gac tgt ggg      2466
   cgc cgc cct gct gcc cga atg aac aaa agg atc ctt gga ggt cgg acg      2514
   agt cgc cct gga agg tgg cca tgg cag tgt tct ctg cag agt gaa ccc      2562
   agt gga cat atc tgt ggc tgt gtc ctc att gcc aag aag tgg gtt ctg      2610
   aca gtt gcc cac tgc ttc gag ggg aga gag aat gct gca gtt tgg aaa      2658
   gtg gtg ctt ggc atc aac aat cta gac cat cca tca gtg ttc atg cag      2706
   aca cgc ttt gtg aag acc atc atc ctg cat ccc cgc tac agt cga gca      2754
   gtg gtg gac tat gac atc agc atc gtt gag ctg agt gaa gac atc agt      2802
   gag act ggc tac gtc cgg cct gtc tgc ttg ccc aac ccg gag cag tgg      2850
   cta gag cct gac acg tac tgc tat atc aca ggc tgg ggc cac atg ggc      2898
   aat aaa atg cca ttt aag ctg caa gag gga gag gtc cgc att att tct      2946
   ctg gaa cat tgt cag tcc tac ttt gac atg aag acc atc acc act cgg      2994
   atg ata tgt gct ggc tat gag tct ggc aca gtt gat tca tgc atg ggt      3042
   gac agc ggt ggg cct ctt gtt tgt gag aag cct gga gga cgg tgg aca      3090
   tta ttt gga tta act tca tgg ggc tcc gtc tgc ttt tcc aaa gtc ctg      3138
   ggg cct ggc gtt tat agt aat gtg tca tat ttc gtc gaa tgg att aaa      3186
   aga cag att tac atc cag acc ttt ctc cta aac taa ttataaggat        3232
   gatcagagac ttttgccagc tacactaaaa gaaaatggcc ttcttgactg tgaagagctg  3292
   cctgcagaga gctgtacaga agcacttttc atggacagaa atgctcaatc gtgcactgca  3352
   aatttgcatg tttgttttgg actaattttt ttcaatttat tttttcacct tcatttttct 3412
   cttatttcaa gttcaatgaa agactttaca aaagcaaaca aagcagactt tgtccttttg  3472
   ccaggcctaa ccatgactgc agcacaaaat tatcgactct ggcgagattt aaaatcaggt  3532
   gctacagtaa caggttatgg aatggtctct tttatcctat cacaaaaaaa gacatagata  3592
   tttaggctga ttaattatct ctaccagttt ttgtttctca agctcagtgc atagtggtaa  3652
   atttcagtgt taacattgga gacttgcttt tctttttctt tttttatacc ccacaattct  3712
   tttttattac acttcgaatt ttagggtaca cgagcacaac gtgcaggtta gttacatatg  3772
   tatacatgtg ccatgttggt gtgctgaacc cagtaactcg tcatttgatt tattaaaagc  3832
   caagataatt tacatgttta aagtatttac tattaccccc ttctaatgtt tgcataattc  3892
   tgagaactga taaaagacag caataaaaga ccagtgtcat ccatttaggt agcaagacat  3952
   attgaatgca aagttcttta gatatcaata ttaacacttg acattattgg accccccatt  4012
   ctggatgtat atcaagatca taattttata gaagagtctc tatagaactg tcctcatagc  4072
   tgggtttgtt caggatatat gagttggctg attgagactg caacaactac atctatattt  4132
   atgggcaata ttttgtttta cttatgtggc aaagaactgg atattaaact ttgcaaaaga  4192
   gaatttagat gagagatgca attttttaaa aagaaaatta atttgcatcc ctcgtttaat   4252
   taaatttatt tttcagtttt cttgcgttca tccataccaa caaagtcata aagagcatat  4312
   tttagagcac agtaagactt tgcatggagt aaaacatttt gtaattttcc tcaaaagatg  4372
   tttaatatct ggtttcttct cattggtaat taaaatttta gaaatgattt ttagctctag  4432
   gccactttac gcaactcaat ttctgaagca attagtggta aaaagtattt ttccccacta  4492
   aaaaacttta aaacacaaat cttcatatat acttaattta attagtcagg catccatttt  4552
   gccttttaaa caactaggat tccctactaa cctccaccag caacctggac tgcctcagca  4612
   ttccaaatag atactacctg caattttata catgtatttt tgtatctttt ctgtgtgtaa  4672
   acatagttga aattcaaaaa gttgtagcaa tttctatact attcatctcc tgtccttcag  4732
   tttgtataaa cctaaggaga gtgtgaaatc cagcaactga attgtggtca cgattgtatg  4792
   aaagttcaag aacatatgtc agttttgtta cagttgtagc tacatactca atgtatcaac  4852
   ttttagcctg ctcaacttag gctcagtgaa atatatatat tatacttatt ttaaataatt  4912
   cttaatacaa ataaaatggt a                                            4933
<210> 62
   <211> 1042
   <212> PRT
   <213> Homo Sapien
<400> 62
<210> 63
   <211> 3696
   <212> DNA
   <213> Homo Sapien
<220>
   <221> CDS
   <222> (41)...(3100)
   <223> Nucleotide sequence encoding human entorkinase
<300>
   <308> GenBank HSU09860
   <309> 1995-06-03
<400> 63
   accagacagt tcttaaatta gcaagccttc aaaaccaaaa atg ggg tcg aaa aga         55
                                              Met Gly Ser Lys Arg
                                              1               5
   ggc ata tct tct agg cat cat tct ctc agc tcc tat gaa atc atg ttt        103
   Gly Ile Ser Ser Arg His His Ser Leu Ser Ser Tyr Glu Ile Met Phe
                   10                  15                  20
   gca gct ctc ttt gcc ata ttg gta gtg ctc tgt gct gga tta att gca        151
   Ala Ala Leu Phe Ala Ile Leu Val Val Leu Cys Ala Gly Leu Ile Ala
               25                  30                  35
   gta tcc tgc ctg aca atc aag gaa tcc caa cga ggt gca gca ctt gga        199
   Val Ser Cys Leu Thr Ile Lys Glu Ser Gln Arg Gly Ala Ala Leu Gly
           40                  45                  50
   cag agt cat gaa gcc aga gcg aca ttt aaa ata aca tcc gga gtt aca        247
   Gln Ser His Glu Ala Arg Ala Thr Phe Lys Ile Thr Ser Gly Val Thr
       55                  60                  65
   tat aat cct aat ttg caa gac aaa ctc tca gtg gat ttc aaa gtt ctt        295
   Tyr Asn Pro Asn Leu Gln Asp Lys Leu Ser Val Asp Phe Lys Val Leu
   70                  75                  80                  85
   gct ttt gac ctt cag caa atg ata gat gag atc ttt cta tca agc aat        343
   Ala Phe Asp Leu Gln Gln Met Ile Asp Glu Ile Phe Leu Ser Ser Asn
                   90                  95                  100
   ctg aag aat gaa tat aag aac tca aga gtt tta caa ttt gaa aat ggc        391
   Leu Lys Asn Glu Tyr Lys Asn Ser Arg Val Leu Gln Phe Glu Asn Gly
               105                 110                 115
   agc att ata gtc gta ttt gac ctt ttc ttt gcc cag tgg gtg tca gat        439
   Ser Ile Ile Val Val Phe Asp Leu Phe Phe Ala Gln Trp Val Ser Asp
           120                 125                 130
   caa aat gta aaa gaa gaa ctg att caa ggc ctt gaa gca aat aaa tcc        487
   Gln Asn Val Lys Glu Glu Leu Ile Gln Gly Leu Glu Ala Asn Lys Ser
       135                 140                 145
   agc caa ctg gtc act ttc cat att gat ttg aac agc gtt gat atc cta        535
   Ser Gln Leu Val Thr Phe His Ile Asp Leu Asn Ser Val Asp Ile Leu
   150                 155                 160                 165
   gac aag cta aca acc acc agt cat ctg gca act cca gga aat gtc tca        583
   Asp Lys Leu Thr Thr Thr Ser His Leu Ala Thr Pro Gly Asn Val Ser
                   170                 175                 180
   ata gag tgc ctg cct ggt tca agt cct tgt act gat gct cta acg tgt        631
   Ile Glu Cys Leu Pro Gly Ser Ser Pro Cys Thr Asp Ala Leu Thr Cys
               185                 190                 195
   ata aaa gct gat tta ttt tgt gat gga gaa gta aac tgt cca gat ggt        679
   Ile Lys Ala Asp Leu Phe Cys Asp Gly Glu Val Asn Cys Pro Asp Gly
           200                 205                 210
   tct gac gaa gac aat aaa atg tgt gcc aca gtt tgt gat gga aga ttt        727
   Ser Asp Glu Asp Asn Lys Met Cys Ala Thr Val Cys Asp Gly Arg Phe
       215                 220                 225
   ttg tta act gga tca tct ggg tct ttc cag gct act cat tat cca aaa      775
   Leu Leu Thr Gly Ser Ser Gly Ser Phe Gln Ala Thr His Tyr Pro Lys
   230                 235                 240                 245
   cct tct gaa aca agt gtt gtc tgc cag tgg atc ata cgt gta aac caa      823
   Pro Ser Glu Thr Ser Val Val Cys Gln Trp Ile Ile Arg Val Asn Gln
                   250                 255                 260
   gga ctt tcc att aaa ctg agc ttc gat gat ttt aat aca tat tat aca      871
   Gly Leu Ser Ile Lys Leu Ser Phe Asp Asp Phe Asn Thr Tyr Tyr Thr
               265                 270                 275
   gat ata tta gat att tat gaa ggt gta gga tca agc aag att tta aga      919
   Asp Ile Leu Asp Ile Tyr Glu Gly Val Gly Ser Ser Lys Ile Leu Arg
           280                 285                 290
   gct tct att tgg gaa act aat cct ggc aca ata aga att ttt tcc aac      967
   Ala Ser Ile Trp Glu Thr Asn Pro Gly Thr Ile Arg Ile Phe Ser Asn
       295                 300                 305
   caa gtt act gcc acc ttt ctt ata gaa tct gat gaa agt gat tat gtt     1015
   Gln Val Thr Ala Thr Phe Leu Ile Glu Ser Asp Glu Ser Asp Tyr Val
   310                 315                 320                 325
   ggc ttt aat gca aca tat act gca ttt aac agc agt gag ctt aat aat     1063
   Gly Phe Asn Ala Thr Tyr Thr Ala Phe Asn Ser Ser Glu Leu Asn Asn
                   330                 335                 340
   tat gag aaa att aat tgt aac ttt gag gat ggc ttt tgt ttc tgg gtc     1111
   Tyr Glu Lys Ile Asn Cys Asn Phe Glu Asp Gly Phe Cys Phe Trp Val
               345                 350                 355
   cag gat cta aat gat gat aat gaa tgg gaa agg att cag gga agc acc     1159
   Gln Asp Leu Asn Asp Asp Asn Glu Trp Glu Arg Ile Gln Gly Ser Thr
           360                 365                 370
   ttt tct cct ttt act gga ccc aat ttt gac cac act ttt ggc aat gct     1207
   Phe Ser Pro Phe Thr Gly Pro Asn Phe Asp His Thr Phe Gly Asn Ala
       375                 380                 385
   tca gga ttt tac att tct acc cca act gga cca gga ggg aga caa gaa     1255
   Ser Gly Phe Tyr Ile Ser Thr Pro Thr Gly Pro Gly Gly Arg Gln Glu
   390                 395                 400                 405
   cga gtg ggg ctt tta agc ctc cct ttg gac ccc act ttg gag cca gct     1303
   Arg Val Gly Leu Leu Ser Leu Pro Leu Asp Pro Thr Leu Glu Pro Ala
                   410                 415                 420
   tgc ctt agt ttc tgg tat cat atg tat ggt gaa aat gtc cat aaa tta     1351
   Cys Leu Ser Phe Trp Tyr His Met Tyr Gly Glu Asn Val His Lys Leu
               425                 430                 435
   agc att aat atc agc aat gac caa aat atg gag aag aca gtt ttc caa     1399
   Ser Ile Asn Ile Ser Asn Asp Gln Asn Met Glu Lys Thr Val Phe Gln
           440                 445                 450
   aag gaa gga aat tat gga gac aat tgg aat tat gga caa gta acc cta     1447
   Lys Glu Gly Asn Tyr Gly Asp Asn Trp Asn Tyr Gly Gln Val Thr Leu
       455                 460                 465
   aat gaa aca gtt aaa ttt aag gtt gct ttt aat gct ttt aaa aac aag      1495
   Asn Glu Thr Val Lys Phe Lys Val Ala Phe Asn Ala Phe Lys Asn Lys
   470                 475                 480                 485
   atc ctg agt gat att gcg ttg gat gac att agc cta aca tat ggg att      1543
   Ile Leu Ser Asp Ile Ala Leu Asp Asp Ile Ser Leu Thr Tyr Gly Ile
                   490                 495                 500
   tgc aat ggg agt ctt tat cca gaa cca act ttg gtg cca act cct cca      1591
   Cys Asn Gly Ser Leu Tyr Pro Glu Pro Thr Leu Val Pro Thr Pro Pro
               505                 510                 515
   cca gaa ctt cct acg gac tgt gga gga cct ttt gag ctg tgg gag cca      1639
   Pro Glu Leu Pro Thr Asp Cys Gly Gly Pro Phe Glu Leu Trp Glu Pro
           520                 525                 530
   aat aca aca ttc agt tct acg aac ttt cca aac agc tac cct aat ctg      1687
   Asn Thr Thr Phe Ser Ser Thr Asn Phe Pro Asn Ser Tyr Pro Asn Leu
       535                 540                 545
   gct ttc tgt gtt tgg att tta aat gca caa aaa gga aag aat ata caa      1735
   Ala Phe Cys Val Trp Ile Leu Asn Ala Gln Lys Gly Lys Asn Ile Gln
   550                 555                 560                 565
   ctt cat ttt caa gaa ttt gac tta gaa aat att aac gat gta gtt gaa      1783
   Leu His Phe Gln Glu Phe Asp Leu Glu Asn Ile Asn Asp Val Val Glu
                   570                 575                 580
   ata aga gat ggt gaa gaa gct gat tcc ttg ctc tta gct gtg tac aca      1831
   Ile Arg Asp Gly Glu Glu Ala Asp Ser Leu Leu Leu Ala Val Tyr Thr
               585                 590                 595
   ggg cct ggc cca gta aag gat gtg ttc tct acc acc aac aga atg act      1879
   Gly Pro Gly Pro Val Lys Asp Val Phe Ser Thr Thr Asn Arg Met Thr
           600                 605                 610
   gtg ctt ctc atc act aac gat gtg ttg gca aga gga ggg ttt aaa gca      1927
   Val Leu Leu Ile Thr Asn Asp Val Leu Ala Arg Gly Gly Phe Lys Ala
       615                 620                 625
   aac ttt act act ggc tat cac ttg ggg att cca gag cca tgc aag gca      1975
   Asn Phe Thr Thr Gly Tyr His Leu Gly Ile Pro Glu Pro Cys Lys Ala
   630                 635                 640                 645
   gac cat ttt caa tgt aaa aat gga gag tgt gtt cca ctg gtg aat ctc      2023
   Asp His Phe Gln Cys Lys Asn Gly Glu Cys Val Pro Leu Val Asn Leu
                   650                 655                 660
   tgt gac ggt cat ctg cac tgt gag gat ggc tca gat gaa gca gat tgt      2071
   Cys Asp Gly His Leu His Cys Glu Asp Gly Ser Asp Glu Ala Asp Cys
               665                 670                 675
   gtg cgt ttt ttc aat ggc aca acg aac aac aat ggt tta gtg cgg ttc      2119
   Val Arg Phe Phe Asn Gly Thr Thr Asn Asn Asn Gly Leu Val Arg Phe
           680                 685                 690
   aga atc cag agc ata tgg cat aca gct tgt gct gag aac tgg acc acc      2167
   Arg Ile Gln Ser Ile Trp His Thr Ala Cys Ala Glu Asn Trp Thr Thr
       695                 700                 705
   cag att tca aat gat gtt tgt caa ctg ctg gga cta ggg agt gga aac      2215
   Gln Ile Ser Asn Asp Val Cys Gln Leu Leu Gly Leu Gly Ser Gly Asn
   710                 715                 720                 725
   tca tca aag cca atc ttc tct acc gat ggt gga cca ttt gtc aaa tta      2263
   Ser Ser Lys Pro Ile Phe Ser Thr Asp Gly Gly Pro Phe Val Lys Leu
               730                 735                 740
   aac aca gca cct gat ggc cac tta ata cta aca ccc agt caa cag tgt      2311
   Asn Thr Ala Pro Asp Gly His Leu Ile Leu Thr Pro Ser Gln Gln Cys
               745                 750                 755
   tta cag gat tcc ttg att cgg tta cag tgt aac cat aaa tct tgt gga      2359
   Leu Gln Asp Ser Leu Ile Arg Leu Gln Cys Asn His Lys Ser Cys Gly
           760                 765                 770
   aaa aaa ctg gca gct caa gac atc acc cca aag att gtt gga gga agt      2407
   Lys Lys Leu Ala Ala Gln Asp Ile Thr Pro Lys Ile Val Gly Gly Ser
       775                 780                 785
   aat gcc aaa gaa ggg gcc tgg ccc tgg gtt gtg ggt ctg tat tat ggc      2455
   Asn Ala Lys Glu Gly Ala Trp Pro Trp Val Val Gly Leu Tyr Tyr Gly
   790                 795                 800                 805
   ggc cga ctg ctc tgc ggc gca tct ctc gtc agc agt gac tgg ctg gtg      2503
   Gly Arg Leu Leu Cys Gly Ala Ser Leu Val Ser Ser Asp Trp Leu Val
                   810                 815                 820
   tcc gcc gca cac tgc gtg tat ggg aga aac tta gag cca tcc aag tgg      2551
   Ser Ala Ala His Cys Val Tyr Gly Arg Asn Leu Glu Pro Ser Lys Trp
               825                 830                 835
   aca gca atc cta ggc ctg cat atg aaa tca aat ctg acc tct cct caa      2599
   Thr Ala Ile Leu Gly Leu His Met Lys Ser Asn Leu Thr Ser Pro Gln
           840                 845                 850
   aca gtc cct cga tta ata gat gaa att gtc ata aac cct cat tac aat      2647
   Thr Val Pro Arg Leu Ile Asp Glu Ile Val Ile Asn Pro His Tyr Asn
       855                 860                 865
   agg cga aga aag gac aac gac att gcc atg atg cat ctg gaa ttt aaa      2695
   Arg Arg Arg Lys Asp Asn Asp Ile Ala Met Met His Leu Glu Phe Lys
   870                 875                 880                 885
   gtg aat tac aca gat tac ata caa cct att tgt tta ccg gaa gaa aat      2743
   Val Asn Tyr Thr Asp Tyr Ile Gln Pro Ile Cys Leu Pro Glu Glu Asn
                   890                 895                 900
   caa gtt ttt cct cca gga aga aat tgt tct att gct ggt tgg ggg acg      2791
   Gln Val Phe Pro Pro Gly Arg Asn Cys Ser Ile Ala Gly Trp Gly Thr
               905                 910                 915
   gtt gta tat caa ggt act act gca aac ata ttg caa gaa gct gat gtt      2839
   Val Val Tyr Gln Gly Thr Thr Ala Asn Ile Leu Gln Glu Ala Asp Val
           920                 925                 930
   cct ctt cta tca aat gag aga tgc caa cag cag atg cca gaa tat aac      2887
   Pro Leu Leu Ser Asn Glu Arg Cys Gln Gln Gln Met Pro Glu Tyr Asn
       935                 940                 945
   att act gaa aat atg ata tgt gca ggc tat gaa gaa gga gga ata gat      2935
   Ile Thr Glu Asn Met Ile Cys Ala Gly Tyr Glu Glu Gly Gly Ile Asp
   950                 955                 960                 965
   tct tgt cag ggg gat tca gga gga cca tta atg tgc caa gaa aac aac      2983
   Ser Cys Gln Gly Asp Ser Gly Gly Pro Leu Met Cys Gln Glu Asn Asn
                   970                 975                 980
   agg tgg ttc ctt gct ggt gtg acc tca ttt gga tac aag tgt gcc ctg      3031
   Arg Trp Phe Leu Ala Gly Val Thr Ser Phe Gly Tyr Lys Cys Ala Leu
               985                 990                 995
   cct aat cgc ccc gga gtg tat gcc agg gtc tca agg ttt acc gaa tgg      3079
   Pro Asn Arg Pro Gly Val Tyr Ala Arg Val Ser Arg Phe Thr Glu Trp
           1000                1005                1010
   ata caa agt ttt cta cat tag cgcatttctt aaactaaaca ggaaagtcgc         3130
   Ile Gln Ser Phe Leu His  *
       1015
   attattttcc cattctactc tagaaagcat ggaaattaag tgtttcgtac aaaaatttta    3190
   aaaagttacc aaaggttttt attcttacct atgtcaatga aatgctaggg ggccagggaa    3250
   acaaaatttt aaaaataata aaattcacca tagcaataca gaataacttt aaaataccat    3310
   taaatacatt tgtatttcat tgtgaacagg tatttcttca cagatctcat ttttaaaatt    3370
   cttaatgatt atttttatta cttactgttg tttaaaggga tgttatttta aagcatatac    3430
   catacactta agaaatttga gcagaattta aaaaagaaag aaaataaatt gtttttccca    3490
   aagtatgtca ctgttggaaa taaactgcca taaattttct agttccagtt tagtttgctg    3550
   ctattagcag aaactcaatt gtttctctgt cttttctatc aaaattttca acatatgcat    3610
   aaccttagta ttttcccaac caatagaaac tatttattgt aagcttatgt cacaggcctg    3670
   gactaaattg attttacgtt cctctt                                         3696
<210> 64
   <211> 1019
   <212> PRT
   <213> Homo Sapien
<400> 64
   Met Gly Ser Lys Arg Gly Ile Ser Ser Arg His His Ser Leu Ser Ser
    1               5                  10                  15
   Tyr Glu Ile Met Phe Ala Ala Leu Phe Ala Ile Leu Val Val Leu Cys
               20                  25                  30
   Ala Gly Leu Ile Ala Val Ser Cys Leu Thr Ile Lys Glu Ser Gln Arg
           35                  40                  45
   Gly Ala Ala Leu Gly Gln Ser His Glu Ala Arg Ala Thr Phe Lys Ile
       50                  55                  60
   Thr Ser Gly Val Thr Tyr Asn Pro Asn Leu Gln Asp Lys Leu Ser Val
   65                  70                  75                  80
   Asp Phe Lys Val Leu Ala Phe Asp Leu Gln Gln Met Ile Asp Glu Ile
                   85                  90                  95
   Phe Leu Ser Ser Asn Leu Lys Asn Glu Tyr Lys Asn Ser Arg Val Leu
               100                 105                 110
   Gln Phe Glu Asn Gly Ser Ile Ile Val Val Phe Asp Leu Phe Phe Ala
           115                 120                 125
   Gln Trp Val Ser Asp Gln Asn Val Lys Glu Glu Leu Ile Gln Gly Leu
       130                 135                 140
   Glu Ala Asn Lys Ser Ser Gln Leu Val Thr Phe His Ile Asp Leu Asn
   145                 150                 155                 160
   Ser Val Asp Ile Leu Asp Lys Leu Thr Thr Thr Ser His Leu Ala Thr
                   165                 170                 175
   Pro Gly Asn Val Ser Ile Glu Cys Leu Pro Gly Ser Ser Pro Cys Thr
               180                 185                 190
   Asp Ala Leu Thr Cys Ile Lys Ala Asp Leu Phe Cys Asp Gly Glu Val
           195                 200                 205
   Asn Cys Pro Asp Gly Ser Asp Glu Asp Asn Lys Met Cys Ala Thr Val
       210                 215                 220
   Cys Asp Gly Arg Phe Leu Leu Thr Gly Ser Ser Gly Ser Phe Gln Ala
   225 230 235 240
   Thr His Tyr Pro Lys Pro Ser Glu Thr Ser Val Val Cys Gln Trp Ile
   245 250 255
   Ile Arg Val Asn Gln Gly Leu Ser Ile Lys Leu Ser Phe Asp Asp Phe
   260 265 270
   Asn Thr Tyr Tyr Thr Asp Ile Leu Asp Ile Tyr Glu Gly Val Gly Ser
   275 280 285
   Ser Lys Ile Leu Arg Ala Ser Ile Trp Glu Thr Asn Pro Gly Thr Ile
   290 295 300
   Arg Ile Phe Ser Asn Gln Val Thr Ala Thr Phe Leu Ile Glu Ser Asp
   305 310 315 320
   Glu Ser Asp Tyr Val Gly Phe Asn Ala Thr Tyr Thr Ala Phe Asn Ser
   325 330 335
   Ser Glu Leu Asn Asn Tyr Glu Lys Ile Asn Cys Asn Phe Glu Asp Gly
   340 345 350
   Phe Cys Phe Trp Val Gln Asp Leu Asn Asp Asp Asn Glu Trp Glu Arg
   355 360 365
   Ile Gln Gly Ser Thr Phe Ser Pro Phe Thr Gly Pro Asn Phe Asp His
   370 375 380
   Thr Phe Gly Asn Ala Ser Gly Phe Tyr Ile Ser Thr Pro Thr Gly Pro
   385 390 395 400
   Gly Gly Arg Gln Glu Arg Val Gly Leu Leu Ser Leu Pro Leu Asp Pro
   405 410 415
   Thr Leu Glu Pro Ala Cys Leu Ser Phe Trp Tyr His Met Tyr Gly Glu
   420 425 430
   Asn Val His Lys Leu Ser Ile Asn Ile Ser Asn Asp Gln Asn Met Glu
   435 440 445
   Lys Thr Val Phe Gln Lys Glu Gly Asn Tyr Gly Asp Asn Trp Asn Tyr
   450 455 460
   Gly Gln Val Thr Leu Asn Glu Thr Val Lys Phe Lys Val Ala Phe Asn
   465 470 475 480
   Ala Phe Lys Asn Lys Ile Leu Ser Asp Ile Ala Leu Asp Asp Ile Ser
   485 490 495
   Leu Thr Tyr Gly Ile Cys Asn Gly Ser Leu Tyr Pro Glu Pro Thr Leu
   500 505 510
   Val Pro Thr Pro Pro Pro Glu Leu Pro Thr Asp Cys Gly Gly Pro Phe
   515 520 525
   Glu Leu Trp Glu Pro Asn Thr Thr Phe Ser Ser Thr Asn Phe Pro Asn
   530 535 540
   Ser Tyr Pro Asn Leu Ala Phe Cys Val Trp Ile Leu Asn Ala Gln Lys
   545 550 555 560
   Gly Lys Asn Ile Gln Leu His Phe Gln Glu Phe Asp Leu Glu Asn Ile
   565 570 575
   Asn Asp Val Val Glu Ile Arg Asp Gly Glu Glu Ala Asp Ser Leu Leu
   580 585 590
   Leu Ala Val Tyr Thr Gly Pro Gly Pro Val Lys Asp Val Phe Ser Thr
   595 600 605
   Thr Asn Arg Met Thr Val Leu Leu Ile Thr Asn Asp Val Leu Ala Arg
   610 615 620
   Gly Gly Phe Lys Ala Asn Phe Thr Thr Gly Tyr His Leu Gly Ile Pro
   625 630 635 640
   Glu Pro Cys Lys Ala Asp His Phe Gln Cys Lys Asn Gly Glu Cys Val
   645 650 655
   Pro Leu Val Asn Leu Cys Asp Gly His Leu His Cys Glu Asp Gly Ser
   660 665 670
   Asp Glu Ala Asp Cys Val Arg Phe Phe Asn Gly Thr Thr Asn Asn Asn
   675 680 685
   Gly Leu Val Arg Phe Arg Ile Gln Ser Ile Trp His Thr Ala Cys Ala
   690 695 700
   Glu Asn Trp Thr Thr Gln Ile Ser Asn Asp Val Cys Gln Leu Leu Gly
   705 710 715 720
   Leu Gly Ser Gly Asn Ser Ser Lys Pro Ile Phe Ser Thr Asp Gly Gly
                   725                 730                 735
   Pro Phe Val Lys Leu Asn Thr Ala Pro Asp Gly His Leu Ile Leu Thr
               740                 745                 750
   Pro Ser Gln Gln Cys Leu Gln Asp Ser Leu Ile Arg Leu Gln Cys Asn
           755                 760                 765
   His Lys Ser Cys Gly Lys Lys Leu Ala Ala Gln Asp Ile Thr Pro Lys
       770                 775                 780
   Ile Val Gly Gly Ser Asn Ala Lys Glu Gly Ala Trp Pro Trp Val Val
   785                 790                 795                 800
   Gly Leu Tyr Tyr Gly Gly Arg Leu Leu Cys Gly Ala Ser Leu Val Ser
                   805                 810                 815
   Ser Asp Trp Leu Val Ser Ala Ala His Cys Val Tyr Gly Arg Asn Leu
               820                 825                 830
   Glu Pro Ser Lys Trp Thr Ala Ile Leu Gly Leu His Met Lys Ser Asn
           835                 840                 845
   Leu Thr Ser Pro Gln Thr Val Pro Arg Leu Ile Asp Glu Ile Val Ile
       850                 855                 860
   Asn Pro His Tyr Asn Arg Arg Arg Lys Asp Asn Asp Ile Ala Met Met
   865                 870                 875                 880
   His Leu Glu Phe Lys Val Asn Tyr Thr Asp Tyr Ile Gln Pro Ile Cys
                   885                 890                 895
   Leu Pro Glu Glu Asn Gln Val Phe Pro Pro Gly Arg Asn Cys Ser Ile
               900                 905                 910
   Ala Gly Trp Gly Thr Val Val Tyr Gln Gly Thr Thr Ala Asn Ile Leu
           915                 920                 925
   Gln Glu Ala Asp Val Pro Leu Leu Ser Asn Glu Arg Cys Gln Gln Gln
       930                 935                 940
   Met Pro Glu Tyr Asn Ile Thr Glu Asn Met Ile Cys Ala Gly Tyr Glu
   945                 950                 955                 960
   Glu Gly Gly Ile Asp Ser Cys Gln Gly Asp Ser Gly Gly Pro Leu Met
                   965                 970                 975
   Cys Gln Glu Asn Asn Arg Trp Phe Leu Ala Gly Val Thr Ser Phe Gly
               980                 985                 990
   Tyr Lys Cys Ala Leu Pro Asn Arg Pro Gly Val Tyr Ala Arg Val Ser
           995                 1000                1005
   Arg Phe Thr Glu Trp Ile Gln Ser Phe Leu His
       1010                1015
<210> 65
   <211> 1500
   <212> DNA
   <213> Homo Sapien
<220>
   <221> CDS
   <222> (62)...(1318)
   <223> Nucleotide sequence encoding human airway trypsin-like protease
<300>
   <308> GenBank AB002134
   <309> 1998-06-04
<400> 65
   gagtgggaat ctcaaagcag ttgagtaggc agaaaaaaga acctcttcat taaggattaa     60
   a atg tat agg cca gca cgt gta act tcg act tca aga ttt ctg aat cca    109
     Met Tyr Arg Pro Ala Arg Val Thr Ser Thr Ser Arg Phe Leu Asn Pro
     1               5                   10                  15
   tat gta gta tgt ttc att gtc gtc gca ggg gta gtg atc ctg gca gtc      157
   Tyr Val Val Cys Phe Ile Val Val Ala Gly Val Val Ile Leu Ala Val
                   20                       25                       30
   acc ata gct cta ctt gtt tac ttt tta gct ttt gat caa aaa tct tac     205
   Thr Ile Ala Leu Leu Val Tyr Phe Leu Ala Phe Asp Gln Lys Ser Tyr
           35                  40                  45
   ttt tat agg agc agt ttt caa ctc cta aat gtt gaa tat aat agt cag     253
   Phe Tyr Arg Ser Ser Phe Gln Leu Leu Asn Val Glu Tyr Asn Ser Gln
       50                  55                  60
   tta aat tca cca gct aca cag gaa tac agg act ttg agt gga aga att     301
   Leu Asn Ser Pro Ala Thr Gln Glu Tyr Arg Thr Leu Ser Gly Arg Ile
   65                  70                  75                  80
   gaa tct ctg att act aaa aca ttc aaa gaa tca aat tta aga aat cag     349
   Glu Ser Leu Ile Thr Lys Thr Phe Lys Glu Ser Asn Leu Arg Asn Gln
                   85                  90                  95
   ttc atc aga gct cat gtt gcc aaa ctg agg caa gat ggt agt ggt gtg     397
   Phe Ile Arg Ala His Val Ala Lys Leu Arg Gln Asp Gly Ser Gly Val
               100                 105                 110
   aga gcg gat gtt gtc atg aaa ttt caa ttc act aga aat aac aat gga     445
   Arg Ala Asp Val Val Met Lys Phe Gln Phe Thr Arg Asn Asn Asn Gly
           115                 120                 125
   gca tca atg aaa agc aga att gag tct gtt tta cga caa atg ctg aat     493
   Ala Ser Met Lys Ser Arg Ile Glu Ser Val Leu Arg Gln Met Leu Asn
       130                 135                 140
   aac tct gga aac ctg gaa ata aac cct tca act gag ata aca tca ctt     541
   Asn Ser Gly Asn Leu Glu Ile Asn Pro Ser Thr Glu Ile Thr Ser Leu
   145                 150                 155                 160
   act gac cag gct gca gca aat tgg ctt att aat gaa tgt ggg gcc ggt     589
   Thr Asp Gln Ala Ala Ala Asn Trp Leu Ile Asn Glu Cys Gly Ala Gly
                   165                 170                 175
   cca gac cta ata aca ttg tct gag cag aga atc ctt gga ggc act gag     637
   Pro Asp Leu Ile Thr Leu Ser Glu Gln Arg Ile Leu Gly Gly Thr Glu
               180                 185                 190
   gct gag gag gga agc tgg ccg tgg caa gtc agt ctg cgg ctc aat aat     685
   Ala Glu Glu Gly Ser Trp Pro Trp Gln Val Ser Leu Arg Leu Asn Asn
           195                 200                 205
   gcc cac cac tgt gga ggc agc ctg atc aat aac atg tgg atc ctg aca     733
   Ala His His Cys Gly Gly Ser Leu Ile Asn Asn Met Trp Ile Leu Thr
       210                 215                 220
   gca gct cac tgc ttc aga agc aac tct aat cct cgt gac tgg att gcc     781
   Ala Ala His Cys Phe Arg Ser Asn Ser Asn Pro Arg Asp Trp Ile Ala
   225                 230                 235                 240
   acg tct ggt att tcc aca aca ttt cct aaa cta aga atg aga gta aga     829
   Thr Ser Gly Ile Ser Thr Thr Phe Pro Lys Leu Arg Met Arg Val Arg
                   245                 250                 255
   aat att tta att cat aac aat tat aaa tct gca act cat gaa aat gac     877
   Asn Ile Leu Ile His Asn Asn Tyr Lys Ser Ala Thr His Glu Asn Asp
               260                 265                 270
   att gca ctt gtg aga ctt gag aac agt gtc acc ttt acc aaa gat atc      925
   Ile Ala Leu Val Arg Leu Glu Asn Ser Val Thr Phe Thr Lys Asp Ile
           275                     280                     285
   cat agt gtg tgt ctc cca gct gct acc cag aat att cca cct ggc tct      973
   His Ser Val Cys Leu Pro Ala Ala Thr Gln Asn Ile Pro Pro Gly Ser
       290                     295                     300
   act gct tat gta aca gga tgg ggc gct caa gaa tat gct ggc cac aca     1021
   Thr Ala Tyr Val Thr Gly Trp Gly Ala Gln Glu Tyr Ala Gly His Thr
   305                     310                     315                     320
   gtt cca gag cta agg caa gga cag gtc aga ata ata agt aat gat gta     1069
   Val Pro Glu Leu Arg Gln Gly Gln Val Arg Ile Ile Ser Asn Asp Val
                   325                     330                     335
   tgt aat gca cca cat agt tat aat gga gcc atc ttg tct gga atg ctg     1117
   Cys Asn Ala Pro His Ser Tyr Asn Gly Ala Ile Leu Ser Gly Met Leu
               340                     345                     350
   tgt gct gga gta cct caa ggt gga gtg gac gca tgt cag ggt gac tct     1165
   Cys Ala Gly Val Pro Gln Gly Gly Val Asp Ala Cys Gln Gly Asp Ser
           355                     360                     365
   ggt ggc cca cta gta caa gaa gac tca cgg cgg ctt tgg ttt att gtg     1213
   Gly Gly Pro Leu Val Gln Glu Asp Ser Arg Arg Leu Trp Phe Ile Val
       370                     375                     380
   ggg ata gta agc tgg gga gat cag tgt ggc ctg ccg gat aag cca gga     1261
   Gly Ile Val Ser Trp Gly Asp Gln Cys Gly Leu Pro Asp Lys Pro Gly
   385                     390                     395                     400
   gtg tat act cga gtg aca gcc tac ctt gac tgg att agg caa caa act     1309
   Val Tyr Thr Arg Val Thr Ala Tyr Leu Asp Trp Ile Arg Gln Gln Thr
                   405                     410                     415
   ggg atc tag tgcaacaagt gcatccctgt tgcaaagtct gtatgcaggt             1358
   Gly Ile  *
   gtgcctgtct taaattccaa agctttacat ttcaactgaa aaagaaacta gaaatgtcct  1418
   aatttaacat cttgttacat aaatatggtt taacaaacac tgtttaacct ttctttatta  1478
   ttaaaggttt tctattttct cc                                            1500
<210> 66
   <211> 418
   <212> PRT
   <213> Homo Sapien
<400> 66
   Met Tyr Arg Pro Ala Arg Val Thr Ser Thr Ser Arg Phe Leu Asn Pro
   1               5                   10                  15
   Tyr Val Val Cys Phe Ile Val Val Ala Gly Val Val Ile Leu Ala Val
               20                  25                  30
   Thr Ile Ala Leu Leu Val Tyr Phe Leu Ala Phe Asp Gln Lys Ser Tyr
           35                  40                  45
   Phe Tyr Arg Ser Ser Phe Gln Leu Leu Asn Val Glu Tyr Asn Ser Gln
       50                  55                  60
   Leu Asn Ser Pro Ala Thr Gln Glu Tyr Arg Thr Leu Ser Gly Arg Ile
   65                  70                  75                  80
   Glu Ser Leu Ile Thr Lys Thr Phe Lys Glu Ser Asn Leu Arg Asn Gln
                   85                  90                  95
   Phe Ile Arg Ala His Val Ala Lys Leu Arg Gln Asp Gly Ser Gly Val
               100                 105                 110
   Arg Ala Asp Val Val Met Lys Phe Gln Phe Thr Arg Asn Asn Asn Gly
           115                 120                 125
   Ala Ser Met Lys Ser Arg Ile Glu Ser Val Leu Arg Gln Met Leu Asn
       130                 135                 140
   Asn Ser Gly Asn Leu Glu Ile Asn Pro Ser Thr Glu Ile Thr Ser Leu
   145                 150                 155                 160
   Thr Asp Gln Ala Ala Ala Asn Trp Leu Ile Asn Glu Cys Gly Ala Gly
                   165                 170                 175
   Pro Asp Leu Ile Thr Leu Ser Glu Gln Arg Ile Leu Gly Gly Thr Glu
               180                 185                 190
   Ala Glu Glu Gly Ser Trp Pro Trp Gln Val Ser Leu Arg Leu Asn Asn
           195                 200                 205
   Ala His His Cys Gly Gly Ser Leu Ile Asn Asn Met Trp Ile Leu Thr
       210                 215                 220
   Ala Ala His Cys Phe Arg Ser Asn Ser Asn Pro Arg Asp Trp Ile Ala
   225                 230                 235                 240
   Thr Ser Gly Ile Ser Thr Thr Phe Pro Lys Leu Arg Met Arg Val Arg
                   245                 250                 255
   Asn Ile Leu Ile His Asn Asn Tyr Lys Ser Ala Thr His Glu Asn Asp
               260                 265                 270
   Ile Ala Leu Val Arg Leu Glu Asn Ser Val Thr Phe Thr Lys Asp Ile
           275                 280                 285
   His Ser Val Cys Leu Pro Ala Ala Thr Gln Asn Ile Pro Pro Gly Ser
       290                 295                 300
   Thr Ala Tyr Val Thr Gly Trp Gly Ala Gln Glu Tyr Ala Gly His Thr
   305                 310                 315                 320
   Val Pro Glu Leu Arg Gln Gly Gln Val Arg Ile Ile Ser Asn Asp Val
                   325                 330                 335
   Cys Asn Ala Pro His Ser Tyr Asn Gly Ala Ile Leu Ser Gly Met Leu
               340                 345                 350
   Cys Ala Gly Val Pro Gln Gly Gly Val Asp Ala Cys Gln Gly Asp Ser
           355                 360                 365
   Gly Gly Pro Leu Val Gln Glu Asp Ser Arg Arg Leu Trp Phe Ile Val
       370                 375                 380
   Gly Ile Val Ser Trp Gly Asp Gln Cys Gly Leu Pro Asp Lys Pro Gly
   385                 390                 395                 400
   Val Tyr Thr Arg Val Thr Ala Tyr Leu Asp Trp Ile Arg Gln Gln Thr
                   405                 410                 415
   Gly Ile
<210> 67
   <211> 1783
   <212> DNA
   <213> Homo Sapien
<220>
   <221> CDS
   <222> (246)...(1499)
   <223> Nucleotide sequence encoding human hepsin
<300>
   <308> GenBank M18930
   <309> 1993-06-11
<400> 67
   tcgagcccgc tttccaggga ccctacctga gggcccacag gtgaggcagc ctggcctagc     60
   aggccccacg ccaccgcctc tgcctccagg ccgcccgctg ctgcggggcc accatgctcc   120
   tgcccaggcc tggagactga cccgaccccg gcactacctc gaggctccgc ccccacctgc   180
   tggaccccag ggtcccaccc tggcccagga ggtcagccag ggaatcatta acaagaggca   240
   gtgac atg gcg cag aag gag ggt ggc cgg act gtg cca tgc tgc tcc aga      290
         Met Ala Gln Lys Glu Gly Gly Arg Thr Val Pro Cys Cys Ser Arg
          1               5                  10                  15
   ccc aag gtg gca gct ctc act gcg ggg acc ctg cta ctt ctg aca gcc        338
   Pro Lys Val Ala Ala Leu Thr Ala Gly Thr Leu Leu Leu Leu Thr Ala
                   20                  25                  30
   atc ggg gcg gca tcc tgg gcc att gtg gct gtt ctc ctc agg agt gac        386
   Ile Gly Ala Ala Ser Trp Ala Ile Val Ala Val Leu Leu Arg Ser Asp
               35                  40                  45
   cag gag ccg ctg tac cca gtg cag gtc agc tct gcg gac gct cgg ctc        434
   Gln Glu Pro Leu Tyr Pro Val Gln Val Ser Ser Ala Asp Ala Arg Leu
           50                  55                  60
   atg gtc ttt gac aag acg gaa ggg acg tgg cgg ctg ctg tgc tcc tcg        482
   Met Val Phe Asp Lys Thr Glu Gly Thr Trp Arg Leu Leu Cys Ser Ser
       65                  70                  75
   cgc tcc aac gcc agg gta gcc gga ctc agc tgc gag gag atg ggc ttc        530
   Arg Ser Asn Ala Arg Val Ala Gly Leu Ser Cys Glu Glu Met Gly Phe
    80                  85                  90                  95
   ctc agg gca ctg acc cac tcc gag ctg gac gtg cga acg gcg ggc gcc        578
   Leu Arg Ala Leu Thr His Ser Glu Leu Asp Val Arg Thr Ala Gly Ala
                   100                 105                 110
   aat ggc acg tcg ggc ttc ttc tgt gtg gac gag ggg agg ctg ccc cac        626
   Asn Gly Thr Ser Gly Phe Phe Cys Val Asp Glu Gly Arg Leu Pro His
               115                 120                 125
   acc cag agg ctg ctg gag gtc atc tcc gtg tgt gat tgc ccc aga ggc        674
   Thr Gln Arg Leu Leu Glu Val Ile Ser Val Cys Asp Cys Pro Arg Gly
           130                 135                 140
   cgt ttc ttg gcc gcc atc tgc caa gac tgt ggc cgc agg aag ctg ccc        722
   Arg Phe Leu Ala Ala Ile Cys Gln Asp Cys Gly Arg Arg Lys Leu Pro
       145                 150                 155
   gtg gac cgc atc gtg gga ggc cgg gac acc agc ttg ggc cgg tgg ccg        770
   Val Asp Arg Ile Val Gly Gly Arg Asp Thr Ser Leu Gly Arg Trp Pro
   160                 165                 170                 175
   tgg caa gtc agc ctt cgc tat gat gga gca cac ctc tgt ggg gga tcc        818
   Trp Gln Val Ser Leu Arg Tyr Asp Gly Ala His Leu Cys Gly Gly Ser
                   180                 185                 190
   ctg ctc tcc ggg gac tgg gtg ctg aca gcc gcc cac tgc ttc ccg gag        866
   Leu Leu Ser Gly Asp Trp Val Leu Thr Ala Ala His Cys Phe Pro Glu
               195                 200                 205
   cgg aac cgg gtc ctg tcc cga tgg cga gtg ttt gcc ggt gcc gtg gcc        914
   Arg Asn Arg Val Leu Ser Arg Trp Arg Val Phe Ala Gly Ala Val Ala
           210                 215                 220
   cag gcc tct ccc cac ggt ctg cag ctg ggg gtg cag gct gtg gtc tac        962
   Gln Ala Ser Pro His Gly Leu Gln Leu Gly Val Gln Ala Val Val Tyr
       225                 230                 235
   cac ggg ggc tat ctt ccc ttt cgg gac ccc aac agc gag gag aac agc       1010
   His Gly Gly Tyr Leu Pro Phe Arg Asp Pro Asn Ser Glu Glu Asn Ser
   240                 245                 250                 255
   aac gat att gcc ctg gtc cac ctc tcc agt ccc ctg ccc ctc aca gaa      1058
   Asn Asp Ile Ala Leu Val His Leu Ser Ser Pro Leu Pro Leu Thr Glu
                   260                 265                 270
   tac atc cag cct gtg tgc ctc cca gct gcc ggc cag gcc ctg gtg gat      1106
   Tyr Ile Gln Pro Val Cys Leu Pro Ala Ala Gly Gln Ala Leu Val Asp
               275                 280                 285
   ggc aag atc tgt acc gtg acg ggc tgg ggc aac acg cag tac tat ggc      1154
   Gly Lys Ile Cys Thr Val Thr Gly Trp Gly Asn Thr Gln Tyr Tyr Gly
           290                 295                 300
   caa cag gcc ggg gta ctc cag gag gct cga gtc ccc ata atc agc aat      1202
   Gln Gln Ala Gly Val Leu Gln Glu Ala Arg Val Pro Ile Ile Ser Asn
       305                 310                 315
   gat gtc tgc aat ggc gct gac ttc tat gga aac cag atc aag ccc aag      1250
   Asp Val Cys Asn Gly Ala Asp Phe Tyr Gly Asn Gln Ile Lys Pro Lys
   320                 325                 330                 335
   atg ttc tgt gct ggc tac ccc gag ggt ggc att gat gcc tgc cag ggc      1298
   Met Phe Cys Ala Gly Tyr Pro Glu Gly Gly Ile Asp Ala Cys Gln Gly
                   340                 345                 350
   gac agc ggt ggt ccc ttt gtg tgt gag gac agc atc tct cgg acg cca      1346
   Asp Ser Gly Gly Pro Phe Val Cys Glu Asp Ser Ile Ser Arg Thr Pro
               355                 360                 365
   cgt tgg cgg ctg tgt ggc att gtg agt tgg ggc act ggc tgt gcc ctg      1394
   Arg Trp Arg Leu Cys Gly Ile Val Ser Trp Gly Thr Gly Cys Ala Leu
           370                 375                 380
   gcc cag aag cca ggc gtc tac acc aaa gtc agt gac ttc cgg gag tgg      1442
   Ala Gln Lys Pro Gly Val Tyr Thr Lys Val Ser Asp Phe Arg Glu Trp
       385                 390                 395
   atc ttc cag gcc ata aag act cac tcc gaa gcc agc ggc atg gtg acc      1490
   Ile Phe Gln Ala Ile Lys Thr His Ser Glu Ala Ser Gly Met Val Thr
   400                 405                 410                 415
   cag ctc tga ccggtggctt ctcgctgcgc agcctccagg gcccgaggtg              1539
   Gln Leu  *
   atcccggtgg tgggatccac gctgggccga ggatgggacg tttttcttct tgggcccggt    1599
   ccacaggtcc aaggacaccc tccctccagg gtcctctctt ccacagtggc gggcccactc    1659
   agccccgaga ccacccaacc tcaccctcct gacccccatg taaatattgt tctgctgtct    1719
   gggactcctg tctaggtgcc cctgatgatg ggatgctctt taaataataa agatggtttt    1779
   gatt                                                                 1783
<210> 68
   <211> 417
   <212> PRT
   <213> Homo Sapien
<400> 68
   Met Ala Gln Lys Glu Gly Gly Arg Thr Val Pro Cys Cys Ser Arg Pro
    1              5                   10                  15
   Lys Val Ala Ala Leu Thr Ala Gly Thr Leu Leu Leu Leu Thr Ala Ile
               20                  25                  30
   Gly Ala Ala Ser Trp Ala Ile Val Ala Val Leu Leu Arg Ser Asp Gln
           35                  40                  45
   Glu Pro Leu Tyr Pro Val Gln Val Ser Ser Ala Asp Ala Arg Leu Met
       50                  55                  60
   Val Phe Asp Lys Thr Glu Gly Thr Trp Arg Leu Leu Cys Ser Ser Arg
   65                  70                  75                  80
   Ser Asn Ala Arg Val Ala Gly Leu Ser Cys Glu Glu Met Gly Phe Leu
                   85                  90                  95
   Arg Ala Leu Thr His Ser Glu Leu Asp Val Arg Thr Ala Gly Ala Asn
               100                 105                 110
   Gly Thr Ser Gly Phe Phe Cys Val Asp Glu Gly Arg Leu Pro His Thr
           115                 120                 125
   Gln Arg Leu Leu Glu Val Ile Ser Val Cys Asp Cys Pro Arg Gly Arg
       130                 135                 140
   Phe Leu Ala Ala Ile Cys Gln Asp Cys Gly Arg Arg Lys Leu Pro Val
   145                 150                 155                 160
   Asp Arg Ile Val Gly Gly Arg Asp Thr Ser Leu Gly Arg Trp Pro Trp
                   165                 170                 175
   Gln Val Ser Leu Arg Tyr Asp Gly Ala His Leu Cys Gly Gly Ser Leu
               180                 185                 190
   Leu Ser Gly Asp Trp Val Leu Thr Ala Ala His Cys Phe Pro Glu Arg
           195                 200                 205
   Asn Arg Val Leu Ser Arg Trp Arg Val Phe Ala Gly Ala Val Ala Gln
       210                 215                 220
   Ala Ser Pro His Gly Leu Gln Leu Gly Val Gln Ala Val Val Tyr His
   225                 230                 235                 240
   Gly Gly Tyr Leu Pro Phe Arg Asp Pro Asn Ser Glu Glu Asn Ser Asn
                   245                 250                 255
   Asp Ile Ala Leu Val His Leu Ser Ser Pro Leu Pro Leu Thr Glu Tyr
               260                 265                 270
   Ile Gln Pro Val Cys Leu Pro Ala Ala Gly Gln Ala Leu Val Asp Gly
           275                 280                 285
   Lys Ile Cys Thr Val Thr Gly Trp Gly Asn Thr Gln Tyr Tyr Gly Gln
       290                 295                 300
   Gln Ala Gly Val Leu Gln Glu Ala Arg Val Pro Ile Ile Ser Asn Asp
   305                 310                 315                 320
   Val Cys Asn Gly Ala Asp Phe Tyr Gly Asn Gln Ile Lys Pro Lys Met
                   325                 330                 335
   Phe Cys Ala Gly Tyr Pro Glu Gly Gly Ile Asp Ala Cys Gln Gly Asp
               340                 345                 350
   Ser Gly Gly Pro Phe Val Cys Glu Asp Ser Ile Ser Arg Thr Pro Arg
           355                 360                 365
   Trp Arg Leu Cys Gly Ile Val Ser Trp Gly Thr Gly Cys Ala Leu Ala
       370                 375                 380
   Gln Lys Pro Gly Val Tyr Thr Lys Val Ser Asp Phe Arg Glu Trp Ile
   385                 390                 395                 400
   Phe Gln Ala Ile Lys Thr His Ser Glu Ala Ser Gly Met Val Thr Gln
                   405                 410                 415
   Leu
<210> 69
   <211> 2479
   <212> DNA
   <213> Homo sapien
<220>
   <221> CDS
   <222> (57)...(1535)
   <223> Nucleotide sequence encoding human serine protease
<300>
   <308> GenBank U75329
   <309> 1997-10-10
<400> 69
   gtcatattga acattccaga tacctatcat tactcgatgc tgttgataac agcaag atg     59
                                                                  Met
                                                                    1
   gct ttg aac tca ggg tca cca cca gct att gga cct tac tat gaa aac      107
   Ala Leu Asn Ser Gly Ser Pro Pro Ala Ile Gly Pro Tyr Tyr Glu Asn
                   5                   10                  15
   cat gga tac caa ccg gaa aac ccc tat ccc gca cag ccc act gtg gtc      155
   His Gly Tyr Gln Pro Glu Asn Pro Tyr Pro Ala Gln Pro Thr Val Val
               20                  25                  30
   ccc act gtc tac gag gtg cat ccg gct cag tac tac ccg tcc ccc gtg      203
   Pro Thr Val Tyr Glu Val His Pro Ala Gln Tyr Tyr Pro Ser Pro Val
           35                  40                  45
   ccc cag tac gcc ccg agg gtc ctg acg cag gct tcc aac ccc gtc gtc      251
   Pro Gln Tyr Ala Pro Arg Val Leu Thr Gln Ala Ser Asn Pro Val Val
       50                  55                  60                  65
   tgc acg cag ccc aaa tcc cca tcc ggg aca gtg tgc acc tca aag act      299
   Cys Thr Gln Pro Lys Ser Pro Ser Gly Thr Val Cys Thr Ser Lys Thr
                   70                  75                  80
   aag aaa gca ctg tgc atc acc ttg acc ctg ggg acc ttc ctc gtg gga      347
   Lys Lys Ala Leu Cys Ile Thr Leu Thr Leu Gly Thr Phe Leu Val Gly
               85                  90                  95
   gct gcg ctg gcc gct ggc cta ctc tgg aag ttc atg ggc agc aag tgc      395
   Ala Ala Leu Ala Ala Gly Leu Leu Trp Lys Phe Met Gly Ser Lys Cys
           100                 105                 110
   tcc aac tct ggg ata gag tgc gac tcc tca ggt acc tgc atc aac ccc      443
   Ser Asn Ser Gly Ile Glu Cys Asp Ser Ser Gly Thr Cys Ile Asn Pro
       115                 120                 125
   tct aac tgg tgt gat ggc gtg tca cac tgc ccc ggc ggg gag gac gag      491
   Ser Asn Trp Cys Asp Gly Val Ser His Cys Pro Gly Gly Glu Asp Glu
   130                 135                 140                 145
   aat cgg tgt gtt cgc ctc tac gga cca aac ttc atc ctt cag atg tac      539
   Asn Arg Cys Val Arg Leu Tyr Gly Pro Asn Phe Ile Leu Gln Met Tyr
                   150                 155                 160
   tca tct cag agg aag tcc tgg cac cct gtg tgc caa gac gac tgg aac      587
   Ser Ser Gln Arg Lys Ser Trp His Pro Val Cys Gln Asp Asp Trp Asn
               165                 170                 175
   gag aac tac ggg cgg gcg gcc tgc agg gac atg ggc tat aag aat aat      635
   Glu Asn Tyr Gly Arg Ala Ala Cys Arg Asp Met Gly Tyr Lys Asn Asn
           180                 185                 190
   ttt tac tct agc caa gga ata gtg gat gac agc gga tcc acc agc ttt      683
   Phe Tyr Ser Ser Gln Gly Ile Val Asp Asp Ser Gly Ser Thr Ser Phe
       195                 200                 205
   atg aaa ctg aac aca agt gcc ggc aat gtc gat atc tat aaa aaa ctg      731
   Met Lys Leu Asn Thr Ser Ala Gly Asn Val Asp Ile Tyr Lys Lys Leu
   210                 215                 220                 225
   tac cac agt gat gcc tgt tct tca aaa gca gtg gtt tct tta cgc tgt      779
   Tyr His Ser Asp Ala Cys Ser Ser Lys Ala Val Val Ser Leu Arg Cys
                   230                 235                 240
   tta gcc tgc ggg gtc aac ttg aac tca agc cgc cag agc agg atc gtg      827
   Leu Ala Cys Gly Val Asn Leu Asn Ser Ser Arg Gln Ser Arg Ile Val
               245                 250                 255
   ggc ggt gag agc gcg ctc ccg ggg gcc tgg ccc tgg cag gtc agc ctg      875
   Gly Gly Glu Ser Ala Leu Pro Gly Ala Trp Pro Trp Gln Val Ser Leu
           260                 265                 270
   cac gtc cag aac gtc cac gtg tgc gga ggc tcc atc atc acc ccc gag      923
   His Val Gln Asn Val His Val Cys Gly Gly Ser Ile Ile Thr Pro Glu
       275                 280                 285
   tgg atc gtg aca gcc gcc cac tgc gtg gaa aaa cct ctt aac aat cca      971
   Trp Ile Val Thr Ala Ala His Cys Val Glu Lys Pro Leu Asn Asn Pro
   290                 295                 300                 305
   tgg cat tgg acg gca ttt gcg ggg att ttg aga caa tct ttc atg ttc     1019
   Trp His Trp Thr Ala Phe Ala Gly Ile Leu Arg Gln Ser Phe Met Phe
                   310                 315                 320
   tat gga gcc gga tac caa gta caa aaa gtg att tct cat cca aat tat     1067
   Tyr Gly Ala Gly Tyr Gln Val Gln Lys Val Ile Ser His Pro Asn Tyr
               325                 330                 335
   gac tcc aag acc aag aac aat gac att gcg ctg atg aag ctg cag aag     1115
   Asp Ser Lys Thr Lys Asn Asn Asp Ile Ala Leu Met Lys Leu Gln Lys
           340                 345                 350
   cct ctg act ttc aac gac cta gtg aaa cca gtg tgt ctg ccc aac cca     1163
   Pro Leu Thr Phe Asn Asp Leu Val Lys Pro Val Cys Leu Pro Asn Pro
       355                 360                 365
   ggc atg atg ctg cag cca gaa cag ctc tgc tgg att tcc ggg tgg ggg     1211
   Gly Met Met Leu Gln Pro Glu Gln Leu Cys Trp Ile Ser Gly Trp Gly
   370                 375                 380                 385
   gcc acc gag gag aaa ggg aag acc tca gaa gtg ctg aac gct gcc aag     1259
   Ala Thr Glu Glu Lys Gly Lys Thr Ser Glu Val Leu Asn Ala Ala Lys
                   390                 395                 400
   gtg ctt ctc att gag aca cag aga tgc aac agc aga tat gtc tat gac     1307
   Val Leu Leu Ile Glu Thr Gln Arg Cys Asn Ser Arg Tyr Val Tyr Asp
               405                 410                 415
   aac ctg atc aca cca gcc atg atc tgt gcc ggc ttc ctg cag ggg aac     1355
   Asn Leu Ile Thr Pro Ala Met Ile Cys Ala Gly Phe Leu Gln Gly Asn
           420                 425                 430
   gtc gat tct tgc cag ggt gac agt gga ggg cct ctg gtc act tcg aac     1403
   Val Asp Ser Cys Gln Gly Asp Ser Gly Gly Pro Leu Val Thr Ser Asn
       435                 440                 445
   aac aat atc tgg tgg ctg ata ggg gat aca agc tgg ggt tct ggc tgt     1451
   Asn Asn Ile Trp Trp Leu Ile Gly Asp Thr Ser Trp Gly Ser Gly Cys
   450                 455                 460                 465
   gcc aaa gct tac aga cca gga gtg tac ggg aat gtg atg gta ttc acg      1499
   Ala Lys Ala Tyr Arg Pro Gly Val Tyr Gly Asn Val Met Val Phe Thr
                   470                 475                 480
   gac tgg att tat cga caa atg aag gca aac ggc taa tccacatggt           1545
   Asp Trp Ile Tyr Arg Gln Met Lys Ala Asn Gly  *
               485                 490
   cttcgtcctt gacgtcgttt tacaagaaaa caatggggct ggttttgctt ccccgtgcat    1605
   gatttactct tagagatgat tcagaggtca cttcattttt attaaacagt gaacttgtct    1665
   ggctttggca ctctctgcca tactgtgcag gctgcagtgg ctcccctgcc cagcctgctc    1725
   tccctaaccc cttgtccgca aggggtgatg gccggctggt tgtgggcact ggcggtcaat    1785
   tgtggaagga agagggttgg aggctgcccc cattgagatc ttcctgctga gtcctttcca    1845
   ggggccaatt ttggatgagc atggagctgt cacttctcag ctgctggatg acttgagatg    1905
   aaaaaggaga gacatggaaa gggagacagc caggtggcac ctgcagcggc tgccctctgg    1965
   ggccacttgg tagtgtcccc agcctacttc acaaggggat tttgctgatg ggttcttaga    2025
   gccttagcag ccctggatgg tggccagaaa taaagggacc agcccttcat gggtggtgac    2085
   gtggtagtca cttgtaaggg gaacagaaac atttttgttc ttatggggtg agaatataga     2145
   cagtgccctt ggtgcgaggg aagcaattga aaaggaactt gccctgagca ctcctggtgc    2205
   aggtctccac ctgcacattg ggtggggctc ctgggaggga gactcagcct tcctcctcat    2265
   cctccctgac cctgctccta gcaccctgga gagtgaatgc cccttggtcc ctggcagggc    2325
   gccaagtttg gcaccatgtc ggcctcttca ggcctgatag tcattggaaa ttgaggtcca    2385
   tgggggaaat caaggatgct cagtttaagg tacactgttt ccatgttatg tttctacaca    2445
   ttgatggtgg tgaccctgag ttcaaagcca tctt                                2479
<210> 70
   <211> 492
   <212> PRT
   <213> Homo sapien
<400> 70
   Met Ala Leu Asn Ser Gly Ser Pro Pro Ala Ile Gly Pro Tyr Tyr Glu
    1               5                  10                  15
   Asn His Gly Tyr Gln Pro Glu Asn Pro Tyr Pro Ala Gln Pro Thr Val
               20                  25                  30
   Val Pro Thr Val Tyr Glu Val His Pro Ala Gln Tyr Tyr Pro Ser Pro
           35                  40                  45
   Val Pro Gln Tyr Ala Pro Arg Val Leu Thr Gln Ala Ser Asn Pro Val
       50                  55                  60
   Val Cys Thr Gln Pro Lys Ser Pro Ser Gly Thr Val Cys Thr Ser Lys
   65                  70                  75                  80
   Thr Lys Lys Ala Leu Cys Ile Thr Leu Thr Leu Gly Thr Phe Leu Val
                   85                  90                  95
   Gly Ala Ala Leu Ala Ala Gly Leu Leu Trp Lys Phe Met Gly Ser Lys
               100                 105                 110
   Cys Ser Asn Ser Gly Ile Glu Cys Asp Ser Ser Gly Thr Cys Ile Asn
           115                 120                 125
   Pro Ser Asn Trp Cys Asp Gly Val Ser His Cys Pro Gly Gly Glu Asp
       130                 135                 140
   Glu Asn Arg Cys Val Arg Leu Tyr Gly Pro Asn Phe Ile Leu Gln Met
   145                 150                 155                 160
   Tyr Ser Ser Gln Arg Lys Ser Trp His Pro Val Cys Gln Asp Asp Trp
                   165                 170                 175
   Asn Glu Asn Tyr Gly Arg Ala Ala Cys Arg Asp Met Gly Tyr Lys Asn
               180                 185                 190
   Asn Phe Tyr Ser Ser Gln Gly Ile Val Asp Asp Ser Gly Ser Thr Ser
           195                 200                 205
   Phe Met Lys Leu Asn Thr Ser Ala Gly Asn Val Asp Ile Tyr Lys Lys
       210                 215                 220
   Leu Tyr His Ser Asp Ala Cys Ser Ser Lys Ala Val Val Ser Leu Arg
   225                 230                 235                 240
   Cys Leu Ala Cys Gly Val Asn Leu Asn Ser Ser Arg Gln Ser Arg Ile
                   245                 250                 255
   Val Gly Gly Glu Ser Ala Leu Pro Gly Ala Trp Pro Trp Gln Val Ser
               260                 265                 270
   Leu His Val Gln Asn Val His Val Cys Gly Gly Ser Ile Ile Thr Pro
           275                 280                 285
   Glu Trp Ile Val Thr Ala Ala His Cys Val Glu Lys Pro Leu Asn Asn
       290                 295                 300
   Pro Trp His Trp Thr Ala Phe Ala Gly Ile Leu Arg Gln Ser Phe Met
   305                 310                 315                 320
   Phe Tyr Gly Ala Gly Tyr Gln Val Gln Lys Val Ile Ser His Pro Asn
                   325                 330                 335
   Tyr Asp Ser Lys Thr Lys Asn Asn Asp Ile Ala Leu Met Lys Leu Gln
               340                 345                 350
   Lys Pro Leu Thr Phe Asn Asp Leu Val Lys Pro Val Cys Leu Pro Asn
           355                 360                 365
   Pro Gly Met Met Leu Gln Pro Glu Gln Leu Cys Trp Ile Ser Gly Trp
       370                 375                 380
   Gly Ala Thr Glu Glu Lys Gly Lys Thr Ser Glu Val Leu Asn Ala Ala
   385                 390                 395                 400
   Lys Val Leu Leu Ile Glu Thr Gln Arg Cys Asn Ser Arg Tyr Val Tyr
                   405                 410                 415
   Asp Asn Leu Ile Thr Pro Ala Met Ile Cys Ala Gly Phe Leu Gln Gly
               420                 425                 430
   Asn Val Asp Ser Cys Gln Gly Asp Ser Gly Gly Pro Leu Val Thr Ser
           435                 440                 445
   Asn Asn Asn Ile Trp Trp Leu Ile Gly Asp Thr Ser Trp Gly Ser Gly
       450                 455                 460
   Cys Ala Lys Ala Tyr Arg Pro Gly Val Tyr Gly Asn Val Met Val Phe
   465                 470                 475                 480
   Thr Asp Trp Ile Tyr Arg Gln Met Lys Ala Asn Gly
                   485                 490
<210> 71
   <211> 2079
   <212> DNA
   <213> Homo sapien
<220>
   <221> CDS
   <222> (251)...(1522)
   <223> Nucleotide sequence encoding transmembrane protease, serine 4 (TMPRSS4)
<300>
   <308> GenBank NM016425
   <309> 2000-11-06
<400> 71
   gagaggcagc agcttgttca gcggacaagg atgctgggcg tgagggacca aggcctgccc     60
   tgcactcggg cctcctccag ccagtgctga ccagggactt ctgacctgct ggccagccag    120
   gacctgtgtg gggaggccct cctgctgcct tggggtgaca atctcagctc caggctacag    180
   ggagaccggg aggatcacag agccagcatg gtacaggatc ctgacagtga tcaacctctg    240
   aacagcctcg atg tca aac ccc tgc gca aac ccc gta tcc cca tgg aga       289
              Met Ser Asn Pro Cys Ala Asn Pro Val Ser Pro Trp Arg
               1               5                  10
   cct tca gaa agt gtg ggg atc ccc atc atc ata gca cta ctg agc ctg      337
   Pro Ser Glu Ser Val Gly Ile Pro Ile Ile Ile Ala Leu Leu Ser Leu
       15                  20                  25
   gcg agt atc atc att gtg gtt gtc ctc atc aag gtg att ctg gat aaa      385
   Ala Ser Ile Ile Ile Val Val Val Leu Ile Lys Val Ile Leu Asp Lys
    30                  35                  40                  45
   tac tac ttc ctc tgc ggg cag cct ctc cac ttc atc ccg agg aag cag      433
   Tyr Tyr Phe Leu Cys Gly Gln Pro Leu His Phe Ile Pro Arg Lys Gln
                    50                  55                  60
   ctg tgt gac gga gag ctg gac tgt ccc ttg ggg gag gac gag gag cac      481
   Leu Cys Asp Gly Glu Leu Asp Cys Pro Leu Gly Glu Asp Glu Glu His
                65                  70                  75
   tgt gtc aag agc ttc ccc gaa ggg cct gca gtg gca gtc cgc ctc tcc      529
   Cys Val Lys Ser Phe Pro Glu Gly Pro Ala Val Ala Val Arg Leu Ser
            80                  85                  90
   aag gac cga tcc aca ctg cag gtg ctg gac tcg gcc aca ggg aac tgg      577
   Lys Asp Arg Ser Thr Leu Gln Val Leu Asp Ser Ala Thr Gly Asn Trp
        95                 100                 105
   ttc tct gcc tgt ttc gac aac ttc aca gaa gct ctc gct gag aca gcc      625
   Phe Ser Ala Cys Phe Asp Asn Phe Thr Glu Ala Leu Ala Glu Thr Ala
   110                 115                 120                 125
   tgt agg cag atg ggc tac agc agc aaa ccc act ttc aga gct gtg gag      673
   Cys Arg Gln Met Gly Tyr Ser Ser Lys Pro Thr Phe Arg Ala Val Glu
                   130                 135                 140
   att ggc cca gac cag gat ctg gat gtt gtt gaa atc aca gaa aac agc      721
   Ile Gly Pro Asp Gln Asp Leu Asp Val Val Glu Ile Thr Glu Asn Ser
               145                 150                 155
   cag gag ctt cgc atg cgg aac tca agt ggg ccc tgt ctc tca ggc tcc      769
   Gln Glu Leu Arg Met Arg Asn Ser Ser Gly Pro Cys Leu Ser Gly Ser
           160                 165                 170
   ctg gtc tcc ctg cac tgt ctt gcc tgt ggg aag agc ctg aag acc ccc      817
   Leu Val Ser Leu His Cys Leu Ala Cys Gly Lys Ser Leu Lys Thr Pro
       175                 180                 185
   cgt gtg gtg ggt ggg gag gag gcc tct gtg gat tct tgg cct tgg cag      865
   Arg Val Val Gly Gly Glu Glu Ala Ser Val Asp Ser Trp Pro Trp Gln
   190                 195                 200                 205
   gtc agc atc cag tac gac aaa cag cac gtc tgt gga ggg agc atc ctg      913
   Val Ser Ile Gln Tyr Asp Lys Gln His Val Cys Gly Gly Ser Ile Leu
                   210                 215                 220
   gac ccc cac tgg gtc ctc acg gca gcc cac tgc ttc agg aaa cat acc      961
   Asp Pro His Trp Val Leu Thr Ala Ala His Cys Phe Arg Lys His Thr
               225                 230                 235
   gat gtg ttc aac tgg aag gtg cgg gca ggc tca gac aaa ctg ggc agc     1009
   Asp Val Phe Asn Trp Lys Val Arg Ala Gly Ser Asp Lys Leu Gly Ser
           240                 245                 250
   ttc cca tcc ctg gct gtg gcc aag atc atc atc att gaa ttc aac ccc     1057
   Phe Pro Ser Leu Ala Val Ala Lys Ile Ile Ile Ile Glu Phe Asn Pro
       255                 260                 265
   atg tac ccc aaa gac aat gac atc gcc ctc atg aag ctg cag ttc cca     1105
   Met Tyr Pro Lys Asp Asn Asp Ile Ala Leu Met Lys Leu Gln Phe Pro
   270                 275                 280                 285
   ctc act ttc tca ggc aca gtc agg ccc atc tgt ctg ccc ttc ttt gat      1153
   Leu Thr Phe Ser Gly Thr Val Arg Pro Ile Cys Leu Pro Phe Phe Asp
                   290                 295                 300
   gag gag ctc act cca gcc acc cca ctc tgg atc att gga tgg ggc ttt      1201
   Glu Glu Leu Thr Pro Ala Thr Pro Leu Trp Ile Ile Gly Trp Gly Phe
               305                 310                 315
   acg aag cag aat gga ggg aag atg tct gac ata ctg ctg cag gcg tca      1249
   Thr Lys Gln Asn Gly Gly Lys Met Ser Asp Ile Leu Leu Gln Ala Ser
           320                 325                 330
   gtc cag gtc att gac agc aca cgg tgc aat gca gac gat gcg tac cag      1297
   Val Gln Val Ile Asp Ser Thr Arg Cys Asn Ala Asp Asp Ala Tyr Gln
       335                 340                 345
   ggg gaa gtc acc gag aag atg atg tgt gca ggc atc ccg gaa ggg ggt      1345
   Gly Glu Val Thr Glu Lys Met Met Cys Ala Gly Ile Pro Glu Gly Gly
   350                 355                 360                 365
   gtg gac acc tgc cag ggt gac agt ggt ggg ccc ctg atg tac caa tct      1393
   Val Asp Thr Cys Gln Gly Asp Ser Gly Gly Pro Leu Met Tyr Gln Ser
                   370                 375                 380
   gac cag tgg cat gtg gtg ggc atc gtt agc tgg ggc tat ggc tgc ggg      1441
   Asp Gln Trp His Val Val Gly Ile Val Ser Trp Gly Tyr Gly Cys Gly
               385                 390                 395
   ggc ccg agc acc cca gga gta tac acc aag gtc tca gcc tat ctc aac      1489
   Gly Pro Ser Thr Pro Gly Val Tyr Thr Lys Val Ser Ala Tyr Leu Asn
           400                 405                 410
   tgg atc tac aat gtc tgg aag gct gag ctg taa tgctgctgcc cctttgcagt    1542
   Trp Ile Tyr Asn Val Trp Lys Ala Glu Leu  *
       415                 420
   gctgggagcc gcttccttcc tgccctgccc acctggggat cccccaaagt cagacacaga    1602
   gcaagagtcc ccttgggtac acccctctgc ccacagcctc agcatttctt ggagcagcaa    1662
   agggcctcaa ttcctgtaag agaccctcgc agcccagagg cgcccagagg aagtcagcag    1722
   ccctagctcg gccacacttg gtgctcccag catcccaggg agagacacag cccactgaac    1782
   aaggtctcag gggtattgct aagccaagaa ggaactttcc cacactactg aatggaagca     1842
   ggctgtcttg taaaagccca gatcactgtg ggctggagag gagaaggaaa gggtctgcgc    1902
   cagccctgtc cgtcttcacc catccccaag cctactagag caagaaacca gttgtaatat    1962
   aaaatgcact gccctactgt tggtatgact accgttacct actgttgtca ttgttattac    2022
   agctatggcc actattatta aagagctgtg taacatcaaa aaaaaaaaaa aaaaaaa       2079
<210> 72
   <211> 423
   <212> PRT
   <213> Homo sapien
<400> 72
   Met Ser Asn Pro Cys Ala Asn Pro Val Ser Pro Trp Arg Pro Ser Glu
    1               5                  10                  15
   Ser Val Gly Ile Pro Ile Ile Ile Ala Leu Leu Ser Leu Ala Ser Ile
               20                  25                  30
   Ile Ile Val Val Val Leu Ile Lys Val Ile Leu Asp Lys Tyr Tyr Phe
           35                  40                  45
   Leu Cys Gly Gln Pro Leu His Phe Ile Pro Arg Lys Gln Leu Cys Asp
       50                  55                  60
   Gly Glu Leu Asp Cys Pro Leu Gly Glu Asp Glu Glu His Cys Val Lys
   65                  70                  75                  80
   Ser Phe Pro Glu Gly Pro Ala Val Ala Val Arg Leu Ser Lys Asp Arg
                   85                  90                  95
   Ser Thr Leu Gln Val Leu Asp Ser Ala Thr Gly Asn Trp Phe Ser Ala
               100                 105                 110
   Cys Phe Asp Asn Phe Thr Glu Ala Leu Ala Glu Thr Ala Cys Arg Gln
           115                 120                 125
   Met Gly Tyr Ser Ser Lys Pro Thr Phe Arg Ala Val Glu Ile Gly Pro
       130                 135                 140
   Asp Gln Asp Leu Asp Val Val Glu Ile Thr Glu Asn Ser Gln Glu Leu
   145                 150                 155                 160
   Arg Met Arg Asn Ser Ser Gly Pro Cys Leu Ser Gly Ser Leu Val Ser
                   165                 170                 175
   Leu His Cys Leu Ala Cys Gly Lys Ser Leu Lys Thr Pro Arg Val Val
               180                 185                 190
   Gly Gly Glu Glu Ala Ser Val Asp Ser Trp Pro Trp Gln Val Ser Ile
           195                 200                 205
   Gln Tyr Asp Lys Gln His Val Cys Gly Gly Ser Ile Leu Asp Pro His
       210                 215                 220
   Trp Val Leu Thr Ala Ala His Cys Phe Arg Lys His Thr Asp Val Phe
   225                 230                 235                 240
   Asn Trp Lys Val Arg Ala Gly Ser Asp Lys Leu Gly Ser Phe Pro Ser
                   245                 250                 255
   Leu Ala Val Ala Lys Ile Ile Ile Ile Glu Phe Asn Pro Met Tyr Pro
               260                 265                 270
   Lys Asp Asn Asp Ile Ala Leu Met Lys Leu Gln Phe Pro Leu Thr Phe
           275                 280                 285
   Ser Gly Thr Val Arg Pro Ile Cys Leu Pro Phe Phe Asp Glu Glu Leu
       290                 295                 300
   Thr Pro Ala Thr Pro Leu Trp Ile Ile Gly Trp Gly Phe Thr Lys Gln
   305                 310                 315                 320
   Asn Gly Gly Lys Met Ser Asp Ile Leu Leu Gln Ala Ser Val Gln Val
                   325                 330                 335
   Ile Asp Ser Thr Arg Cys Asn Ala Asp Asp Ala Tyr Gln Gly Glu Val
               340                 345                 350
   Thr Glu Lys Met Met Cys Ala Gly Ile Pro Glu Gly Gly Val Asp Thr
           355                 360                 365
   Cys Gln Gly Asp Ser Gly Gly Pro Leu Met Tyr Gln Ser Asp Gln Trp
       370                 375                 380
   His Val Val Gly Ile Val Ser Trp Gly Tyr Gly Cys Gly Gly Pro Ser
   385                 390                 395                 400
   Thr Pro Gly Val Tyr Thr Lys Val Ser Ala Tyr Leu Asn Trp Ile Tyr
                   405                 410                 415
   Asn Val Trp Lys Ala Glu Leu
               420

## Description

### RELATED APPLICATIONS

Benefit of priority is claimed to U.S. provisional application Serial No. 60/179,982, to Edwin L. Madison and Edgar O. Ong, filed February 3, 2000, entitled "NUCLEOTIDE AND PROTEIN SEQUENCES OF A TRANSMEMBRANE SERINE PROTEASE AND METHODS BASED THEREOF"; to U.S. provisional application Serial No. 60/183,542, to Edwin L. Madison and Edgar O. Ong, filed February 18, 2000, entitled "NUCLEOTIDE AND PROTEIN SEQUENCES OF A TRANSMEMBRANE SERINE PROTEASE AND METHODS BASED THEREOF"; to U.S. provisional application Serial No. 60/213,124, to Edwin L. Madison and Edgar O. Ong, filed June 22, 2000, entitled "NUCLEOTIDE AND PROTEIN SEQUENCES OF A TRANSMEMBRANE SERINE PROTEASE AND METHODS BASED THEREOF"; to U.S. provisional application Serial No. 60/220,970, to Edwin L. Madison and Edgar O. Ong, filed July 26, 2000, entitled "NUCLEOTIDE AND PROTEIN SEQUENCES OF A TRANSMEMBRANE SERINE PROTEASE AND METHODS BASED THEREOF"; and to U.S. provisional application Serial No. 60/234,840 to Edwin L. Madison, Edgar O. Ong and Jiunn-Chern Yeh, filed September 22, 2000, entitled "NUCLEIC ACID MOLECULES ENCODING TRANSMEMBRANE SERINE PROTEASES, THE ENCODED PROTEINS AND METHODS BASED THEREON" is claimed herein. Benefit of priority is also claimed to U.S. application Serial No. 09/657,968, to Edwin L. Madison, Joseph Edward Semple, Gary Samuel Coombs, John Eugene Reiner, Edgar O. Ong, Gian Luca Araldi, filed September 8, 2000, entitled "INHIBITORS OF SERINE PROTEASE ACTIVITY OF MATRIPTASE OR MTSP1 ". This application is a continuation-in-part of U.S. application Serial No. 09/657,986.

This application is related to U.S. provisional application Serial No. 60/166,391 to Edwin L. Madison and Edgar O. Ong, filed November 18, 1999 entitled "NUCLEOTIDE AND PROTEIN SEQUENCES OF PROTEASE DOMAINS OF ENDOTHELIASE AND METHODS BASED THEREON". This a application is also related to International PCT application No. PCT/USOO/31803, filed November 17, 2000.

### FIELD OF INVENTION

Nucleic acid molecules that encode proteases and portions thereof, particularly protease domains are provided. Also provided are prognostic, diagnostic and therapeutic methods using the proteases and domains thereof and the encoding nucleic acid molecules.

### BACKGROUND OF THE INVENTION AND OBJECTS THEREOF

Cancer a leading cause of death in the United States, developing in one in three Americans; one of every four Americans dies of cancer. Cancer is characterized by an increase in the number of abnormal neoplastic cells, which proliferate to form a tumor mass, the invasion of adjacent tissues by these neoplastic tumor cells, and the generation of malignant cells that metastasize via the blood or lymphatic system to regional lymph nodes and to distant sites.

Among the hallmarks of cancer is a breakdown in the communication among tumor cells and their environment. Normal cells do not divide in the absence of stimulatory signals, cease dividing in the presence of inhibitory signals. Growth-stimulatory and growth-inhibitory signals, are routinely exchanged between cells within a tissue. In a cancerous, or neoplastic, state, a cell acquires the ability to "override" these signals and to proliferate under conditions in which normal cells do not grow.

In order to proliferate tumor cells acquire a number of distinct aberrant traits reflecting genetic alterations. The genomes of certain well-studied tumors carry several different independently altered genes, including activated oncogenes and inactivated tumor suppressor genes. Each of these genetic changes appears to be responsible for imparting some of the traits that, in the aggregate, represent the full neoplastic phenotype.

A variety of biochemical factors have been associated with different phases of metastasis. Cell surface receptors for collagen, glycoproteins such as laminin, and proteoglycans, facilitate tumor cell attachment, an important step in invasion and metastases. Attachment triggers the release of degradative enzymes which facilitate the penetration of tumor cells through tissue barriers. Once the tumor cells have entered the target tissue, specific growth factors are required for further proliferation. Tumor invasion (or progression) involves a complex series of events, in which tumor cells detach from the primary tumor, break down the normal tissue surrounding it, and migrate into a blood or lymphatic vessel to be carried to a distant site. The breaking down of normal tissue barriers is accomplished by the elaboration of specific enzymes that degrade the proteins of the extracellular matrix that make up basement membranes and stromal components of tissues.

A class of extracellular matrix degrading enzymes have been implicated in tumor invasion. Among these are the matrix metalloproteinases (MMP). For example, the production of the matrix metalloproteinase stromelysin is associated with malignant tumors with metastatic potential (see, *e.g*., McDonnell et al. (1990) Smnrs. in Cancer Biology 1:107-115; McDonnell et al. (1990) Cancer and Metastasis Reviews 9:309-319).

The capacity of cancer cells to metastasize and invade tissue is facilitated by degradation of the basement membrane. Several proteinase enzymes, including the MMPs, have been reported to facilitate the process of invasion of tumor cells. MMPs are reported to enhance degradation of the basement membrane, which thereby permits tumorous cells to invade tissues. For example, two major metalloproteinases having molecular weights of about 70 kDa and 92 kDa appear to enhance ability of tumor cells to metastasize.

### Type II Transmembrane Serine Proteases (TTSPs)

In addition to the MMPs, serine proteases have been implicated in neoplastic disease progression. Most serine proteases, which are either secreted enzymes or are sequestered in cytoplasmic storage organelles, have roles in blood coagulation, wound healing, digestion, immune responses and tumor invasion and metastasis. A class cell surface proteins designated type II transmembrane serine proteases, which are membrane-anchored proteins with N-terminal extracellular domains, has been identified. As cell surface proteins, they are positioned to play a role in intracellular signal transduction and in mediating cell surface proteolytic events.

Cell surface proteolysis is a mechanism for the generation of biologically active proteins that mediate a variety of cellular functions. These membrane-anchored proteins, include a disintegrin-like and metalloproteinase (ADAM) and membrane-type matrix metalloproteinase (MT-MMP). In mammals, at least 17 members of the family are known, including seven in humans (see, Hooper et al. (2001) J. Biol. Chem. 276:857-860). These include: corin (accession nos. AF133845 and AB013874; see, Yan et al. (1999) J. Biol. Chem. 274:14926-14938; Tomia et al. (1998) J. Biochem. 124:784-789; Uan et al. (2000) Proc. Natl. Acad. Sci. U.S.A. 97:8525-8529); enterpeptidase (also designated enterokinase; accession no. U09860 for the human protein; see, Kitamoto et al. (1995) Biochem. 27: 4562-4568; Yahagi et al. (1996) Biochem. Biophys. Res. Commun. 219:806-812; Kitamoto et al. (1994) Proc. Natl. Acad. Sci. U.S.A. 91:7588-7592; Matsushima et al. (1994) J. Biol. Chem. 269:19976-19982;); human airway trypsin-like protease (HAT; accession no. AB002134; see Yamaoka et al. J. Biol. Chem. 273:11894-11901); MTSP1 and matriptase (also called TADG-15; see SEQ ID Nos. 1 and 2; accession nos. AF133086/AF118224, AF04280022; Takeuchi et al. (1999) Proc. Natl. Acad. Sci. U.S.A. 96:11054-1161; Lin et al. (1999) J. Biol. Chem. 274:18231-18236; Takeuchi et al. (2000) J. Biol. Chem. 275:26333-26342; and Kim et al. (1999) Immunogenetics 49:420-429); hepsin (see, accession nos. M18930, AF030065, X70900; Leytus et al. (1988) Biochem. 27: 11895-11901; Vu et al. (1997) J. Biol. Chem. 272:31315-31320; and Farley et al. (1993) Biochem. Biophys. Acta 1773:350-352; and see, U.S. Patent No. 5,972,616); TMPRS2 (see, Accession Nos. U75329 and AF1 13596; Paoloni-Giacobino et al. (1997) Genomics 44:309-320; and Jacquinet et al. (2000) FEBS Lett. 468: 93-100); and TMPRSS4 (see, Accession No. NM 016425; Wallrapp et al. (2000) Cancer 60:2602-2606).

Serine proteases, including transmembrane serine proteases, have been implicated in processes involved in neoplastic development and progression. While the precise role of these proteases has not been elaborated, serine proteases and inhibitors thereof are involved in the control of many intra- and extracellular physiological processes, including degradative actions in cancer cell invasion, metastatic spread, and neovascularization of tumors, that are involved in tumor progression. It is believed that proteases are involved in the degradation of extracellular matrix (ECM) and contribute to tissue remodeling, and are necessary for cancer invasion and metastasis. The activity and/or expression of some proteases have been shown to correlate with tumor progression and development.

For example, a membrane-type serine protease MTSP1 (also called matriptase; see SEQ ID Nos. 1 and 2 from U.S. Patent No. 5,972,616; and GenBank Accession No. AF118224; (1999) J. Biol. Chem. 274:18231-18236; U.S. Patent No. 5,792,616; see, also Takeuchi (1999) Proc. Natl. Acad. Sci. U.S.A. 96:11054-1161) that is expressed in epithelial cancer and normal tissue (Takeucuhi et al. (1999) Proc. Natl. Acad. Sci. USA, 96(20):11054-61) has been identified. Matriptase was originally identified in human breast cancer cells as a major gelatinase (see, U.S. Patent No. 5,482,848), a type of matrix metalloprotease (MMP). It has been proposed that it plays a role in the metastasis of breast cancer. Its primary cleavage specificity is Arg-Lys residues. Matriptase also is expressed in a variety of epithelial tissues with high levels of activity and/or expression in the human gastrointestinal tract and the prostate.

Prostate-specific antigen (PSA), a kallikrein-like serine protease, degrades extracellular matrix glycoproteins fibronectin and laminin, and, has been postulated to facilitate invasion by prostate cancer cells (Webber et al. (1995) Clin. Cancer Res., 1(10):1089-94). Blocking PSA proteolytic activity with PSA-specific monoclonal antibodies results in a dose-dependent decrease *in vitro* in the invasion of the reconstituted basement membrane Matrigel by LNCaP human prostate carcinoma cells which secrete high levels of PSA.

Hepsin, a cell surface serine protease identified in hepatoma cells, is overexpressed in ovarian cancer (Tanimoto et al. (1997) Cancer Res., 57(14):2884-7). The hepsin transcript appears to be abundant in carcinoma tissue and is almost never expressed in normal adult tissue, including normal ovary. It has been suggested that hepsin is frequently overexpressed in ovarian tumors and therefore may be a candidate protease in the invasive process and growth capacity of ovarian tumor cells.

A serine protease-like gene, designated normal epithelial cell-specific 1 (NES1) (Liu et al., Cancer Res., 56(14):3371-9 (1996)) has been identified. Although expression of the NES1 mRNA is observed in all normal and immortalised nontumorigenic epithelial cell lines, the majority of human breast cancer cell lines show a drastic reduction or a complete lack of its expression. The structural similarity of NES1 to polypeptides known to regulate growth factor activity and a negative correlation of NES1 expression with breast oncogenesis suggest a direct or indirect role for this protease-like gene product in the suppression of tumorigenesis.

Lu et al ([1997] J Biol chem.. 272: 31293-31300) discloses the purification of bovine enteropeptidase which is a dimer comprising a light chain disulphide-linked to a heavy chain. It further discloses that the light chain which forms the serine protease domain is active when isolated alone, but has greatly reduced activity towards trypsinogen in the absence of the heavy chain.

WO99/4212 Discloses a dimeric extracellular serine protease designated TADGI which is an MTSP1 varient overexpressed in tumours.

Hence transmembrane serine proteases appear to be involved in the etiology and pathogenesis of tumors. There is a need to further elucidate their role in these processes and to identify additional transmembrane proteases.

Therefore, it is an object herein to provide transmembrane serine protease (MTSP) proteins and nucleic

acids encoding such MTSP proteases that are involved in the regulation of or participate in tumorigenesis and/or cacinogenesis. It is also an object herein to provide prognostic, diagnostic, therapeutic screening methods using the such proteases and the nucleic acids encoding such proteases.

### SUMMARY OF THE INVENTION

Provided herein are isolated protease domains of the Type II Transmembrane Serine Protease family, (TTSP) (also referred to herein as MTSPs), and more particularly TTSP family members whose functional activity differs in tumor cells from non-tumor cells in the same tissue. For example, the MTSPs include those that are activated and/or expressed in tumor cells at different levels, typically higher, from non-tumor cells; and those from cells in which substrates therefore differ in tumor cells from non-tumor cells or otherwise after the specificity of the MTSP.

The MTSP family as intended herein does not include any membrane anchored or spanning proteases that are expressed on endothelial cells.

Included among the MTSPs are several heretofore unidentified MTSP family members, designated herein as MTSP3 and MTSP4 and a new form of a protein designated herein as MTSP6 as described in the claims. In addition to the protease domains of each of MTSP3 and MTSP4, the full-length proteins, including those that results from splice variants, zymogens and activated forms, and uses thereof, are also provided.

The protease domains as provided herein are single-chain polypeptides, with an N-terminus (such as IV, VV, IL and II) generated at the cleavage site (generally having the consensus sequence R↓VVGG, R↓IVGG, R↓IVNG, R↓ILGG, R↓VGLL, R↓ILGG or a variation thereof: an N-terminus R↓V or R↓I, where the arrow represents the cleavage point) when the zymogen is activated. To identify the protease domain an RI should be identified, and then the following amino acids compared to the above noted motif.

The protease domains generated herein, however, do not result from activation, which produces a two chain activated product, but rather are single chain polypeptides with the N-terminus include the consensus sequence ↓VVGG, ↓IVGG, ↓VGLL, ↓ILGG or ↓IVNG or other such motif at the N-terminus. As shown herein, such polypeptides, although not the result of activation and not double-chain forms, exhibit proteolytic (catalytic) activity. These protease domain polypeptides are used in assays to screen for agents that modulate the activity of the MTSP. Such assays are also provided herein. In exemplary assays, the affects of test compounds in the ability of a protease domains to proteolytically cleave a known substrate, typically a fluorescently, chromogenically or otherwise detectably labeled substrate, are assessed. Agents, generally compounds, particularly small molecules, that modulate the activity of the protease domain are candidate compounds for modulating the activity of the MTSP. The protease domains provided herein include, the single chain region having an N-terminus at the cleavage site for activation of the zymogen, through the C-terminus, or C-terminal truncated portions thereof that exhibit proteolytic activity as a single-chain polypeptide in *in vitro* proteolysis assays, of any MTSP family member, preferably from a mammal, including and most preferably human, that, for example, is expressed in tumor cells at different levels from non-tumor cells, and that is not expressed on an endothelial cell. These include, but are not limited to : MTSP1 (or matriptase), MTSP3, MTSP4 and MTSP6. Other MTSP protease domains of interest herein, particularly for use in *in vitro* drug screening proteolytic assays, include, but are not limited to: corin (accession nos. AF133845 and AB013874; see, Yan et al. (1999) J. Biol. Chem. 274:14926-14938; Tomia et al. (1998) J. Biochem. 124:784-789; Uan et al. (2000) Proc. Natl. Acad. Sci. U.S.A. 97:8525-8529; SEQ ID Nos. 61 and 62 for the human protein); enterpeptidase (also designated enterokinase; accession no. U09860 for the human protein; see, Kitamoto et al. (1995) Biochem. 27: 4562-4568; Yahagi et al. (1996) Biochem. Biophys. Res. Commun. 219:806-812; Kitamoto et al. (1994) Proc. Natl. Acad. Sci. U.S.A. 91:7588-7592; Matsushima et al. (1994) J. Biol. Chem. 269:19976-19982; see SEQ ID Nos. 63 and 64 for the human protein); human airway trypsin-like protease (HAT; accession no. AB002134; see Yamaoka et al. J. Biol. Chem. 273:11894-11901; SEQ ID Nos. 65 and 66 for the human protein); hepsin (see, accession nos. M18930, AF030065, X70900; Leytus et al. (1988) Biochem. 27: 11895-11901; Vu et al. (1997) J. Biol. Chem. 272:31315-31320; and Farley et al. (1993) Biochem. Biophys. Acta 1173:350-352; SEQ ID Nos. 67 and 68 for the human protein); TMPRS2 (see, Accession Nos. U75329 and AF113596; Paoloni-Giacobino et al. (1997) Genomics 44:309-320; and Jacquinet et al. (2000) FEBS Lett. 468: 93-100; SEQ ID Nos. 69 and 70 for the human protein) TMPRSS4 (see, Accession No. NM 016425; Wallrapp et al. (2000) Cancer 60:2602-2606; SEQ ID Nos. 71 and 72 for the human protein); and TADG-12 (also designated MTSP6, see SEQ ID Nos. 11 and 12; see International PCT application No. WO 00/52044, which claims priority to U.S. application Serial No. 09/261,416).

Also provided are muteins of the single chain protease domains and MTSPs, particularly muteins in which the Cys residue in the protease domain that is free (*i.e*., does not form disulfide linkages with any other Cys residue in the protein) is substituted with another amino acid substitution, preferably with a conservative amino acid substitution or a substitution that does not eliminate the

activity. Hence, provided herein are members of a family of transmembrane serine protease (MTSP) proteins, and functional domains, especially protease (or catalytic) domains thereof, muteins and other derivatives and analogs thereof.

Exemplary MTSPs (see, *e.g.,* SEQ ID No. 1-12, 49 and 50) are provided herein, as are the single chain protease domains thereof as follows: SEQ ID Nos. 1, 2, 49 and 50 set forth amino acid and nucleic acid sequences of MTSP1 and the protease domain thereof; SEQ ID No. 3 sets forth the MTSP3 nucleic acid sequence and SEQ ID No. 4 the encoded MTSP3 amino acids; SEQ ID No. 5 MTSP4 a nucleic acid sequence of the protease domain arid SEQ ID No. 6 the encoded MTSP4 amino acid protease domain; SEQ ID No. 7 MTSP4-L a nucleic acid sequence and SEQ ID No. 8 the encoded MTSP4-L amino acid sequence; SEQ ID No. 9 an MTSP4-S encoding nucleic acid sequence and SEQ ID No. 10 the encoded MTSP4-S amino acid sequence; and SEQ ID No. 11 an MTSP6 encoding nucleic acid sequence and SEQ ID No. 12 the encoded MTSP6 amino acid sequence. The single chain protease domains of each are delineated below.

Provided herein are members of a family of membrane serine proteases (MTSP) that are expressed in certain tumor or cancer cells such lung, prostate, colon and breast cancers. In particular, it is shown herein, that MTSPs, particularly, MTSP3, MTSP4 and MTSP6 are expressed in lung carcinoma, breast carcinoma, colon adenocarcinoma and/or ovarian carcinomas as well as in certain normal cells and tissues (see *e.g*., EXAMPLES for tissue-specific expression profiles of each protein exemplified herein). The MTSPs that are of particular interest herein, are those that are expressed in tumor cells, for example, those that appear to be expressed at different levels in tumor cells from normal cells, or whose functional activity is different in tumor cells from normal cells, such as by an alteration in a substrate therefor, or a cofactor. Hence the MTSP provided herein can serve as diagnostic markers for certain tumors. The level of activated MTSP3, MTSP4 and MTSP6 can be diagnostic of prostate cancer. In addition, MTSP4 is expressed and/or activated in lymphomas, leukemias, lung cancer, breast, prostrate and colon cancers. MTSP6 is activated and/or expressed in breast, lung, prostate, colon and ovarian cancers. Furthermore, compounds that modulate the activity of these MTSPs, as assessed by the assays provided herein, particularly the *in vitro* proteolytic assays that use the single chain protease domains, are potential therapeutic candidates for treatment of various malignancies and neoplastic disease.

Also provided herein are methods of screening for compounds that modulate, including inhibit, antagonize, agonize or otherwise alter the activity of the MTSPs. Of particular interest is the extracellular domain of these MTSPs that includes the proteolytic (catalytic) portion of the protein.

MTSP proteins, including, but not limited to, MTSP3, MTSP4, and MTSP6, including splice variants thereof, and nucleic acids encoding MTSPs, and domains, derivatives and analogs thereof are provided herein. Single chain protease domains, in the N-terminal is that which would be generated by activation of the zymogen, from any MTSP, particularly those that are not expressed in endothelial cells and that are expressed in tumor cells are also provided.

Also provided are methods for screening for compounds that modulate the activity of any MTSP. The compounds are identified by contacting them with the MTSP and a substrate for the MTSP. A change in the amount of substrate cleaved in the presence of the compounds compared to that in the absence of the compound indicates that the compound modulates the activity of the MTSP. Such compounds are selected for further analyses or for use to modulate the activity of the MTSP, such as inhibitors or agonists. The compounds can also be identified by contacting the substrates with a cell that expresses the MTSP or the extracellular domain or proteolytically active portion thereof. For assays in which the extracellular domain or a proteolytically active portion thereof is employed, the MTSP is any MTSP that is expressed on cells, other than endothelial cells, including, but not limited to MTSP1, MTSP3, MTSP4 and MTSP6.

Pharmaceutical composition containing the protease domains of an MTSP protein, and the MTSP proteins, MTSP3, MTSP4 and MTSP6 are provided herein in a pharmaceutically acceptable carrier or excipient are provided herein.

Conjugates containing a) a MTSP protease domain in single chain from; and b) a targeting agent linked to the MTSP directly or via a linker, wherein the agent facilitates: i) affinity isolation or purification of the conjugate; ii) attachment of the conjugate to a surface; iii) detection of the conjugate; or iv) targeted delivery to a selected tissue or cell, is provided herein. The conjugate can contain a plurality of agents linked thereto. The conjugate can be a chemical conjugate; and it can be a fusion protein.

In yet another embodiment, the targeting agent is a protein or peptide fragment. The protein or peptide fragment can include a protein binding sequence, a nucleic acid binding sequence, a lipid binding sequence, a polysaccharide binding sequence, or a metal binding sequence.

### BRIEF DESCRIPTION OF DRAWINGS

Figure 1 illustrates the domain organization of the MTSP3;
Figure 2 illustrates the domain organization of the MTSP4 splice variants and domains thereof; MTSP4-L includes a transmembrane domain, a CUB domain, a low density lipoprotein receptor (LDLR) domains, and a serine protease catalytic domain; MTSP4-S lacking the portion between amino acids 136-279.
Figure 3 depicts the domain organization of MTSP6.

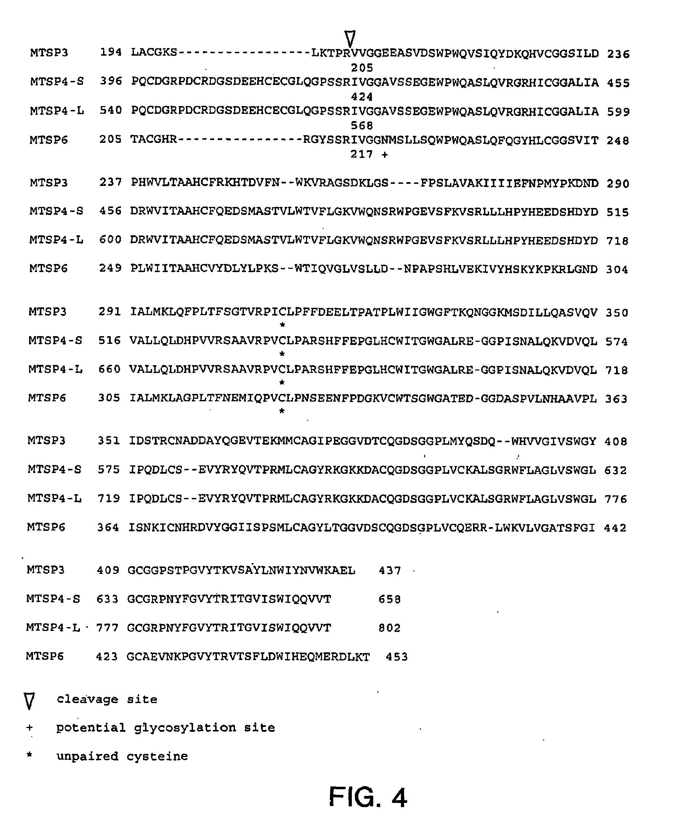

Figure 4 provides an alignment of the C-terminal portions of MTSP3, the two splice variant-encoded forms of MTSP4, and MTSP6, that encompasses the protease domains thereof; the figure shows the cleavage sites, which form the N-terminus of the protease domain of each protein; a potential glycosylation site is noted and the free Cys residues in the protease domain of each are noted (*). Muteins of each protein may be prepared by replacing the residues in the glycosylation site, particularly the N residue, and the free Cys residues, with preferably conservative amino acid residues. Such muteins are also provided herein.

### DETAILED DESCRIPTION OF THE INVENTION

### A. DEFINITIONS

Unless defined otherwise, all technical and scientific terms used herein have the same meaning as is commonly understood by one of ordinary skill in the art to which this invention belongs. All patents, applications, published applications and other publications and sequences from GenBank and other data bases referred to herein are incorporated by reference in their entirety.

As used herein, the abbreviations for any protective groups, amino acids and other compounds, are, unless indicated otherwise, in accord with their common usage, recognized abbreviations, or the IUPAC-IUB Commission on Biochemical Nomenclature (see, (1972) Biochem. 11:942-944).

As used herein, serine protease refers to a diverse family of proteases wherein a serine residue is involved in the hydrolysis of proteins or peptides. The serine residue can be part of the catalytic triad mechanism, which includes a serine, a histidine and an aspartic acid in the catalysis, or be part of the hydroxyl/ε-amine or hydroxyl/α-amine catalytic dyad mechanism, which involves a serine and a lysine in the catalysis.

As used herein, "transmembrane serine protease (MTSP)" refers to a family of transmembrane serine proteases that share common structural features as described herein (see, also Hooper et al. (2001) J. Biol. Chem.276:857-860). Thus, reference, for example, to "MTSP" encompasses all proteins encoded by the MTSP gene family, including but are not limited to: MTSP1, MTSP3, MTSP4 and MTSP6, or an equivalent molecule obtained from any other source or that has been prepared synthetically or that exhibits the same activity. Other MTSPs include, but are not limited to, corin, enterpeptidase, human airway trypsin-like protease (HAT), MTSP1, TMPRSS2, and TMPRSS4. Sequences of encoding nucleic molecules and the encoded amino acid sequences of exemplary MTSPs and/or domains thereof are set forth in SEQ ID Nos. 1-12, 49, 50 and 61-72. The term also encompass MTSPs with conservative amino acid substitutions that do not substantially alter activity of each member, and also encompasses splice variants thereof. Suitable conservative substitutions of amino acids are known to those of skill in this art and may be made generally without altering the biological activity of the resulting molecule. Of particular interest are MTSPs of mammalian, including human, origin. Those of skill in this art recognize that, in general, single amino acid substitutions in non-essential regions of a polypeptide do not substantially alter biological activity (see, e.g., Watson et al. Molecular Biology of the Gene, 4th Edition, 1987, The Bejacmin/Cummings Pub. co., p.224).

As used herein, a "protease domain of an MTSP" refers to the protease domain of MTSP that is located within the extracellular domain of a MTSP and exhibits serine proteolytic activity. It includes at least the smallest fragment thereof that acts catalytically as a single chain form. Hence it is at least the minimal portion of the extracellular domain that exhibits proteolytic activity as assessed by standard assays *in vitro* assays. Those of skill in this art recognize that such protease domain is the portion of the protease that is structurally equivalent to the trypsin or chymotrypsin fold.

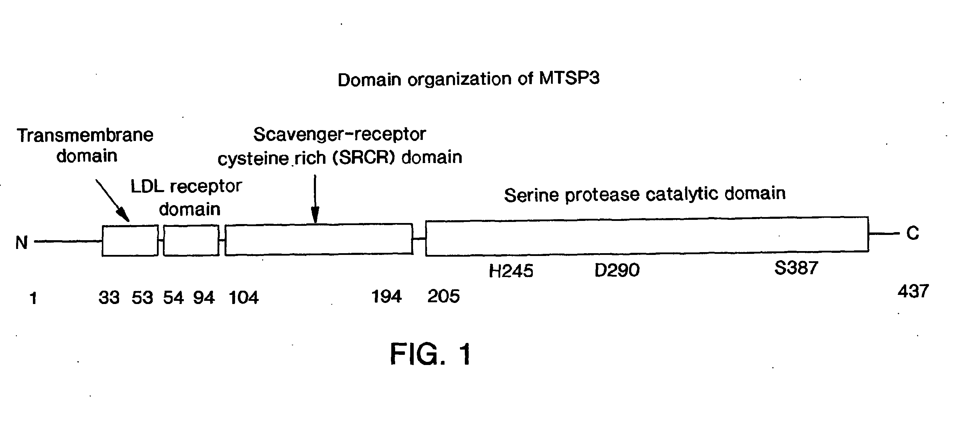

Exemplary MTSP proteins, with the protease domains indicated, are illustrated in Figures 1-3, Smaller portions thereof that retain protease activity are contemplated. The protease domains vary in size and constitution, including insertions and deletions in surface loops. They retain conserved structure, including at least one of the active site triad, primary specificity pocket, oxyanion hole and/or other features of serine protease domains of proteases. Thus, for purposes herein, the protease domain is a portion of a MTSP, as defined herein, and is homologous to a domain of other MTSPs, such as corin, enterpeptidase, human airway trypsin-like protease (HAT), MTSP1, TMPRSS2, and TMPRSS4, which have been previously identified; it was not recognized, however, that an isolated single chain form of the protease domain could function proteolytically in *in vitro* assays. As with the larger class of enzymes of the chymotrypsin (S1) fold (see, *e.g*., Internet accessible MEROPS data base), the MTSPs protease domains share a high degree of amino acid sequence identity. The His, Asp and Ser residues necessary for activity are present in conserved motifs. The activation site, which results in the N-terminus of second chain in the two chain forms is has a conserved motif and readily can be identified (see, *e.g*., amino acids 801-806, SEQ ID No. 62, amino acids 406-410, SEQ ID No. 64; amino acids 186-190, SEQ ID No. 66; amino acids 161-166, SEQ ID No. 68; amino acids 255-259, SEQ ID No. 70; amino acids 190-194, SEQ ID No. 72).

As used herein, the catalytically active domain of an MTSP refers to the protease domain. Reference to the protease domain of an MTSP refers includes the single and double-chain forms of any of these proteins. The zymogen form of each protein is single chain form, which can be converted to the active two chain form by cleavage. The protease domain may also be converted to a two chain form. By active form is meant a form active *in vivo.*

Significantly, it is shown herein, that, at least *in vitro,* the single chain forms of the MTSPs and the catalytic domains or proteolytically active portions thereof (typically C-terminal truncations) thereof exhibit protease activity. Hence provided herein are isolated single chain forms of the protease domains of MTSPs and their use in *in vitro* drug screening assays for identification of agents that modulate the activity thereof.

As used herein an MTSP3, whenever referenced herein, includes at least one or all of or any combination of:
a polypeptide encoded by the sequence of nucleotides set forth in SEQ ID No. 3;
a polypeptide encoded by a sequence of nucleotides that hybridizes under conditions of low, moderate or high stringency to the sequence of nucleotides set forth in SEQ ID No. 3;
a polypeptide that comprises the sequence of amino acids set forth as amino acids 205-437 of SEQ ID No. 4;
a polypeptide that comprises a sequence of amino acids having at least about 85% or 90% sequence identity with the sequence of amino acids set forth in SEQ ID No. 4; and/or
a splice variant of the MTSP3 set forth in SEQ ID Nos. 3 and 4.

The MTSP3 may be from any animal, particularly a mammal, and includes but are not limited to, humans, rodents, fowl, ruminants and other animals. The full length zymogen or double chain activated form is contemplated or any domain thereof, including the protease domain, which can be a double chain activated form, or a single chain form.

As used herein an MTSP4, whenever referenced herein, includes at least one or all of or any combination of:
a polypeptide encoded by the sequence of nucleotides set forth in any of SEQ ID No. 5, 7 or 9;
a polypeptide encoded by a sequence of nucleotides that hybridizes under conditions of low, moderate or high stringency to the sequence of nucleotides set forth in any of SEQ ID Nos. 5, 7 or 9;
a polypeptide that comprises the sequence of amino acids set forth in any of SEQ ID Nos. 6, 8 or 10;
a polypeptide that comprises a sequence of amino acids having at least about 85% or 90% or 95% sequence identity with the sequence of amino acids set forth in SEQ ID No. 6, 8 or 10; and/or
a splice variant of the MTSP4s set forth in SEQ ID Nos. 7-10.

The MTSP4 may be from any animal, particularly a mammal, and includes but are not limited to, humans, rodents, fowl, ruminants and other animals. The full length zymogen or double chain activated form is contemplated or any domain thereof, including the protease domain, which can be a double chain activated form, or a single chain form.

As used herein an MTSP6, whenever referenced herein, includes at least one or all of or any combination of:
a polypeptide encoded by the sequence of nucleotides set forth in any of SEQ ID No. 11;
a polypeptide encoded by a sequence of nucleotides that hybridizes under conditions of low, moderate or high stringency to the sequence of nucleotides set forth in any of SEQ ID Nos. 11;
a polypeptide that comprises the sequence of amino acids set forth in any of SEQ ID Nos. 12;
a polypetide that comprises a sequence of amino acids having at least about 90% or 95% or 98% sequence identity with the sequence of amino acids set forth in SEQ ID No. 12; and/or
a splice variant of the MTSP4s set forth in SEQ ID No. 12.

The MTSP6 may be from any animal, particularly a mammal, and includes but are not limited to, humans, rodents, fowl, ruminants and other animals. The full length zymogen or double chain activated form is contemplated or any domain thereof, including the protease domain, which can be a double chain activated form, or a single chain form. Of particular interest herein is the MTSP6 of SEQ ID No. 12.

As used herein, a human protein is one encoded by DNA present in the genome of a human, including all allelic variants and conservative variations as long as they are not variants found in other mammals.

As used herein, a "nucleic acid encoding a protease domain or catalytically active portion of a MTSP" shall be construed as referring to a nucleic acid encoding only the recited single chain protease domain or active portion thereof, and not the other contiguous portions of the MTSP as a continuous sequence.

As used herein, a CUB domain is a motif that mediates protein-protein interactions in complement components C1 r/C1 s and has also been identified in various proteins involved in developmental processes.

As used herein, catalytic activity refers to the activity of the MTSP as a serine proteases. Function of the MTSP refers to its function in tumor biology, including promotion of or involvement in tumorigenesis, metastasis or carcinogenesis, and also roles in signal transduction.

As used herein, a "propeptide" or "pro sequence" is sequence of amino acids positioned at the amino terminus of a mature biologically active polypeptide. When so-positioned, the resulting polypeptide is called a zymogen. Zymogens, generally, are biologically inactive and can be converted to mature active polypeptides by catalytic or autocatalytic cleavage of the propeptide from the zymogen. A zymogen is an enzymatically inactive protein that is converted to a proteolytic enzyme by the action of an activator. Cleavage may be effected autocatalytically.

As used herein, "disease or disorder" refers to a pathological condition in an organism resulting from, *e.g.,* infection or genetic defect, and characterized by identifiable symptoms.

As used herein, neoplasm (neoplasia) refers to abnormal new growth, and thus means the same as *tumor,* which may be benign or malignant. Unlike *hyperplasia,* neoplastic proliferation persists even in the absence of the original stimulus.

As used herein, neoplastic disease refers to any disorder involving cancer, including tumor development, growth, metastasis and progression.

As used herein, cancer refers to a general term for diseases caused by any type of malignant tumor.

As used herein, malignant, as applies to tumors, refers to primary tumors that have the capacity of *metastasis* with loss of *growth control* and *positional control.*

As used herein, an anti-cancer agent (used interchangeable with "anti-tumor or anti-neoplastic agent") refers to any agents used in the anti-cancer treatment. These include any agents, when used alone or in combination with other compounds, that can alleviate, reduce, ameliorate, prevent, or place or maintain in a state of remission of clinical symptoms or diagnostic markers associated with neoplastic disease, tumor and cancer, and can be used in methods, combinations and compositions provided herein. Non-limiting examples of anti-neoplastic agents include anti-angiogenic agents, alkylating agents, antimetabolite, certain natural products, platinum coordination complexes, anthracenediones, substituted ureas, methylhydrazine derivatives, adrenocortical suppressants, certain hormones, antagonists and anti-cancer polysaccharides.

As used herein, a splice variant refers to a variant produced by differential processing of a primary transcript of genomic DNA that results in more than one type of mRNA. Splice variants of MTSPs are provided herein.

As used herein, angiogenesis is intended to broadly encompass the totality of processes directly or indirectly involved in the establishment and maintenance of new vasculature (neovascularization), including, but not limited to, neovascularization associated with tumors.

As used herein, anti-angiogenic treatment or agent refers to any therapeutic regimen and compound, when used alone or in combination with other treatment or compounds, that can alleviate, reduce, ameliorate, prevent, or place or maintain in a state of remission of clinical symptoms or diagnostic markers associated with undesired and/or uncontrolled angiogenesis. Thus, for purposes herein an anti-angiogenic agent refers to an agent that inhibits the establishment or maintenance of vasculature. Such agents include, but are not limited to, anti-tumor agents, and agents for treatments of other disorders associated with undesirable angiogenesis, such as diabetic retinopathies, restenosis, hyperproliferative disorders and others.

As used herein, non-anti-angiogenic anti-tumor agents refer to anti-tumor agents that do not act primarily by inhibiting angiogenesis.

As used herein, pro-angiogenic agents are agents that promote the establishment or maintenance of the vasculature. Such agents include agents for treating cardiovascular disorders, including heart attacks and strokes.

As used herein, undesired and/or uncontrolled angiogenesis refers to pathological angiogenesis wherein the influence of angiogenesis stimulators outweighs the influence of angiogenesis inhibitors. As used herein, deficient angiogenesis refers to pathological angiogenesis associated with disorders where there is a defect in normal angiogenesis resulting in aberrant angiogenesis or an absence or substantial reduction in angiogenesis.

As used herein, endotheliase refers to a mammalian protein, including humans, that has a transmembrane domain and is expressed on the surface of endothelial cells and includes a protease domain, particularly an extracellular protease domain, and is preferably a serine protease. Thus, reference, for example, to endotheliase encompasses all proteins encoded by the endotheliase gene family, or an equivalent molecule obtained from any other source or that has been prepared synthetically or that exhibits the same activity. The endotheliase gene family are transmembrane proteases expressed in endothelial cells. Endotheliases are excluded from the MTSPs contemplated herein.

As used herein, the protease domain of an endotheliase refers to the polypeptide portion of the endotheliase that is the extracellular portion that exhibits protease activity. The protease domain is a polypeptide that includes at least the minimum number of amino acids, generally more than 50 or 100, required for protease activity. Protease activity may be assessed empirically, such as by testing the polypeptide for its ability to act as a protease. Assays, such as the assays described in the EXAMPLES, employing a known substrate in place of the test compounds may be used. Furthermore, since proteases, particularly serine proteases, have characteristic structures and sequences or motifs, the protease domain may be readily identified by such structure and sequence or motif.

As used herein, the protease domain of an MTSP protein refers to the protease domain of an MTSP that is located within or is the extracellular domain of an MTSP and exhibits serine proteolytic activity. Hence it is at least the minimal portion of the extracellular domain that exhibits proteolytic activity as assessed by standard assays *in vitro.* It refers, herein, to a single chain form heretofore thought to be inactive.

Exemplary protease domains include at least a sufficient portion of sequences of amino acids set forth as amino acids 615-855 in SEQ ID No. 2 (encoded by nucleotides 1865-2587 in SEQ ID No. 1; see also SEQ ID Nos. 49 and 50) from MTSP1, amino acids 205-437 of SEQ ID NO. 4 from MTSP3, SEQ ID No. 6, which sets forth the protease domain of MTSP4, and amino acids 217-443 of SEQ ID No. 11 from MTSP6. Also contemplated are nucleic acid molecules that encode polypeptide that has proteolytic activity in an *in vitro* proteolysis assay and that have at least 80%, 85%, 90% or 95% sequence identity with the full length of a protease domain of an MTSP protein, or that hybridize along their full length to a nucleic acids that encode a protease domain, particularly under conditions of moderate, generally high, stringency.

For each of these protease domains, residues at the N-terminus can be critical for activity, since it has been shown that an Asp in the N-terminus of such proteases is essential for formation of the catalytically active conformation upon activation cleavage of the zymogen form of the protease. It is shown herein that the protease domain of the singles chain form of the protease is catalytically active. Hence the protease domain will require the N-terminal amino acids; the c-terminus portion may be truncated. The amount that can be removed can be determined empirically by testing the protein for protease activity in an *in vitro* assays that assesses catalytic cleavage.

Hence smaller portions of the protease domains, particularly the single chain domains, thereof that retain protease activity are contemplated. Such smaller versions will generally be C-terminal truncated versions of the protease domains. The protease domains vary in size and constitution, including insertions and deletions in surface loops. Such domains exhibit conserved structure, including at least one structural feature, such as the active site triad, primary specificity pocket, oxyanion hole and/or other features of serine protease domains of proteases. Thus, for purposes herein, the protease domain is a single chain portion of an MTSP, as defined herein, but is homologous in its structural features and retention of sequence of similarity or homology the protease domain of chymotrypsin or trypsin. Most significantly, the polypeptide will exhibit proteolytic activity as a single chain.

As used herein, by homologous means about greater than 25% nucleic acid sequence identity, preferably 25% 40%, 60%, 80%, 90% or 95%. The terms "homology" and "identity" are often used interchangeably. In general, sequences are aligned so that the highest order match is obtained (see, *e.g*.: Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; Carillo et al. (1988) SIAM J Applied Math 48:1073).

By sequence identity, the number of conserved amino acids are determined by standard alignment algorithms programs, and are used with default gap penalties established by each supplier. Substantially homologous nucleic acid molecules would hybridize typically at moderate stringency or at high stringency all along the length of the nucleic acid of interest. Also contemplated are nucleic acid molecules that contain degenerate codons in place of codons in the hybridizing nucleic acid molecule.

Whether any two nucleic acid molecules have nucleotide sequences that are at least 80%, 85%, 90%, 95%, 96%, 97%, 98% or 99% "identical" can be determined using known computer algorithms such as the "FAST A" program, using for example, the default parameters as in Pearson et al. (1988) Proc. Natl. Acad. Sci. USA 85:2444 (other programs include the GCG program package (Devereux, J., et al., Nucleic Acids Research 12(I):387 (1984)), BLASTP, BLASTN, FASTA (Atschul, S.F., et al., J Molec Biol 215:403 (1990); Guide to Huge Computers, Martin J. Bishop, ed., Academic Press, San Diego, 1994, and Carillo et al. (1988) SIAM J Applied Math 48:1073). For example, the BLAST function of the National Center for Biotechnology Information database may be used to determine identity. Other commercially or publicly available programs include, DNAStar "MegAlign" program (Madison, WI) and the University of Wisconsin Genetics Computer Group (UWG) "Gap" program (Madison WI)). Percent homology or identity of proteins and/or nucleic acid moleucles may be determined, for example, by comparing sequence information using a GAP computer program (*e.g.,* Needleman et al. (1970) J. Mol. Biol. 48:443, as revised by Smith and Waterman ((1981) Adv. Appl. Math. 2:482). Briefly, the GAP program defines similarity as the number of aligned symbols (i.e., nucleotides or amino acids) which are similar, divided by the total number of symbols in the shorter of the two sequences. Default parameters for the GAP program may include: (1) a unary comparison matrix (containing a value of 1 for identities and 0 for non-identities) and the weighted comparison matrix of Gribskov et al. (1986) Nucl. Acids Res. 14:6745, as described by Schwartz and Dayhoff, eds., ATLAS OF PROTEIN SEQUENCE AND STRUCTURE, National Biomedical Research Foundation, pp. 353-358 (1979); (2) a penalty of 3.0 for each gap and an additional 0.10 penalty for each symbol in each gap; and (3) no penalty for end gaps.

Therefore, as used herein, the term "identity" represents a comparison between a test and a reference polypeptide or polynucleotide. For example, a test polypeptide may be defined as any polypeptide that is 90% or more identical to a reference polypeptide. As used herein, the term at least "90% identical to" refers to percent identities from 90 to 99.99 relative to the reference polypeptides. Identity at a level of 90% or more is indicative of the fact that, assuming for exemplification purposes' a test and reference polynucleotide length of 100 amino acids are compared. No more than 10% (i.e., 10 out of 100) amino acids in the test polypeptide differs from that of the reference polypeptides. Similar comparisons may be made between a test and reference polynucleotides. Such differences may be represented as point mutations randomly distributed over the entire length of an amino acid sequence or they may be clustered in one or more locations of varying length up to the maximum allowable, e.g. 10/100 amino acid difference (approximately 90% identity). Differences are defined as nucleic acid or amino acid substitutions, or deletions. At level of homologies or identities above about 85-90%, the result should be independent of the program and gap parameters set; such high levels of identity readily can be assess, often without relying on software.

As used herein, primer refers to an oligonucleotide containing two or more deoxyribonucleotides or ribonucleotides, preferably more than three, from which synthesis of a primer extension product can be initiated. Experimental conditions conducive to synthesis include the presence of nucleoside triphosphates and an agent for polymerization and extension, such as DNA polymerase, and a suitable buffer, temperature and pH.

As used herein, animals include any animal, such as, but are not limited to, goats, cows, deer, sheep, rodents, pigs and humans. Non-human animals, exclude humans as the contemplated animal. The MTSPs provided herein are from any source, animal, plant, prokaryotic and fungal. Preferred MTSPs are of animal origin, preferably mammalian origin.

As used herein, genetic therapy involves the transfer of heterologous DNA to the certain cells, target cells, of a mammal, particularly a human, with a disorder or conditions for which such therapy is sought. The DNA is introduced into the selected target cells in a manner such that the heterologous DNA is expressed and a therapeutic product encoded thereby is produced. Alternatively, the heterologous DNA may in some manner mediate expression of DNA that encodes the therapeutic product, or it may encode a product, such as a peptide or RNA that in some manner mediates, directly or indirectly, expression of a therapeutic product. Genetic therapy may also be used to deliver nucleic acid encoding a gene product that replaces a defective gene or supplements a gene product produced by the mammal or the cell in which it is introduced. The introduced nucleic acid may encode a therapeutic compound, such as a growth factor inhibitor thereof, or a tumor necrosis factor or inhibitor thereof, such as a receptor therefor, that is not normally produced in the mammalian host or that is not produced in therapeutically effective amounts or at a therapeutically useful time. The heterologous DNA encoding the therapeutic product may be modified prior to introduction into the cells of the afflicted host in order to enhance or otherwise alter the product or expression thereof. Genetic therapy may also involve delivery of an inhibitor or repressor or other modulator of gene expression.

As used herein, heterologous DNA is DNA that encodes RNA and proteins that are not normally produced *in vivo* by the cell in which it is expressed or that mediates or encodes mediators that alter expression of endogenous DNA by affecting transcription, translation, or other regulatable biochemical processes. Heterologous DNA may also be referred to as foreign DNA. Any DNA that one of skill in the art would recognize or consider as heterologous or foreign to the cell in which is expressed is herein encompassed by heterologous DNA. Examples of heterologous DNA include, but are not limited to, DNA that encodes traceable marker proteins, such as a protein that confers drug resistance, DNA that encodes therapeutically effective substances, such as anti-cancer agents, enzymes and hormones, and DNA that encodes other types of proteins, such as antibodies. Antibodies that are encoded by heterologous DNA may be secreted or expressed on the surface of the cell in which the heterologous DNA has been introduced.

Hence, herein heterologous DNA or foreign DNA, includes a DNA molecule not present in the exact orientation and position as the counterpart DNA molecule found in the genome. It may also refer to a DNA molecule from another organism or species (*i.e.*, exogenous).

As used herein, a therapeutically effective product is a product that is encoded by heterologous nucleic acid, typically DNA, that, upon introduction of the nucleic acid into a host, a product is expressed that ameliorates or eliminates the symptoms, manifestations of an inherited or acquired disease or that cures the disease.

As used herein, recitation that a polypeptide consists essentially of the protease domain means that the only MTSP portion of the polypeptide is a protease domain or a catalytically active portion thereof. The polypeptide may optionally, and generally will, include additional non-MTSP-derived sequences of amino acids.

As used herein, cancer or tumor treatment or agent refers to any therapeutic regimen and/or compound that, when used alone or in combination with other treatments or compounds, can alleviate, reduce, ameliorate, prevent, or place or maintain in a state of remission of clinical symptoms or diagnostic markers associated with deficient angiogenesis.

As used herein, domain refers to a portion of a molecule, *e.g*., proteins or nucleic acids, that is structurally and/or functionally distinct from other portions of the molecule.

As used herein, protease refers to an enzyme catalyzing hydrolysis of proteins or peptides. For purposes herein, the protease domain is a single chain form of an MTSP protein. For MTSP3 and MTSP4 the protease domain also includes two chain forms.

As used herein, catalytic activity refers to the activity of the MTSP as a protease as assessed in *in vitro* proteolytic assays that detect proteolysis of a selected substrate.

As used herein, nucleic acids include DNA, RNA and analogs thereof, including protein nucleic acids (PNA) and mixture thereof. Nucleic acids can be single or double stranded. When referring to probes or primers, optionally labeled, with a detectable label, such as a fluorescent or radiolabel, single-stranded molecules are contemplated. Such molecules are typically of a length such that they are statistically unique and of a low copy number (typically less than 5, preferably less than 3) for probing or priming a library. Generally a probe or primer contains at least 14, 16 or 30 contiguous sequence complementary to or identical to a gene of interest. Probes and primers can be 10, 20, 30, 50, 100 or more nucleic acids long.

As used herein, nucleic acid encoding a fragment or portion of an MTSP refers to a nucleic acid encoding only the recited fragment or portion of MTSP, and not the other contiguous portions of the MTSP.

As used herein, heterologous or foreign DNA and RNA are used interchangeably and refer to DNA or RNA that does not occur naturally as part of the genome in which it is present or which is found in a location or locations in the genome that differ from that in which it occurs in nature. Heterologous nucleic acid is generally not endogenous to the cell into which it is introduced, but has been obtained from another cell or prepared synthetically. Generally, although not necessarily, such nucleic acid encodes RNA and proteins that are not normally produced by the cell in which it is expressed. Any DNA or RNA that one of skill in the art would recognize or consider as heterologous or foreign to the cell in which it is expressed is herein encompassed by heterologous DNA. Heterologous DNA and RNA may also encode RNA or proteins that mediate or alter expression of endogenous DNA by affecting transcription, translation, or other regulatable biochemical processes.

As used herein, operative linkage of heterologous DNA to regulatory and effector sequences of nucleotides, such as promoters, enhancers, transcriptional and translational stop sites, and other signal sequences refers to the relationship between such DNA and such sequences of nucleotides. For example, operative linkage of heterologous DNA to a promoter refers to the physical relationship between the DNA and the promoter such that the transcription of such DNA is initiated from the promoter by an RNA polymerase that specifically recognizes, binds to and transcribes the DNA in reading frame.

As used herein, a sequence complementary to at least a portion of an RNA, with reference to antisense oligonucleotides, means a sequence having sufficient complementarily to be able to hybridize with the RNA, preferably under moderate or high stringency conditions, forming a stable duplex; in the case of double-stranded MTSP antisense nucleic acids, a single strand of the duplex DNA may thus be tested, or triplex formation may be assayed. The ability to hybridize depends on the degree of complementarily and the length of the antisense nucleic acid. Generally, the longer the hybridizing nucleic acid, the more base mismatches with a MTSP encoding RNA it can contain and still form a stable duplex (or triplex, as the case may be). One skilled in the art can ascertain a tolerable degree of mismatch by use of standard procedures to determine the melting point of the hybridized complex.

For purposes herein, conservative amino acid substitutions may be made in any of MTSPs and protease domains thereof provided that the resulting protein exhibits protease activity. Conservative amino acid substitutions, such as those set forth in Table 1, are those that do not eliminate proteolytic activity. Suitable conservative substitutions of amino acids are known to those of skill in this art and may be made generally without altering the biological activity of the resulting molecule. Those of skill in this art recognize that, in general, single amino acid substitutions in non-essential regions of a polypeptide do not substantially alter biological activity (see, e.g., Watson et al. Molecular Biology of the Gene, 4th Edition, 1987, The Bejacmin/Cummings Pub. co., p.224). Also included within the definition, is the catalytically active fragment of an MTSP, particularly a single chain protease portion. Conservative amino acid substitutions are made, for example, in accordance with those set forth in TABLE 1 as follows:

**TABLE 1**

| **Original residue** | **Conservative substitution** |
|---|---|
| Ala (A) | Gly; Ser, Abu |
| Arg (R) | Lys, orn |
| Asn (N) | Gln; His |
| Cys (C) | Ser |
| Gln (Q) | Asn |
| Glu (E) | Asp |
| Gly (G) | Ala; Pro |
| His (H) | Asn; Gln |
| Ile (I) | Leu; Val; Met; Nle; Nva |
| Leu (L) | Ile; Val; Met; Nle; Nv |
| Lys (K) | Arg; Gln; Glu |
| Met (M) | Leu; Tyr; Ile; NLe Val |
| Ornitine | Lys; Arg |
| Phe (F) | Met; Leu; Tyr |
| Ser (S) | Thr |
| Thr (T) | Ser |
| Trp (W) | Tyr |
| Tyr (Y) | Trp; Phe |
| Val (V) | Ile; Leu; Met; Nle; Nv |

Other substitutions are also permissible and may be determined empirically or in accord with known conservative substitutions.

As used herein, Abu is 2-aminobutyric acid; Orn is ornithine.

As used herein, the amino acids, which occur in the various amino acid sequences appearing herein, are identified according to their well-known, three-letter or one-letter abbreviations. The nucleotides, which occur in the various DNA fragments, are designated with the standard single-letter designations used routinely in the art.

As used herein, a splice variant refers to a variant produced by differential processing of a primary transcript of genomic DNA that results in more than one type of mRNA.

As used herein, a probe or primer based on a nucleotide sequence disclosed herein, includes at least 10, 14, preferably at least 16 or 30 or 100 contiguous sequence of nucleotides of SEQ ID Nos. 1, 3, 5, 7, 9 or 11.

As used herein, amelioration of the symptoms of a particular disorder by administration of a particular pharmaceutical composition refers to any lessening, whether permanent or temporary, lasting or transient that can be attributed to or associated with administration of the composition.

As used herein, antisense polynucleotides refer to synthetic sequences of nucleotide bases complementary to mRNA or the sense strand of double stranded DNA. Admixture of sense and antisense polynucleotides under appropriate conditions leads to the binding of the two molecules, or hybridization. When these polynucleotides bind to (hybridize with) mRNA, inhibition of protein synthesis (translation) occurs. When these polynucleotides bind to double stranded DNA, inhibition of RNA synthesis (transcription) occurs. The resulting inhibition of translation and/or transcription leads to an inhibition of the synthesis of the protein encoded by the sense strand. Antisense nucleic acid molecule typically contain a sufficient number of nucleotides to specifically bind to a target nucleic acid, generally at least 5 contiguous nucleotides, often at least 14 or 16 or 30 contiguous nucleotides or modified nucleotides complementary to the coding portion of a nucleic acid molecule that encodes a gene of interest, for example, nucleic acid encoding a single chain protease domain of an MTSP.

As used herein, an array refers to a collection of elements, such as antibodies, containing three or more members. An addressable array is one in which the members of the array are identifiable, typically by position on a solid phase support. Hence, in general the members of the array will be immobilized to discrete identifiable loci on the surface of a solid phase.

As used herein, antibody refers to an immunoglobulin, whether natural or partially or wholly synthetically produced, including any derivative thereof that retains the specific binding ability the antibody. Hence antibody includes any protein having a binding domain that is homologous or substantially homologous to an immunoglobulin binding domain. Antibodies include members of any immunoglobulin claims, including IgG, IgM, IgA, IgD and IgE.

As used herein, antibody fragment refers to any derivative of an antibody that is less then full length, retaining at least a portion of the full-length antibody's specific binding ability. Examples of antibody fragments include,but are not limited to, Fab, Fab', Flab)₂, single-chain Fvs (scFV), FV, dsFV diabody and Fd fragments. The fragment can include multiple chains linked together, such as by disulfide bridges. An antibody fragment generally contains at least about 50 amino acids and typically at least 200 amino acids.

As used herein, an Fv antibody fragment is composed of one variable heavy domain (V_{H}) and one variable light domain linked by noncovalent interactions.

As used herein, a dsFV refers to an Fv with an engineered intermolecular disulfide bond, which stabilizes the V_{H}-V_{L} pair.

As used herein, an F(ab)₂ fragment is an antibody fragment that results from digestion of an immunoglobulin with pepsin at pH 4.0-4.5; it may be recombinantly produced.

As used herein, Fab fragments is an antibody fragment that results from digestion of an immunoglobulin with papain; it may be recombinantly produced.

As used herein, scFVs refer to antibody fragments that contain a variable light chain (V_{L}) and variable heavy chain (V_{H}) covalently connected by a polypeptide linker in any order. The linker is of a length such that the two variable domains are bridged without substantial interference. Preferred linkers are (Gly-Ser)ₙ residues with some Glu or Lys residues dispersed throughout to increase solubility.

As used herein, humanized antibodies refer to antibodies that are modified to include human sequences of amino acids so that administration to a human will not provoke an immune response. Methods for preparation of such antibodies are known. For example, the hybridoma that expresses the monoclonal antibody is altered by recombinant DNA techniques to express an antibody in which the amino acid composition of the non-variable regions is based on human antibodies. Computer programs have been designed to identify such regions.

As used herein, diabodies are dimeric scFV; diabodies typically have shorter peptide linkers than scFvs, and they preferentially dimerize.

As used herein, humanized antibodies refer to antibodies that are modified to include human sequences of amino acids so that administration to a human will not provoke an immune response. Methods for preparation of such antibodies are known. For example, the hybridoma that expresses the monoclonal antibody is altered by recombinant DNA techniques to express an antibody in which the amino acid composition of the non-variable regions is based on human antibodies. Computer programs have been designed to identify such regions.

As used herein, production by recombinant means by using recombinant DNA methods means the use of the well known methods of molecular biology for expressing proteins encoded by cloned DNA.

As used herein the term assessing is intended to include quantitative and qualitative determination in the sense of obtaining an absolute value for the activity of an MTSP, or a domain thereof, present in the sample, and also of obtaining an index, ratio, percentage, visual or other value indicative of the level of the activity. Assessment may be direct or indirect and the chemical species actually detected need not of course be the proteolysis product itself but may for example be a derivative thereof or some further substance.

As used herein, biological activity refers to the in vivo activities of a compound or physiological responses that result upon in vivo administration of a compound, composition or other mixture. Biological activity, thus, encompasses therapeutic effects and pharmaceutical activity of such compounds, compositions and mixtures. Biological activities may be observed in in vitro systems designed to test or use such activities. Thus, for purposes herein the biological activity of a luciferase is its oxygenase activity whereby, upon oxidation of a substrate, light is produced.

As used herein, a combination refers to any association between two or among more items.

As used herein, a composition refers to any mixture. It may be a solution, a suspension, liquid, powder, a paste, aqueous, non-aqueous or any combination thereof.

As used herein, a conjugate refers to the compounds provided herein that include one or more MTSPs, particularly single chain protease domains thereof, and one or more targeting agents. These conjugates include those produced by recombinant means as fusion proteins, those produced by chemical means, such as by chemical coupling, through, for example, coupling to sulfhydryl groups, and those produced by any other method whereby at least one MTSP, or a domain thereof, is linked, directly or indirectly via linker(s) to a targeting agent.

As used herein, a targeting agent, is any moiety, such as a protein or effective portion thereof, that provides specific binding of the conjugate to a cell surface receptor, which, preferably, internalizes the conjugate or MTSP portion thereof. A targeting agent may also be one that promotes or facilitates, for example, affinity isolation or purification of the conjugate; attachment of the conjugate to a surface; or detection of the conjugate or complexes containing the conjugate.

As used herein, an antibody conjugate refers to a conjugate in which the targeting agent is an antibody.

As used herein, humanized antibodies refer to antibodies that are modified to include human sequences of amino acids so that administration to a human will not provoke an immune response. Methods for preparation of such antibodies are known. For example, the hybridoma that expresses the monoclonal antibody is altered by recombinant DNA techniques to express an antibody in which the amino acid composition of the non-variable regions is based on human antibodies. Computer programs have been designed to identify such regions.

As used herein, derivative or analog of a molecule refers to a portion derived from or a modified version of the molecule.

As used herein, fluid refers to any composition that can flow. Fluids thus encompass compositions that are in the form of semi-solids, pastes, solutions, aqueous mixtures, gels, lotions, creams and other such compositions.

As used herein, an effective amount of a compound for treating a particular disease is an amount that is sufficient to ameliorate, or in some manner reduce the symptoms associated with the disease. Such amount may be administered as a single dosage or may be administered according to a regimen, whereby it is effective. The amount may cure the disease but, typically, is administered in order to ameliorate the symptoms of the disease. Repeated administration may be required to achieve the desired amelioration of symptoms.

As used herein equivalent, when referring to two sequences of nucleic acids means that the two sequences in question encode the same sequence of amino acids or equivalent proteins. When equivalent is used in referring to two proteins or peptides, it means that the two proteins or peptides have substantially the same amino acid sequence with only conservative amino acid substitutions (see, *e.g*., Table 1, above) that do not substantially alter the activity or function of the protein or peptide. When equivalent refers to a property, the property does not need to be present to the same extent [e.g., two peptides can exhibit different rates of the same type of enzymatic activity], but the activities are preferably substantially the same. Complementary, when referring to two nucleotide sequences, means that the two sequences of nucleotides are capable of hybridizing, preferably with less than 25%, more preferably with less than 15%, even more preferably with less than 5%, most preferably with no mismatches between opposed nucleotides. Preferably the two molecules will hybridize under conditions of high stringency.

As used herein, an agent that modulates the activity of a protein or expression of a gene or nucleic acid either decreases or increases or otherwise alters the activity of the protein or, in some manner up- or down-regulates or otherwise alters expression of the nucleic acid in a cell.

As used herein, inhibitor of an the activity of an MTSP encompasses any substances that prohibit or decrease production, post-translational modification(s), maturation, or membrane localization of the MTSP or any substances that interfere with or decrease the proteolytic efficacy of thereof, particular of a single chain form *in vitro.*

As used herein, a method for treating or preventing neoplastic disease means that any of the symptoms, such as the tumor, metastasis thereof, the vascularization of the tumors or other parameters by which the disease is characterized are reduced, ameliorated, prevented, placed in a state of remission, or maintained in a state of remission. It also means that the hallmarks of neoplastic disease and metastasis may be eliminated, reduced or prevented by the treatment. Non-limiting examples of the hallmarks include uncontrolled degradation of the basement membrane and proximal extracellular matrix, migration, division, and organization of the endothelial cells into new functioning capillaries, and the persistence of such functioning capillaries.

As used herein, operatively linked or operationally associated refers to the functional relationship of DNA with regulatory and effector sequences of nucleotides, such as promoters, enhancers, transcriptional and translational stop sites, and other signal sequences. For example, operative linkage of DNA to a promoter refers to the physical and functional relationship between the DNA and the promoter such that the transcription of such DNA is initiated from the promoter by an RNA polymerase that specifically recognizes, binds to and transcribes the DNA. In order to optimize expression and/or *in vitro* transcription, it may be necessary to remove, add or alter 5' untranslated portions of the clones to eliminate extra, potential inappropriate alternative translation initiation (*i.e.*, start) codons or other sequences that may interfere with or reduce expression, either at the level of transcription or translation. Alternatively, consensus ribosome binding sites (see, e.g., Kozak J. Biol. Chem. 266:19867-19870 (1991)) can be inserted immediately 5' of the start codon and may enhance expression. The desirability of (or need for) such modification may be empirically determined.

As used herein, pharmaceutically acceptable salts, esters or other derivatives of the conjugates include any salts, esters or derivatives that may be readily prepared by those of skill in this art using known methods for such derivatization and that produce compounds that may be administered to animals or humans without substantial toxic effects and that either are pharmaceutically active or are prodrugs.

As used herein, a prodrug is a compound that, upon in vivo administration, is metabolized or otherwise converted to the biologically, pharmaceutically or therapeutically active form of the compound. To produce a prodrug, the pharmaceutically active compound is modified such that the active compound will be regenerated by metabolic processes. The prodrug may be designed to alter the metabolic stability or the transport characteristics of a drug, to mask side effects or toxicity, to improve the flavor of a drug or to alter other characteristics or properties of a drug. By virtue of knowledge of pharmacodynamic processes and drug metabolism in vivo, those of skill in this art, once a pharmaceutically active compound is known, can design prodrugs of the compound (see, e.g., Nogrady (1985) Medicinal Chemistry A Biochemical Approach, Oxford University Press, New York, pages 388-392).

As used herein, a drug identified by the screening methods provided herein refers to any compound that is a candidate for use as a therapeutic or as lead compound for designed a therapeutic. Such compounds can be small molecules, including small organic molecules, peptides, peptide mimetics, antisense molecules, antibodies, fragments of antibodies, recombinant antibodies and other such compound which can serve as drug candidate or lead compound.

As used herein, production by recombinant means by using recombinant DNA methods means the use of the well known methods of molecular biology for expressing proteins encoded by cloned DNA.

As used herein, a promoter region or promoter element refers to a segment of DNA or RNA that controls transcription of the DNA or RNA to which it is operatively linked. The promoter region includes specific sequences that are sufficient for RNA polymerase recognition, binding and transcription initiation. This portion of the promoter region is referred to as the promoter. In addition, the promoter region includes sequences that modulate this recognition, binding and transcription initiation activity of RNA polymerase. These sequences may be *cis* acting or may be responsive to *trans* acting factors. Promoters, depending upon the nature of the regulation, may be constitutive or regulated. Exemplary promoters contemplated for use in prokaryotes include the bacteriophage T7 and T3 promoters.

As used herein, a receptor refers to a molecule that has an affinity for a given ligand. Receptors may be naturally-occurring or synthetic molecules. Receptors may also be referred to in the art as anti-ligands. As used herein, the receptor and anti-ligand are interchangeable. Receptors can be used in their unaltered state or as aggregates with other species. Receptors may be attached, covalently or noncovalently, or in physical contact with, to a binding member, either directly or indirectly via a specific binding substance or linker. Examples of receptors, include, but are not limited to: antibodies, cell membrane receptors surface receptors and internalizing receptors, monoclonal antibodies and antisera reactive with specific antigenic determinants [such as on viruses, cells, or other materials], drugs, polynucleotides, nucleic acids, peptides, cofactors, lectins, sugars, polysaccharides, cells, cellular membranes, and organelles.

Examples of receptors and applications using such receptors, include but are not restricted to:
a) enzymes: specific transport proteins or enzymes essential to survival of microorganisms, which could serve as targets for antibiotic [ligand] selection;
b) antibodies: identification of a ligand-binding site on the antibody molecule that combines with the epitope of an antigen of interest may be investigated; determination of a sequence that mimics an antigenic epitope may lead to the development of vaccines of which the immunogen is based on one or more of such sequences or lead to the development of related diagnostic agents or compounds useful in therapeutic treatments such as for auto-immune diseases
c) nucleic acids: identification of ligand, such as protein or RNA, binding sites;
d) catalytic polypeptides: polymers, preferably polypeptides, that are capable of promoting a chemical reaction involving the conversion of one or more reactants to one or more products; such polypeptides generally include a binding site specific for at least one reactant or reaction intermediate and an active functionality proximate to the binding site, in which the functionality is capable of chemically modifying the bound reactant [see, e.g., U.S. Patent No. 5,215,899];
e) hormone receptors: determination of the ligands that bind with high affinity to a receptor is useful in the development of hormone replacement therapies; for example, identification of ligands that bind to such receptors may lead to the development of drugs to control blood pressure; and
f) opiate receptors: determination of ligands that bind to the opiate receptors in the brain is useful in the development of less-addictive replacements for morphine and related drugs.

As used herein, sample refers to anything which may contain an analyte for which an analyte assay is desired. The sample may be a biological sample, such as a biological fluid or a biological tissue. Examples of biological fluids include urine, blood, plasma, serum, saliva, semen, stool, sputum, cerebral spinal fluid, tears, mucus, amniotic fluid or the like. Biological tissues are aggregate of cells, usually of a particular kind together with their intercellular substance that form one of the structural materials of a human, animal, plant, bacterial, fungal or viral structure, including connective, epithelium, muscle and nerve tissue. Examples of biological tissues also include organs, tumors, lymph nodes, arteries and individual cell(s).

As used herein: stringency of hybridization in determining percentage mismatch is as follows:
1) high stringency: 0.1 x SSPE, 0.1% SDS, 65°C
2) medium stringency: 0.2 x SSPE, 0.1 % SDS, 50°C
3) low stringency: 1.0 x SSPE, 0.1 % SDS, 50°C

Those of skill in this art know that the washing step selects for stable hybrids and also know the ingredients of SSPE (see, e.g., Sambrook, E.F. Fritsch, T. Maniatis, in: Molecular Cloning, A Laboratory Manual, Cold Spring Harbor Laboratory Press (1989), vol. 3, p. B.13, see, also, numerous catalogs that describe commonly used laboratory solutions). SSPE is pH 7.4 phophate-buffered 0.18 NaCl. Further, those of skill in the art recognize that the stability of hybrids is determined by Tₘ, which is a function of the sodium ion concentration and temperature (Tₘ = 81.5° C-16.6(log₁₀[Na⁺]) + 0.41(%G+C)-600/l)), so that the only parameters in the wash conditions critical to hybrid stability are sodium ion concentration in the SSPE (or SSC) and temperature.

It is understood that equivalent stringencies may be achieved using alternative buffers, salts and temperatures. By way of example and not limitation, procedures using conditions of low stringency are as follows (see also Shilo and Weinberg, Proc. Natl. Acad. Sci. USA, 78:6789-6792 (1981)): Filters containing DNA are pretreated for 6 hours at 40°C in a solution containing 35% formamide, 5X SSC, 50 mM Tris-HCl (pH 7.5), 5 mM EDTA, 0.1% PVP, 0.1% Ficoll, 1% BSA, and 500 µg/ml denatured salmon sperm DNA (10X SSC is 1.5 M sodium chloride, and 0.15 M sodium citrate, adjusted to a pH of 7).

Hybridizations are carried out in the same solution with the following modifications: 0.02% PVP, 0.02% Ficoll, 0.2% BSA, 100 µg/ml salmon sperm DNA, 10% (wt/vol) dextran sulfate, and 5-20 X 10⁶ cpm ³²P-labeled probe is used. Filters are incubated in hybridization mixture for 18-20 hours at 40°C, and then washed for 1.5 hours at 55°C in a solution containing 2X SSC, 25 mM Tris-HCl (pH 7.4), 5 mM EDTA, and 0.1 % SDS. The wash solution is replaced with fresh solution and incubated an additional 1.5 hours at 60°C. Filters are blotted dry and exposed for autoradiography. If necessary, filters are washed for a third time at 65-68°C and reexposed to film. Other conditions of low stringency which may be used are well known in the art (*e.g*., as employed for cross-species hybridizations).

By way of example and not way of limitation, procedures using conditions of moderate stringency is provided. For example, but not limited to, procedures using such conditions of moderate stringency are as follows: Filters containing DNA are pretreated for 6 hours at 55 °C in a solution containing 6X SSC, 5X Denhart's solution, 0.5% SDS and 100 µg/ml denatured salmon sperm DNA. Hybridizations are carried out in the same solution and 5-20 X 10⁶ cpm ³²P-labeled probe is used. Filters are incubated in hybridization mixture for 18-20 hours at 55°C, and then washed twice for 30 minutes at 60°C in a solution containing 1X SSC and 0.1% SDS. Filters are blotted dry and exposed for autoradiography. Other conditions of moderate stringency which may be used are well-known in the art. Washing of filters is done at 37°C for 1 hour in a solution containing 2X SSC, 0.1% SDS.

By way of example and not way of limitation, procedures using conditions of high stringency are as follows: Prehybridization of filters containing DNA is carried out for 8 hours to overnight at 65 °C in buffer composed of 6X SSC, 50 mM Tris-HCl (pH 7.5), 1 mM EDTA, 0.02% PVP, 0.02% Ficoll, 0.02% BSA, and 500 µg/ml denatured salmon sperm DNA. Filters are hybridized for 48 hours at 65 °C in prehybridization mixture containing 100 µg/ml denatured salmon sperm DNA and 5-20 X 10⁶ cpm of ³²P-labeled probe. Washing of filters is done at 37°C for 1 hour in a solution containing 2X SSC, 0.01 % PVP, 0.01% Ficoll, and 0.01% BSA. This is followed by a wash in 0.1 X SSC at 50°C for 45 minutes before autoradiography. Other conditions of high stringency which may be used are well known in the art.

The term substantially identical or homologous or similar varies with the context as understood by those skilled in the relevant art and generally means at least 70%, preferably means at least 80%, more preferably at least 90%, and most preferably at least 95% identity.

As used herein, substantially identical to a product means sufficiently similar so that the property of interest is sufficiently unchanged so that the substantially identical product can be used in place of the product.

As used herein, substantially pure means sufficiently homogeneous to appear free of readily detectable impurities as determined by standard methods of analysis, such as thin layer chromatography (TLC), gel electrophoresis and high performance liquid chromatography (HPLC), used by those of skill in the art to assess such purity, or sufficiently pure such that further purification would not detectably alter the physical and chemical properties, such as enzymatic and biological activities, of the substance. Methods for purification of the compounds to produce substantially chemically pure compounds are known to those of skill in the art. A substantially chemically pure compound may, however, be a mixture of stereoisomers or isomers. In such instances, further purification might increase the specific activity of the compound.

As used herein, target cell refers to a cell that expresses an MTSP *in vivo.*

As used herein, test substance refers to a chemically defined compound (*e.g*., organic molecules, inorganic molecules, organic/inorganic molecules, proteins, peptides, nucleic acids, oligonucleotides, lipids, polysaccharides, saccharides, or hybrids among these molecules such as glycoproteins, etc.) or mixtures of compounds (*e.g*., a library of test compounds, natural extracts or culture supernatants, etc.) whose effect on an MTSP, particularly a single chain form that includes the protease domain or a sufficient portion thereof for activity, as determined by *in vitro* method, such as the assays provided herein.

As used herein, the terms a therapeutic agent, therapeutic regimen, radioprotectant, chemotherapeutic mean conventional drugs and drug therapies, including vaccines, which are known to those skilled in the art. Radiotherapeutic agents are well known in the art.

As used herein, treatment means any manner in which the symptoms of a conditions, disorder or disease are ameliorated or otherwise beneficially altered. Treatment also encompasses any pharmaceutical use of the compositions herein.

As used herein, vector (or plasmid) refers to discrete elements that are used to introduce heterologous DNA into cells for either expression or replication thereof. The vectors typically remain episomal, but may be designed to effect integration of a gene or portion thereof into a chromosome of the genome. Also contemplated are vectors that are artificial chromosomes, such as yeast artificial chromosomes and mammalian artificial chromosomes. Selection and use of such vehicles are well known to those of skill in the art. An expression vector includes vectors capable of expressing DNA that is operatively linked with regulatory sequences, such as promoter regions, that are capable of effecting expression of such DNA fragments. Thus, an expression vector refers to a recombinant DNA or RNA construct, such as a plasmid, a phage, recombinant virus or other vector that, upon introduction into an appropriate host cell, results in expression of the cloned DNA. Appropriate expression vectors are well known to those of skill in the art and include those that are replicable in eukaryotic cells and/or prokaryotic cells and those that remain episomal or those which integrate into the host cell genome.

As used herein, protein binding sequence refers to a protein or peptide sequence that is capable of specific binding to other protein or peptide sequences generally, to a set of protein or peptide sequences or to a particular protein or peptide sequence.

As used herein, epitope tag refers to a short stretch of amino acid residues corresponding to an epitope to facilitate subsequent biochemical and immunological analysis of the epitope tagged protein or peptide. Epitope tagging is achieved by appending the sequence of the epitope tag to the protein-encoding sequence in an appropriate expression vector. Epitope tagged proteins can be affinity purified using highly specific antibodies raised against the tags.

As used herein, metal binding sequence refers to a protein or peptide sequence that is capable of specific binding to metal ions generally, to a set of metal ions or to a particular metal ion.

As used herein, a composition refers to a any mixture. It may be a solution, a suspension, liquid, powder, a paste, aqueous, non-aqueous or any combination thereof.

As used herein, a combination refers to any association between two or among more items.

As used herein, fluid refers to any composition that can flow. Fluids thus encompass compositions that are in the form of semi-solids, pastes, solutions, aqueous mixtures, gels, lotions, creams and other such compositions.

As used herein, a cellular extract refers to a preparation or fraction which is made from a lysed or disrupted cell.

As used herein, an agent is said to be randomly selected when the agent is chosen randomly without considering the specific sequences involved in the association of a protein alone or with its associated substrates, binding partners, etc. An example of randomly selected agents is the use a chemical library or a peptide combinatorial library, or a growth broth of an organism.

As used herein, an agent is the to be rationally selected or designed when the agent is chosen on a non-random basis which takes into account the sequence of the target site and/or its conformation in connection with the agent's action. As described in the Examples, there are proposed binding sites for serine protease and (catalytic) sites in the protein having SEQ ID NO:3 or SEQ ID NO:4. Agents can be rationally selected or rationally designed by utilizing the peptide sequences that make up these sites. For example, a rationally selected peptide agent can be a peptide whose amino acid sequence is identical to the ATP or calmodulin binding sites or domains.

For clarity of disclosure, and not by way of limitation, the detailed description is divided into the subsections that follow.

### B. MTSP PROTEINS, MUTEINS, DERIVATIVES AND ANALOGS THEREOF MTSPs

The MTSPs are a family of transmembrane serine proteases that are found in mammals and also other species that share a number of common structural features including: a proteolytic extracellular C-terminal domain; a transmembrane domain, with a hydrophobic domain near the N-terminus; a short cytoplasmic domain; and a variable length stem region containing modular domains. The proteolytic domains share sequence homology including conserved his, asp, and ser residues necessary for catalytic activity that are present in conserved motifs. The MTSPs are synthesized as zymogens, and activated to double chain forms by cleavage. It is shown herein that the single chain proteolytic domain can function *in vitro* and, hence is useful in *in vitro* assays for identifying agents that modulate the activity of members of this family. Also provided are family members designated MTSP3, MTSP4 and an MTSP6 variant.

The MTSP family is a target for therapeutic intervention and also some, may serve as diagnostic markers for tumor development, growth and/or progression. As discussed, the members of this family are involved in proteolytic processes that are implicated in tumor development, growth and/or progression. This implication is based upon their functions as proteolytic enzymes in processes related to ECM degradative pathways. In addition, their levels of expression or level of activation or their apparent activity resulting from substrate levels or alterations in substrates and levels thereof differs in tumor cells and non-tumor cells in the same tissue. Hence, protocols and treatments that alter their activity, such as their proteolytic acitivities and roles in signal transduction, and/or their expression, such as by contacting them with a compound that modulates their activity and/or expression, could impact tumor development, growth and/or progression. Also, in some instances, the level of activation and/or expression may be altered in tumors, such as lung carcinoma, colon adenocarcinoma and ovarian carcinoma.

The MTSP may serve as a diagnostic marker for tumors. It is shown herein, that MTSP3 and MTSP4 and the MTSP6 variant provided herein are expressed and/or activated in certain tumors: hence their activation or expression may serve as a diagnostic marker for tumor development, growth and/or progression. In other instances the MTSP protein can exhibit altered activity by virtue of a change in activity or expression of a co-factor therefor or a substrate therefor. In addition, in some instances, these MTSPS and/or variants thereof may be shed from cell surfaces. Detection of the shed MTSPS, particularly the extracellular domains, in body fluids, such as serum, blood, saliva, cerebral spinal fluid, synovial fluid and interstitial fluids, urine, sweat and other such fluids and secretions, may serve as a diagnostic tumor marker. In particular, detection of higher levels of such shed polypeptides in a subject compared to a subject known not to have any neoplastic disease or compared to earlier samples from the same subject, can be indicative of neoplastic disease in the subject.

Provided herein are isolated substantially pure single polypeptides that contain the protease domain of an MTSP as a single chain. The MTSPs contemplated herein are not expressed on endothelial cells, and, preferably, are expressed on tumor cells, typically at a level that differs from the level in which they are expressed in the non-tumor cell of the same type. Hence, for example, if the MTSP is expressed in an ovarian tumor cell, to be of interest herein with respect to ovarian cancer, it is expressed at the same level in non-tumor ovarian cells. MTSP protease domains include the single chain protease domains of MTSP1, MTSP3, MTSP4, MTSP6 and other such proteases, including, but are not limited to, corin, enterpeptidase, human airway trypsin-like protease (HAT), MTSP1, TMPRS2, and TMPRSS4.

Provided are the protease domains or proteins that include a portion of an MTSP that is the protease domain of any MTSP, particularly an MTSP1, MTSP3, MTSP4 and MTSP6. The protein can also include other non-MTSP sequences of amino acids, but will include the protease domain or a sufficient portion thereof to exhibit catalytic activity in any *in vitro* assay that assess such protease activity, such as any provided herein.

in specific aspects, the MTSP protease domains, and portions thereof, are from or based on animal MTSPS, including, but are not limited to, rodent, such as mouse and rat; fowl, such as chicken; ruminants, such as goats, cows, deer, sheep; ovine, such as pigs; and humans.

In particular, MTSP derivatives can be made by altering their sequences by substitutions, additions or deletions that provide for functionally equivalent molecules. Due to the degeneracy of nucleotide coding sequences, other nucleic sequences which encode substantially the same amino acid sequence as a *MTSP* gene can be used. These include but are not limited to nucleotide sequences comprising all or portions of *MTSP* genes that are altered by the substitution of different codons that encode the amino acid residue within the sequence, thus producing a silent change. Likewise, the MTSP derivatives include, but are not limited to, those containing, as a primary amino acid sequence, all or part of the amino acid sequence of MTSP, including altered sequences in which functionally equivalent amino acid residues are substituted for residues within the sequence resulting in a silent change. For example, one or more amino acid residues within the sequence can be substituted by another amino acid of a similar polarity which acts as a functional equivalent, resulting in a silent alteration. Substitutes for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid (see, *e.g.,* Table 1).

In a preferred embodiment, the substantially purified MTSP protease is encoded by a nucleic acid that hybridizes to the a nucleic acid molecule containing the protease domain encoded by the nucleotide sequence set forth in any of SEQ. ID Nos. 1, 3, 5, 7, 9 or 11 under at least moderate, generally high, stringency conditions, such that the protease domain encoding nucleic acid thereof hybridizes along its full length. In preferred embodiments the substantially purified MTSP protease is a single chain polypeptide that includes substantially the sequence of amino acids set forth in any SEQ ID Nos. 2, 4, 6, 8, 10 and 12 that encodes the protease domain, Specific sequences for the following human MTSPs and domains thereof are provided as follows: SEQ ID No. 3 MTSP3 nucleic acid sequence; SEQ ID No. 4 MTSP3 amino acid sequence; SEQ ID No. 5 MTSP4 nucleic acid encoding the protease domain; SEQ ID No. 6 MTSP4 amino acid sequence of the protease domain; SEQ ID No. 7 MTSP4-L nucleic acid sequence; SEQ ID No. 8 MTSP4-L amino acid sequence; SEQ ID No. 9 MTSP4-S nucleic acid sequence; SEQ ID No. 10 MTSP4-S amino acid sequence; SEQ ID No. 11 MTSP6 nucleic acid sequence; SEQ ID No. 12 MTSP6 amino acid sequence. SEQ ID No. 1 sets forth the nucleic acid sequence of the long form of MTSP1; SEQ ID No. 2 the encoded amino acid sequence; SEQ ID No. 49 sets forth the sequence of a protease domain of an MTSP1, and SEQ ID No. 50 the sequence of the encoded single chain protease domain thereof. Figures 1-3 depict the structural organization of the MTSP3, MTSP4 and MTSP6, respectively.

In particular, exemplary protease domains include at least a sufficient portion of sequences of amino acids set forth as amino acids 615-855 in SEQ ID No. 2 (encoded by nucleotides 1865-2587 in SEQ ID No. 1; see also SEQ ID Nos. 49 and 50) from MTSP1 (matriptase), amino acids 205-437 of SEQ ID NO. 4 from MTSP3, SEQ ID No. 6, which sets forth the protease domain of MTSP4, and amino acids 217-443 of SEQ ID No. 11 from MTSP6.

The isolated polypeptides contain the MTSP protease domain or a catalytically active portion thereof and do not contain additional MTSP. Hence isolated, substantially pure protease domains or catalytically active portion thereof in single chain form of MTSPs are provided. The protease domains may be included in a longer protein, but such longer protein is not the MTSP zymogen.

Thus, MTSP3 and MTSP4 proteins are provided. For these proteins, the domains, fragments, derivatives or analogs that are functionally active, *i.e.,* capable of exhibiting one or more functional activities associated with the MTSP protein, *e.g.,* serine protease activity, immunogenicity and antigenicity, are provided. As discussed above, the protease domains thereof are also provided.

### Exemplary MTSPs

The above discussion provides an overview and some details of the exemplified MTSPs. The following discussion provides additional details (see, also, EXAMPLES).

### MTSP1 (matriptase)

Matriptase is a trypsin-like serine protease with broad spectrum cleavage activity and two potential regulatory modules. It was named "matriptase" because its ability to degrade the extra-cellular matrix and its trypsin-like activity. When isolated from breast cancer cells (or T-47D cell conditioned medium), matriptase has been reported to be primarily in an uncomplexed form. Matriptase has been isolated from human milk; when isolated from human milk, matriptase was reported to be in one of two complexed forms, 95 kDa (the predominant form) and 110 kDa; uncomplexed matriptase was not detected. (Liu, et al., J. Biol. Chem. 274(26):18237-18242 (1999).) It has been proposed that matriptase exists as an uncomplexed protease when in its active state. In breast milk, matriptase has been reported to exist in complex with a fragment of hepatocyte growth factor inhibitor-1 (HAI-1), a Kuntz-type serine protease inhibitor having activity against trypsin-like serine proteases.

Ecotin and Ecotin M84R/M85R are macromolecular inhibitors of serine proteases of the chymotrypsin fold and inhibit ductal branching, morphogenesis and differentiation of the explanted ductal prostate. PC-3 is a cell line derived from prostate cancer epithelial cells. Ecotin and M84R/M85R ecotin were found to decrease tumor size and metastasis in PC-3 implanted nude mice.

Matriptase has been isolated and its encoding nucleic acids cloned from T-47D human breast cancer cell-conditioned medium (Lin et al. (1999) J. Biol. Chem. 274:18231-18236). Upon analysis of the cDNA, it was determined that the full length protease has 683 amino acids and contains three main structural regions: a serine protease domain near the carboxyl-terminal region, four tandem low-density lipoprotein receptor domains, and two tandem complement subcomponents C1r and C1s.

Studies to identify additional serine proteases made by cancer cells were done using PC-3 cells. A serine protease termed "MT-SP1 ", reported to be a transmembrane protease was cloned (Takeuchi et al. (1999) Proc. Natl. Acad. Sci. U.S.A. 96:11054-11061). It was subsequently found the originally identified matriptase sequence is included in the translated sequence of the cDNA that encodes MT-SP1. The matriptase cDNA was reported to be a partial MT-SP1 cDNA and to lack 516 of the coding nucleotides (Takeuchi, et al., J. Biol. Chem 275:26333-26342 (2000).) Since the reported matriptase encoding cDNA sequence encoded a possible initiating methionine, it was proposed that alternative splicing could yield a protein lacking the N-terminal region of MTSP1.

Matriptase and MT-SP1 demonstrate trypsin-like protease activity and are Type II transmembrane proteins with a common extracellular protease domain. Studies of substrate specificity of MT-SP1 reveal that protease-activated receptor 2 (PAR2) and single-chain urokinase-type plasminogen activator (sc-uPA) are macromolecular substrates of MT-SP1. PAR2 is functions in inflammation, cytoprotection and/or cell adhesion, while sc-uPa is functions in tumor cell invasion and metastasis.

An exemplary nucleotide sequences encoding a human MTSP1 is set forth in SEQ ID Nos 1 and 2 (see also SEQ ID Nos. 49 and 50 for the protease domain thereof). As previously noted SEQ ID No. 1 sets for an MTSP1-encoding nucleic acid sequence. This sequence is the longer version and includes the protease domain, which is common to both variants Nucleic acids encoding the MTSP that hybridizes to the nucleotide sequence set forth in SEQ ID No. 1 can be obtained by any method known in the art, *e.g,* by PCR amplification using synthetic primers that hybridizes to the 3' and 5' ends of the sequence and/or by cloning from a cDNA or genomic library using a PCR amplification product or an oligonucleotide specific for the gene sequence (*e.g.,* as described in Section C herein). Homologs (*e.g*., nucleic acids of the above-listed genes of species other than human) or other related sequences (*e.g*., paralogs) and muteins can be obtained by low, moderate or high stringency hybridization with all or a portion of the particular sequence provided as a probe using methods well known in the art for nucleic acid hybridization and cloning. '

Isolated single chain protease domains of MTSP1 proteins from animals are provided herein. As shown herein, the single chain protease domain is catalytically active and can be used in a variety of drug screening assays, particularly *in vitro* proteolytic assays. Exemplary MTSP protease domains are set forth as the amino acids (615-855 of SEQ ID No. 2) encoded by nucleotides 1865-2587 of SEQ ID No. 1 (see, also, SEQ ID Nos. 49 and 50). The MTSP1 single chain protease domain is catalytically active

### MTSP3

A nucleic acid that encodes a MTSP, designated MTSP3 is provided. In particular, the nucleic acid includes an open reading frame within the following sequence of nucleotides set forth in SEQ ID No. 3. In particular the protein is encoded by the open reading frame that begins at nucleotide 261 and ends at 1574.

Also included are substantially purified MTSP3 single chain protease domains. These are encoded by a nucleic acid that includes sequence encoding a protease domain that exhibits proteolytic activity and that hybridizes to a nucleic acid molecule having a nucleotide sequence set forth in SEQ ID No. 3, typically under moderate, generally under high stringency, conditions and most preferably along the full length of the protease domain. Splice variants are also contemplated herein.

The isolated nucleic acid fragment hybridizes to the nucleic acid having the nucleotide sequence set forth in SEQ ID No: 3 under high stringency conditions, and preferably comprises the sequence of nucleotides set forth in any of SEQ ID Nos. 3 or comprises a portion thereof that encodes a serine protease catalytic domain or comprises nucleic acid molecule that encodes the protein encoded by SEQ ID NO, 4.

The isolated nucleic acid fragment is DNA, including genomic or cDNA, or is RNA, or can include other components, such as protein nucleic acid. The isolated nucleic acid may include additional components, such as heterologous or native promoters, and other transcriptional and translational regulatory sequences, these genes may be linked to other genes, such as reporter genes or other indicator genes or genes that encode indicators.

Also provided is an isolated nucleic acid molecule that includes the sequence of molecules that is complementary to the nucleotide sequence encoding the MTSP or the portion thereof.

Hence provided herein are polypeptides that are encoded by such nucleic acid molecules. Included among those polypeptides are the MTSP3 protease domain or a polypeptide with conservative amino acid changes such that the specificity and protease activity remains substantially unchange. In particular, a substantially purified mammalian MTSP protein is provided that has a serine protease catalytic domain.

Also provided is a substantial purified protein comprising a sequence of amino acids that has at least 60%, more preferably at least about 90%, most preferably at least about 95%, identity to the MTSP3, wherein the percentage identity is determined using standard algorithms and gap penalties that maximize the percentage identity. The human MTSP3 protein is most preferred, although other mammalian MTSP3 proteins are contemplated.

### MTSP4

Among the proteins provided herein is MTSP4. MTSP4 is highly expressed in the liver, and is expressed in substantially lower levels in other tissues (see, EXAMPLES). It is also expressed in non-liver-derived tumors (see EXAMPLES), including Burkitt's lymphoma, colorectal adenocarcinoma

(SW480), lung carcinoma (A549), and in leukemic cells, indicating a role in one or more of tumor progression, tumor invasion, tumor growth and tumor metastases.

Included are substantially purified MTSP4 single chain protease domains. These are encoded by a nucleic acid that includes sequence encoding a protease domain that exhibits proteolytic activity and that hybridizes to a nucleic acid molecule having a nucleotide sequence set forth in SEQ ID Nos. 5, 7 and 9, typically under moderate, generally under high stringency, conditions and most preferably along the full length of the protease domain.

The isolated nucleic acid fragment may hybridizes to the nucleic acid having the nucleotide sequence set forth in SEQ ID No: 5, 7 or 9 under high stringency conditions, and preferably comprises the sequence of nucleotides set forth in any of SEQ ID Nos. 5, 7 or 9 comprises a portion thereof that encodes a serine protease catalytic domain or comprises nucleic acid molecule that encodes the protein encoded by SEQ ID NO, 6, 9 or 10..

The isolated nucleic acid fragment is DNA, including genomic or cDNA, or is RNA, or can include other components, such as protein nucleic acid. The isolated nucleic acid may include additional components, such as heterologous or native promoters, and other transcriptional and translational regulatory sequences, these genes may be linked to other genes, such as reporter genes or other indicator genes or genes that encode indicators.

Also provided is an isolated nucleic acid molecule that includes the sequence of molecules that is complementary to the nucleotide sequence encoding and MTSP4 or the portion thereof.

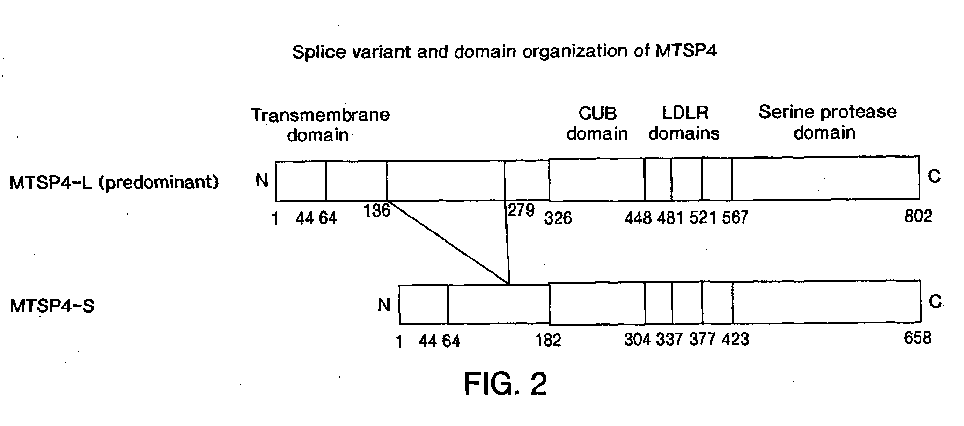

In particular nucleic acid molecules encoding two forms of MTSP4 are provide. The encoded proteins are multi-domain, type II membrane-type serine proteases and include a transmembrane domain at the N terminus followed by a CUB domain, 3 LDLR domains and a trypsin-like serine protease domain at the C terminus. The difference between the two forms, which are splice variants, is the absence in MTSP4-S of a 432-bp nucleotide sequence between the transmembrane and the CUB domains (see FIGURE 2; see, also SEQ ID Nos. 5-10).

Also provided is a nucleic acid that encodes the extracellular protease domain of an MTSP4 is provided. Both forms of MTSP4 exemplified herein include a protease domain in common (see SEQ ID Nos. 5 and 6).

In particular, the extracellular protease domain of the MTSP4 proteins is encoded by the open reading frame that begins at nucleotide 1 and ends at 708 (TGA) (SEQ ID No. 5. This open reading frame encodes a portion of the MSTP4 protein and includes the protease domain. Full length MSTP4 proteins (SEQ ID Nos. 7 and 9) include the above domain. The extracellular protease domain, as a single chain, and also an activated double chain, exhibit protease activity. The disulfide bonds that form that two chain form of MTSP forms are likely between Cys415 and Cys535 for MTSP4-S, and between Cys559 and Cys679 for MTSP4-L.

For use of the single chain protease domain thereof, it is of interest to replace the free Cys (i.e. Cys535 (Cys679)) in the protease domain with another amino acid, such as any amino acid that does not alter the function (such change is likely to be any amino acid).

### MTSP6

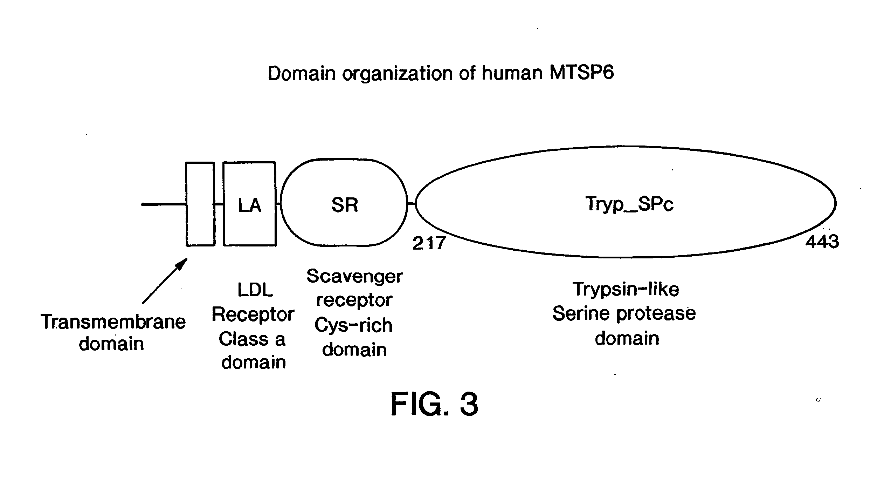

Nucleic acid and the encoded MTSP6 protein of an exemplary MTSP6 are also provided. The respective sequences are set forth in SEQ ID Nos. 11 and 12. The MTSP6 DNA and protein sequences were analyzed using DNA Strider (version 1.2). The ORF encoding the MTSP6 variant provided herein is composed of 1,362 bp, which translate into a 453-amino acid protein. MTSP6 is a multi-domain, type-II membrane-type serine protease containing a transmembrane domain (amino acids 48-68) at the N-terminus followed by a LDLRa domain (LDL receptor domain class a) (amino acids 72-108), a SR domain (Scavenger receptor Cys-rich domain)(amino acids 109-205), and a trypsin-like serine protease domain (amino acids 216-443) (see FIGURE 3)).

International PCT application No. WO 00/52044 describes MTSPs that resemble the MTSP6 provided herein. The polypeptide provided therein differs at single amino acid positions, such as 90 in SEQ ID No. 12 (Ala is replaced with a Thr), and significantly from the instant MTSP6 in that ten amino acids (amino acid nos. 46-55 in SEQ ID No. 12) are replaced with the eleven amino acids; phe glu val phe ser gln ser ser ser leu gly (SEQ ID No. 59) resulting in a protein that is one 454 amino acids long.

There are a few other amino acid sequence differences and a number of nucleic acid sequence differences. Significantly, there are substantial differences in the protease domain at amino acids 368-394 (368 ICNHRDVYGGIISPSMLCAGYLTGGVD----- 394; SEQ ID No. 12) are replaced at position 369-396 with animo acids: 369 DLOPQ--GRVRWHHLPLHALRGLPDGWRWN 396, where the differences from 368-394 (Seq ID No. 12) are indicated.

In addition, a second C-terminus truncated variant with an altered protease domain is identified in the PCT application. The variant is the same as the 454 variant through amino acid 261 thereof (corresponding to 160 of SEQ ID No. 12 herein), followed by 33 amino acids (see SEQ ID No. 60 herein) that differ by virtue of a frame shift.

### C. Tumor specificity and tissue expression profiles

Each MTSP has a characteristic tissue expression profile; the MTSPs in particular, although not exclusively expressed or activated in tumors, exhibit characteristic tumor tissue expression or activation profiles. In some instances, MTSPs may have different activity in a tumor cell from a non-tumor cell by virtue of a change in a substrate or cofactor thereof or other factor that would alter the apparent functional activity of the MTSP. Hence each can serve as a diagnostic marker for particular tumors, by virtue of a level of activity and/or expression or function in a subject (i.e. a mammal, particularly a human) with neoplastic disease, compared to a subject or subjects that do not have the neoplastic disease. In addition, detection of activity (and/or expression) in a particular tissue can be indicative of neoplastic disease. Shed MTSPs in body fluids can be indicative of neoplastic disease. Also, by virtue of the activity and/or expression profiles of each, they can serve as therapeutic targets, such as by administration of modulators of the activity thereof, or, as by administration of a prodrug specifically activated by one of the MTSPs.

### Tissue expression profiles

### MTSP3

The MTSP3 transcript was detected in lung carcinoma (LX-1), colon adenocarcinoma (CX-1), colon adenocarcinoma (GI-112) and ovarian carcinoma (GI-102). No apparent signal was detected in another form of lung carcinoma (GI-117), breast carcinoma (GI-101), pancreatic adenocarcinoma (GI-103) and prostatic adenocarcinoma (PC3).

### MTSP1 is expressed in breast cancers.

### MTSP4

The MTSP4 transcript, a DNA fragment encoding part of the LDL receptor domain and the protease domain was used to probe an RNA blot composed of 76 different human tissues (catalog number 7775-1; human multiple tissue expression (MTE) array; CLONTECH). As in the northern analysis of gel blot, a very strong signal was observed in the liver. Signals in other tissues were observed in (decreasing signal level): fetal liver > heart = kidney = adrenal gland = testis = fetal heart and kidney = skeletal muscle = bladder = placenta > brain = spinal cord = colon = stomach = spleen = lymph node = bone marrow = trachea = uterus = pancreas = salivary gland = mammary gland = lung. MTSP4 is also expressed less abundantly in several tumor cell lines including HeLa S3 = leukemia K-562 = Burkitt's lymphomas (Raji and Daudi) = colorectal adenocarcinoma (SW480) > lung carcinoma (A549) = leukemia MOLT-4 = leukemia HL-60. PCR of the MTSP4 transcript from cDNA libraries made from several human primary tumors xenografted in nude mice (human tumor multiple tissue cDNA panel, catalog number K1522-1, CLONTECH) was performed using MTSP4-specific primers. The MTSP4 transcript was detected in breast carcinoma (GI-101), lung carcinoma (LX-1), colon adenocarcinoma (GI-112) and pancreatic adenocarcinoma (GI-103). No apparent signal was detected in another form of lung carcinoma (GI-117), colon adenocarcinoma (CX-1), ovarian carcinoma (GI-102) and prostatic adenocarcinoma (PC3). The MTSP4 transcript was also detected in LNCaP and PC-3 prostate cancer cell lines as well as in HT-1080 human fibrosarcoma cell line.

### Gene expression profile of MTSP6 in normal and tumor tissues

To obtain information regarding the gene expression profile of the MTSP6 transcript, a 495 bp DNA fragment obtained from PCR reaction with primers Ch17-NSP-3 and NSP-4AS was used to probe an RNA blot composed of 76 different human tissues (catalog number 7775-1; human multiple tissue expression (MTE) array; CLONTECH). The strongest signal was observed in duodenum. Signal in other tissues were observed in (decreased signal level): Stomach > trachea = mammary gland = thyroid gland =salivary gland = pituitary gland = pancreas > kidney > lung > jejunum = ileum = ilocecum = appendix = fetal kidney > fetal lung. Very weak signals can also be detected in several other tissues.

MTSP6 is also expressed in several tumor cell lines including HeLa S3 > colorectal adenocarcinoma (SW480) > leukemia MOLT-4 > leukemia K-562. PCR analysis of the MTSP6 transcript from cDNA libraries made from several human primary tumors xenografted in nude mice (human tumor multiple tissue cDNA panel, catalog number K1522-1, CLONTECH) was performed using MTSP6-specific primers (Ch17-NSP-3 and Ch17-NSP2AS). The MTSP6 transcript was strongly detected in lung carcinoma (LX-1), moderately detected in pancreatic adenocarcinoma (GI-103), weakly detected in ovarian carcinoma (GI-102); and very weakly detected in colon adenocarcinoma (GI-112 and CX-1), breast carcinoma (GI-101), lung carcinoma (GI-117) and prostatic adenocarcinoma (PC3). The MTSP6 transcript was also detected in breast cancer cell line MDA-MB-231, prostate cancer cell line PC-3, but not in HT-1080 human fibrosarcoma cell line. MTSP6 is also expressed in mammary gland carcinoma cDNA (Clontech). MTSP6 is also over expressed in ovarian tumor cells.

### D. Identification and isolation of MTSP protein genes

The MTSP proteins, or domains thereof, can be obtained by methods well known in the art for protein purification and recombinant protein expression. Any method known to those of skill in the art for identification of nucleic acids that encode desired genes may be used. Any method available in the art can be used to obtain a full length (*i.e*., encompassing the entire coding region) cDNA or genomic DNA clone encoding an MTSP protein. In particular, the polymerase chain reaction (PCR) can be used to amplify a sequence identified as being differentially expressed in normal and tumor cells or tissues, *e.g.*, nucleic acids encoding an MTSP protein (SEQ. NOs: 1-12), in a genomic or cDNA library. Oligonucleotide primers that hybridize to sequences at the 3' and 5' termini of the identified sequences can be used as primers to amplify by PCR sequences from a nucleic acid sample (RNA or DNA), preferably a cDNA library, from an appropriate source (*e.g.*, tumor or cancer tissue).

PCR can be carried out, *e.g.*, by use of a Perkin-Elmer Cetus thermal cycler and Taq polymerase (Gene Amp^{™}). The DNA being amplified can include mRNA or cDNA or genomic DNA from any eukaryotic species. One can choose to synthesize several different degenerate primers, for use in the PCR reactions. It is also possible to vary the stringency of hybridization conditions used in priming the PCR reactions, to amplify nucleic acid homologs (*e.g.*, to obtain *MTSP protein* sequences from species other than humans or to obtain human sequences with homology to MTSP protein) by allowing for greater or lesser degrees of nucleotide sequence similarity between the known nucleotide sequence and the nucleic acid homolog being isolated. For cross species hybridization, low stringency conditions are preferred. For same species hybridization, moderately stringent conditions are preferred. After successful amplification of the nucleic acid containing all or a portion of the identified MTSP protein sequence or of a nucleic acid encoding all or a portion of an MTSP protein homolog, that segment may be molecularly cloned and sequenced, and used as a probe to isolate a complete cDNA or genomic clone. This, in turn, will permit the determination of the gene's complete nucleotide sequence, the analysis of its expression, and the production of its protein product for functional analysis. Once the nucleotide sequence is determined, an open reading frame encoding the MTSP protein gene protein product can be determined by any method well known in the art for determining open reading frames, for example, using publicly available computer programs for nucleotide sequence analysis. Once an open reading frame is defined, it is routine to determine the amino acid sequence of the protein encoded by the open reading frame. In this way, the nucleotide sequences of the entire MTSP protein genes as well as the amino acid sequences of MTSP protein proteins and analogs may be identified.

Any eukaryotic cell potentially can serve as the nucleic acid source for the molecular cloning of the MTSP protein gene. The nucleic acids can be isolated from vertebrate, mammalian, human, porcine, bovine, feline, avian, equine, canine, as well as additional primate sources, insects, plants, etc. The DNA may be obtained by standard procedures known in the art from cloned DNA (*e.g.*, a DNA "library"), by chemical synthesis, by cDNA cloning, or by the cloning of genomic DNA, or fragments thereof, purified from the desired cell (see, for example, Sambrook et al., 1989, Molecular Cloning, A Laboratory Manual, 2d Ed., Cold Spring Harbor Laboratory Press, Cold Spring Harbor, New York; Glover, D.M. (ed.), 1985, DNA Cloning: A Practical Approach, MRL Press, Ltd., Oxford, U.K. Vol. I, II). Clones derived from genomic DNA may contain regulatory and intron DNA regions in addition to coding regions; clones derived from cDNA will contain only exon sequences. Whatever the source, the gene should be molecularly cloned into a suitable vector for propagation of the gene.

In the molecular cloning of the gene from genomic DNA, DNA fragments are generated, some of which will encode the desired gene. The DNA may be cleaved at specific sites using various restriction enzymes. Alternatively, one may use DNAse in the presence of manganese to fragment the DNA, or the DNA can be physically sheared, for example, by sonication. The linear DNA fragments can then be separated according to size by standard techniques, including but not limited to, agarose and polyacrylamide gel electrophoresis and column chromatography.

Once the DNA fragments are generated, identification of the specific DNA fragment containing the desired gene may be accomplished in a number of ways. For example, a portion of the MTSP protein (of any species) gene (*e.g.*, a PCR amplification product obtained as described above or an oligonucleotide having a sequence of a portion of the known nucleotide sequence) or its specific RNA, or a fragment thereof be purified and labeled, and the generated DNA fragments may be screened by nucleic acid hybridization to the labeled probe (Benton and Davis, Science 196:180 (1977); Grunstein and Hogness, Proc. Natl. Acad. Sci. U.S.A. 72:3961 (1975)). Those DNA fragments with substantial homology to the probe will hybridize. It is also possible to identify the appropriate fragment by restriction enzyme digestion(s) and comparison of fragment sizes with those expected according to a known restriction map if such is available or by DNA sequence analysis and comparison to the known nucleotide sequence of MTSP protein. Further selection can be carried out on the basis of the properties of the gene. Alternatively, the presence of the gene may be detected by assays based on the physical, chemical, or immunological properties of its expressed product. For example, cDNA clones, or DNA clones which hybrid-select the proper mRNA, can be selected which produce a protein that, *e.g.*, has similar or identical electrophoretic migration, isolectric focusing behavior, proteolytic digestion maps, antigenic properties, serine protease activity. If an anti-MTSP protein antibody is available, the protein may be identified by binding of labeled antibody to the putatively MTSP protein synthesizing clones, in an ELISA (enzyme-linked immunosorbent assay)-type procedure.

Alternatives to isolating the MTSP protein genomic DNA include, but are not limited to, chemically synthesizing the gene sequence from a known sequence or making cDNA to the mRNA that encodes the MTSP protein. For example, RNA for cDNA cloning of the MTSP protein gene can be isolated from cells expressing the protein. The identified and isolated nucleic acids can then be inserted into an appropriate cloning vector. A large number of vector-host systems known in the art may be used. Possible vectors include, but are not limited to, plasmids or modified viruses, but the vector system must be compatible with the host cell used. Such vectors include, but are not limited to, bacteriophages such as lambda derivatives, or plasmids such as pBR322 or pUC plasmid derivatives or the Bluescript vector (Stratagene, La Jolla, CA). The insertion into a cloning vector can, for example, be accomplished by ligating the DNA fragment into a cloning vector which has complementary cohesive termini. I the complementary restriction sites used to fragment the DNA are not present in the cloning vector, the ends of the DNA molecules may be enzymatically modified. Alternatively, any site desired may be produced by ligating nucleotide sequences (linkers) onto the DNA termini; these ligated linkers may comprise specific chemically synthesized oligonucleotides encoding restriction endonuclease recognition sequences. In an alternative method, the cleaved vector and MTSP protein gene may be modified by homopolymeric tailing. Recombinant molecules can be introduced into host cells via transformation, transfection, infection, electroporation, etc., so that many copies of the gene sequence are generated.

In an alternative method, the desired gene may be identified and isolated after insertion into a suitable cloning vector in a "shot gun" approach. Enrichment for the desired gene, for example, by size fractionization, can be done before insertion into the cloning vector.

In specific embodiments, transformation of host cells with recombinant DNA molecules that incorporate the isolated MTSP protein gene, cDNA, or synthesized DNA sequence enables generation of multiple copies of the gene. Thus, the gene may be obtained in large quantities by growing transformants, isolating the recombinant DNA molecules from the transformants and, when necessary, retrieving the inserted gene from the isolated recombinant DNA.

### E. Vectors, plasmids and cells that contain nucleic acids encoding an MTSP protein or protease domain thereof and expression of MTSP proteins

### Vectors and cells

For recombinant expression of one or more of the MTSP proteins, the nucleic acid containing all or a portion of the nucleotide sequence encoding the MTSP protein can be inserted into an appropriate expression vector, *i.e*., a vector that contains the necessary elements for the transcription and translation of the inserted protein coding sequence. The necessary transcriptional and translational signals can also be supplied by the native promoter for MTSP genes, and/or their flanking regions.

Also provided are vectors that contain nucleic acid encoding the MTSPs. Cells containing the vectors are also provided. The cells include eukaryotic and prokaryotic cells, and the vectors are any suitable for use therein.

Prokaryotic and eukaryotic cells, including endothelial cells, containing the vectors are provided. Such cells include bacterial cells, yeast cells, fungal cells. plant cells, insect cells and animal cells. The cells are used to produce an MTSP protein or protease domain thereof by growing the above-described cells under conditions whereby the encoded MTSP protein or protease domain of the MTSP protein is expressed by the cell, and recovering the expressed protease domain protein. For purposes herein, the protease domain is preferably secreted into the medium.

In one embodiment, the vectors include a sequence of nucleotides that encodes a polypeptide that has protease activity and contains all or a portion of only the protease domain, or multiple copies thereof, of an MTSP protein are provided. Also provided are vectors that comprise a sequence of nucleotides that encodes the protease domain and additional portions of an MTSP protein up to and including a full length MTSP protein, as well as multiple copies thereof, are also provided. The vectors may selected for expression of the MTSP protein or protease domain thereof in the cell or such that the MTSP protein is expressed as a transmembrane protein. Alternatively, the vectors may include signals necessary for secretion of encoded proteins. When the protease domain is expressed the nucleic acid is preferably linked to a secretion signal, such as the *Saccharomyces cerevisiae a* mating factor signal sequence or a portion thereof.

A variety of host-vector systems may be used to express the protein coding sequence. These include but are not limited to mammalian cell systems infected with virus (*e.g.* vaccinia virus, adenovirus, etc.); insect cell systems infected with virus (*e.g.* baculovirus); microorganisms such as yeast containing yeast vectors; or bacteria transformed with bacteriophage, DNA, plasmid DNA, or cosmid DNA. The expression elements of vectors vary in their strengths and specificities. Depending on the host-vector system used, any one of a number of suitable transcription and translation elements may be used.

Any methods known to those of skill in the art for the insertion of DNA fragments into a vector may be used to construct expression vectors containing a chimeric gene containing appropriate transcriptional/translational control signals and protein coding sequences. These methods may include *in vitro* recombinant DNA and synthetic techniques and *in vivo* recombinants (genetic recombination). Expression of nucleic acid sequences encoding MTSP protein, or domains, derivatives, fragments or homologs thereof, may be regulated by a second nucleic acid sequence so that the genes or fragments thereof are expressed in a host transformed with the recombinant DNA molecule(s). For example, expression of the proteins may be controlled by any promoter/enhancer known in the art. In a specific embodiment, the promoter is not native to the genes for MTSP protein. Promoters which may be used include but are not limited to the SV40 early promoter (Bernoist and Chambon, Nature 290:304-310 (1981)), the promoter contained in the 3' long terminal repeat of Rous sarcoma virus (Yamamoto et al., Cell 22:787-797 (1980)), the herpes thymidine kinase promoter (Wagner et al., Proc. Natl. Acad. Sci. USA 78:1441-1445 (1981)), the regulatory sequences of the metallothionein gene (Brinster et al., Nature 296:39-42 (1982)); prokaryotic expression vectors such as the β-lactamase promoter (Villa-Kamaroff et al., Proc. Natl. Acad. Sci. USA 75:3727-3731 1978)) or the *tac* promoter (DeBoer et al., Proc. Natl. Acad. Sci. USA 80:21-25 (1983)); see also "Useful Proteins from Recombinant Bacteria": in Scientific American 242:79-94 (1980)); plant expression vectors containing the nopaline synthetase promoter (Herrar-Estrella et al., Nature 303:209-213 (1984)) or the cauliflower mosaic virus 35S RNA promoter (Garder et al., Nucleic Acids Res. 9:2871 (1981)), and the promoter of the photosynthetic enzyme ribulose bisphosphate carboxylase (Herrera-Estrella et al., Nature 310:115-120 (1984)); promoter elements from yeast and other fungi such as the Gal4 promoter, the alcohol dehydrogenase promoter, the phosphoglycerol kinase promoter, the alkaline phosphatase promoter, and the following animal transcriptional control regions that exhibit tissue specificity and have been used in transgenic animals: elastase I gene control region which is active in pancreatic acinar cells (Swift et al., Cell 38:639-646 (1984); Ornitz et al., Cold Spring Harbor Symp. Quant. Biol. 50:399-409 (1986); MacDonald, Hepatology 7:425-515 (1987)); insulin gene control region which is active in pancreatic beta cells (Hanahan et al., Nature 315:115-122 (1985)), immunoglobulin gene control region which is active in lymphoid cells (Grosschedl et al., Cell 38:647-658 (1984); Adams et al., Nature 318:533-538 (1985); Alexander et al., Mol. Cell Biol. 7:1436-1444 (1987)), mouse mammary tumor virus control region which is active in testicular, breast, lymphoid and mast cells (Leder et al., Cell 45:485-495 (1986)), albumin gene control region which is active in liver (Pinckert et al., Genes and Devel. 1:268-276 (1987)), alpha-fetoprotein gene control region which is active in liver (Krumlauf et al., Mol. Cell. Biol. 5:1639-1648 (1985); Hammer et al., Science 235:53-58 1987)), alpha-1 antitrypsin gene control region which is active in liver (Kelsey et al., Genes and Devel. 1:161-171 (1987)), beta globin gene control region which is active in myeloid cells (Mogram et al., Nature 315:338-340 (1985); Kollias et al., Cell 46:89-94 (1986)), myelin basic protein gene control region which is active in oligodendrocyte cells of the brain (Readhead et al., Cell 48:703-712 (1987)), myosin light chain-2 gene control region which is active in skeletal muscle (Sani, Nature 314:283-286 (1985)), and gonadotrophic releasing hormone gene control region which is active in gonadotrophs of the hypothalamus (Mason et al., Science 234:1372-1378 (1986)).

In a specific embodiment, a vector is used that contains a promoter operably linked to nucleic acids encoding an MTSP domain, fragment, one or more origins of replication, and optionally, one or more selectable markers (*e.g.*, an antibiotic resistance gene). Expression vectors containing the coding sequences portions of an MTSP protein, is made, for example, by subcloning the coding portions into the EcoRI restriction site of each of the three pGEX vectors (glutathione S-transferase expression vectors (Smith and Johnson, Gene 7:31-40 (1988)). This allows for the expression of products in the correct reading frame. Preferred vectors and systems for expression of the protease domains of the MTSP proteins are well known *Pichia* vectors (available, for example, from Invitrogen, San Diego, CA), particularly those designed for secretion of the encoded proteins. One exemplary vector is described in the EXAMPLES.

Plasmids for transformation of E. coli cells, include, for example, the pET expression vectors (see, U.S patent 4,952,496; available from NOVAGEN, Madison, WI; see, also literature published by Novagen describing the system). Such plasmids include pET 11 a, which contains the T7lac promoter, T7 terminator, the inducible E. coli lac operator, and the lac repressor gene; pET 12a-c, which contains the T7 promoter, T7 terminator, and the E. coli ompT secretion signal; and pET 15b and pET19b (NOVAGEN, Madison, WI), which contain a His-Tag^{™} leader sequence for use in purification with a His column and a thrombin cleavage site that permits cleavage following purification over the column; the T7-lac promoter region and the T7 terminator.

The vectors are introduced into host cells, such as *Pichia* cells and bacterial cells, such as *E. coli,* and the proteins expressed therein. Preferred *Pichia* strains, include, for example, GS115. Preferred bacterial hosts contain chromosomal copies of DNA encoding T7 RNA polymerase operably linked to an inducible promoter, such as the lacUV promoter (see, U.S. Patent No. 4,952,496). Such hosts include, but are not limited to, the lysogenic E. coli strain BL21 (DE3).

### Expression and production of proteins

The MTSP domains, derivatives and analogs be produced by various methods known in the art. For example, once a recombinant cell expressing an MTSP protein, or a domain, fragment or derivative thereof, is identified, the individual gene product can be isolated and analyzed. This is achieved by assays based on the physical and/or functional properties of the protein, including, but not limited to, radioactive labeling of the product followed by analysis by gel electrophoresis, immunoassay, cross-linking to marker-labeled product. The MTSP protein proteins may be isolated and purified by standard methods known in the art (either from natural sources or recombinant host cells expressing the complexes or proteins), including but not restricted to column chromatography (*e.g.*, ion exchange, affinity, gel exclusion, reversed-phase high pressure, fast protein liquid, etc.), differential centrifugation, differential solubility, or by any other standard technique used for the purification of proteins. Functional properties may be evaluated using any suitable assay known in the art.

Alternatively, once an MTSP protein or its domain or derivative is identified, the amino acid sequence of the protein can be deduced from the nucleotide sequence of the gene which encodes it. As a result, the protein or its domain or derivative can be synthesized by standard chemical methods known in the art (*e.g.* see Hunkapiller et al, Nature 310:105-111 (1984)).

Manipulations of MTSP protein sequences may be made at the protein level. Also contemplated herein are MTSP protein proteins, domains thereof, derivatives or analogs or fragments thereof, which are differentially modified during or after translation, *e.g.*, by glycosylation, acetylation, phosphorylation, amidation, derivatization by known protecting/blocking groups, proteolytic cleavage, linkage to an antibody molecule or other cellular ligand, etc. Any of numerous chemical modifications may be carried out by known techniques, including but not limited to specific chemical cleavage by cyanogen bromide, trypsin, chymotrypsin, papain, V8 protease, NaBH₄, acetylation, formylation, oxidation, reduction, metabolic synthesis in the presence of tunicamycin, etc.

In addition, domains, analogs and derivatives of an MTSP protein can be chemically synthesized. For example, a peptide corresponding to a portion of an MTSP protein, which includes the desired domain or which mediates the desired activity *in vitro* can be synthesized by use of a peptide synthesizer. Furthermore, if desired, nonclassical amino acids or chemical amino acid analogs can be introduced as a substitution or addition into the MTSP protein sequence. Non-classical amino acids include but are not limited to the D-isomers of the common amino acids, a-amino isobutyric acid, 4-aminobutyric acid, Abu, 2-aminobutyric acid, ε-Abu, e-Ahx, 6-amino hexanoic acid, Aib, 2-amino isobutyric acid, 3-amino propionoic acid, ornithine, norleucine, norvaline, hydroxyproline, sarcosine, citrulline, cysteic acid, t-butylglycine, t-butylalanine, phenylglycine, cyclohexylalanine, β-alanine, fluoro-amino acids, designer amino acids such as β-methyl amino acids, Ca-methyl amino acids, Na-methyl amino acids, and amino acid analogs in general. Furthermore, the amino acid can be D (dextrorotary) or L (levorotary).

In cases where natural products are suspected of being mutant or are isolated from new species, the amino acid sequence of the MTSP protein isolated from the natural source, as well as those expressed *in vitro,* or from synthesized expression vectors *in vivo* or *in vitro,* can be determined from analysis of the DNA sequence, or alternatively, by direct sequencing of the isolated protein. Such analysis may be performed by manual sequencing or through use of an automated amino acid sequenator.

### Modifications

A variety of modification of the MTSP proteins and domains are contemplated herein. An MTSP-encoding nucleic acid molecule modified by any of numerous strategies known in the art (Sambrook et al., 1990, Molecular Cloning, A Laboratory Manual, 2d ed., Cold Spring Harbor Laboratory, Cold Spring Harbor, New York). The sequences can be cleaved at appropriate sites with restriction endonuclease(s), followed by further enzymatic modification if desired, isolated, and ligated *in vitro.* In the production of the gene encoding a domain, derivative or analog of MTSP, care should be taken to ensure that the modified gene retains the original translational reading frame, uninterrupted by translational stop signals, in the gene region where the desired activity is encoded.

Additionally, the MTSP-encoding nucleic acid molecules can be mutated *in vitro* or *in vivo,* to create and/or destroy translation, initiation, and/or termination sequences, or to create variations in coding regions and/or form new restriction endonuclease sites or destroy pre-existing ones, to facilitate further *in vitro* modification. Also, as described herein muteins with primary sequence alterations, such as replacements of Cys residues and elimination of glycosylation sites are contemplated. Such mutations may be effected by any technique for mutagenesis known in the art, including, but not limited to, chemical mutagenesis and *in vitro* site-directed mutagenesis (Hutchinson et al., J. Biol. Chem. 253:6551-6558 (1978)), use of TAB® linkers (Pharmacia). For example, an MTSP protein or domain thereof is modified to include a fluorescent label. In other examples, the MTSP protein is modified to have a heterofunctional reagent, such heterofunctional reagents can be used to crosslink the members of the complex.

The MTSP proteins may be isolated and purified by standard methods known in the art (either from natural sources or recombinant host cells expressing the complexes or proteins), including but not restricted to column chromatography (*e.g*., ion exchange, affinity, gel exclusion, reversed-phase high pressure, fast protein liquid, etc.), differential centrifugation, differential solubility, or by any other standard technique used for the purification of proteins. Functional properties may be evaluated using any suitable assay known in the art.

Alternatively, once a MTSP or its domain or derivative is identified, the amino acid sequence of the protein can be deduced from the nucleotide sequence of the gene which encodes it. As a result, the protein or its domain or derivative can be synthesized by standard chemical methods known in the art (*e.g.,* see Hunkapiller et al, Nature, 310:105-111 (1984)).

Manipulations of MTSP sequences may be made at the protein level. MTSP domains, derivatives or analogs or fragments, which are differentially modified during or after translation, *e.g.*, by glycosylation, acetylation, phosphorylation, amidation, derivatization by known protecting/blocking groups, proteolytic cleavage, linkage to an antibody molecule and other cellular ligand, are contemplated herein. Any of numerous chemical modifications may be carried out by known techniques, including but not limited to specific chemical cleavage by cyanogen bromide, trypsin, chymotrypsin, papain, V8 protease, NaBH₄, acetylation, formylation, oxidation, reduction, metabolic synthesis in the presence of tunicamycin, etc.

In addition, domains, analogs and derivatives of a MTSP can be chemically synthesized. For example, a peptide corresponding to a portion of a MTSP, which comprises the desired domain or which mediates the desired activity *in vitro* can be synthesized by use of a peptide synthesizer.

Furthermore, if desired, nonclassical amino acids or chemical amino acid analogs can be introduced as a substitution or addition into the MTSP sequence. Non-classical amino acids include but are not limited to the D-isomers of the common amino acids, a-amino isobutyric acid, 4-aminobutyric acid, Abu, 2-aminobutyric acid, ε-Abu, e-Ahx, 6-amino hexanoic acid, Aib, 2-amino isobutyric acid, 3-amino propionoic acid, ornithine, norleucine, norvaline, hydroxyproline, sarcosine, citrulline, cysteic acid, t-butylglycine, t-butylalanine, phenylglycine, cyclohexylalanine, β-alanine, fluoro-amino acids, designer amino acids such as β-methyl amino acids, Ca-methyl amino acids, Na-methyl amino acids, and amino acid analogs in general. Furthermore, the amino acid can be D (dextrorotary) or L (levorotary).

### F. SCREENING METHODS

The single chain protease domains, as shown herein, can be used in a variety of methods to identify compounds that modulate the activity thereof. For MTSPs that exhibit higher activity or expression in tumor cells, compounds that inhibit the proteolytic activity are of particular interest. For any MTSPs that are active at lower levels in tumor cells, compounds or agents that enhance the activity are potentially of interest. In all instances the identified compounds will include agents that are candidate cancer treatments.

Several types of assays are exemplified and described herein. It is understood that the protease domains may be used in other assays. It is shown here, however, that the single chain protease domains exhibit catalytic activity. As such they are ideal for *in vitro* screening assays.

They may also be used in binding assays.

The MTSP3, MTSP4 and MTSP6 single chain protease domains are contemplated for use in any screening assay known to those of skill in the art, including those provided herein. Hence the following description, if directed to proteolytic assays is intended to apply to use of a single chain protease domain or a catalytically active portion thereof of any MTSP, including an MTSP3, MTSP4 or an MTSP6. Other assays, such as binding assays are provided herein, particularly for use with an MTSP3, MTSP4 or MTSP6, including any variants, such as splice variants thereof. MTSP3 and MTSP4 are of most interest in such assays.

### 1. Catalytic Assays for identification of agents that modulate the protease activity of an MTSP protein

Methods for identifying a modulator of the catalytic activity of an MTSP, single chain protease domain or catalytically active portion thereof, are provided herein. The methods can be practiced by: a) contacting the MTSP, single-chain domain, with a substrate of the MTSP in the presence of a test substance, and detecting the proteolysis of the substrate, whereby the activity of the MTSP is assessed, and comparing the activity to a control. For example, the control can be the activity of the MTSP assessed by contacting an MTSP single-chain domain, with a substrate of the MTSP, and detecting the proteolysis of the substrate, whereby the activity of the MTSP is assessed. The results in the presence and absence of the test compounds are compared. A difference in the activity indicates that the test substance modulates the activity of the MTSP.

in one embodiment a plurality of the test substances are screened simultaneously in the above screening method. In another embodiment, the MTSP is isolated from a target cell as a means for then identifying agents that are potentially specific for the target cell.

In still another embodiment, The test substance is a therapeutic compound, and whereby a difference of the MTSP activity measured in the presence and in the absence of the test substance indicates that the target cell responds to the therapeutic compound.

One method include the steps of (a) contacting the MTSP protease domain thereof with one or a plurality of test compounds under conditions conducive to interaction between the ligand and the compounds; and (b) identifying one or more compounds in the plurality that specifically binds to the ligand.

Another method provided herein includes the steps of a) contacting an MTSP protease domain thereof with a substrate of the MTSP protein, and detecting the proteolysis of the substrate, whereby the activity of the MTSP protein is assessed; b) contacting the MTSP protein with a substrate of the MTSP protein in the presence of a test substance, and detecting the proteolysis of the substrate, whereby the activity of the MTSP protein is assessed; and c) comparing the activity of the MTSP protein assessed in steps a) and b), whereby the activity measured in step a) differs from the activity measured in step b) indicates that the test substance modulates the activity of the MTSP protein.

In another embodiment, a plurality of the test substances are screened simultaneously. In comparing the activity of an MTSP protein in the presence and absence of a test substance to assess whether the test substance is a modulator of the MTSP protein, it is unnecessary to assay the activity in parallel, although such parallel measurement is preferred. It is possible to measure the activity of the MTSP protein at one time point and compare the measured activity to a historical value of the activity of the MTSP protein.

For instance, one can measure the activity of the MTSP protein in the presence of a test substance and compare with historical value of the activity of the MTSP protein measured previously in the absence of the test substance, and *vice versa.* This can be accomplished, for example, by providing the activity of the MTSP protein on an insert or pamphlet provided with a kit for conducting the assay.

Methods for selecting substrates for a particular MTSP are described in the EXAMPLES, and particular proteolytic assays are exemplified.

Combinations and kits containing the combinations optionally including instructions for performing the assays are provided. The combinations include an MTSP protein and a substrate of the MTSP protein to be assayed; and, optionally reagents for detecting proteolysis of the substrate. The substrates, which are typically proteins subject to proteolysis by a particular MTSP protein, can be identified empirically by testing the ability of the MTSP protein to cleave the test substrate. Substrates that are cleaved most effectively (i.e., at the lowest concentrations and/or fastest rate or under desirable conditions), are identified.

### 2. Binding assays

Also provided herein are methods for identification and isolation of agents, particularly compounds that bind to MTSPs. The assays are designed to identify agents that bind to the zymogen form, the single chain isolated protease domain (or a protein, other than an MTSP protein, that contains the protease domain of an MTSP protein), and to the activated form, including the activated form derived from the full length zymogen or from an extended protease domain. The identified compounds are candidates or leads for identification of compounds for treatments of tumors and other disorders and diseases involving aberrant angiogenesis. The MTSP proteins used in the methods include any MTSP protein as defined herein, and preferably use MTSP single chain domain or proteolytically active portion thereof.

A variety of methods are provided herein. These methods may be performed in solution or in solid phase reactions in which the MTSP protein(s) or protease domain(s) thereof are linked, either directly or indirectly via a linker, to a solid support. Screening assays are described in the Examples, and these assays have been used to identify candidate compounds.

For purposes herein, all binding assays described above are provided for MTSP3, MTSP4 and MTSP6. For MTSP1 (including any variant thereof) and thereof) and other such proteases, binding assays that employ the isolated single chain protease domain or a protein containing such domain (other than the MTSP from which the protease is derived) are provided.

Methods for identifying an agent, such as a compound, that specifically binds to an MTSP single chain protease domain or an MTSP, such as an MTSP3, MTSP4 or an MTSP6, are provided herein. The method can be practiced by (a) contacting the MTSP with one or a plurality of test agents under conditions conducive to binding between the MTSP and an agent; and (b) identifying one or more agents within the plurality that specifically binds to the MTSP.

For example, in practicing such methods the MTSP polypeptide is mixed with a potential binding partner or an extract or fraction of a cell under conditions that allow the association of potential binding partners with the polypeptide. After mixing, peptides, polypeptides, proteins or other molecules that have become associated with an MTSP are separated from the mixture. The binding partner that bound to the MTSP can then be removed and further analyzed. To identify and isolate a binding partner, the entire protein, for instance the entire disclosed protein of SEQ ID Nos. 6, 8 10 or 12 can be used. Alternatively, a fragment of the protein can be used.

A variety of methods can be used to obtain cell extracts. Cells can be disrupted using either physical or chemical disruption methods. Examples of physical disruption methods include, but are not limited to, sonication and mechanical shearing. Examples of chemical lysis methods include, but are not limited to, detergent lysis and enzyme lysis. A skilled artisan can readily adapt methods for preparing cellular extracts in order to obtain extracts for use in the present methods.

Once an extract of a cell is prepared, the extract is mixed with the MTSP under conditions in which association of the protein with the binding partner can occur. A variety of conditions can be used, the most preferred being conditions that closely resemble conditions found in the cytoplasm of a human cell. Features such as osmolarity, pH, temperature, and the concentration of cellular extract used, can be varied to optimize the association of the protein with the binding partner.

After mixing under appropriate conditions, the bound complex is separated from the mixture. A variety of techniques can be used to separate the mixture. For example, antibodies specific to an MTSP can be used to immunoprecipitate the binding partner complex. Alternatively, standard chemical separation techniques such as chromatography and density/sediment centrifugation can be used.

After removing the non-associated cellular constituents in the extract, the binding partner can be dissociated from the complex using conventional methods. For example, dissociation can be accomplished by altering the salt concentration or pH of the mixture.

To aid in separating associated binding partner pairs from the mixed extract, the MTSP can be immobilized on a solid support. For example, the protein can be attached to a nitrocellulose matrix or acrylic beads. Attachment of the protein or a fragment thereof to a solid support aids in separating peptide/binding partner pairs from other constituents found in the extract. The identified binding partners can be either a single protein or a complex made up of two or more proteins.

Alternatively, the nucleic acid molecules encoding the single chain proteases can be used in a yeast two-hybrid system. The yeast two-hybrid system has been used to identify other protein partner pairs and can readily be adapted to employ the nucleic acid molecules herein described.

Another *in vitro* binding assay, particularly for an MTSP3, MTSP4 or an MTSP6 uses a mixture of a polypeptide that contains at least the catalytic domain of one of these proteins and one or more candidate binding targets or substrates. After incubating the mixture under appropriate conditions, one determines whether the MTSP or a polypeptide fragment thereof containing the catalytic domain binds with the candidate substrate. For cell-free binding assays, one of the components includes or is coupled to a detectable label. The label may provide for direct detection, such as radioactivity, luminescence, optical or electron density, *etc*., or indirect detection such as an epitope tag, an enzyme, *etc*. A variety of methods may be employed to detect the label depending on the nature of the label and other assay components. For example, the label may be detected bound to the solid substrate or a portion of the bound complex containing the label may be separated from the solid substrate, and the label thereafter detected.

### 3. Detection of signal transduction

The cell surface location of the MTSPs suggests a role for some or all of these proteins in signal transduction. Assays for assessing signal transduction are well known to those of skill in the art, and may be adapted for use with the MTSP protein.

Assays for identifying agents that effect or alter signal transduction mediated by an MTSP, particularly the full length or a sufficient portion to anchor the extracellular domain or a function portion thereof of an MTSP on the surface of a cell are provided. Such assays, include, for example, transcription based assays in which modulation of a transduced signal is assessed by detecting an effect on an expression from a reporter gene (see, *e.g.*, U.S. Patent No. 5,436,128).

### 4. Methods for identifying Agents that Modulate the Expression a Nucleic Acid Encoding an MTSP, particularly an MTSP3, MTSP4 or MTSP6

Methods for identifying agents that modulate the expression of a nucleic acid encoding an MTSP, particularly an MTSP3, MTSP4 or MTSP are exemplified. Such assays use any available means of monitoring for changes in the expression level of the nucleic acids encoding an MTSP, such as MTSP3 or MTSP4.

In one assay format, cell lines that contain reporter gene fusions between the open reading frame of MTSP3, MTSP4 or MTSP6 or a domain thereof, particularly the protease domain and any assayable fusion partner may be prepared. Numerous assayable fusion partners are known and readily available including the firefly luciferase gene and the gene encoding chloramphenicol acetyltransferase (Alam et al., Anal. Biochem. 188: 245-54 (1990)). Cell lines containing the reporter gene fusions are then exposed to the agent to be tested under appropriate conditions and time. Differential expression of the reporter gene between samples exposed to the agent and control samples identifies agents which modulate the expression of a nucleic acid encoding an MTSP3, MTSP4 or MTSP6.

Additional assay formats may be used to monitor the ability of the agent to modulate the expression of a nucleic acid encoding an MTSP3, MTSP4 or MTSP6. For instance, mRNA expression may be monitored directly by hybridization to the nucleic acids. Cell lines are exposed to the agent to be tested under appropriate conditions and time and total RNA or mRNA is isolated by standard procedures (see, *e.g.*, Sambrook et al. (1989) MOLECULAR CLONING: A LABORATORY MANUAL, 2nd Ed. Cold Spring Harbor Laboratory Press). Probes to detect differences in RNA expression levels between cells exposed to the agent and control cells may be prepared from the nucleic acids. It is preferable, but not necessary, to design probes which hybridize only with target nucleic acids under conditions of high stringency. Only highly complementary nucleic acid hybrids form under conditions of high stringency. Accordingly, the stringency of the assay conditions determines the amount of complementarity which should exist between two nucleic acid strands in order to form a hybrid. Stringency should be chosen to maximize the difference in stability between the probe:target hybrid and potential probe:non-target hybrids.

Probes may be designed from the nucleic acids through methods known in the art. For instance, the G + C content of the probe and the probe length can affect probe binding to its target sequence. Methods to optimize probe specificity are commonly available (see, *e.g.*, Sambrook et al. (1989) MOLECULAR CLONING: A LABORATORY MANUAL, 2nd Ed. Cold Spring Harbor Laboratory Press); and Ausubel et al. (1995) CURRENT PROTOCOLS IN MOLECULAR BIOLOGY, Greene Publishing Co., NY).

Hybridization conditions are modified using known methods (see, *e.g.*, Sambrook et al. (1989) MOLECULAR CLONING: A LABORATORY MANUAL, 2nd Ed. Cold Spring Harbor Laboratory Press); and Ausubel et a/. (1995) CURRENT PROTOCOLS IN MOLECULAR BIOLOGY, Greene Publishing Co., NY), as required for each probe. Hybridization of total cellular RNA or RNA enriched for polyA RNA can be accomplished in any available format. For instance, total cellular RNA or RNA enriched for polyA RNA can be affixed to a solid support, and the solid support exposed to at least one probe .comprising at least one, or part of one of the nucleic acid molecules under conditions in which the probe will specifically hybridize. Alternatively, nucleic acid fragments comprising at least one, or part of one of the sequences can be affixed to a solid support, such as a porous glass wafer. The glass wafer can then be exposed to total cellular RNA or polyA RNA from a sample under conditions in which the affixed sequences will specifically hybridize. Such glass wafers and hybridization methods are widely available, for example, those disclosed by Beattie (WO 95/11755). By examining for the ability of a given probe to specifically hybridize to an RNA sample from an untreated cell population and from a cell population exposed to the agent, agents which up or down regulate the expression of a nucleic acid encoding the protein having the sequence of SEQ ID NO:3 or SEQ ID NO:4 are identified.

### 5. Methods for Identifying Agents that Modulate at Least One Activity of an MTPS, such as MTSP3, MTSP4 or MTSP6

Methods for identifying agents that modulate at least one activity of a an MTSP, such as an MTSP3, MTSP4 or MTSP6 are described. Such methods or assays may use any means of monitoring or detecting the desired activity.

In one format, the relative amounts of a protein between a cell population that has been exposed to the agent to be tested compared to an un-exposed control cell population may be assayed (*e.g*., a prostate cancer cell line, a lung cancer cell line, a colon cancer cell line or a breast cancer cell line). In this format, probes, such as specific antibodies, are used to monitor the differential expression of the protein in the different cell populations. Cell lines or populations are exposed to the agent to be tested under appropriate conditions and time. Cellular lysates may be prepared from the exposed cell line or population and a control, unexposed cell line or population. The cellular lysates are then analyzed with the probe.

For example, N- and C- terminal fragments of the MTSP can be expressed in bacteria and used to search for proteins which bind to these fragments. Fusion proteins, such as His-tag or GST fusion to the N- or C-terminal regions of the MTSP, such as an MTSP3, MTSP4 or an MTSP6, can be prepared for use as a substrate. These fusion proteins can be coupled to, for example, Glutathione-Sepharose beads and then probed with cell lysates. Prior to lysis, the cells may be treated with a candidate agent which may modulate an MTSP, such as an MTSP3, MTSP4 or an MTSP6, or proteins that interact with domains thereon. Lysate proteins binding to the fusion proteins can be resolved by SDS-PAGE, isolated and identified by protein sequencing or mass spectroscopy, as is known in the art.

Antibody probes are prepared by immunizing suitable mammalian hosts in appropriate immunization protocols using the peptides, polypeptides or proteins if they are of sufficient length (*e.g.*, 4, 5, 6, 7, 8, 9, 10, 11, 12, 13, 14, 15, 20, 25, 30, 35, 40 or more consecutive amino acids the MTSP protein, such as an MTSP3, an MTSP4 or an MTSP6), or if required to enhance immunogenicity, conjugated to suitable carriers. Methods for preparing immunogenic conjugates with carriers, such as bovine serum albumin (BSA), keyhole limpet hemocyanin (KLH), or other carrier proteins are well known in the art. In some circumstances, direct conjugation using, for example, carbodiimide reagents may be effective; in other instances linking reagents such as those supplied by Pierce Chemical Co., Rockford, IL, may be desirable to provide accessibility to the hapten. Hapten peptides can be extended at either the amino or carboxy terminus with a Cys residue or interspersed with cysteine residues, for example, to facilitate linking to a carrier. Administration of the immunogens is conducted generally by injection over a suitable time period and with use of suitable adjuvants, as is generally understood in the art. During the immunization schedule, titers of antibodies are taken to determine adequacy of antibody formation.

Anti-peptide antibodies can be generated using synthetic peptides corresponding to, for example, the carboxy terminal amino acids of the MTSP. Synthetic peptides can be as small as 1-3 amino acids in length, but are preferably at least 4 or more amino acid residues long. The peptides can be coupled to KLH using standard methods and can be immunized into animals, such as rabbits or ungulate. Polyclonal antibodies can then be purified, for example using Actigel beads containing the covalently bound peptide.

While the polyclonal antisera produced in this way may be satisfactory for some applications, for pharmaceutical compositions, use of monoclonal preparations is preferred. Immortalized cell lines which secrete the desired monoclonal antibodies may be prepared using the standard method of Kohler et al., (Nature 256: 495-7 (1975)) or modifications which effect immortalization of lymphocytes or spleen cells, as is generally known. The immortalized cell lines secreting the desired antibodies are screened by immunoassay in which the antigen is the peptide hapten, polypeptide or protein. When the appropriate immortalized cell culture secreting the desired antibody is identified, the cells can be cultured either *in vitro* or by production *in vivo* via ascites fluid. Of particular interest, are monoclonal antibodies that recognize the catalytic domain of an MTSP, such as an MTSP3, MTSP4 or an MTSP6.

Additionally, the zymogen or two-chain forms the MTSP can be used to make monoclonal antibodies which recognize conformation epitopes. For peptide-directed monoclonal antibodies, peptides from the C1r/C1s domain can be used to generate anti-C1r/C1s domain monoclonal antibodies which can thereby block activation of the zymogen to the two-chain form of the MTSP. This domain can similarly be the substrate for other non-antibody compounds which bind to these preferred domains on either the single-chain or double-chain forms of the MTSP3, MTSP4 or MTSP6, and thereby modulate the activity of thereof or prevent its activation.

The desired monoclonal antibodies are then recovered from the culture supernatant or from the ascites supernatant. Fragments of the monoclonals or the polyclonal antisera which contain the immunologically significant portion can be used as antagonists, as well as the intact antibodies. Use of immunologically reactive fragments, such as the Fab, Fab', of F(ab')₂ fragments are often preferable, especially in a therapeutic context, as these fragments are generally less immunogenic than the whole immunoglobulin.

The antibodies or fragments may also be produced. Regions that bind specifically to the desired regions of receptor can also be produced in the context of chimeras with multiple species origin.

Agents that are assayed in the above method can be randomly selected or rationally selected or designed.

The agents can be, as examples, peptides, small molecules, and carbohydrates. A skilled artisan can readily recognize that there is no limit as to the structural nature of the agents.

The peptide agents can be prepared using standard solid phase (or solution phase) peptide synthesis methods, as is known in the art. In addition, the DNA encoding these peptides may be synthesized using commercially available oligonucleotide synthesis instrumentation and produced recombinantly using standard recombinant production systems. The production using solid phase peptide synthesis is necessitated if non-gene-encoded amino acids are to be included.

### G. Assay formats and selection of test substances

A variety of formats and detection protocols are known for performing screening assays. Any such formats and protocols may be adapted for identifying modulators of MTSP protein activities. The following includes a discussion of exemplary protocols.

### 1. High throughput screening assays

Although the above-described assay can be conducted where a single MTSP protein is screened, and/or a single test substance is screened for in one assay, the assay is preferably conducted in a high throughput screening mode, *i.e.,* a plurality of the MTSP proteins are screened against and/or a plurality of the test substances are screened for simultaneously (*See generally,* High Throughput Screening: The Discovery of Bioactive Substances (Devlin, Ed.) Marcel Dekker, 1997; Sittampalam et al., Curr. Opin. Chem. Biol., 1(3):384-91 (1997); and Silverman et al., Curr. Opin. Chem. Biol., 2(3):397-403 (1998)). For example, the assay can be conducted in a multi-well (*e.g.,* 24-, 48-, 96-, or 384-well), chip or array format.

High-throughput screening (HTS) is the process of testing a large number of diverse chemical structures against disease targets to identify "hits" (Sittampalam et al., Curr. Opin. Chem. Biol., 1(3):384-91 (1997)). Current state-of-the-art HTS operations are highly automated and computerized to handle sample preparation, assay procedures and the subsequent processing of large volumes of data.

Detection technologies employed in high-throughput screens depend on the type of biochemical pathway being investigated (Sittampalam et al., Curr. Opin. Chem. Bio/., 1(3):384-91 (1997)). These methods include, radiochemical methods, such as the scintillation proximity assays (SPA), which can be adapted to a variety of enzyme assays (Lerner et al., J. Biomol. Screening, 1:135-143 (1996); Baker et al., Anal. Biochem., 239:20-24 (1996); Baum et al., Anal. Biochem., 237:129-134 (1996); and Sullivan et al., J. Biomol. Screening, 2:19-23 (1997)) and protein-protein interaction assays (Braunwalder et al., J. Biomol. Screening, 1:23-26 (1996); Sonatore et al., Anal. Biochem., 240:289-297 (1996); and Chen et al., J. Biol. Chem., 271:25308-25315 (1996)), and non-isotopic detection methods, including but are not limited to, colorimetric and luminescence detection methods, resonance energy transfer (RET) methods, time-resolved fluorescence (HTRF) methods, cell-based fluorescence assays, such as fluorescence resonance energy transfer (FRET) procedures (see, *e.g*., Gonzalez et al., Biophys. J., 69:1272-1280 (1995)), fluorescence polarization or anisotropy methods (see, *e.g.,* Jameson et al., Methods Enzymol., 246:283-300 (1995); Jolley, J. Biomol. Screening, 1:33-38 (1996); Lynch et al., Anal. Biochem., 247:77-82 (1997)), fluorescence correlation spectroscopy (FCS) and other such methods.

### 2. Test Substances

Test compounds, including small molecules and libraries and collections thereof can be screened in the above-described assays and assays described below to identify compounds that modulate the activity an MTSP protein. Rational drug design methodologies that rely on computational chemistry may be used to screen and identify candidate compounds.

The compounds identified by the screening methods include inhibitors, including antagonists, and may be agonists Compounds for screening are any compounds and collections of compounds available, know or that can be prepared.

### a. Selection of Compounds

Compounds can be selected for their potency and selectivity of inhibition of serine proteases, especially MTSP protein. As described herein, and as generally known, a target serine protease and its substrate are combined under assay conditions permitting reaction of the protease with its substrate. The assay is performed in the absence of test compound, and in the presence of increasing concentrations of the test compound. The concentration of test compound at which 50% of the serine protease activity is inhibited by the test compound is the IC₅₀ value (Inhibitory Concentration) or EC₅₀ (Effective Concentration) value for that compound. Within a series or group of test compounds, those having lower IC₅₀ or EC₅₀ values are considered more potent inhibitors of the serine protease than those compounds having higher IC₅₀ or EC₅₀ values. The IC₅₀ measurement is often used for more simplistic assays, whereas the EC₅₀ is often used for more complicated assays, such as those employing cells.

Preferred compounds according to this aspect have an IC₅₀ value of 100 nM or less as measured in an *in vitro* assay for inhibition of MTSP protein activity. Especially preferred compounds have an IC₅₀ value of less than 100 nM.

The test compounds also are evaluated for selectivity toward a serine protease. As described herein, and as generally known, a test compound is assayed for its potency toward a panel of serine proteases and other enzymes and an IC₅₀ value or EC₅₀ value is determined for each test compound in each assay system. A compound that demonstrates a low IC₅₀ value or EC₅₀ value for the target enzyme, *e.g.*, MTSP protein, and a higher IC₅₀ value or EC₅₀ value for other enzymes within the test panel (*e.g.*, urokinase tissue plasminogen activator, thrombin, Factor Xa), is considered to be selective toward the target enzyme. Generally, a compound is deemed selective if its IC₅₀ value or EC₅₀ value in the target enzyme assay is at least one order of magnitude less than the next smallest IC₅₀ value or EC₅₀ value measured in the selectivity panel of enzymes.

Presently preferred compounds have an IC₅₀ value of 100 nM or less as measured in an *in vitro* assay for inhibition of urokinase activity. Especially preferred compounds have an IC₅₀ value in the *in vitro* urokinase inhibition assay that is at least one order of magnitude smaller than the IC₅₀ value measured in the *in vitro* tPA inhibition assay. Compounds having a selectivity ratio of IC₅₀ u-PA assay: IC₅₀ MTSP protein assay of greater than 100 are especially preferred.

Compounds are also evaluated for their activity *in vivo.* The type of assay chosen for evaluation of test compounds will depend on the pathological condition to be treated or prevented by use of the compound, as well as the route of administration to be evaluated for the test compound.

For instance, to evaluate the activity of a compound to reduce tumor growth through inhibition of MTSP protein, the procedures described by Jankun et al., Canc. Res., 57:559-563 (1997) to evaluate PAI-I can be employed. Briefly, the ATCC cell lines DU145 and LnCaP are injected into SCID mice. After tumors are established, the mice are given test compound according to a dosing regime determined from the compound's *in vitro* characteristics. The Jankun *et a*/. compound was administered in water. Tumor volume measurements are taken twice a week for about five weeks. A compound is deemed active if an animal to which the compound was administered exhibited decreased tumor volume, as compared to animals receiving appropriate control compounds.

Another *in vivo* experimental model designed to evaluate the effect of p-aminobenzamidine, a swine protease inhibitor, on reducing tumor volume is described by Billström et al., Int. J. Cancer, 61:542-547 (1995).

To evaluate the ability of a compound to reduce the occurrence of, or inhibit, metastasis, the procedures described by Kobayashi et al., Int. J. Canc., 57:727-733d (1994) can be employed. Briefly, a murein xenograft selected for high lung colonization potential in injected into C57B1/6 mice i.v. (experimental metastasis) or s.c. into the abdominal wall (spontaneous metastasis). Various concentrations of the compound to be tested can be admixed with the tumor cells in Matrigel prior to injection. Daily i.p. injections of the test compound are made either on days 1-6 or days 7-13 after tumor inoculation. The animals are sacrificed about three or four weeks after tumor inoculation, and the lung tumor colonies are counted. Evaluation of the resulting data permits a determination as to efficacy of the test compound, optimal dosing and route of administration.

The activity of the tested compounds toward decreasing tumor volume and metastasis can be evaluated in model described in Rabbani et al., Int. J. Cancer 63:840-845 (1995) to evaluate their inhibitor. There, Mat LyLu tumor cells were injected into the flank of Copenhagen rats. The animals were implanted with osmotic minipumps to continuously administer various doses of test compound for up to three weeks. The tumor mass and volume of experimental and control animals were evaluated during the experiment, as were metastatic growths. Evaluation of the resulting data permits a determination as to efficacy of the test compound, optimal dosing, and route of administration. Some of these authors described a related protocol in Xing et al., Canc. Res., 57:3585-3593 (1997).

To evaluate the inhibitory activity of a compound, a rabbit cornea neovascularization model can be employed. Avery et al., Arch. Ophthalmo/., 108:1474-1475 (1990) describe anesthetizing New Zealand albino rabbits and then making a central corneal incision and forming a radial corneal pocket. A slow release prostaglandin pellet was placed in the pocket to induce neovascularization. Test compound was administered i.p. for five days, at which time the animals were sacrificed. The effect of the test compound is evaluated by review of periodic photographs taken of the limbus, which can be used to calculate the area of neovascular response and, therefore, limbal neovascularization. A decreased area of neovascularization as compared with appropriate controls indicates the test compound was effective at decreasing or inhibiting neovascularization.

An angiogenesis model used to evaluate the effect of a test compound in preventing angiogenesis is described by Min et al., Canc. Res., 56:2428-2433 (1996). C57BL6 mice receive subcutaneous injections of a Matrigel mixture containing bFGF, as the angiogenesis-inducing agent, with and without the test compound. After five days, the animals are sacrificed and the Matrigel plugs, in which neovascularization can be visualized, are photographed. An experimental animal receiving Matrigel and an effective dose of test compound will exhibit less vascularization than a control animal or an experimental animal receiving a less- or non-effective does of compound.

An *in vivo* system designed to test compounds for their ability to limit the spread of primary tumors is described by Crowley et al., Proc. Natl. Acad. Sci., 90:5021-5025 (1993). Nude mice are injected with tumor cells (PC3) engineered to express CAT (chloramphenicol acetyltransferase). Compounds to be tested for their ability to decrease tumor size and/or metastases are administered to the animals, and subsequent measurements of tumor size and/or metastatic growths are made. In addition, the level of CAT detected in various organs provides an indication of the ability of the test compound to inhibit metastasis; detection of less CAT in tissues of a treated animal versus a control animal indicates less CAT-expressing cells migrated to that tissue.

*In vivo* experimental modes designed to evaluate the inhibitory potential of a test serine protease inhibitors, using a tumor cell line F3II, the to be highly invasive, are described by Alonso et al., Breast Canc. Res. Treat., 40:209-223 (1996). This group describes *in vivo* studies for toxicity determination, tumor growth, invasiveness, spontaneous metastasis, experimental lung metastasis, and an angiogenesis assay.

The CAM model (chick embryo chorioallantoic membrane model), first described by L. Ossowski in 1998 (J. Cell Bio/., 107:2437-2445 (1988)), provides another method for evaluating the urokinase inhibitory activity of a test compound. In the CAM model, tumor cells invade through the chorioallantoic membrane containing CAM with tumor cells in the presence of several serine protease inhibitors results in less or no invasion of the tumor cells through the membrane. Thus, the CAM assay is performed with CAM and tumor cells in the presence and absence of various concentrations of test compound. The invasiveness of tumor cells is measured under such conditions to provide an indication of the compound's inhibitory activity. A compound having inhibitory activity correlates with less tumor invasion.

The CAM model is also used in a standard assay of angiogenesis (*i.e.,* effect on formation of new blood vessels (Brooks et al., Methods in Molecular Biology, 129:257-269 (1999)). According to this model, a filter disc containing an angiogenesis inducer, such as basic fibroblast growth factor (bFDG) is placed onto the CAM. Diffusion of the cytokine into the CAM induces local angiogenesis, which may be measured in several ways such as by counting the number of blood vessel branch points within the CAM directly below the filter disc. The ability of identified compounds to inhibit cytokine-induced angiogenesis can be tested using this model. A test compound can either be added to the filter disc that contains the angiogenesis inducer, be placed directly on the membrane or be administered systemically. The extent of new blood vessel formation in the presence and/or absence of test compound can be compared using this model. The formation of fewer new blood vessels in the presence of a test compound would be indicative of anti-angiogenesis activity. Demonstration of anti-angiogenesis activity for inhibitors of an MTSP protein indicates a role in angiogenesis for that MTSP protein.

### b. Known serine protease inhibitors

Compounds for screening can be serine protease inhibitors, which can be tested for their ability to inhibit the activity of an MTSP, particularly an MTSP3, MTSP4, or MTSP6.

Exemplary, but not limiting serine proteases, include the following known serine protease inhibitors are used in the screening assays: Serine Protease Inhibitor 3 (SPI-3) (Chen, M.C., et al., Citokine, 11(11):856-862 (1999)); Aprotinin (lijima, R., et al., J. Biochem. (Tokyo), 126(5):912-916 (1999)); Kazaltype serine protease inhibitor-like proteins (Niimi, T., et al., Eur. J. Biochem., 266(1):282-292 (1999)); Kunitz-type serine protease inhibitor (Ravichandran, S., et al., Acta Crystallogr. D. Biol. Crystallogr., 55(11):1814-1821 (1999)); Tissue factor pathway inhibitor-2/Matrix-associated serine rotease inhibitor (TFPI-2/MSPI), (Liu, Y., et al., Arch. Biochem. Biophys., 370(1):112-8 (1999)); Bukunin, (Cui, C.Y., et al., J. Invest. Dermatol., 113(2):182-8 (1999)); Nafmostat mesilate (Ryo, R., et al., Vox Sang., 76(4):241-6 (1999)); TPCK (Huang, Y., et al., Oncogene, 18(23):3431-9 (1999)); A synthetic cotton-bound serine protease inhibitor (Edwards, J.V., et al., Wound Repair Regen., 7(2):106-18 (1999)); FUT-175 (Sawada, M., et al., Stroke, 30(3):644-50 (1999)); Combination of serine protease inhibitor FUT-0175 and thromboxane synthetase inhibitor OKY-046 (Kaminogo, M., et al., Neuro/. Med. Chir. (Tokyo), 38(11):704-8; discussion 708-9 (1998)); The rat serine protease inhibitor 2.1 gene (LeCam, A., et al., Biochem. Biophys. Res. Commun., 253(2):311-4 (1998)); A new intracellular serine protease inhibitor expressed in the rat pituitary gland complexes with granzyme B (Hill, R.M., et al., FEBS Lett., 440(3):361-4 (1998)); 3,4-Dichloroisocoumarin (Hammed, A., et al., Proc. Soc. Exp. Bio/. Med., 219(2):132-7 (1998)); LEX032 (Bains, A.S., et al., Eur. J. Pharmacol., 356(1):67-72 (1998)); N-tosyl-L-phenylalanine chloromethyl ketone (Dryjanski, M., et al., Biochemistry, 37(40):14151-6 (1998)); Mouse gene for the serine protease inhibitor neuroserpin (P112) (Berger, P., et al., Gene, 214(1-2):25-33 (1998)); Rat serine protease inhibitor 2.3 gene (Paul, C., et al., Eur. J. Biochem., 254(3):538-46 (1998)); Ecotin (Yang, S.Q., et al., J. Mol. Bio/., 279(4):945-57 (1998)); A 14 kDa plant-related serine protease inhibitor (Roch, P., et al., Dev. Comp. Immunol., 22(1):1-12 (1998)); Matrix-associated serine protease inhibitor TFPI-2/33 kDa MSPI (Rao, C.N., et al., Int. J. Cancer, 76(5):749-56 (1998)); ONO-3403 (Hiwasa, T., et al., Cancer Lett., 126(2):221-5 (1998)); Bdellastasin (Moser, M., et al., Eur. J. Biochem., 253(1):212-20 (1998)); Bikunin (Xu, Y., et al., J. Mol. Biol., 276(5):955-66 (1998)); Nafamostat mesilate (Mellgren, K., et al., Thromb. Haemost., 79(2):342-7 (1998)); The growth hormone dependent serine protease inhibitor, Spi 2.1 (Maake, C., et al., Endocrinology, 138(12):5630-6 (1997)); Growth factor activator inhibitor type 2, a Kunitz-type serine protease inhibitor (Kawaguchi, T., et al., J. Bio/. Chem., 272(44):27558-64 (1997)); Heat-stable serine protease inhibitor protein from ovaries of the desert locust, Schistocerga gregaria (Hamdaoui, A., et al., Biochem. Biophys. Res. Commun., 238(2):357-60 (1997)); Bikunin, (Delaria, K.A., et al., J. Bio/. Chem., 272(18):12209-14 (1997)); Human placental bikunin (Marlor, C.W., et al., J. Bio/. Chem., 272(10):12202-8 (1997)); Hepatocyte growth factor activator inhibitor, a novel Kunitz-type serine protease inhibitor (Shimomura, T., et al., J. Bio/. Chem., 272(10):6370-6 (1997)); FUT-187, oral serine protease inhibitor, (Shiozaki, H., et al., Gan To Kaguku Ryoho, 23(14): 1971-9 (1996)); Extracellular matrix-associated serine protease inhibitors (Mr 33,000, 31,000, and 27,000 (Rao, C.N., et al., Arch. Biochem. Biophys., 335(1):82-92 (1996)); An irreversible isocoumarin serine protease inhibitor (Palencia, D.D., et al., Bio/. Reprod., 55(3):536-42 (1996)); 4-(2-aminoethyl)-benzenesulfonyl fluoride (AEBSF) (Nakabo, Y., et al., J. Leukoc. Bio/., 60(3):328-36 (1996)); Neuroserpin (Osterwalder, T., et al., EMBO J., 15(12):2944-53 (1996)); Human serine protease inhibitor alpha-1-antitrypsin (Forney, J.R., et al., J. Parasitol.. 82(3):496-502 (1996)); Rat serine protease inhibitor 2.3 (Simar-Blanchet, A.E., et al., Eur. J. Biochem., 236(2):638-48 (1996)); Gebaxate mesilate (parodi, F., et al., J. Cardiothorac. Vasc. Anesth., 10(2):235-7 (1996)); Recombinant serine protease inhibitor, CPTI II (Stankiewicz, M., et al., (Acta Biochim. Pol., 43(3):525-9 (1996)); A cysteine-rich serine protease inhibitor (Guamerin II) (Kim, D.R., et al., J. Enzym. Inhib., 10(2):81-91 (1996)); Diisopropylfluorophosphate (Lundqvist, H., et al., Inflamm. Res., 44(12):510-7 (1995)); Nexin 1 (Yu, D.W., et al., J. Cell Sci., 108(Pt 12):3867-74 (1995)); LEX032 (Scalia, R., et al., Shock, 4(4):251-6 (1995)); Protease nexin I (Houenou, L.J., et al., Proc. Natl. Acad. Sci. U.S.A., 92(3):895-9 (1995)); Chymase-directed serine protease inhibitor (Woodard S.L., et al., J. Immunol., 153(11):5016-25 (1994)); N-alpha-tosyl-L-lysyl-chloromethyl ketone (TLCK) (Bourinbaiar, A.S., et al., Cell Immunol., 155(1):230-6 (1994)); Smpi56 (Ghendler, Y., et al., Exp. Parasitol., 78(2):121-31 (1994)); Schistosoma haematobium serine protease (Blanton, R.E., et al., Mol. Biochem. Parasitol., 63(1):1-11 (1994)); Spi-1 (Warren, W.C., et al., Mol. Cell Endocrinol., 98(1):27-32 (1993)); TAME (Jessop, J.J., et al., Inflammation, 17(5):613-31 (1993)); Antithrombin III (Kalaria, R.N., et al., Am. J. Pathol., 143(3):886-93 (1993)); FOY-305 (Ohkoshi, M., et al., Anticancer Res., 13(4):963-6 (1993)); Camostat mesilate (Senda, S., et al., lantern. Med., 32(4):350-4 (1993)); Pigment epithelium-derived factor (Steele, F.R., et al., Proc. Natl. Acad. Sci. U.S.A., 90(4):1526-30 (1993)); Antistasin (Holstein, T.W., et al., FEBS Lett., 309(3):288-92 (1992)); The vaccinia virus K2L gene encodes a serine protease inhibitor (Zhou, J., et al., Virology, 189(2):678-86 (1992)); Bowman-Birk serine-protease inhibitor (Werner, M.H., et al., J. Mol. Biol., 225(3):873-89 (1992); FUT-175 (Yanamoto, H., et al., Neurosurgery, 30(3):358-63 (1992)); FUT-175; (Yanamoto, H., et al., Neurosurgery, 30(3):351-6, discussion 356-7 (1992)); PAI-I (Yreadwell, B.V., et al., J. Orthop. Res., 9(3):309-16 (1991)); 3,4-Dichloroisocoumarin (Rusbridge, N.M., et al., FEBS Lett., 268(1):133-6 (1990)); Alpha 1-antichymotrypsin (Lindmark, B.E., et al., Am. Rev. Respir. Des., 141(4 Pt 1):884-8 (1990)); P-toluenesulfonyl-L-arginine methyl ester (TAME) (Scuderi, P., J. Immunol., 143(1):168-73 (1989)); Aprotinin (Seto, S., et al., Adv. Exp. Med. Bio/., 247B:49-54 (1989)); Alpha 1-antichymotrypsin (Abraham, C.R., et al., Cell, 52(4):487-501 (1988)); Contrapsin (Modha, J., et al., Parasitology, 96 (Pt 1):99-109 (1988)); (FOY-305) (Yamauchi, Y., et al., Hiroshima J. Med. Sci., 36(1):81-7 No abstract available (1987)); Alpha 2-antiplasmin (Holmes, W.E., et al., J. Biol. Chem., 262(4):1659-64 (1987)); 3,4-dichloroisocoumarin (Harper, J.W., et al., Biochemistry, 24((8):1831-41 (1985)); Diisoprophylfluorophosphate (Tsutsui, K., et al., Biochem. Biophys. Res. Commun., 123(1):271-7 (1984)); Gabexate mesilate (Hesse, B., et al., Pharmacol. Res. Commun., 16(7):637-45 (1984)); Phenyl methyl sulfonyl fluoride (Dufer, J., et al., Scand. J. Haematol., 32(1):25-32 (1984)); Aprotinin (Seto, S., et al., Hypertension, 5(6):893-9 (1983)); Protease inhibitor CI-2 (McPhalen, C.A., et al., J. Mol. Bio/., 168(2):445-7 (1983)); Phenylmethylsulfonyl fluoride (Sekar V., et al., Biochem. Biophys. Res. Commun., 89(2):474-8 (1979)); PGE1 (Feinstein, M.D., et al., Prostaglandine, 14(6):1075-93 (1977)

### c. Combinatorial libraries and other libraries

The source of compounds for the screening assays, can be libraries, including, but are not limited to, combinatorial libraries. Methods for synthesizing combinatorial libraries and characteristics of such combinatorial libraries are known in the art (*See generally,* Combinatorial Libraries: Synthesis, Screening and Application Potential (Cortese Ed.) Walter de Gruyter, Inc., 1995; Tietze and Lieb, Curr. Opin. Chem. Bio/., 2(3):363-71 (1998); Lam, Anticancer Drug Des., 12(3):145-67 (1997); Blaney and Martin, Curr. Opin. Chem. Bio/., 1(1):54-9 (1997); and Schultz and Schultz, Biotechnol. Prog., 12(6):729-43 (1996)).

Methods and strategies for generating diverse libraries, primarily peptideand nucleotide-based oligomer libraries, have been developed using molecular biology methods and/or simultaneous chemical synthesis methodologies (*see*, *e.g.,* Dower et al., Annu. Rep. Med. Chem., 26:271-280 (1991); Fodor et al., Science, 251:767-773 (1991); Jung et al., Angew. Chem. Ind. Ed. Engl., 31:367-383 (1992); Zuckerman et al., Proc. Natl. Acad. Sci. USA, 89:4505-4509 (1992); Scott et al., Science, 249:386-390 (1990); Devlin et al., Science, 249:404-406 (1990); Cwirla et al., Proc. Natl. Acad. Sci. USA, 87:6378-6382 (1990); and Gallop et al., J. Medicinal Chemistry, 37:1233-1251 (1994)). The resulting combinatorial libraries potentially contain millions of compounds and that can be screened to identify compounds that exhibit a selected activity.

The libraries fall into roughly three categories: fusion-protein-displayed peptide libraries in which random peptides or proteins are presented on the surface of phage particles or proteins expressed from plasmids; support-bound synthetic chemical libraries in which individual compounds or mixtures of compounds are presented on insoluble matrices, such as resin beads (*see, e.g.,* Lam et al., Nature, 354:82-84 (1991)) and cotton supports (*see, e.g.,* Eichler et al., Biochemistry 32:11035-11041 (1993)); and methods in which the compounds are used in solution (*see, e.g.,* Houghten et al., Nature, 354:84-86 (1991); Houghten et al., BioTechniques, 313:412-421 (1992); and Scott et al., Curr. Opin. Biotechnol., 5:40-48 (1994)). There are numerous examples of synthetic peptide and oligonucleotide combinatorial libraries and there are many methods for producing libraries that contain non-peptidic small organic molecules. Such libraries can be based on basis set of monomers that are combined to form mixtures of diverse organic molecules or that can be combined to form a library based upon a selected pharmacophore monomer.

Either a random or a deterministic combinatorial library can be screened by the presently disclosed and/or claimed screening methods. In either of these two libraries, each unit of the library is isolated and/or immobilized on a solid support. In the deterministic library, one knows *a priori* a particular unit's location on each solid support. In a random library, the location of a particular unit is not known *a priori* although each site still contains a single unique unit. Many methods for preparing libraries are known to those of skill in this art (see, *e.g.,* Geysen et al., Proc. Natl. Acad. Sci. USA, 81:3998-4002 (1984), Houghten et al., Proc. Natl. Acad Sci. USA, 81:5131-5135 (1985)). Combinatorial library generated by the any techniques known to those of skill in the art are contemplated (see, *e.g.,* Table 1 of Schultz and Schultz, Biorechnol. Prog., 12(6):729-43 (1996)) for screening; Bartel et al., Science, 261:1411-1418 (1993); Baumbach et al. BioPharm, (May):24-35 (1992); Bock et al. Nature, 355:564-566 (1992); Borman, S., Combinatorial chemists focus on small molecules molecular recognition, and automation, Chem. Eng. News, 2(12):29 (1996); Boublik, et al., Eukaryotic Virus Display: Engineering the Major Surface Glycoproteins of the Autographa California Nuclear Polyhedrosis Virus (ACNPV) for the Presentation of Foreign Proteins on the Virus Surface, Bio/Technology, 13:1079-1084 (1995); Brenner, et al., Encoded Combinatorial Chemistry, Proc. Natl. Acad Sci. U.S.A., 89:5381-5383 (1992); Caflisch, et al., Computational Combinatorial Chemistry for De Novo Ligand Design: Review and Assessment, Perspect. Drug Discovery Des., 3:51-84 (1995); Cheng, et al., Sequence-Selective Peptide Binding with a Peptido-A,B-trans-steroidal Receptor Selected from an Encoded Combinatorial Library, J. Am. Chem. Soc., 118:1813-1814 (1996); Chu, et al., Affinity Capillary Electrophoresis to Identify the Peptide in A Peptide Library that Binds Most Tightly to Vancomycin, J. Org. Chem., 58:648-652 (1993); Clackson, et al., Making Antibody Fragments Using Phage Display Libraries, Nature, 352:624-628 (1991); Combs, et al., Protein Structure-Based Combinatorial Chemistry: Discovery of Non-Peptide Binding Elements to Src SH3 Domain, J. Am. Chem. Soc., 118:287-288 (1996); Cwirla, et al., Peptides On Phage: A Vast Library of Peptides for Identifying Ligands, Proc. Natl. Acad Sci. U.S.A., 87:6378-6382 (1990); Ecker, et al., Combinatorial Drug Discovery: Which Method will Produce the Greatest Value, Bio/Technology, 13:351-360 (1995); Ellington, et al., In Vitro Selection of RNA Molecules That Bind Specific Ligands, Nature, 346:818-822 (1990); Ellman, J.A., Variants of Benzodiazephines, J. Am. Chem. Soc., 114:10997 (1992); Erickson, et al., The Proteins; Neurath, H., Hill, R.L., Eds.: Academic: New York, 1976; pp. 255-257; Felici, et al., J. Mol. Biol., 222:301-310 (1991); Fodor, et al., Light-Directed, Spatially Addressable Parallel Chemical Synthesis, Science, 251:767-773 (1991); Francisco, et al., Transport and Anchoring of Beta-Lactamase to the External Surface of E. Coli., Proc. Natl. Acad. Sci. U.S.A., 89:2713-2717 (1992); Georgiou, et al., Practical Applications of Engineering Gram-Negative Bacterial Cell Surfaces, TIBTECH, 11:6-10 (1993); Geysen, et al., Use of peptide synthesis to probe viral antigens for epitopes to a resolution of a single amino acid, Proc. Natl. Acad. Sci. U.S.A., 81:3998-4002 (1984); Glaser, et al., Antibody Engineering by Condon-Based Mutagenesis in a Filamentous Phage Vector System, J. Immunol., 149:3903-3913 (1992); Gram, et al., In vitro selection and affinity maturation of antibodies from a naive combinatorial immunoglobulin library, Proc. Natl. Acad. Sci., 89:3576-3580 (1992); Han, et al., Liquid-Phase Combinatorial Synthesis, Proc. Natl. Acad. Sci. U.S.A., 92:6419-6423 (1995); Hoogenboom, et al., Multi-Subunit Proteins on the Surface of Filamentous Phage: Methodologies for Displaying Antibody (Fab) Heavy and Light Chains, Nucleic Acids Res., 19:4133-4137 (1991); Houghten, et al., General Method for the Rapid Solid-Phase Synthesis of Large Numbers of Peptides: Specificity of Antigen-Antibody Interaction at the Level of Individual Amino Acids, Proc. Natl. Acad. Sci. U.S.A., 82:5131-5135 (1985); Houghten, et al., The Use of Synthetic Peptide Combinatorial Libraries for the Determination of Peptide Ligands in Radio-Receptor Assays-Opiod-Peptides, Bioorg. Med. Chem. Lett., 3:405-412 (1993); Houghten, et al., Generation and Use of Synthetic Peptide Combinatorial Libraries for Basic Research and Drug Discovery, Nature, 354:84-86 (1991); Huang, et al., Discovery of New Ligand Binding Pathways in Myoglobin by Random Mutagenesis, Nature Struct. Biol., 1:226-229 (1994); Huse, et al., Generation of a Large Combinatorial Library of the Immunoglobulin Repertoire In Phage Lambda, Science, 246:1275-1281 (1989); Janda, K.D., New Strategies for the Design of Catalytic Antibodies, Biotechnol. Prog., 6:178-181 (1990); Jung, et al., Multiple Peptide Synthesis Methods and Their Applications, Angew. Chem. Int. Ed. Engl., 31:367-486 (1992); Kang, et al., Linkage of Recognition and Replication Functions By Assembling Combinatorial Antibody Fab Libraries Along Phage Surfaces, Proc. Natl. Acad. Sci. U.S.A., 88:4363-4366 (1991a); Kang, et al., Antibody Redesign by Chain Shuffling from Random Combinatorial Immunoglobulin Libraries, Proc. Natl. Acad. Sci. U.S.A., 88:11120-11123 (1991 b); Kay, et al., An M13 Phage Library Displaying Random 38-Amino-Acid-Peptides as a Source of Novel Sequences with Affinity to Selected Targets Genes, Gene, 128:59-65 (1993); Lam, et al., A new type of synthetic peptide library for identifying ligand-binding activity, Nature, 354:82-84 (1991) (published errata apear in Nature, 358:434 (1992) and Nature, 360:768 (1992); Lebl, et al., One Bead One Structure Combinatorial Libraries, Biopolymers (Pept. Sci.), 37:177-198 (1995); Lerner, et al., Antibodies without Immunization, Science, 258:1313-1314 (1992); Li, et al., Minimization of a Polypeptide Hormone, Science, 270:1657-1660 (1995); Light, et al., Display of Dimeric Bacterial Alkaline Phosphatase on the Major Coat Protein of Filamentous Bacteriophage, Bioorg. Med. Chem. Lett., 3:1073-1079 (1992); Little, et al., Bacterial Surface Presentation of Proteins and Peptides: An Alternative to Phage Technology, Trends Biotechnol., 11:3-5 (1993); Marks, et al., By-Passing Immunization. Human Antibodies from V-Gene Libraries Displayed on Phage, J. Mol. Bio/., 222:581-597 (1991); Matthews, et al., Substrate Phage: Selection of Protease Substrates by Monovalent Phage Display, Science, 260:1113-1117 (1993); McCafferty, et al., Phage Enzymes: Expression and Affinity Chromatography of Functional Alkaline Phosphatase on the Surface of Bacteriophage, Protein Eng., 4:955-961 (1991); Menger, et al., Phosphatase Catalysis Developed Via Combinatorial Organic Chemistry, J. Org. Chem., 60:6666-6667 (1995); Nicolaou, et al., Angew. Chem. Int. Ed. Engl., 34:2289-2291 (1995); Oldenburg, et al., Peptide Ligands for A Sugar-Binding Protein Isolated from a Random Peptide Library, Proc. Natl. Acad. Sci. U.S.A., 89:5393-5397 (1992); Parmley, et al., Antibody-Selectable Filamentous fd Phage Vectors: Affinity Purification of Target Genes, Genes, 73:305-318 (1988); Pinilla, et al., Synthetic Peptide Combinatorial Libraries (SPCLS)--Identification of the Antigenic Determinant of Beta-Endorphin Recognized by Monoclonal Antibody-3E7, Gene, 128:71-76 (1993); Pinilla, et al., Review of the Utility of Soluble Combinatorial Libraries, Biopolymers, 37:221-240 (1995); Pistor, et al., Expression of Viral Hemegglutinan On the Surface of E. Coli., Klin. Wochenschr., 66:110-116 (1989); Pollack, et al., Selective Chemical Catalysis by an Antibody, Science, 234:1570-1572 (1986); Rigler, et al., Fluorescence Correlations, Single Molecule Detection and Large Number Screening: Applications in Biotechnology, J. Biotechnol., 41:177-186 (1995); Sarvetnick, et al., Increasing the Chemical Potential of the Germ-Line Antibody Repertoire, Proc. Natl. Acad. Sci. U.S.A., 90:4008-4011 (1993); Sastry, et al., Cloning of the Immunological Repertiore in Escherichia Coli for Generation of Monoclonal Catalytic Antibodies: Construction of a Heavy Chain Variable Region-Specific cDNA Library, Proc. Natl. Acad. Sci. U.S.A., 86:5728-5732 (1989); Scott, et al., Searching for Peptide Ligands with an Epitope Library, Science, 249:386-390 (1990); Sears, et al., Engineering Enzymes for Bioorganic Synthesis: Peptide Bond Formation, Biotechnol. Prog., 12:423-433 (1996); Simon, et. al., Peptides: A Modular Approach to Drug Discovery, Proc. Natl. Acad. Sci. U.S.A., 89:9367-9371 (1992); Still, et al., Discovery of Sequence-Selective Peptide Binding by Synthetic Receptors Using Encoded Combinatorial Libraries, Acc. Chem. Res., 29:155-163 (1996); Thompson, et al., Synthesis and Applications of Small Molecule Libraries, Chem. Rev., 96:555-600 (1996); Tramontano, et al., Catalytic Antibodies, Science, 234:1566-1570 (1986); Wrighton, et al., Small Peptides as Potent Mimetics of the Protein Hormone Erythropoietin, Science, 273:458-464 (1996); York, et al., Combinatorial mutagenesis of the reactive site region in plasminogen activator inhibitor I, J. Bio/. Chem., 266:8595-8600 (1991); Zebedee, et al., Human Combinatorial Antibody Libraries to Hepatitis B Surface Antigen, Proc. Natl. Acad Sci. U.S.A., 89:3175-3179 (1992); Zuckerman, et al., Identification of Highest-Affinity Ligands by Affinity Selection from Equimolar Peptide Mixtures Generated by Robotic Synthesis, Proc. Natl. Acad. Sci. U.S.A., 89:4505-4509 (1992).

For example, peptides that bind to an MTSP protein or a protease domain of an MTSP protein can be identified using phage display libraries. In an exemplary embodiment, this method can include a) contacting phage from a phage library with the MTSP protein or a protease domain thereof; (b) isolating phage that bind to the protein; and (c) determining the identity of at least one peptide coded by the isolated phage to identify a peptide that binds to an MTSP protein.

### H, Modulators of the activity of MTSP proteins

Compounds, identified by screening or produced using the MTSP proteins or protease domain in other screening methods, that modulate the activity of an MTSP are discussed herein. These compounds act by directly interacting with the MTSP protein or by altering transcription or translation thereof. Such molecules include, but are not limited to, antibodies that specifically react with an MTSP protein, particularly with the protease domain thereof, antisense nucleic acids that alter expression of the MTSP protein, antibodies, peptide mimetics and other such compounds.

### 1. Antibodies

Antibodies, including polyclonal and monoclonal antibodies, that specifically bind to the MTSP proteins particularly to the single chain protease domains thereof are described. The antibody may be is a monoclonal antibody, and specifically binds to the protease domain of the MTSP protein. In particular embodiments, antibodies to each of the single chain of protease domain of MTSP1, MTSP3, MTSP4 and MTSP6. Antibodies that specifically bind to any domain of MTSP3 or MTSP4, and to double chain forms thereof are also described.

The MTSP protein and domains, fragments, homologs and derivatives thereof may be used as immunogens to generate antibodies that specifically bind such immunogens. Such antibodies include but are not limited to polyclonal, monoclonal, chimeric, single chain, Fab fragments, and an Fab expression library. Antibodies to human MTSP protein may be produced. Complexes formed from fragments of MTSP protein, which fragments contain the serine protease domain, may be used as immunogens for antibody production.

Various procedures known in the art may be used for the production of polyclonal antibodies to MTSP protein, its domains, derivatives, fragments or analogs. For production of the antibody, various host animals can be immunized by injection with the native MTSP protein or a synthetic version, or a derivative of the foregoing, such as a cross-linked MTSP protein. Such host animals include but are not limited to rabbits, mice, rats, etc. Various adjuvants can be used to increase the immunological response, depending on the host species, and include but are not limited to Freund's (complete and incomplete), mineral gels such as aluminum hydroxide, surface active substances such as lysolecithin, pluronic polyols, polyanions, peptides, oil emulsions, dinitrophenol, and potentially useful human adjuvants such as bacille Calmette-Guerin (BCG) and corynebacterium parvum.

For preparation of monoclonal antibodies directed towards an MTSP protein or domains, derivatives, fragments or analogs thereof, any technique that provides for the production of antibody molecules by continuous cell lines in culture may be used. Such techniques include but are not restricted to the hybridoma technique originally developed by Kohler and Milstein (Nature 256:495-497 (1975)), the trioma technique, the human B-cell hybridoma technique (Kozbor et al., Immunology Today 4:72 (1983)), and the EBV hybridoma technique to produce human monoclonal antibodies (Cole et al., in Monoclonal Antibodies and Cancer Therapy, Alan R. Liss, Inc., pp. 77-96 (1985)). In an additional embodiment, monoclonal antibodies can be produced in germ-free animals utilizing recent technology (PCT/US90/02545). Human antibodies may be used and can be obtained by using human hybridomas (Cote et al., Proc. Natl. Acad. Sci. USA 80:2026-2030 (1983)). Or by transforming human B cells with EBV virus *in vitro* (Cole et al., in Monoclonal Antibodies and Cancer Therapy, Alan R. Liss, Inc., pp. 77-96 (1985)). Techniques developed for the production of "chimeric antibodies" (Morrison et al., Proc. Natl. Acad. Sci. USA 81:6851-6855 (1984); Neuberger et al., Nature 312:604-608 (1984); Takeda et al., Nature 314:452-454 (1985)) by splicing the genes from a mouse antibody molecule specific for the MTSP protein together with genes from a human antibody molecule of appropriate biological activity can be used.

Techniques described for the production of single chain antibodies (U.S. patent 4,946,778) can be adapted to produce MTSP protein-specific single chain antibodies. An additional embodiment uses the techniques described for the construction of Fab expression libraries (Huse et al., Science 246:1275-1281 (1989)) to allow rapid and easy identification of monoclonal Fab fragments with the desired specificity for MTSP protein or MTSP protein, or domains, derivatives, or analogs thereof. Non-human antibodies can be "humanized" by known methods (*see*, *e.g.,* U.S. Patent No. 5,225,539).

Antibody fragments that contain the idiotypes of MTSP protein can be generated by techniques known in the art. For example, such fragments include but are not limited to: the F(ab')2 fragment which can be produced by pepsin digestion of the antibody molecule; the Fab' fragments that can be generated by reducing the disulfide bridges of the F(ab')2 fragment, the Fab fragments that can be generated by treating the antibody molecular with papain and a reducing agent, and Fv fragments.

In the production of antibodies, screening for the desired antibody can be accomplished by techniques known in the art, *e.g.,* ELISA (enzyme-linked immunosorbent assay). To select antibodies specific to a particular domain of the MTSP protein one may assay generated hybridomas for a product that binds to the fragment of the MTSP protein that contains such a domain

The foregoing antibodies can be used in methods known in the art relating to the localization and/or quantitation of MTSP proteins, *e.g.,* for imaging these proteins, measuring levels thereof in appropriate physiological samples, in diagnostic methods, etc.

Anti-MTSP protein antibodies, or fragments thereof, containing the binding domain may be used as therapeutic agents.

### 2. Peptides and Peptide Mimetics

Provided herein are methods for identifying molecules that bind to and modulate the protease activity of MTSP proteins. Included among molecules that bind to MTSPs, particularly the single chain protease domain or catalytically active fragments thereof, are peptides and peptide mimetics. Peptide mimetics are molecules or compounds that mimic the necessary molecular conformation of a ligand or polypeptide for specific binding to a target molecule such as, *e.g.*, an MTSP protein. In an exemplary embodiment, the peptides or peptide mimetics bind to the protease domain of the MTSP protein. Such peptides and peptide mimetics include those of antibodies that specifically bind an MTSP protein and, preferably, bind to the protease domain of an MTSP protein. The peptides and peptide mimetics identified by methods provided herein can be agonists or antagonists of MTSP proteins.

Such peptides and peptide mimetics may be useful for diagnosing, treating, preventing, and screening for a disease or disorder associated with MTSP protein activity in a mammal. In addition, the peptides and peptide mimetics may be useful for identifying, isolating, and purifying molecules or compounds that modulate the activity of an MTSP protein, or specifically bind to an MTSP protein, preferably, the protease domain of an MTSP protein. Low molecular weight peptides and peptide mimetics can have strong binding properties to a target molecule, *e.g.*, an MTSP protein or, preferably, to the protease domain of an MTSP protein.

Peptides and peptide mimetics that bind to MTSP proteins as described herein can be administered to mammals, including humans, to modulate MTSP protein activity.

Accordingly, the peptides and peptide mimetics that bind to an MTSP protein can be used generating pharmaceutical compositions containing, as an active ingredient, at least one of the peptides or peptide mimetics in association with a pharmaceutical carrier or diluent. The compounds can be administered, for example, by oral, pulmonary, parental (intramuscular, intraperitoneal, intravenous (IV) or subcutaneous injection), inhalation (via a fine powder formulation), transdermal, nasal, vaginal, rectal, or sublingual routes of administration and can be formulated in dosage forms appropriate for each route of administration (see, *e.g.,* International PCT application Nos. WO 93/25221 and WO 94/17784; and European Patent Application 613,683).

Peptides and peptide mimetics that bind to MTSP proteins are useful *in vitro* as unique tools for understanding the biological role of MTSP proteins, including the evaluation of the many factors thought to influence, and be influenced by, the production of MTSP protein. Such peptides and peptide mimetics are also useful in the development of other compounds that bind to and modulate the activity of an MTSP protein, because such compounds provide important information on the relationship between structure and activity that should facilitate such development.

The peptides and peptide mimetics are also useful as competitive binders in assays to screen for new MTSP proteins or MTSP protein agonists. In such assay embodiments, the compounds can be used without modification or can be modified in a variety of ways; for example, by labeling, such as covalently or non-covalently joining a moiety which directly or indirectly provides a detectable signal. In any of these assays, the materials thereto can be labeled either directly or indirectly. Possibilities for direct labeling include label groups such as: radiolabels such as ¹²⁵I enzymes (U.S. Pat. No. 3,645,090) such as peroxidase and alkaline phosphatase, and fluorescent labels (U.S. Pat. No. 3,940,475) capable of monitoring the change in fluorescence intensity, wavelength shift, or fluorescence polarization. Possibilities for indirect labeling include biotinylation of one constituent followed by binding to avidin coupled to one of the above label groups. The compounds may also include spacers or linkers in cases where the compounds are to be attached to a solid support.

Moreover, based on their ability to bind to an MTSP protein, the peptides and peptide mimetics can be used as reagents for detecting MTSP proteins in living cells, fixed cells, in biological fluids, in tissue homogenates, in purified, natural biological materials, etc. For example, by labelling such peptides and peptide mimetics, one can identify cells having MTSP proteins. In addition, based on their ability to bind an MTSP protein, the peptides and peptide mimetics can be used in situ staining, FACS (fluorescence-activated cell sorting), Western blotting, ELISA, etc. In addition, based on their ability to bind to an MTSP protein, the peptides and peptide mimetics can be used in purification of MTSP protein polypeptides or in purifying cells expressing the MTSP protein polypeptides, *e.g.,* a polypeptide encoding the protease domain of an MTSP protein.

The peptides and peptide mimetics can also be used as commercial reagents for various medical research and diagnostic uses.

The activity of the peptides and peptide mimetics can be evaluated either *in vitro* or *in vivo* in one of the numerous models described in McDonald (1992) Am. J. of Pediatric Hematology/Oncology, 14:8-21, which is incorporated herein by reference.

### 3. Peptide and peptide mimetic therapy

Peptides and peptide mimetics that can bind to MTSP proteins or the protease domain of MTSP proteins and modulate the activity thereof, or have MTSP protein activity, can be used for treatment of neoplastic diseases. The peptides and peptide mimetics may be delivered, *in vivo* or *ex vivo,* to the cells of a subject in need of treatment. Further, peptides which have MTSP protein activity can be delivered, *in vivo* or *ex vivo,* to cells which carry mutant or missing alleles encoding the MTSP protein gene. Any of the techniques described herein or known to the skilled artisan can be used for preparation and *in vivo* or *ex vivo* delivery of such peptides and peptide mimetics that are substantially free of other human proteins. For example, the peptides can be readily prepared by expression in a microorganism or synthesis *in vitro.*

The peptides or peptide mimetics can be introduced into cells, *in vivo* or *ex vivo,* by microinjection or by use of liposomes, for example. Alternatively, the peptides or peptide mimetics may be taken up by cells, *in vivo* or *ex vivo,* actively or by diffusion. In addition, extracellular application of the peptide or peptide mimetic may be sufficient to effect treatment of a neoplastic disease. Other molecules, such as drugs or organic compounds, that: 1) bind to an MTSP protein or protease domain thereof; or 2) have a similar function or activity to an MTSP protein or protease domain thereof, may be used in methods for treatment.

### 4. Rational drug design

The goal of rational drug design is to produce structural analogs of biologically active polypeptides or peptides of interest or of small molecules or peptide mimetics with which they interact (e.g., agonists, antagonists, inhibitors) in order to fashion drugs which are, e.g., more active or stable forms thereof; or which, e.g., enhance or interfere with the function of a polypeptide *in vivo* (e.g., an MTSP protein). In one approach, one first determines the three-dimensional structure of a protein of interest (e.g., an MTSP protein or polypeptide having a protease domain) or, for example, of a MTSP protein-ligand complex, by X-ray crystallography, by computer modeling or most typically, by a combination of approaches (see, *e.g.,* Erickson *et al.* 1990). Also, useful information regarding the structure of a polypeptide may be gained by modeling based on the structure of homologous proteins. In addition, peptides can be analyzed by an alanine scan. In this technique, an amino acid residue is replaced by Ala, and its effect on the peptide's activity is determined. Each of the amino acid residues of the peptide is analyzed in this manner to determine the important regions of the peptide.

Also, a polypeptide or peptide that binds to an MTSP protein or, preferably, the protease domain of an MTSP protein, can be selected by a functional assay, and then the crystal structure of this polypeptide or peptide can be determined. The polypeptide can be, for example, an antibody specific for an MTSP protein or the protein domain of an MTSP protein. This approach can yield a pharmacore upon which subsequent drug design can be based. Further, it is possible to bypass the crystallography altogether by generating anti-idiotypic polypeptides or peptides, (anti-ids) to a functional, pharmacologically active polypeptide or peptide that binds to an MTSP protein or protease domain of an MTSP protein. As a mirror image of a mirror image, the binding site of the anti-ids is expected to be an analog of the original target molecule, e.g., an MTSP protein or polypeptide having an MTSP protein. The anti-id could then be used to identify and isolate peptides from banks of chemically or biologically produced banks of peptides. Selected peptides would then act as the pharmacore.

Thus, one may design drugs which have, e.g., improved activity or stability or which act as modulators (e.g., inhibitors, agonists, antagonists, etc.) of MTSP protein activity, and are useful in the methods, particularly the methods for diagnosis, treatment, prevention, and screening of a neoplastic disease. By virtue of the availability of cloned MTSP protein sequences, sufficient amounts of the MTSP protein polypeptide may be made available to perform such analytical studies as X-ray crystallography. In addition, the knowledge of the amino acid sequence of an MTSP protein or the protease domain thereof, e.g., the protease domain encoded by the amino acid sequence of SEQ ID NO: 2, can provide guidance on computer modeling techniques in place of, or in addition to, X-ray crystallography.

### Methods of identifying peptides and peptide mimetics that bind to MTSP proteins

Peptides having a binding affinity to the MTSP protein polypeptides provided herein (*e.g*., an MTSP protease domain of an MTSP protein) can be readily identified, for example, by random peptide diversity generating systems coupled with an affinity enrichment process. Specifically, random peptide diversity generating systems include the "peptides on plasmids" system (see, *e.g.,* U.S. Patent Nos. 5,270,170 and 5,338,665); the "peptides on phage" system (see, *e.g.,* U.S. Patent No. 6,121,238 and Cwirla,et al. (1990) Proc. Natl. Acad. Sci. U.S.A. 87:6378-6382); the "polysome system;" the "encoded synthetic library (ESL)" system; and the "very large scale immobilized polymer synthesis" system (see, *e.g.,* U.S. Patent No. 6,121,238; and Dower et al. (1991) An. Rep. Med. Chem. 26:271-280

For example, using the procedures described above, random peptides can generally be designed to have a defined number of amino acid residues in length (*e.g*., 12). To generate the collection of oligonucleotides encoding the random peptides, the codon motif (NNK)x, where N is nucleotide A, C, G, or T (equimolar; depending on the methodology employed, other nucleotides can be employed), K is G or T (equimolar), and x is an integer corresponding to the number of amino acids in the peptide (*e.g*., 12) can be used to specify any one of the 32 possible codons resulting from the NNK motif: 1 for each of 12 amino acids, 2 for each of 5 amino acids, 3 for each of 3 amino acids, and only one of the three stop codons. Thus, the NNK motif encodes all of the amino acids, encodes only one stop codon, and reduces codon bias.

The random peptides can be presented, for example, either on the surface of a phage particle, as part of a fusion protein containing either the pIII or the pVIII coat protein of a phage fd derivative (peptides on phage) or as a fusion protein with the Lacl peptide fusion protein bound to a plasmid (peptides on plasmids). The phage or plasmids, including the DNA encoding the peptides, can be identified and isolated by an affinity enrichment process using immobilized MTSP protein polypeptide having a protease domain. The affinity enrichment process, sometimes called "panning," typically involves multiple rounds of incubating the phage, plasmids, or polysomes with the immobilized MTSP protein polypeptide, collecting the phage, plasmids, or polysomes that bind to the MTSP protein polypeptide (along with the accompanying DNA or mRNA), and producing more of the phage or plasmids (along with the accompanying Lacl-peptide fusion protein) collected.

### Characteristics of peptides and peptide mimetics

Typically, the molecular weight of preferred peptides or peptide mimetics is from about 250 to about 8,000 daltons. If the peptides are oligomerized, dimerized and/or derivatized with a hydrophilic polymer (*e.g*., to increase the affinity and/or activity of the compounds), the molecular weights of such peptides can be substantially greater and can range anywhere from about 500 to about 120,000 daltons, more preferably from about 8,000 to about 80,000 daltons. Such peptides can comprise 9 or more amino acids wherein the amino acids are naturally occurring or synthetic (non-naturally occurring) amino acids. One skilled in the art would know how to determine the affinity and molecular weight of the peptides and peptide mimetics suitable for therapeutic and/or diagnostic purposes (*e.g.*, see Dower et al., U.S. Patent No. 6,121,238).

The peptides may be covalently attached to one or more of a variety of hydrophilic polymers. Suitable hydrophilic polymers include, but are not limited to, polyalkylethers as exemplified by polyethylene glycol and polypropylene glycol, polylactic acid, polyglycolic acid, polyoxyalkenes, polyvinylalcohol, polyvinylpyrrolidone, cellulose and cellulose derivatives, dextran and dextran derivatives, etc. When the peptide compounds are derivatized with such polymers, their solubility and circulation half-lives can be increased with little, if any, diminishment in their binding activity. The peptide compounds may be dimerized and each of the dimeric subunits can be covalently attached to a hydrophilic polymer. The peptide compounds can be PEGylated, i.e., covalently attached to polyethylene glycol (PEG).

Peptide analogs are commonly used in the pharmaceutical industry as non-peptide drugs with properties analogous to those of the template peptide. These types of non-peptide compounds are termed "peptide mimetics" or "peptidomimetics" (Luthman et al., A Textbook of Drug Design and Development, 14:386-406, 2nd Ed., Harwood Academic Publishers (1996); Joachim Grante (1994) Angew. Chem. Int. Ed. Engl., 33:1699-1720; Fauchere (1986) J. Adv. Drug Res., 15:29; Veber and Freidinger (1985) TINS, p. 392; and Evans et al. (1987) J. Med. Chem. 30:1229). Peptide mimetics that are structurally similar to therapeutically useful peptides may be used to produce an equivalent or enhanced therapeutic or prophylactic effect. Preparation of peptidomimetics and structures thereof are known to those of skill in this art.

Systematic substitution of one or more amino acids of a consensus sequence with a D-amino acid of the same type (*e.g*., D-lysine in place of L-lysine) may be used to generate more stable peptides. In addition, constrained peptides containing a consensus sequence or a substantially identical consensus sequence variation may be generated by methods known in the art (Rizo et al. (1992) An. Rev. Biochem., 61:387, incorporated herein by reference); for example, by adding internal cysteine residues capable of forming intramolecular disulfide bridges which cyclize the peptide.

Those skilled in the art would appreciate that modifications may be made to the peptides and mimetics without deleteriously effecting the biological or functional activity of the peptide. Further, the skilled artisan would know how to design non-peptide structures in three dimensional terms, that mimic the peptides that bind to a target molecule, *e.g.,* an MTSP protein or, preferably, the protease domain of MTSP proteins (see, *e.g.,* Eck and Sprang (1989) J. Biol. Chem., 26: 17605-18795).

When used for diagnostic purposes, the peptides and peptide mimetics may be labeled with a detectable label and, accordingly, the peptides and peptide mimetics without such a label can serve as intermediates in the preparation of labeled peptides and peptide mimetics. Detectable labels can be molecules or compounds, which when covalently attached to the peptides and peptide mimetics, permit detection of the peptide and peptide mimetics *in vivo,* for example, in a patient to whom the peptide or peptide mimetic has been administered, or *in vitro, e.g.,* in a sample or cells. Suitable detectable labels are well known in the art and include, by way of example, radioisotopes, fluorescent labels (*e.g*., fluorescein), and the like. The particular detectable label employed is not critical and is selected relative to the amount of label to be employed as well as the toxicity of the label at the amount of label employed. Selection of the label relative to such factors is well within the skill of the art.

Covalent attachment of a detectable label to the peptide or peptide mimetic is accomplished by conventional methods well known in the art. For example, when the ¹²⁵I radioisotope is employed as the detectable label, covalent attachment of ¹²⁵I to the peptide or the peptide mimetic can be achieved by incorporating the amino acid tyrosine into the peptide or peptide mimetic and then iodinating the peptide (see, *e.g.,* Weaner et al. (1994) Synthesis and Applications of Isotopically Labelled Compounds, pp. 137-140). If tyrosine is not present in the peptide or peptide mimetic, incorporation of tyrosine to the N or C terminus of the peptide or peptide mimetic can be achieved by well known chemistry. Likewise, ³²P can be incorporated onto the peptide or peptide mimetic as a phosphate moiety through, for example, a hydroxyl group on the peptide or peptide mimetic using conventional chemistry.

Labeling of peptidomimetics usually involves covalent attachment of one or more labels, directly or through a spacer (*e.g.*, an amide group), to non-interfering position(s) on the peptidomimetic that are predicted by quantitative structure-activity data and/or molecular modeling. Such non-interfering positions generally are positions that do not form direct contacts with the macromolecules(s) to which the peptidomimetic binds to produce the therapeutic effect. Derivatization (*e.g*., labeling) of peptidomimetics should not substantially interfere with the desired biological or pharmacological activity of the peptidomimetic.

### 6. Methods of preparing peptides and peptide mimetics

Peptides that bind to MTSP proteins can be prepared by classical methods known in the art, for example, by using standard solid phase techniques. The standard methods include exclusive solid phase synthesis, partial solid phase synthesis methods, fragment condensation, classical solution synthesis, and even by recombinant DNA technology (see, *e.g.,* Merrifield (1963) J. Am. Chem. Soc., 85:2149, incorporated herein by reference.)

Using the "encoded synthetic library" or "very large scale immobilized polymer synthesis" systems (see, *e.g*., U.S. Patent No. 5,925,525, and 5,902,723); one can not only determine the minimum size of a peptide with the activity of interest, one can also make all of the peptides that form the group of peptides that differ from the preferred motif (or the minimum size of that motif) in one, two, or more residues. This collection of peptides can then be screened for ability to bind to the target molecule, *e.g*., and MTSP protein or, preferably, the protease domain of an MTSP protein. This immobilized polymer synthesis system or other peptide synthesis methods can also be used to synthesize truncation analogs and deletion analogs and combinations of truncation and deletion analogs of the peptide compounds.

These procedures can also be used to synthesize peptides in which amino acids other than the 20 naturally occurring, genetically encoded amino acids are substituted at one, two, or more positions of the peptide. For instance, naphthylalanine can be substituted for tryptophan, facilitating synthesis. Other synthetic amino acids that can be substituted into the peptides include L-hydroxypropyl, L-3, 4-dihydroxy-phenylalanyl, d amino acids such as L-d-hydroxylysyl and D-d-methylalanyl, L-α-methylalanyl, β amino acids, and isoquinolyl. D amino acids and non-naturally occurring synthetic amino acids can also be incorporated into the peptides (see, *e.g.,* Roberts et al. (1983) Unusual Amino/Acids in Peptide Synthesis, 5(6):341-449).

The peptides may also be modified by phosphorylation (see, *e.g.,* W. Bannwarth et al. (1996) Biorganic and Medicinal Chemistry Letters, 6(17):2141-2146), and other methods for making peptide derivatives (see, *e.g.,* Hruby et al. (1990) Biochem. J., 268(2):249-262). Thus, peptide compounds also serve as a basis to prepare peptide mimetics with similar biological activity.

Those of skill in the art, recognize that a variety of techniques are available for constructing peptide mimetics with the same or similar desired biological activity as the corresponding peptide compound but with more favorable activity than the peptide with respect to solubility, stability, and susceptibility to hydrolysis and proteolysis (see, *e.g.,* Morgan et al. (1989) An. Rep. Med. Chem., 24:243-252). Methods for preparing peptide mimetics modified at the N-terminal amino group, the C-terminal carboxyl group, and/or changing one or more of the amido linkages in the peptide to a non-amido linkage are known to those of skill in the art.

Amino terminus modifications include alkylating, acetylating, adding a carbobenzoyl group, forming a succinimide group, etc. (see, *e.g.,* Murray et al. (1995) Burger's Medicinal Chemistry and Drug Discovery, 5th ed., Vol. 1, Manfred E. Wolf, ed., John Wiley and Sons, Inc.). C-terminal modifications include mimetics wherein the C-terminal carboxyl group is replaced by an ester, an amide or modifications to form a cyclic peptide.

In addition to N-terminal and C-terminal modifications, the peptide compounds, including peptide mimetics, can advantageously be modified with or covalently coupled to one or more of a variety of hydrophilic polymers. It has been found that when peptide compounds are derivatized with a hydrophilic polymer, their solubility and circulation half-lives may be increased and their immunogenicity is masked, with little, if any, diminishment in their binding activity. Suitable nonproteinaceous polymers include, but are not limited to, polyalkylethers as exemplified by polyethylene glycol and polypropylene glycol, polylactic acid, polyglycolic acid, polyoxyalkenes, polyvinylalcohol, polyvinylpyrrolidone, cellulose and cellulose derivatives, dextran and dextran derivatives, etc. Generally, such hydrophilic polymers have an average molecular weight ranging from about 500 to about 100,000 daltons, more preferably from about 2,000 to about 40,000 daltons and, even more preferably, from about 5,000 to about 20,000 daltons. The hydrophilic polymers also can have an average molecular weights of about 5,000 daltons, 10,000 daltons and 20,000 daltons.

Methods for derivatizing peptide compounds or for coupling peptides to such polymers have been described (see, *e.g.,* Zallipsky (1995) Bioconjugate Chem., 6:150-165; Monfardini et al. (1995) Bioconjugate Chem., 6:62-69; U.S. Pat. No. 4,640,835; U.S. Pat. No. 4,496,689; U.S. Pat. No. 4,301,144; U.S. Pat. No. 4,670,417; U.S. Pat. No. 4,791,192; U.S. Pat. No. 4,179,337 and WO 95/34326, all of which are incorporated by reference in their entirety herein).

Other methods for making peptide derivatives are described, for example, in Hruby et al. (1990), Biochem J., 268(2):249-262, which is incorporated herein by reference. Thus, the peptide compounds also serve as structural models for non-peptidic compounds with similar biological activity. Those of skill in the art recognize that a variety of techniques are available for constructing compounds with the same or similar desired biological activity as a particular peptide compound but with more favorable activity with respect to solubility, stability, and susceptibility to hydrolysis and proteolysis (see, *e.g.,* Morgan et al. (1989) An. Rep. Med. Chem., 24:243-252, incorporated herein by reference). These techniques include replacing the peptide backbone with a backbone composed of phosphonates, amidates, carbamates, sulfonamides, secondary amines, and N-methylamino acids.

Peptide compounds may exist in a cyclized form with an intramolecular disulfide bond between the thiol groups of the cysteines. Alternatively, an intermolecular disulfide bond between the thiol groups of the cysteines can be produced to yield a dimeric (or higher oligomeric) compound. One or more of the cysteine residues may also be substituted with a homocysteine.

### 1. CONJUGATES

A conjugate, containing: a) a single chain protease domain (or proteolytically active portion thereof) of an MTSP protein or an MTSP3, MTSP4 or MTSP6 ; and b) a targeting agent linked to the MTSP protein directly or via a linker, wherein the agent facilitates: i) affinity isolation or purification of the conjugate; ii) attachment of the conjugate to a surface; iii) detection of the conjugate; or iv) targeted delivery to a selected tissue or cell, is provided herein. The conjugate can be a chemical conjugate or a fusion protein mixture thereof.

The targeting agent is preferably a protein or peptide fragment, such as a tissue specific or tumor specific monoclonal antibody or growth factor or fragment thereof linked either directly or via a linker to an MTSP protein protease domain . The targeting agent may also be a protein or peptide fragment that contains a protein binding sequence, a nucleic acid binding sequence, a lipid binding sequence, a polysaccharide binding sequence, or a metal binding sequence, or a linker for attachment to a solid support. In a particular embodiment, the conjugate contains a) the MTSP or portion thereof, as described herein; and b) a targeting agent linked to the MTSP protein directly or via a linker.

Conjugates, such as fusion proteins and chemical conjugates, of the MTSP protein with a protein or peptide fragment (or plurality thereof) that functions, for example, to facilitate affinity isolation or purification of the MTSP protein domain, attachment of the MTSP protein domain to a surface, or detection of the MTSP protein domain are provided. The conjugates can be produced by chemical conjugation, such as via thiol linkages, but are preferably produced by recombinant means as fusion proteins. In the fusion protein, the peptide or fragment thereof is linked to either the N-terminus or C-terminus of the MTSP protein domain. In chemical conjugates the peptide or fragment thereof may be linked anywhere that conjugation can be effected, and there may be a plurality of such peptides or fragments linked to a single MTSP protein domain or to a plurality thereof.

The targeting agent is preferably for *in vitro* delivery to a cell or tissue, and includes agents such as cell or tissue-specific antibodies, growth factors and other factors expressed on specific cells; and other cell or tissue specific agents the promote directed delivery of a linked protein.

Most preferably the targeting agent specifically delivers the MTSP protein to selected cells by interaction with a cell surface protein and internalization of conjugate or MTSP protein portion thereof. These conjugate are used in a variety of methods and are particularly suited for use in methods of activation of prodrugs, such as prodrugs that upon cleavage by the particular MTSP protein are cytotoxic. The prodrugs are administered prior to simultaneously with or subsequently to the conjugate. Upon delivery to the targeted cells, the protease activates the prodrug, which then exhibits is therapeutic effect, such as a cytotoxic effect.

### 1. Conjugation

Conjugates with linked MTSP protein domains can be prepared either by chemical conjugation, recombinant DNA technology, or combinations of recombinant expression and chemical conjugation. The MTSP protein domains and the targeting agent may be linked in any orientation and more than one targeting agents and/or MTSP protein domains may be present in a conjugate.

### a. Fusion proteins

Fusion proteins are proved herein. A fusion protein contains: a) one or a plurality of domains of an MTSP proteins and b) a targeting agent. The fusion proteins are preferably produced by recombinant expression of nucleic acids that encode the fusion protein.

### b. Chemical conjugation

To effect chemical conjugation herein, the MTSP protein domain is linked via one or more selected linkers or directly to the targeting agent. Chemical conjugation must be used if the targeted agent is other than a peptide or protein, such a nucleic acid or a non-peptide drug. Any means known to those of skill in the art for chemically conjugating selected moieties may be used.

### 2. Linkers

Linkers for two purposes are contemplated herein. The conjugates may include one or more linkers between the MTSP protein portion and the targeting agent. Additionally, linkers are used for facilitating or enhancing immobilization of an MTSP protein or portion thereof on a solid support, such as a microtiter plate, silicon or silicon-coated chip, glass or plastic support, such as for high throughput solid phase screening protocols.

Any linker known to those of skill in the art for preparation of conjugates may be used herein. These linkers are typically used in the preparation of chemical conjugates; peptide linkers may be incorporated into fusion proteins.

Linkers can be any moiety suitable to associate a domain of MTSP protein and a targeting agent. Such linkers and linkages include, but are not limited to, peptidic linkages, amino acid and peptide linkages, typically containing between one and about 60 amino acids, more generally between about 10 and 30 amino acids, chemical linkers, such as heterobifunctional cleavable cross-linkers, including but are not limited to, N-succinimidyl (4-iodoacetyl)-aminobenzoate, sulfosuccinimydil (4-iodoacetyl)-aminobenzoate, 4-succinimidyl-oxycarbonyl-a-(2-pyridyldithio)toluene, sulfosuccinimidyl-6- [a-methyl-a-(pyridyldithiol)-toluamido] hexanoate, N-succinimidyl-3-(-2-pyridyldithio) - propionate, succinimidyl 6[3(-(-2-pyridyldithio)-proprionamido] hexanoate, sulfosuccinimidyl 6[3(-(-2-pyridyldithio)-propionamido] hexanoate, 3-(2-pyridyldithio)-propionyl hydrazide, Ellman's reagent, dichlorotriazinic acid, and S-(2-thiopyridyl)-L-cysteine. Other linkers include, but are not limited to peptides and other moieties that reduce stearic hindrance between the domain of MTSP protein and the targeting agent, intracellular enzyme substrates, linkers that increase the flexibility of the conjugate, linkers that increase the solubility of the conjugate, linkers that increase the serum stability of the conjugate, photocleavable linkers and acid cleavable linkers.

Other exemplary linkers and linkages that are suitable for chemically linked conjugates include, but are not limited to, disulfide bonds, thioether bonds, hindered disulfide bonds, and covalent bonds between free reactive groups, such as amine and thiol groups. These bonds are produced using heterobifunctional reagents to produce reactive thiol groups on one or both of the polypeptides and then reacting the thiol groups on one polypeptide with reactive thiol groups or amine groups to which reactive maleimido groups or thiol groups can be attached on the other. Other linkers include, acid cleavable linkers, such as bismaleimideothoxy propane, acid labile-transferrin conjugates and adipic acid dihydrazide, that would be cleaved in more acidic intracellular compartments; cross linkers that are cleaved upon exposure to UV or visible light and linkers, such as the various domains, such as C_{H}1, C_{H}2, and C_{H}3, from the constant region of human IgG₁ (see, Batra et al. Molecular Immunol., 30:379-386 (1993)). In some embodiments, several linkers may be included in order to take advantage of desired properties of each linker.

Chemical linkers and peptide linkers may be inserted by covalently coupling the linker to the domain of MTSP protein and the targeting agent. The heterobifunctional agents, described below, may be used to effect such covalent coupling. Peptide linkers may also be linked by expressing DNA encoding the linker and TA, linker and targeted agent, or linker, targeted agent and TA as a fusion protein. Flexible linkers and linkers that increase solubility of the conjugates are contemplated for use, either alone or with other linkers are also contemplated herein.

### a) Acid cleavable, photocleavable and heat sensitive linkers

Acid cleavable linkers, photocleavable and heat sensitive linkers may also be used, particularly where it may be necessary to cleave the domain of MTSP protein to permit it to be more readily accessible to reaction. Acid cleavable linkers include, but are not limited to, bismaleimideothoxy propane; and adipic acid dihydrazide linkers (see, *e.g.,* Fattom et al. (1992) Infection & Immun. 60:584-589) and acid labile transferrin conjugates that contain a sufficient portion of transferrin to permit entry into the intracellular transferrin cycling pathway (see, *e.g*., Welhöner et al. (1991) J. Biol. Chem. 266:4309-4314).

Photocleavable linkers are linkers that are cleaved upon exposure to light (see, *e.g.,* Goldmacher et al. (1992) Bioconj. Chem. 3:104-107, which linkers are herein incorporated by reference), thereby releasing the targeted agent upon exposure to light. Photocleavable linkers that are cleaved upon exposure to light are known (see, *e.g.,* Hazum et al. (1981) in Pept., Proc. Eur. Pept. Symp., 16th, Brunfeldt, K (Ed), pp. 105-110, which describes the use of a nitrobenzyl group as a photocleavable protective group for cysteine; Yen et al. (1989) Makromol. Chem 190:69-82, which describes water soluble photocleavable copolymers, including hydroxypropylmethacrylamide copolymer, glycine copolymer, fluorescein copolymer and methylrhodamine copolymer; Goldmacher et al. (1992) Bioconj. Chem. 3:104-107, which describes a cross-linker and reagent that undergoes photolytic degradation upon exposure to near UV light (350 nm); and Senter et al. (1985) Photochem. Photobiol 42:231-237, which describes nitrobenzyloxycarbonyl chloride cross linking reagents that produce photocleavable linkages), thereby releasing the targeted agent upon exposure to light. Such linkers would have particular use in treating dermatological or ophthalmic conditions that can be exposed to light using fiber optics. After administration of the conjugate, the eye or skin or other body part can be exposed to light, resulting in release of the targeted moiety from the conjugate. Such photocleavable linkers are useful in connection with diagnostic protocols in which it is desirable to remove the targeting agent to permit rapid clearance from the body of the animal.

### b) Other linkers for chemical conjugation

Other linkers, include trityl linkers, particularly, derivatized trityl groups to generate a genus of conjugates that provide for release of therapeutic agents at various degrees of acidity or alkalinity. The flexibility thus afforded by the ability to preselect the pH range at which the therapeutic agent will be released allows selection of a linker based on the known physiological differences between tissues in need of delivery of a therapeutic agent (see, *e.g.,* U.S. Patent No. 5,612,474). For example, the acidity of tumor tissues appears to be lower than that of normal tissues.

### c) Peptide linkers

The linker moieties can be peptides. Peptide linkers can be employed in fusion proteins and also in chemically linked conjugates. The peptide typically has from about 2 to about 60 amino acid residues, for example from about 5 to about 40, or from about 10 to about 30 amino acid residues. The length selected will depend upon factors, such as the use for which the linker is included.

Peptide linkers are advantageous when the targeting agent is proteinaceous. For example, the linker moiety can be a flexible spacer amino acid sequence, such as those known in single-chain antibody research. Examples of such known linker moieties include, but are not limited to, peptides, such as (GlyₘSer)ₙ and (SerₘGly)ₙ, in which n is 1 to 6, preferably 1 to 4, more preferably 2 to 4, and m is 1 to 6, preferably 1 to 4, more preferably 2 to 4, enzyme cleavable linkers and others.

Additional linking moieties are described, for example, in Huston et al., Proc. Natl. Acad. Sci. U.S.A. 85:5879-5883, 1988; Whitlow, M., et al., Protein Engineering 6:989-995, 1993; Newton et al., Biochemistry 35:545-553, 1996; A. J. Cumber et al., Bioconj. Chem. 3:397-401, 1992; Ladurner et al., J. Mol. Biol. 273:330-337, 1997; and U.S. Patent. No. 4,894,443. In some embodiments, several linkers may be included in order to take advantage of desired properties of each linker.

### 3. Targeting agents

Any agent that facilitates detection, immobilization, or purification of the conjugate is contemplated for use herein. For chemical conjugates any moiety that has such properties is contemplated; for fusion proteins, the targeting agent is a protein, peptide or fragment thereof that sufficient to effects the targeting activity. Preferred targeting agents are those that deliver the MTSP protein or portion thereof to selected cells and tissues. Such agents include tumor specific monoclonal antibodies and portions thereof, growth factors, such as FGF, EGF, PDGF, VEGF, cytokines, including chemokines, and other such agents.

### 4. Nucleic acids, plasmids and cells

Isolated nucleic acid fragments encoding fusion proteins are described but do not form part of the invention. The nucleic acid fragment that encodes the fusion protein includes: a) nucleic acid encoding a protease domain of an MTSP protein encoded by a nucleic acid that hybridizes to a nucleic acid having the nucleotide sequence set forth in the SEQ. ID NO:1; and b) nucleic acid encoding a protein, peptide or effective fragment thereof that facilitates: i) affinity isolation or purification of the fusion protein; ii) attachment of the fusion protein to a surface; or iii) detection of the fusion protein. Preferably, the nucleic acid is DNA..

Plasmids for replication and vectors for expression that contain the above nucleic acid fragments are also described. Cells containing the plasmids and vectors are also described. The cells can be any suitable host including, but are not limited to, bacterial cells, yeast cells, fungal cells, plant cells, insect cell and animal cells. The nucleic acids, plasmids, and cells containing the plasmids can be prepared according to methods known in the art including any described herein.

Also described are methods for producing the above fusion proteins. An exemplary method includes the steps of growing, i.e. culturing the cells so that the proliferate, cells containing a plasmid encoding the fusion protein under conditions whereby the fusion protein is expressed by the cell, and recovering the expressed fusion protein. Methods for expressing and recovering recombinant proteins are well known in the art (*See generally,* Current Protocols in Molecular Biology (1998) § 16, John Wiley & Sons, Inc.) and such methods can be used for expressing and recovering the expressed fusion proteins. Preferably, the recombinant expression and recovery methods disclosed in Section B can be used.

The recovered fusion proteins can be isolated or purified by methods known in the art such as centrifugation, filtration, chromatograph, electrophoresis, immunoprecipitation, etc., or by a combination thereof (*See generally,* Current Protocols in Molecular Biology (1998) § 10, John Wiley & Sons, Inc.). The recovered fusion protein may be isolated or purified through affinity binding between the protein or peptide fragment of the fusion protein and an affinity binding moiety. As discussed in the above sections regarding the construction of the fusion proteins, any affinity binding pairs can be constructed and used in the isolation or purification of the fusion proteins. For example, the affinity binding pairs can be protein binding sequences/protein, DNA binding sequences/DNA sequences, RNA binding sequences/RNA sequences, lipid binding sequences/lipid, polysaccharide binding sequences/polysaccharide, or metal binding sequences/metal.

### 5. Immobilization and supports or substrates therefor

In certain embodiments, where the targeting agents are designed for linkage to surfaces, the MTSP protein can be attached by linkage such as ionic or covalent, non-covalent or other chemical interaction, to a surface of a support or matrix material. Immobilization may be effected directly or via a linker. The MTSP protein may be immobilized on any suitable support, including, but are not limited to, silicon chips, and other supports described herein and known to those of skill in the art. A plurality of MTSP protein or protease domains thereof may be attached to a support, such as an array (*i.e*., a pattern of two or more) of conjugates on the surface of a silicon chip or other chip for use in high throughput protocols and formats.

It is also noted that the domains of the MTSP protein can be linked directly to the surface or via a linker without a targeting agent linked thereto. Hence chips containing arrays of the domains of the MTSP protein.

The matrix material or solid supports contemplated herein are generally any of the insoluble materials known to those of skill in the art to immobilize ligands and other molecules, and are those that used in many chemical syntheses and separations. Such supports are used, for example, in affinity chromatography, in the immobilization of biologically active materials, and during chemical syntheses of biomolecules, including proteins, amino acids and other organic molecules and polymers. The preparation of and use of supports is well known to those of skill in this art; there are many such materials and preparations thereof known. For example, naturally-occurring support materials, such as agarose and cellulose, may be isolated from their respective sources, and processed according to known protocols, and synthetic materials may be prepared in accord with known protocols.

The supports are typically insoluble materials that are solid, porous, deformable, or hard, and have any required structure and geometry, including, but not limited to: beads, pellets, disks, capillaries, hollow fibers, needles, solid fibers, random shapes, thin films and membranes. Thus, the item may be fabricated from the matrix material or combined with it, such as by coating all or part of the surface or impregnating particles.

Typically, when the matrix is particulate, the particles are at least about 10-2000 µM, but may be smaller or larger, depending upon the selected application. Selection of the matrices will be governed, at least in part, by their physical and chemical properties, such as solubility, functional groups, mechanical stability, surface area swelling propensity, hydrophobic or hydrophilic properties and intended use.

If necessary, the support matrix material can be treated to contain an appropriate reactive moiety. In some cases, the support matrix material already containing the reactive moiety may be obtained commercially. The support matrix material containing the reactive moiety may thereby serve as the matrix support upon which molecules are linked. Materials containing reactive surface moieties such as amino silane linkages, hydroxyl linkages or carboxysilane linkages may be produced by well established surface chemistry techniques involving silanization reactions, or the like. Examples of these materials are those having surface silicon oxide moieties, covalently linked to gamma-aminopropylsilane, and other organic moieties; N-[3-(triethyoxysilyl)propyl]phthelamic acid; and bis-(2-hydroxyethyl)aminopropyltriethoxysilane. Exemplary of readily available materials containing amino group reactive functionalities, include, but are not limited to, para-aminophenyltriethyoxysilane. Also derivatized polystyrenes and other such polymers are well known and readily available to those of skill in this art (*e.g*., the Tentagel^{®} Resins are available with a multitude of functional groups, and are sold by Rapp Polymere, Tubingen, Germany; see, U.S. Patent No. 4,908,405 and U.S. Patent No. 5,292,814; *see, also* Butz et al., Peptide Res., 7:20-23 (1994); and Kleine et al., Immunobiol., 190:53-66 (1994)).

These matrix materials include any material that can act as a support matrix for attachment of the molecules of interest. Such materials are known to those of skill in this art, and include those that are used as a support matrix. These materials include, but are not limited to, inorganics, natural polymers, and synthetic polymers; including, but are not limited to: cellulose, cellulose derivatives, acrylic resins, glass, silica gels, polystyrene, gelatin, polyvinyl pyrrolidone, co-polymers of vinyl and acrylamide, polystyrene cross-linked with divinylbenzene and others (*see,* Merrifield, Biochemistry, 3:1385-1390 (1964)), polyacrylamides, latex gels, polystyrene, dextran, polyacrylamides, rubber, silicon, plastics, nitrocellulose, celluloses, natural sponges. Of particular interest herein, are highly porous glasses (*see, e.g.,* U.S. Patent No. 4,244,721) and others prepared by mixing a borosilicate, alcohol and water.

Synthetic supports include, but are not limited to: acrylamides, dextran-derivatives and dextran co-polymers, agarose-polyacrylamide blends, other polymers and co-polymers with various functional groups, methacrylate derivatives and co-polymers, polystyrene and polystyrene copolymers (*see, e.g.,* Merrifield, Biochemistry, 3:1385-1390 (1964); Berg et al., in Innovation Perspect. Solid Phase Synth. Collect. Pap., Int. Symp., 1 st, Epton, Roger (Ed), pp. 453-459 (1990); Berg et al., Pept., Proc. Eur. Pept. Symp., 20th, Jung, G. et al. (Eds), pp. 196-198 (1989); Berg et al., J. Am. Chem. Soc., 111:8024-8026 (1989); Kent et al., Isr. J. Chem., 17:243-247 (1979); Kent et al., J. Org. Chem., 43:2845-2852 (1978); Mitchell et al., Tetrahedron Lett., 42:3795-3798 (1976); U.S. Patent No. 4,507,230; U.S. Patent No. 4,006,117; and U.S. Patent No. 5,389,449). Such materials include those made from polymers and co-polymers such as polyvinylalcohols, acrylates and acrylic acids such as polyethylene-co-acrylic acid, polyethylene-co-methacrylic acid, polyethylene-co-ethylacrylate, polyethylene-co-methyl acrylate, polypropylene-co-acrylic acid, polypropylene-co-methyl-acrylic acid, polypropylene-co-ethylacrylate, polypropylene-co-methyl acrylate, polyethylene-co-vinyl acetate, polypropylene-co-vinyl acetate, and those containing acid anhydride groups such as polyethylene-co-maleic anhydride and polypropylene-co-maleic anhydride. Liposomes have also been used as solid supports for affinity purifications (Powell et al. Biotechnol. Bioeng., 33:173 (1989)).

Numerous methods have been developed for the immobilization of proteins and other biomolecules onto solid or liquid supports (*see, e.g.,* Mosbach, Methods in Enzymology, 44 (1976); Weetall, Immobilized Enzymes, Antigens, Antibodies, and Peptides, (1975); Kennedy et al., Solid Phase Biochemistry, Analytical and Synthetic Aspects, Scouten, ed., pp. 253-391 (1983); *see, generally,* Affinity Techniques. Enzyme Purification: Part B. Methods in Enzymology, Vol. 34, ed. W. B. Jakoby, M. Wilchek, Acad. Press, N.Y. (1974); and Immobilized Biochemicals and Affinity Chromatography, Advances in Experimental Medicine and Biology, vol. 42, ed. R. Dunlap, Plenum Press, N.Y. (1974)).

Among the most commonly used methods are absorption and adsorption or covalent binding to the support, either directly or via a linker, such as the numerous disulfide linkages, thioether bonds, hindered disulfide bonds, and covalent bonds between free reactive groups, such as amine and thiol groups, known to those of skill in art (*see, e.g.,* the PIERCE CATALOG, ImmunoTechnology Catalog & Handbook, 1992-1993, which describes the preparation of and use of such reagents and provides a commercial source for such reagents; Wong, Chemistry of Protein Conjugation and Cross Linking, CRC Press (1993); *see also* DeWitt et al., Proc. Natl. Acad. Sci. U.S.A., 90:6909 (1993); Zuckermann et al., J. Am. Chem. Soc., 114:10646 (1992); Kurth et al., J. Am. Chem. Soc., 116:2661 (1994); Ellman et al., Proc. Natl. Acad. Sci. U.S.A., 91:4708 (1994); Sucholeiki, Tetrahedron Lttrs., 35:7307 (1994); Su-Sun Wang, J. Org. Chem., 41:3258 (1976); Padwa et al., J. Org. Chem., 41:3550 (1971); and Vedejs et al., J. Org. Chem., 49:575 (1984), which describe photosensitive linkers).

To effect immobilization, a composition containing the protein or other biomolecule is contacted with a support material such as alumina, carbon, an ion-exchange resin, cellulose, glass or a ceramic. Fluorocarbon polymers have been used as supports to which biomolecules have been attached by adsorption (*see*, U.S. Patent No. 3,843,443; Published International PCT Application WO/86 03840).

### K. PHARMACEUTICAL COMPOSITIONS AND MODES OF ADMINISTRATION

### 2. Formulations and route of administration

The peptides and agents are preferably formulated as pharmaceutical compositions, preferably for single dosage administration. The concentrations of the peptides in the formulations are effective for delivery of an amount, upon administration, that is effective for the intended treatment. Typically, the compositions are formulated for single dosage administration. To formulate a composition, the weight fraction of a peptide or mixture thereof is dissolved, suspended, dispersed or otherwise mixed in a selected vehicle at an effective concentration such that the treated condition is relieved or ameliorated. Pharmaceutical carriers or vehicles suitable for administration of the compounds provided herein include any such carriers known to those skilled in the art to be suitable for the particular mode of administration.

In addition, the peptides may be formulated as the sole pharmaceutically active ingredient in the composition or may be combined with other active ingredients. Liposomal suspensions, including tissue-targeted liposomes, may also be suitable as pharmaceutically acceptable carriers. These may be prepared according to methods known to those skilled in the art. For example, liposome formulations may be prepared as described in U.S. Patent No. 4,522,811.

The active peptide is included in the pharmaceutically acceptable carrier in an amount sufficient to exert a therapeutically useful effect in the absence of undesirable side effects on the patient treated. The therapeuticlly effective concentration may be determined empirically by testing the peptides in known in vitro and in vivo systems, such as the assays provided herein.

The concentration of active peptide in the drug composition will depend on absorption, inactivation and excretion rates of the active compound, the physicochemical characteristics of the compound, the dosage schedule, and amount administered as well as other factors known to those of skill in the art.

Typically a therapeutically effective dosage is contemplated. The amounts administered may be on the order of 0.001 to 1 mg/ml, preferably about 0.005-0.05 mg/ml, more preferably about 0.01 mg/ml, of blood volume. Pharmaceutical dosage unit forms are prepared to provide from about 1 mg to about 1000 mg and preferably from about 10 to about 500 mg, more preferably about 25-75 mg of the essential active ingredient or a combination of essential ingredients per dosage unit form. The precise dosage can be empirically determined.

The active ingredient may be administered at once, or may be divided into a number of smaller doses to be administered at intervals of time. It is understood that the precise dosage and duration of treatment is a function of the disease being treated and may be determined empirically using known testing protocols or by extrapolation from in vivo or in vitro test data. It is to be noted that concentrations and dosage values may also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or use of the claimed compositions and combinations containing them.

Preferred pharmaceutically acceptable derivatives include acids, salts, esters, hydrates, solvates and prodrug forms. The derivative is typically selected such that its pharmacokinetic properties are superior to the corresponding neutral compound.

Thus, effective concentrations or amounts of one or more of the peptides provided herein or pharmaceutically acceptable derivatives thereof are mixed with a suitable pharmaceutical carrier or vehicle for systemic, topical or local administration to form pharmaceutical compositions. Peptides are included in an amount effective for ameliorating or treating the disorder for which treatment is contemplated. The concentration of peptide in the composition will depend on absorption, inactivation, excretion rates of the active compound, the dosage schedule, amount administered, particular formulation as well as other factors known to those of skill in the art.

Solutions or suspensions used for parenteral, intradermal, subcutaneous, or topical application can include any of the following components: a sterile diluent, such as water for injection, saline solution, fixed oil, polyethylene glycol, glycerine, propylene glycol or other synthetic solvent; antimicrobial agents, such as benzyl alcohol and methyl parabens; antioxidants, such as ascorbic acid and sodium bisulfite; chelating agents, such as ethylenediaminetetraacetic acid (EDTA); buffers, such as acetates, citrates and phosphates; and agents for the adjustment of tonicity such as sodium chloride or dextrose. Parenteral preparations can be enclosed in ampules, disposable syringes or single or multiple dose vials made of glass, plastic or other suitable material.

In instances in which the peptides exhibit insufficient solubility, methods for solubilizing compounds may be used. Such methods are known to those of skill in this art, and include, but are not limited to, using cosolvents, such as dimethylsulfoxide (DMSO), using surfactants, such as Tween^{®}, or dissolution in aqueous sodium bicarbonate. Derivatives of the peptides, such as prodrugs of the peptides may also be used in formulating effective pharmaceutical compositions. For ophthalmic indications, the compositions are formulated in an ophthalmically acceptable carrier. For the ophthalmic uses herein, local administration, either by topical administration or by injection is preferred. Time release formulations are also desirable. Typically, the compositions are formulated for single dosage administration, so that a single dose administers an effective amount.

Upon mixing or addition of the peptide with the vehicle, the resulting mixture may be a solution, suspension, emulsion or other composition. The form of the resulting mixture depends upon a number of factors, including the intended mode of administration and the solubility of the compound in the selected carrier or vehicle. If necessary, pharmaceutically acceptable salts or other derivatives of the compounds are prepared.

The peptide is included in the pharmaceutically acceptable carrier in an amount sufficient to exert a therapeutically useful effect in the absence of undesirable side effects on the patient treated. It is understood that number and degree of side effects depends upon the condition for which the peptides are administered. For example, certain toxic and undesirable side effects are tolerated when treating life-threatening illnesses that would not be tolerated when treating disorders of fester consequence.

The peptides can also be mixed with other active materials, that do not impair the desired action, or with materials that supplement the desired action known to those of skill in the art. The formulations of the compounds and agents for use herein include those suitable for oral, rectal, topical, inhalational, buccal (*e.g*., sublingual), parenteral (*e.g.,* subcutaneous, intramuscular, intradermal, or intravenous), transdermal administration or any route. The most suitable route in any given case will depend on the nature and severity of the condition being treated and on the nature of the particular active compound which is being used. The formulations are provided for administration to humans and animals in unit dosage forms, such as tablets, capsules, pills, powders, granules, sterile parenteral solutions or suspensions, and oral solutions or suspensions, and oil-water emulsions containing suitable quantities of the compounds or pharmaceutically acceptable derivatives thereof. The pharmaceutically therapeutically active peptides and derivatives thereof are typically formulated and administered in unit-dosage forms or multiple-dosage forms. Unit-dose forms as used herein refers to physically discrete units suitable for human and animal subjects and packaged individually as is known in the art. Each unit-dose contains a predetermined quantity of the therapeutically active compound sufficient to produce the desired therapeutic effect, in association with the required pharmaceutical carrier, vehicle or diluent. Examples of unit-dose forms include ampoules and syringes and individually packaged tablets or capsules. Unit-dose forms may be administered in fractions or multiples thereof. A multiple-dose form is a plurality of identical unit-dosage forms packaged in a single container to be administered in segregated unit-dose form. Examples of multiple-dose forms include vials, bottles of tablets or capsules or bottles of pints or gallons. Hence, multiple dose form is a multiple of unit-doses which are not segregated in packaging.

The composition can contain along with the active ingredient: a diluent such as lactose, sucrose, dicalcium phosphate, or carboxymethylcellulose; a lubricant, such as magnesium stearate, calcium stearate and talc; and a binder such as starch, natural gums, such as gum acaciagelatin, glucose, molasses, polvinylpyrrolidine, celluloses and derivatives thereof, povidone, crospovidones and other such binders known to those of skill in the art. Liquid pharmaceutically administrable compositions can, for example, be prepared by dissolving, dispersing, or otherwise mixing an active compound as defined above and optional pharmaceutical adjuvants in a carrier, such as, for example, water, saline, aqueous dextrose, glycerol, glycols, ethanol, and the like, to thereby form a solution or suspension. If desired, the pharmaceutical composition to be administered may also contain minor amounts of nontoxic auxiliary substances such as wetting agents, emulsifying agents, or solubilizing agents, pH buffering agents and the like, for example, acetate, sodium citrate, cyclodextrine derivatives, sorbitan monolaurate, triethanolamine sodium acetate, triethanolamine oleate, and other such agents. Methods of preparing such dosage forms are known, or will be apparent, to those skilled in this art (see, *e.g*., Remington's Pharmaceutical Sciences, Mack Publishing Company, Easton, Pa., 15th Edition, 1975). The composition or formulation to be administered will contain a quantity of the active compound in an amount sufficient to alleviate the symptoms of the treated subject.

Dosage forms or compositions containing active ingredient in the range of 0.005% to 100% with the balance made up from non-toxic carrier may be prepared. For oral administration, the pharmaceutical compositions may take the form of, for example, tablets or capsules prepared by conventional means with pharmaceutically acceptable excipients such as binding agents (*e.g*., pregelatinized maize starch, polyvinyl pyrrolidone or hydroxypropyl methylcellulose); fillers (*e.g*., lactose, microcrystalline cellulose or calcium hydrogen phosphate); lubricants (*e.g*., magnesium stearate, talc or silica); disintegrants (*e.g*., potato starch or sodium starch glycolate); or wetting agents (*e.g*., sodium lauryl sulphate). The tablets may be coated by methods well-known in the art.

The pharmaceutical preparation may also be in liquid form, for example, solutions, syrups or suspensions, or may be presented as a drug product for reconstitution with water or other suitable vehicle before use. Such liquid preparations may be prepared by conventional means with pharmaceutically acceptable additives such as suspending agents (*e.g*., sorbitol syrup, cellulose derivatives or hydrogenated edible fats); emulsifying agents (*e.g*., lecithin or acacia); non-aqueous vehicles (*e.g*., almond oil, oily esters, or fractionated vegetable oils); and preservatives (*e.g*., methyl or propyl-p-hydroxybenzoates or sorbic acid).

Formulations suitable for rectal administration are preferably presented as unit dose suppositories. These may be prepared by admixing the active compound with one or more conventional solid carriers, for example, cocoa butter, and then shaping the resulting mixture.

Formulations suitable for topical application to the skin or to the eye preferably take the form of an ointment, cream, lotion, paste, gel, spray, aerosol and oil. Carriers which may be used include vaseline, lanoline, polyethylene glycols, alcohols, and combinations of two or more thereof. The topical formulations may further advantageously contain 0.05 to 15 percent by weight of thickeners selected from among hydroxypropyl methyl cellulose, methyl cellulose, polyvinylpyrrolidone, polyvinyl alcohol, poly (alkylene glycols), poly/hydroxyalkyl, (meth)acrylates or poly(meth)acrylamides. A topical formulation is often applied by instillation or as an ointment into the conjunctival sac. It can also be used for irrigation or lubrication of the eye, facial sinuses, and external auditory meatus. It may also be injected into the anterior eye chamber and other places. The topical formulations in the liquid state may be also present in a hydrophilic three-dimensional polymer matrix in the form of a strip, contact lens, and the like from which the active components are released.

For administration by inhalation, the compounds for use herein can be delivered in the form of an aerosol spray presentation from pressurized packs or a nebulizer, with the use of a suitable propellant, *e.g*., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Capsules and cartridges of, *e.g*., gelatin, for use in an inhaler or insufflator may be formulated containing a powder mix of the compound and a suitable powder base such as lactose or starch.

Formulations suitable for buccal (sublingual) administration include, for example, lozenges containing the active compound in a flavored base, usually sucrose and acacia or tragacanth; and pastilles containing the compound in an inert base such as gelatin and glycerin or sucrose and acacia.

The compounds may be formulated for parenteral administration by injection, *e.g*., by bolus injection or continuous infusion. Formulations for injection may be presented in unit dosage form, *e.g*., in ampules or in multi-dose containers, with an added preservative. The compositions may be suspensions, solutions or emulsions in oily or aqueous vehicles, and may contain formulatory agents such as suspending, stabilizing and/or dispersing agents. Alternatively, the active ingredient may be in powder form for reconstitution with a suitable vehicle, *e.g*., sterile pyrogen-free water or other solvents, before use.

Formulations suitable for transdermal administration may be presented as discrete patches adapted to remain in intimate contact with the epidermis of the recipient for a prolonged period of time. Such patches suitably contain the active compound as an optionally buffered aqueous solution of, for example, 0.1 to 0.2 M concentration with respect to the active compound. Formulations suitable for transdermal administration may also be delivered by iontophoresis (*see, e.g.,* Pharmaceutical Research 3 (6), 318 (1986)) and typically take the form of an optionally buffered aqueous solution of the active compound.

The pharmaceutical compositions may also be administered by controlled release means and/or delivery devices (see, *e.g.,* in U.S. Patent Nos. 3,536,809; 3,598,123; 3,630,200; 3,845,770; 3,847,770; 3,91 6,899; 4,008,719; 4,687,610; 4,769,027; 5,059,595; 5,073,543; 5,720,548; 5,354,566; 5,591,767; 5,639,476; 5,674,533 and 5,733,566).

Desirable blood levels may be maintained by a continuous infusion of the active agent as ascertained by plasma levels. It should be noted that the attending physician would know how to and when to terminate, interrupt or adjust therapy to lower dosage due to toxicity, or bone marrow, liver or kidney dysfunctions. Conversely, the attending physician would also know how to and when to adjust treatment to higher levels if the clinical response is not adequate (precluding toxic side effects).

The efficacy and/or toxicity of the MTSP protein, alone or in combination with other agents can also be assessed by the methods known in the art (See generally, O'Reilly, Investigational New Drugs, 15:5-13 (1997)).

The peptides may be prepared with carriers that protect the compound against rapid elimination from the body, such as time release formulations or coatings.

Finally, the MTSP protease domains or compositions containing any of the preceding agents may be packaged as articles of manufacture containing packaging material, a compound or suitable derivative thereof provided herein, which is effective for treatment of a diseases or disorders contemplated herein, within the packaging material, and a label that indicates that the compound or a suitable derivative thereof is for treating the diseases or disorders contemplated herein. The label can optionally include the disorders for which the therapy is warranted.

### L. METHODS OF TREATMENT

The compounds identified by the methods herein are used for treating or preventing neoplastic diseases in an animal, particularly a mammal, including a human.

The following examples are included for illustrative purposes only and are not intended to limit the scope of the invention.

### EXAMPLE 1

### Cloning of MTSP3, cloning and mutagenesis of the Protease domain of MTSP3

### 1. Identification and cloning of MTSP3

### a. Identification of EST clones AI924527 and AI924182 as part of a serine protease MTSP3

DNA encoding the protease domain of the protease designated MTSP1 was independently cloned from the human prostatic adenocarcinoma cell line, PC-3, using degenerate oligonucleotide primers, then sequenced and characterized (see EXAMPLE 6). The sequence of the sense degenerate primer used in cloning MTSP1 was 5'-TGGRT(I)VT(I)WS(I)GC(I)RC(I)CAYTG-3' (SEQ ID No: 13), and that of the anti-sense was 5'-(I)GG(I)CC(I)CC(I)SWRTC(I)CCYT(I)RCA(I)GHRTC-3' (SEQ ID No:14), where R = A,G; V = G,A,C; W = A,T; S = G,C; Y = C,T; H = A,T,C. The primer sequences correspond to two highly conserved regions in all serine proteases and should amplify PCR products ranging from 400 to 500 base pairs. MTSP1 was subsequently found to be identical to matriptase (Genbank accession number AF1 18224; see also Takeuchi et al., Proc. Natl. Acad. Sci. USA, 96(20):11054-61 (1999); and Lin et al., J. Biol. Chem., 274(26):18231-6 1999).

Using the protein sequence of the protease domain of the serine protease MTSP1, the EST database (dbEST) at the National Center for Biotechnology Information (Bethesda, MD; www.ncbi.nlm.nih.gov) was searched for EST clones that contain similar or identical sequences to MTSP1 using the search algorithm tblastn. The tblastn algorithm compares a protein query sequence against a nucleotide sequence database dynamically translated in all six reading frames (both strands). The sequences for two identical EST clones (NCI_CGAP_Lu19 AI924527 and AI924182) derived from human lung tumor tissue showed 43% identity with the MTSP1 protein sequence. Subsequent search of GenBank and SwissProt database for the EST sequence AI924527 and AI924182 did not show any matching sequence to MTSP1, indicating that the sequence contained in these EST clones AI924527 and AI924182 may be portions of a new serine protease.

### b. PCR cloning of a cDNA fragment of another membrane type serine protease MTSP3

The double-stranded Marathon-Ready(tm) cDNA library derived from human lung carcinoma (LX-1) was obtained from Clontech (Palo Alto, CA; catalog # 7495-1) and used as a template. Two primers, 5'-TCACCGAGAAGATGATGTGTGCAGGCATCC-3' (SEQ ID No:15) (sense primer), and 5'-GGGACAGGGGCTGTAAGGCAGGGAATGAG-3' (SEQ ID No:16) (antisense primer), were used to amplify a ∼360 bp DNA fragment. The PCR product was separated on a 2% agarose gel and purified using a gel extraction kit (catalog number 28706; QIAquick gel extraction kit; Qiagen). The purified DNA fragment was ligated into TA vectors (catalog number K4500-01; TOPO-TA cloning kit, Invitrogen, Carlsbad, CA). After transformation into E. coli cells, plasmids were isolated and analyzed by digestion with EcoRI restriction enzyme. Clones that had inserted DNA were further characterized by sequencing using a fluorescent dye-based DNA sequencing method (catalog number 4303149; BigDye terminator cycle sequencing kit with AmpliTaq DNA polymerase; Perkin Elmer, LincoIn, CA).

The DNA sequence obtained was analyzed and has 43% identity with the MTSP1 protein sequence. This indicates that the LX-1 cDNA library contains a desired nucleic acid molecule. It was used to isolate a cDNA clone encompassing a full length protease.

### c. 5'- and 3'- rapid amplification of cDNA ends (RACE)

To obtain the full-length cDNA that encoded this serine protease, hereafter called MTSP3, 5'- and 3'-RACE reactions were performed. The Marathon-Ready cDNA library from human lung carcinoma (LX-1) was used to isolate the 5' and 3' ends of the cDNA encoding MTSP3. Marathon-Ready cDNA is specifically made for RACE reactions. Two gene specific primers were used: 5'-CCCGCAGCCATAGCCCCAGCTAACG-3' (SEQ ID No. 17) for 5'-RACE reaction and 5'-GCAGACGATGCGTACCAGGGGGAAGTC-3' (SEQ ID No. 18) for 3'-RACE reaction. Two fragments, approximately 1.8 kbp and 0.85 kbp, were isolated that correspond to the missing 5' and 3' end sequences, respectively. These fragments were subcloned as described above. They were further confirmed by Southern analysis using an internal cDNA fragment encompassing the 2 primers used in the RACE reactions as probe and by DNA sequence analysis.

### d. PCR amplification of cDNA encoding full-length protease domain of MTSP3

To obtain the cDNA fragment encoding the protease domain of MTSP3, an end-to-end PCR amplification using gene-specific primers was used. The two primers used were: 5'-CTCGAGAAAAGAGTGGTGGGTGGGGAGGAGGCCTCTGTG-3' (SEQ ID No. 19) for the 5' end and 5'-GCGGCCGCATTACAGCTCAGCCTTCCAGAC-3' (SEQ ID No. 20) for the 3' end. The 5' primer contains the sequence (underlined) that encodes the start of the MTSP3 protease domain (VVGGEEASV). The 3' primer contains the stop codon (underlined) of MTSP3. A ∼700-bp fragment was amplified and subcloned into a Pichia pastoris expression vector, pPIC9K.

### e. C310S mutagenesis of MTSP3

To eliminate the free cysteine (at position 310 in SEQ ID No. 4) that exists when the protease domain of the MTSP3 protein is expressed or the zymogen is activated, the free cysteine at position 310 (see SEQ ID No. 3), which is Cys122 if a chymotrypsin numbering scheme is used, was replaced with a serine. The resulting vector was designated pPIC9K:MTSP3C122S.

The gene encoding the protease domain of MTSP3 was mutagenized by PCR SOE (PCR-based splicing by overlap extension) to replace the unpaired cysteine at position 310 (122 chymotrypsin numbering system) with a serine. Two overlapping gene fragments, each containing the TCT codon for serine at position 310 were PCR amplified using the following primers: for the 5' gene fragment, TCTCTCGAGAAAAGAGTGGTGGGTGGGTGGGGAGGAGGCCTCTGTG SEQ ID No. 51 and GCTCCTCATCAAAGAAGGGCAGAGAGATGGGCCTGACTGTGCC SEQ ID No. 52; for the 3' gene fragment, ATTCGCGGCCGCATTACAGCTCAGCCTTCCAGAC (SEQ ID No. 53) and GGCACAGTCAGGCCCATCTCTCTGCCCTTCTTTGATGAGGAGC (SEQ ID No. 54). The amplified gene fragments were purified on a 1% agarose gel, mixed and reamplified by PCR to produce the full length coding sequence for MTSP3 C122S. This sequence was then cut with restriction enzymes NotI and XhoI, and ligated into vector pPic9K.

### 2. Sequence analysis

All derived DNA and protein sequences were analyzed using MacVector (version 6.5; Oxford Molecular Ltd., Madison, WI). The full-length cDNA encoding MTSP3 is composed of 2,137 base pairs containing the longest open reading frame of 1,314 base pairs which translate to a 437-amino acid protein sequence. The cDNA fragment (nt 873-1,574) encoding the protease domain of MTSP3 is composed of 702 base pairs which translate to a 233-amino acid protein sequence plus the stop codon. The DNA sequence and the translated protein sequence of MTSP3 are shown in SEQ ID Nos. 3 and 4, respectively.

### 3. Construction of the Expression Vectors

DNA encoding MTSP3 full length protein containing the C310S point mutation (i.e., MTSP3C122S) was cloned from pPIC9K:MTSP3C122S. The primers MTSP3:
5' GAATTCCATATGCCGCGCTTTAAAGTGGTGGGTGGGGAGGAGGCC SEQ ID No. 47 (containing a Ndel restriction site) and MTSP3-3' GGGATACCCGTTACAGCTCAGCCTTCCAGAC 5' SEQ ID No. 48 (containing a BamHI restriction site) were used to PCR amplify the human MTSP3C122S protease domain utilizing a plasmin recognition sequence (PRFK) for zymogen activation. Amplification was conducted in a total volume of 50 µl containing 10 mM KCI, 20 mM Tris-HCI (pH 8.8 at 25 °C), 10 mM (NH₄)₂SO₄, 2.0 mM MgSO₄, 0.1% Triton X-100, 0.3 mM dNTPs, 5.0 units of vent DNA polymerase, and 100 pmol of primers. The reaction mixtures were heated to 95 °C for 5 min, followed by 25-30 cycles of 95, 60, and 75 °C for 30 s each and a final extension at 75 °C for 2 min.

PCR products were purified using a QIAquick PCR purification kit (QIAGEN Inc., Chatsworth, CA). Full-length oligonucleotides were doubly digested with 10 units BamHI and 20 units Ndel for 2 h at 37 °C. The digested fragments were purified on a 1.3% agarose gel and stained with ethidium bromide. The band containing the MTSP3C122S encoding DNA was excised and purified using a QIAEX II gel extraction kit.

The MTSP3C122S encoding DNA was then cloned into the Ndel and BamHI sites of the pET19b vector (Novagen) using standard methods. This vector allows the fusion of a HIS₆ tag for purification by metal affinity chromatography (MAC). Competent XL1 Blue cells (Stratagene) were transformed with the pET19b-MTSP3C122S vector and used to produce plasmid stocks. Proper insertion and DNA sequence were confirmed by fluorescent thermal dye DNA sequencing methods as well as restriction digests.

### 4. Protein Expression, Purification, and Refolding

Overexpression of the gene product was achieved in *E. coli* strain BL21 (DE3) (Novagen, Madison WI) containing the DNAY plasmid for rare codon optimization (see, *e.g*., Garcia et al. (1986) Cell 45:.453-459). Cells were grown at 37 °C in (2xYT) media supplemented with carbenicillin and kanamycin to a final concentrations of 50 ug/ml and 34 ug/ml, respectively. One liter cultures were inoculated with 10 mL of an overnight culture grown in the same media. Cells were allowed to grow to a density of 0.6 - 1.0 OD₆₀₀ before the addition of IPTG (final concentration 1.0 mM). Cells were grown an additional 4 hours before harvesting.

The cell pellet was resuspended in 20 mL of lysis buffer (50 mM Na₂HPO₄, 300 mM NaCl, pH 7.4). The cell suspension was treated with 10-20 mg lysozyme and incubated at 37°C for 1 hour. DNasel was then added (1-2mg) with mixing until the solution was no longer viscous. The solution was then transferred to a Rosette flask and sonicated, on ice, at high power for 15 min. Inclusion bodies were pelleted by centrifugation at 20K rpm (∼48,000 g) at 4°C for 30 min.

Inclusion bodies were washed by douncing 2 times in 50 mM Na₂HPO₄, 300 mM NaCl, 5% LADO, pH 7.4 followed by 2 times in 50 mM Na₂HPO₄, 300 mM NaCl, pH 7.4. Inclusion bodies (∼ 500 mg) are solubilized in 25 mL 6 M GuHCl, 100 mM tris-HCl, 20 mM βMe, pH 8.0. This solution was spun at 20K rpm for 30 minutes to pull down any particulate matter. This solution was passed through a 0.2 µM filter and diluted to 100 mL in solubilization buffer.

MTSP3C122S was refolded by slowly adding the inclusion body mixture to 8 L of refolding buffer (100 mM tris-HCl, 150 mM NaCl, 5 mM GSH, 0.05 mM GSSG, 1 M arginine, pH 8.0) using a peristaltic pump. The refolding mixture was allowed to stir at 4°C for 7 days or until the thiol concentration was below 1 mM as detected by Ellman's reagent. The solution was filtered through a 5 µM filter, concentrated by ultrafiltration and the buffer exchanged into MAC equilibration buffer (50 mM Na₂HPO₄, 300 mM NaCl, 10 mM imidazole, pH 8.0) by crossflow filtration. The resulting solution was passed through a 0.2 µM filter and further purified on a FPLC (Amersham-Pharmacia) using Pharmacia chelating sepharose. The solution was loaded onto the nickel loaded MAC at a flow rate of 1.0 mL/min and eluted with a linear gradient of 1.0 mM -1.0 M imidazole in 50 mM Na₂HPO₄, 300 mM NaCl, pH 8.0. Protein containing fractions were determined by SDS-PAGE and subsequently pooled and frozen at -80°C.

Small amounts of purified MTSP3C122S were activated using plasmin sepharose for 30 min. at 37°C. The resin was spun down at 14K rpm for 5 min. and the protein solution removed. The resulting solution was screened for activity against of series of protease substrates; spec-tpa, spec-pl, spec-UK, spec-fXIIa (American Diagnostica), S-2238, S-2266 (Kabi Diagnostica), S-2586, S-2366, S-2444, S-2288, S-2251, S-2302, S-2765, S-2222, spec-THE (Chromogenix), spec-fVIIa (Pentapharm). MTSP3C122S cleaved several of these substrates efficiently but was most active towards Spec-fXlla, Spec-tPA, S-2765, Spec-fVlla and S-2444.

### 5. Gene expression profile of the serine protease MTSP3 in normal and tumor tissues

To obtain information regarding the tissue distribution of the MTSP3 transcripts, the DNA insert encoding the MTSP3 protease domain was used to probe a RNA blot composed of 76 different human tissues (catalog number 7775-1; human multiple tissue expression (MTE) array; CLONTECH, Palo Alto, CA). The expression pattern observed in decreasing signal level was: trachea = colon (descending) = esophagus > colon (ascending) > colon (transverse) = rectum > ileum > duodenum > jejunum > bladder > ilocecum > stomach > kidney > appendix. It is also expressed less abundantly in fetal kidney, and in two tumor cell lines, HeLa S3 and leukemia, K-562. Northern analysis using RNA blots (catalog numbers 7780-1, 7765-1 & 7782-1; human 12-lane, human muscle and human digestive system multiple tissue northern (MTN) blots; CLONTECH) confirmed that the expression was detected most abundantly in the colon, moderately in the esophagus, small intestine, bladder and kidney, and less abundantly in stomach and rectum. A single transcript of ∼2.2 kb was detected.

Amplification of the MTSP3 transcript in several human primary tumors xenografted in mouse was performed using gene-specific primers. The MTSP3 transcript was detected in lung carcinoma (LX-1), colon adenocarcinoma (CX-1), colon adenocarcinoma (GI-112) and ovarian carcinoma (GI-102). No apparent signal was detected in another form of lung carcinoma (GI-117), breast carcinoma (GI-101), pancreatic adenocarcinoma (GI-103) and prostatic adenocarcinoma (PC3).

### EXAMPLE 2

### Identification of genomic clone of MTSP4

Using the nucleotide sequence encoding the protease domain of the serine protease MTSP1 (also called matriptase), the protein database (SWISSPROT) at the National Center for Biotechnology information (Bethesda, MD; <http://www.ncbi.nlm.nih.gov>) was searched for similar or identical sequence to MTSP1 using the search algorithm blastx. The blastx algorithm compares the six-frame conceptual translation products of a nucleotide query sequence (both strands) against a protein sequence database. A protein encoding sequence (CAA18442) that has 37% identity to the MTSP1 protein sequence that was found to include a putative LDL-receptor domain and a trypsin-like serine protease domain was identified. This protein-encoding sequence (hereinafter referred to as MTSP4) was found to be encoded by a genomic clone (AL022314) derived from human chromosome 22 sequenced by the Sanger Centre Chromosome 22 Mapping Group and deposited into the public database as part of the Human Genome Project. Subsequent search of the GenBank database showed that no identical sequence has been deposited. A search of the EST database also did not show any matching human sequence, indicating that no human EST clone exists in the public database. Mouse EST clones (AI391417 and AA208793) are present and showed 88% identity to the serine protease at the nucleotide level.

### PCR cloning of a genomic DNA fragment of MTSP4 for use as hybridization probe

In order to obtain tissue distribution profile of MTSP4 as well as to identify a tissue source for subsequent cloning of the cDNA, a genomic fragment was amplified from human genomic DNA using two gene-specific primers, 5'-CCTCCACGGTGCTGTGGACCGTGTTCC-3' (5' primer) SEQ ID No. 21 and 5'-CCTCGCGCAAGGCGCCCCAGCCCG-3' (3' primer) SEQ ID No. 22. These two primers amplified a 265-base pair fragment within a single exon of MTSP4. The fragment was then used as a hybridization probe on human tissue northern blot (human 12-lane multiple tissue northern (MTN) blot (catalog number 7780-1); CLONTECH, Palo Alto, CA). A prominent band (∼ 2.6 kb) was detected in liver. Relatively weaker signals were obtained from the brain, heart, skeletal muscle and kidney. Since human liver showed a very strong signal, this tissue was selected for the amplification of the MTSP4 cDNA.

### 5'- and 3'- rapid amplification of cDNA ends (RACE)

To obtain a full-length clone encoding MTSP4, 5'- and 3'-RACE reactions were performed. The Marathon-Ready cDNA library from human liver (CLONTECH) was used to isolate the 5' and 3' ends of the cDNA encoding MTSP4. Marathon-Ready cDNA clones are specifically made for RACE reactions. Two gene specific primers were used: 5'-GCGTGGCGTCACCTGGTAGCGATAGACCTCGC -3' (SEQ ID No. 23) for 5'-RACE reaction and 5'-CCTCCACGGTGCTGTGGACCGTGTTCC-3' (SEQ ID No. 24) for 3'-RACE reaction. No fragment was obtained from the initial 5'-RACE reaction.

The 3'-RACE reaction, however, produced a ∼ 1.5 kbp fragment. A nested PCR reaction was used on the initial 5'-RACE reaction products to obtain part of the 5' end of MTSP4. The nested 5' gene-specific primer used was 5'-CCTCGCGCAAGGCGCCCCAGCCCG-3' (SEQ ID No. 25) and produced a ∼ 0.8 kbp fragment. The fragments were subcloned into pCR2.1-TOPO TA cloning vector (Invitrogen, Carlsbad, CA). The resulting clones were analyzed by Southern analysis using the internal genomic fragment encompassing the primers used in the RACE reactions as probe and by DNA sequence analysis. Sequence analysis of the 5'-RACE product showed that the potential initiation codon was still missing.

To obtain the 5' cDNA end that encodes the N terminus of MTSP4, the publicly available genomic sequence of chromosome 22 was searched for sequence corresponding to the sequence obtained in the 5'-RACE clone. The resulting genomic sequence was translated and the protein sequence was compared to that derived from the translated sequence of the 5'-RACE clone. After determining the overlapping sequences, a gene-specific oligonucleotide primer (5'-TCATCGGCCAGAGGGTGATCAGTGAG-3') SEQ ID No. 26 corresponding to the sequence upstream of the potential initiation codon and another gene-specific oligonucleotide primer (5'-CCTCCTCAGTGCATAGGCATCAAACCAG-3') SEQ ID No. 27 corresponding to a sequence within the overlapping region were used to amplify the missing 5' cDNA of MTSP4 from the human liver cDNA library.

### Splice variants and domain organization of MTSP4

At least two cDNA fragments were consistently obtained during PCR amplification, indicating multiple splice variants of MTSP4. Subcloning and sequence analysis revealed that a longer, more abundant form, MTSP4-L and a shorter form, MTSP4-S. The encoded proteins are multi-domain, type II membrane-type serine proteases and include a transmembrane domain at the N terminus followed by a CUB domain, 3 LDLR domains and a trypsin-like serine protease domain at the C terminus. The difference between these two forms of MTSP4 is the absence in MTSP4-S of a 432-bp nucleotide sequence between the transmembrane and the CUB domains (see FIGURE 2).

### PCR amplification of cDNA encoding full-length protease domain of MTSP4

To obtain a cDNA fragment encoding the protease domain of MTSP4, an end-to-end PCR amplification using gene-specific primers and the Marathon-Ready cDNA library from human liver was used. The two primers used were: 5'-TCTCTCGAGAAAAGAATTGTTGGTGGAGCTGTGTCCTCCGAG -3' (SEQ ID No. 28 ) for the 5' end and 5'-AGGTGGGCCTTGCTTTGCAGGGGGGCAGTTC-3' for the 3' end SEQ ID NO. 29). The 5' primer contained the sequence that encodes the start of the MTSP4 protease domain (IVGGAVSSE). The 3' primer corresponds to the sequence just downstream of the stop codon. A ∼740-bp fragment was amplified, subcloned into pCR2.1-TOPO TA cloning vector and sequenced.

### Gene expression profile of MTSP4 in normal and tumor tissues

To obtain information regarding the gene expression profile of the MTSP4 transcript, a DNA fragment encoding part of the LDL receptor domain and the protease domain was used to probe an RNA blot composed of 76 different human tissues (catalog number 7775-1; human multiple tissue expression (MTE) array; CLONTECH). As in the northern analysis of gel blot, a very strong signal was observed in the liver. Signals in other tissues were observed in (decreasing signal level): fetal liver > heart = kidney = adrenal gland = testis = fetal heart and kidney = skeletal muscle = bladder = placenta > brain = spinal cord = colon = stomach = spleen = lymph node = bone marrow = trachea = uterus = pancreas = salivary gland = mammary gland = lung. MTSP4 is also expressed less abundantly in several tumor cell lines including HeLa S3 = leukemia K-562 = Burkitt's lymphomas (Raji and Daudi) = colorectal adenocarcinoma (SW480) > lung carcinoma (A549) = leukemia MOLT-4 = leukemia HL-60. PCR of the MTSP4 transcript from cDNA libraries made from several human primary tumors xenografted in nude mice (human tumor multiple tissue cDNA panel, catalog number K1522-1, CLONTECH) was performed using MTSP4-specific primers. The MTSP4 transcript was detected in breast carcinoma (GI-101), lung carcinoma (LX-1), colon adenocarcinoma (GI-112) and pancreatic adenocarcinoma (GI-103). No apparent signal was detected in another form of lung carcinoma (GI-117), colon adenocarcinoma (CX-1), ovarian carcinoma (GI-102). and prostatic adenocarcinoma (PC3). The MTSP4 transcript was also detected in LNCaP and PC-3 prostate cancer cell lines as well as in HT-1080 human fibrosarcoma cell line.

### Sequence analysis

MTSP4 DNA and protein sequences were analyzed using MacVector (version 6.5; Oxford Molecular Ltd., Madison, WI). The ORF of MTSP4-L includes 2,409 bp, which translate to a 802-amino acid protein, while the ORF of MTSP4-S is composed of 1,977 bp which translate to a 658-amino acid protein. The cDNA encoding the protease domain in both forms is composed of 708 bp which translate to a 235-amino acid protein sequence (see, SEQ ID No. 6) The DNA sequences and the translated protein sequences of MTSP4-L and MTSP4-S, and of the protease domain of MTSP4 are set forth in SEQ ID Nos. 8, 10 and 6, respectively.

### EXAMPLE 3

### Cloning of MTSP6

### Identification of genomic clone of MTSP6

Using the protein sequence of the protease domain of the serine protease MTSP4 (see EXAMPLE 2), the non-redundant database (all non-redundant GenBank CDS translations + PDB + SwissProt + PIR + PRF) at the National Center for Biotechnology Information (Bethesda, MD; <http://www.ncbi.nim.nih.gov>) was searched for sequences that were similar or identical to MTSP4 using the search algorithm tblastn. The tblastn algorithm compares a protein query sequence against a nucleotide sequence database dynamically translated in all reading frames. A protein (55 amino acids), which has 60% identity with the query MTSP4 sequence (55 amino acids), was obtained from the translation of genomic sequence of AC015555 (nucleotide #15553 to 15717). This protein hereafter is referred to as MTSP6. Subsequent search of the GenBank database showed that no cDNA encoding MTSP6 has been deposited.

The gene exhibiting highest homology to MTSP6 was human transmembrane serine protease 2 (GenBank accession number U75329; Swissprot accession number 015393), which showed 66% identity to MTSP6 within the 45 amino acid regions compared. Consequently, the nucleotide sequence encoding the MTSP6 protease domain was obtained by comparing the protein sequence of human transmembrane serine protease 2 protease domain with the nucleotide sequence of AC015555 translated in six reading frames. The protein sequence obtained from the translated nucleotide sequence of MTSP6 revealed an overall 50% identity with human transmembrane serine protease 2. A search of the EST database indicated the presence of seven MTSP6 EST clones (AA883068, AW591433, A1978874, AI469095, A1935487, AA534591 and AI758271).

### Cloning of human MTSP6 full-length cDNA

To obtain cDNA encoding the region of the MTSP6 protease domain identified by database searches described above, two gene-specific primers, Ch17-NSP-1, 5'-TCACGCATCGTGGGTGGAACATGTCC-3' (5' primer) SEQ ID NO. 30 nd Ch17-NSP-2AS, 5'- ACCCACCTCCATCTGCTCGTGGATCC-3' SEQ ID NO. 31 (3' primer), were used for PCR. These two primers amplified a 708-base pair fragment from human mammary gland carcinoma cDNA (Clontech Marathon-Ready cDNA, Cat. No. 7493-1).

To obtain the remaining, unknown cDNA of MTSP6, 5'- and 3'-RACE reactions were performed on the human mammary gland carcinoma. Marathon-Ready cDNA is specifically made for RACE reactions. The first RACE reactions were performed by PCR using Marathon cDNA adaptor primer 1 (AP1) with gene specific primers, Ch17-NSP-2AS, 5'-ACCCACCTCCATCTGCTCGTGGATCC-3' SEQ ID NO. 31 for 5'-RACE reaction and Ch17-NSP-1, 5'-TCACGCATCGTGGGTGGAACATGTCC-3' SEQ ID NO. 30 for 3'-RACE reaction. The PCR products were purified from agarose gel. A second nested PCR was then performed using Marathon cDNA adaptor primer 2 (AP2) with gene specific primer Ch17-NSP-3AS, 5'- CCACAGCCTCCTCTCTTGACACACCAG-3' SEQ ID No. 32 for 5'-RACE reaction (using first 5'-RACE product as template) and Ch17-NSP-3 5'-ACGCCCCTGTGGATCATCACTGCTGC-3' SEQ ID No. 33 for 3'-RACE reaction (using first 3'-RACE product as template). First 5'- and 3'-RACE products were also used as template for PCR reactions using primers Ch17-NSP-3 and Ch17-NSP-4AS to obtain a cDNA fragment for use as a probe. PCR products from RACE reactions which were larger than 700 bp were cut out and purified from agarose gel and subcloned into pCR2.1-TOPO cloning vector (Invitrogen, Carlsbad, CA). Colony hybridization was then performed to identify positive colonies containing MTSP6 sequence. Positive clones were identified by colony hybridization using the 495 bp DNA fragment obtained from PCR reaction (with primers Ch17-NSP-3 and Ch17-NSP-4AS) and by DNA sequencing.

Sequence analysis of the 5'-RACE products indicated that an additional 420 bp of upstream sequence were obtained. The potential initial codon was not present in the 5'-RACE sequence. Another round of nested 5'-RACE reaction was performed using AP2 and a gene specific primer (designed based on the new RACE sequence) Ch17-NSP-5AS 5'-TCCCTCCCTCACATATACTGAGTGGTG-3' SEQ ID No. 34, using the PCR products obtained from the first 5'-RACE as template. A PCR product of 367 bp using Ch17-NSP-6 5'-CGACTGCTCAGGGAAGTCAGATGTCG-3' SEQ ID NO. 35 (designed based on the new 5'-RACE sequence) and Ch17-NSP-5AS was used to identify the positive clones. An additional sequence of 480 bp was obtained from the second 5'-RACE products. A potential ATG start codon was observed within a sequence of GTCACCATGG (nucleotides 262-272 of SEQ ID No. 12, which appears to be a Kozak sequence (GCC (A/G) CCAUGG), indicating that this ATG is likely the initiation codon for MTSP6.

The 3'-RACE reaction to obtain the rest of the 3' end of MTSP6 was not successful using Marathon Ready human mammary gland carcinoma cDNA. The sequence of the 3'-RACE products obtained was exclusively that of an MTSP6 cDNA truncated with the Marathon AP2 primer sequence within the coding region.

The 3'-end sequence of MTSP6 was obtained by PCR using Ch17-NSP-3 (5'-ACGCCCCTGTGGATCATCACTGCTGC-3'; SEQ ID NO. 33) and Ch17-NSP-4 (5'-CTGGTGTGTCAAGAGAGGAGGCTGTGG-3'; SEQ ID NO. 37) with an antisense primer Ch17-NSP-7AS (5'-ACTCAGGTGGCTACTTATCCCCTTCCTC-3'; SEQ ID NO. 38) designed based on the sequence of an EST clone AA883068, which apparently covers the 3'-end of MTSP6 sequence, and human small intestine cDNA (Clontech) as template. Two PCR products (650 bp and 182 bp, respectively) were obtained and DNA sequence analysis indicated that both PCR products contained a stop codon.

### Sequence analysis and domain organization of MTSP6

The MTSP6 DNA and protein sequences were analyzed using DNA Strider (version 1.2). The ORF of MTSP6 is composed of 1,362 bp, which translate into a 453-amino acid protein. Protein sequence analysis using the SMART (Simple Modular Architecture Research Tool) program at http://smart.embl-heidelberg.de predicts that MTSP6 is a multi-domain, type-II membrane-type serine protease containing of a transmembrane domain (amino acids 48-68) at the N terminus followed by a LDLRa domain (LDL receptor domain class a) (amino acids 72-108), a SR domain (Scavenger receptor Cys-rich domain)(amino acids 109-205), and a trypsin-like serine protease domain (amino acids 216-443) (see FIGURE 3).

### Gene expression profile of MTSP6 in normal and tumor tissues

To obtain information regarding the gene expression profile of the MTSP6 transcript, a 495 bp DNA fragment obtained from PCR reaction with primers Ch17-NSP-3 and NSP-4AS was used to probe an RNA blot composed of 76 different human tissues (catalog number 7775-1; human multiple tissue expression (MTE) array; CLONTECH). The strongest signal was observed in duodenum. Signal in other tissues were observed in (decreased signal level): Stomach > trachea = mammary gland = thyroid gland = salivary gland = pituitary gland = pancreas > kidney > lung > jejunum = ileum = ilocecum = appendix = fetal kidney > fetal lung. Very weak signals can also be detected in several other tissues. MTSP6 is also expressed in several tumor cell lines including HeLa S3 > colorectal adenocarcinoma (SW480) > leukemia MOLT-4 > leukemia K-562. PCR analysis of the MTSP6 transcript from cDNA libraries made from several human primary tumors xenografted in nude mice (human tumor multiple tissue cDNA panel, catalog number K1522-1, CLONTECH) was performed using MTSP6-specific primers (Ch17-NSP-3 and Ch17-NSP2AS). The MTSP6 transcript was strongly detected in lung carcinoma (LX-1), moderately detected in pancreatic adenocarcinoma (GI-103), weakly detected in ovarian carcinoma (GI-102); and very weakly detected in colon adenocarcinoma (GI-112 and CX-1), breast carcinoma (GI-101), lung carcinoma (GI-117) and prostatic adenocarcinoma (PC3). The MTSP6 transcript was also detected in breast cancer cell line MDA-MB-231, prostate cancer cell line PC-3, but not in HT-1080 human fibrosarcoma cell line. MTSP6 is also expressed in mammary gland carcinoma cDNA (Clontech).

### EXAMPLE 4

### Expression of the protease MTSP domains

The DNA encoding each of the MTSP 3 and 4 protease domains was cloned into a derivative of the *Pichia pastoris* vector pPIC9K (available from Invitrogen; see SEQ ID NO. 45). Plasmid pPIC9k features include the 5' AOX1 promoter fragment at 1-948; 5' AOX1 primer site at 855-875; alpha-factor secretion signal(s) at 949-1218; alpha-factor primer site at 1152-1172; multiple cloning site at 1192-1241; 3' AOX1 primer site at 1327-1347; 3' AOX1 transcription termination region at 1253-1586; HIS4 ORF at 4514-1980; kanamycin resistance gene at 5743-4928; 3' AOX1 fragment at 6122-6879; ColE1 origin at 7961-7288; and the ampicillin resistance gene at 8966-8106. The plasmid used herein is derived from pPIC9K by eliminating the XhoI site in the kanamycin resistance gene and the resulting vector is herein designated pPIC9KX.

### Primers used for PCR amplification of protease domain and subcloning into the XhoI/NotI sites of Pichia vector

### MTSP3

5' primer (with XhoI site [underlined]) SEQ ID No. 39
   5' TCTCTCGAGAAAAGAGTGGTGGGTGGGGAGGAGGCCTCTGTG 3'
3' primer (with NotI site [underlined]) SEQ ID No. 40
   5' ATTCGCGGCCGCATTACAGCTCAGCCTTCCAGAC 3'

### MTSP4-S and MTSP4-L

5' primer (with XhoI site [underlined]) SEQ ID No. 41
   5'TCTCTCGAGAAAAGAATTGTTGGTGGAGCTGTGTCCTCCGAG
3' primer with NotI site SEQ ID No. 42
   5' ATTCGCGGCCGCTCAGGTCACCACTTGCTGGATCCAG 3'

### MTSP6

MTSP6 was cloned into the *E. coli* TOPO vector (pcR^{®} 2.1 TOPO™, SEQ ID No. 46, Invitrogen, Carlsbad, CA; the TOPO^{®} TA Cloning^{®} Kit is designed form cloning Taq-amplified PRCR products).
5' primer (with XhoI site [underlined]) SEQ ID No. 43
   5' CTCGAGAAACGCATCGTGGGTGGAAACATGTCCTTG 3'
3' primer NotI site comes from E. coli TOPO vector SEQ ID No. 44:
   5' ACTCAGGTGGCTACTTATCCCCTTCCTC 3'

### EXAMPLE 5

### Assays for identification of candidate compounds that modulate that activity of an MTSP

### Assay for identifying inhibitors

The ability of test compounds to act as inhibitors of catalytic activity of an MTSP, including MTSP1, MTSP3, MTSP4, MTSP6 can be assessed in an amidolytic assay. The inhibitor-induced inhibition of amidolytic activity by a recombinant MTSP or the protease domain portions thereof, can be measured by IC50 values in such an assay.

An exemplary assay buffer is HBSA (10 mM Hepes, 150mM sodium chloride, pH 7.4, 0.1% bovine serum albumin). All reagents were from Sigma Chemical Co. (St. Louis, MO), unless otherwise indicated. Two IC50 assays at 30-minute (a 30-minute preincubation of test compound and enzyme) and at 0-minutes (no preincubation of test compound and enzyme) are conducted. For the IC50 assay at 30-minute, the following reagents are combined in appropriate wells of a Corning microtiter plate: 50 microliters of HBSA, 50 microliters of the test compound, diluted (covering a broad concentration range) in HBSA (or HBSA alone for uninhibited velocity measurement), and 50 microliters of the MTSP or protease domain thereof diluted in buffer, yielding a final enzyme concentration of about 100-500 pM. Following a 30-minute incubation at ambient temperature, the assay is initiated by the addition of 50 microliters of a substrate for the particular MTSP (see, *e.g*., table and discussion below) and reconstituted in deionized water, followed by dilution in HBSA prior to the assay) were added to the wells, yielding a final volume of 200 microliters and a final substrate concentration of 300 µM (about 1.5-times Km).

For an IC50 assay at 0-minute, the same reagents are combined: 50 microliters of HBSA, 50 microliters of the test compound, diluted (covering the identical concentration range) in HBSA (or HBSA alone for uninhibited velocity measurement), and 50 microliters of the substrate, such as a chromogenic substrate. The assay is initiated by the addition of 50 microliters of MTSP. The final concentrations of all components are identical in both IC50 assays (at 30-and 0-minute incubations).

The initial velocity of the substrate hydrolysis is measured in both assays by, for example for a chromogenic substrate, as the change of absorbance at a particular wavelength, using a Thermo Max¿ Kinetic Microplate Reader (Molecular Devices) over a 5 minute period, in which less than 5% of the added substrate was used. The concentration of added inhibitor, which caused a 50% decrease in the initial rate of hydrolysis was defined as the respective IC50 value in each of the two assays (30-and 0-minute).

### Another assay for identifying inhibitors

Test compounds for inhibition of the protease activity of the protease domain of is assayed in Costar 96 well tissue culture plates (Corning NY). Approximately 2-3 nM the MTSP or protease domain thereof is mixed with varying concentrations of inhibitor in 29.2 mM Tris, pH 8.4, 29.2 mM imidazole, 217 mM NaCl (100 mL final volume), and allowed to incubate at room temperature for 30 minutes. 400 mM substrate is added, and the reaction monitored in a SpectraMAX Plus microplate reader (Molecular Devices, Sunnyvale CA) by following the change in a parameter correlated with hydrolysis, such as absorbance for a chromogenic substrate for 1 hour at 37° C.

### ASSAY FOR SCREENING MTSP6

The protease domain of MTSP6 expressed in Pichia pastoris is assayed for inhibition by various compounds in Costar 96 well tissue culture plates (Corning NY). Approximately 1-20 nM MTSP6 is mixed with varying concentrations of inhibitor in 29.2 mM Tris, pH 8.4, 29.2 mM Imidazole, 217 mM NaCl (100 µL final volume), and allowed to incubate at room temperature for 30 minutes. 500 µM substrate Spectrozyme t-PA (American Diagnostica" Greenwich, CT) is added, and the reaction is monitored in a SpectraMAX Plus microplate reader (Molecular Devices, Sunnyvale CA) by measuring the change in absorbance at 405 nm for 30 minutes at 37°C.

### Identification of substrates

Particular substrates for use in the assays can be identified empirically by testing substrates. The following list of substrates are exemplary of those that can be tested.

| Substrate name | Structure |
|---|---|
| S 2366 | pyroGlu-Pro-Arg-pNA.HCl |
| spectrozyme t-PA | CH₃SO₂-D-HHT-Gly-Arg-pNA.AcOH |
| N-p-tosyl-Gly-Pro-Arg-pNA | N-p-tosyl-Gly-Pro-Arg-pNA |
| Benzoyl-Val-Gly-Arg-pNA | Benzoyl-Val-Gly-Arg-pNA |
| Pefachrome t-PA | CH₃SO₂-D-HHT-Gly-Arg-pNA |
| S 2765 | N-α-Z-D-Arg-Gly-Arg-pNA.2HCl |
| S 2444 | pyroGlu-Gly-Arg-pNA.HCl |
| S 2288 | H-D-Ile-Pro-Arg-pNA.2HCl |
| spectrozyme UK | Cbo-L-(γ)Glu(α-t-BuO)-Gly-Arg-pNA.2AcOH |
| S 2302 | H-D-Pro-Phe-Arg-pNA.2HCl |
| S 2266 | H-D-Val-Leu-Arg-pNA.2HCl |
| S 2222 | Bz-Ile-Glu(g-OR)-Gly-Arg-pNA. HCl R=H(50%) and R = CH₃(50%) |
| Chromozyme PK | Benzoyl-Pro-Phe-Arg-pNA |
| S 2238 | H-D-Phe-Pip-Arg-pNA.2HCl |
| S 2251 | H-D-Val-Leu-Lys-pNA.2HCl |
| Spectrozyme PI | H-D-Nle-HHT-Lys-pNA.2AcOH |
| | Pyr-Arg-Thr-Lys-Arg-AMC |
| | H-Arg-Gln-Arg-Arg-AMC |
| | Boc-Gln-Gly-Arg-AMC |
| | Z-Arg-Arg-AMC |
| Spectrozyme THE | H-D-HHT-Ala-Arg-pNA.2AcOH |
| Spectrozyme fXlla | H-D-CHT-Gly-Arg-pNA.2AcOH |
| | CVS 2081-6 (MeSO₂-dPhe-Pro-Arg-pNA) |
| | Pefachrome fVIIa (CH₃SO₂-D-CHA-But-Arg-pNA) |

| | |
|---|---|
| pNA = para-nitranilide (chromogenic) AMC = amino methyl coumarin (fluorescent) | |

If none of the above substrates are cleaved, a coupled assay, described above, can be used. Briefly, test the ability of the protease to activate and enzyme, such as plasminogen and trypsinogen. To perform these assays, the single chain protease is incubated with a zymogen, such as plasminogen or trypsinogen, in the presence of the a known substrate, such, lys-plasminogen, for the zymogen. If the single chain activates the zymogen, the activated enzyme, such as plasmin and trypsin, will degrade the substrate therefor.

### EXAMPLE 6

### Isolation and Cloning of Matriptase

### A. Cell type and growth of cells

Human prostate adenocarcinoma cell line, PC-3, was purchased from ATCC (catalog number CRL-1435; Manassas, VA). The cells were cultured at 37°C, 5% CO₂ in Ham's F-12K growth medium (catalog number 9077; Irvine) supplemented with 2 mM L-glutamine and 10% fetal bovine serum. All subsequent cell manipulations were carried out according to the manufacturer's instructions. PC-3 cells were allowed to grow to about 90% confluence, and were then washed briefly with 1 x phosphate buffered saline.

### B. Isolation of total RNA, and purification and enrichment of polyA⁺ RNA

PC-3 cells were lysed in Trizol reagent (catalog number 15596; Life Technologies, Rockville, MD) and total RNA was isolated according to the manufacturer's protocol. The concentration of total RNA was estimated from absorbance reading at 260 nm. PolyA⁺ RNA was purified and enriched using oligo-dT beads (catalog number 70061; Oligotex, Qiagen, Valencia, CA).

### C. Reverse-transcription and polymerase chain reaction (PCR)

PC-3-derived polyA⁺ RNA was converted to single-stranded cDNA (sscDNA) by reverse transcription using ProSTAR first-strand RT-PCR kit (catalog number 200420; Stratagene, La Jolla, CA) and SuperScript II RNase H⁻ reverse transcriptase (catalog number 18064-022; Life Technologies). After reverse transcription, an aliquot of PC-3 sscDNA (4 µL) was subjected to PCR using 2 mM each of the sense and anti-sense degenerate oligonucleotide primers and *Taq* polymerase (catalog number 201203; Qiagen). Total reaction volume was 100 µL. The sequence of the sense primer was 5'-TGGRT(I)VT(I)WS(I)GC(I)RC(I)CAYTG-3' (SEQ ID No. 13) and that of the antisense was 5'(I)GG(I)CC(I)CC(I)SWRTC(I)CCYT(I)RCA(I)GHRTC-3' (SEQ ID No. 14), where R = A,G; V = G,A,C; W = A,T; S = G,C; Y = C,T; H =A,T,C. The primer sequences correspond to two highly conserved regions in all chymotrypsin-like serine proteases and amplify PCR products ranging from approximately 400 to 500 base pairs.

### D. Clone screening and sequencing

The PCR products were separated on a 2% agarose gel and purified using a gel extraction kit (catalog number 28706; QIAquick gel extraction kit; Qiagen). The purified DNA fragments were ligated into pCR2.1-TOPO (catalog number K4500-01; Invitrogen, Carlsbad, CA). After transformation into *E. coli* cells, plasmid DNA was isolated and analyzed by digestion with *Eco*RI restriction enzyme. Clones that had inserted nucleic acid were further characterized by sequencing using a fluorescent dye-based DNA sequencing method (catalog number 4303149; BigDye terminator cycle sequencing kit with Ampli *Taq* DNA polymerase; Perkin Elmer, Lincoln**,** CA). A total of 31 clones were sequenced and analyzed. All sequences were analyzed by a multiple nucleotide sequence alignment algorithm (blastn) (www.ncbi.nlm.nih.gov/blast) to identify identical or closely related DNA deposited in GenBank (NCBI, Bethesda, MD). Those that did not show significant homology were further analyzed using blastx, which compares the six-frame conceptual translation products of a nucleotide sequence (both strands) against a protein sequence database (SwissProt). Eight clones yielded identical cDNA fragments that encode MTSP1. MTSP1 was subsequently found to be identical to matriptase (GenBank accession number AF118224).

### E. Rapid amplification of cDNA ends (RACE) and gene-specific amplification of MTSP1

To obtain DNA encoding the complete protease domain of MTSP1, RACE and gene-specific amplification reactions were performed. A human prostate Marathon-Ready cDNA (catalog # 7418-1; Clontech) was used to isolate part of the cDNA encoding MTSP1. Marathon-Ready cDNA is prepared to contain a known hybridization sequence at the 5' and 3' ends of the sscDNA. The 3' region of MTSP1 cDNA was obtained by a 3'-RACE reaction using a gene specific primer, 5'-CACCCCTTCTTCAATGACTTCACCTTCG-3' (SEQ ID No. 55). The 5' end of the MTSP1 protease domain was obtained by gene-specific amplification reaction using two MTSP1-specific primers, 5'-TACCTCTCCTACGACTCC-3' (SEQ ID No. 56) for the sense primer and 5'-GAGGTTCTCGCAGGTGGTCTGGTTG-3' (SEQ ID No. 57) for the antisense primer. The sequences for these two primers were obtained from the human SNC19 mRNA sequence. The 3'-RACE reaction and gene-specific PCR produced DNA fragments that were > 1 kbp in size. These fragments were subcloned into pCR2.1-TOPO (Invitrogen, San Diego, CA). After transformation into *E. coli* cells, plasmid DNA was isolated and analyzed by digestion with *Eco*RI restriction enzyme. Clones that had inserts were characterized by Southern blot analysis (using the internal cDNA fragment as probe) and by DNA sequence analysis.

### F. PCR amplification of cDNA encoding the protease domain of MTSP1

To obtain a cDNA fragment encoding the entire protease domain of MTSP1, an end-to-end PCR amplification using gene-specific primers was used. The two primers used were: 5'-CTCGAGAAAAGAGTTGTTGGGGGCACGGATGCGGATGAG-3' (SEQ ID No. 58) for the 5' end and 5'-GCGGCCGCACTATACCCCAGTGTTCTCTTTGATCCA-3' (SEQ ID No. 36 for the 3' end. The 5' primer contained the sequence that encodes the start of the MTSP1 protease domain (VVGGTDADE) (SEQ. ID. NO. 10). The 3' primer contained the stop codon of MTSP1. A ∼800-bp fragment was amplified, purified and subcloned into the *Pichia pastoris* expression vector, pPIC9K, resulting in pPIC9K-MTSP1.

### G. Gene expression profile of MTSP1 in normal tissues, cancer cells and cancer tissues

To obtain information regarding the tissue distribution and gene expression level of MTSP1, the DNA insert from pPIC9K-MTSP1 was used to probe a blot containing RNA from 76 different human tissues (catalog number 7775-1; human multiple tissue expression (MTE) array; CLONTECH, Palo Alto, CA). Significant expression was observed in the colon (ascending, transverse and descending), rectum, trachea, esophagus and duodenum. Moderate expression levels were observed in the jejunum, ileum, ilocecum, stomach, prostate, pituitary gland, appendix, kidney, lung, placenta, pancreas, thyroid gland, salivary gland, mammary gland, fetal kidney, and fetal lung. Lower expression levels were seen in the spleen, thymus, peripheral blood leukocyte, lymph node, bone marrow, bladder, uterus, liver, adrenal gland, fetal heart, fetal liver, fetal spleen, and fetal thymus. A significant amount of the MTSP1 transcript was also detected in colorectal adenocarcinoma cell line (SW480), Burkitt's lymphoma cell line (Daudi), and leukemia cell line (HL-60). RT-PCR of the MTSP1 transcript in several human primary tumors xenografted in athymic nude mice was performed using gene-specific primers. A high level of MTSP1 transcript was detected in colon adenocarcinoma (CX-1) and pancreatic adenocarcinoma (GI-103). Moderate levels were observed in another colon adenocarcinoma (GI-112), ovarian carcinoma (GI-102), lung carcinoma (LX-1), and breast carcinoma (GI-101). Another lung carcinoma (GI-117) expressed a low level of the MTSP1 transcript. A similar RT-PCR was performed to detect the presence of the MTSP1 transcript in PC-3 and LNCaP cell lines. Both cell lines expressed significant amounts of MTSP1 transcript.

### H. Sequence analysis

All derived DNA and protein sequences were analyzed using MacVector (version 6.5; Oxford Molecular Ltd., Madison, WI). The cDNA encoding the protease domain of MTSP1 is composed of 726 base pairs which translate into a 241-amino acid protein sequence (rMAP) (see SEQ ID No. 1, 2, 49 and 50).

### EXAMPLE 7

### Production of Recombinant Serine Protease Domain of Matriptase or MTSP1 (rMAP)

### A. Fermentation

The production of multi-milligram amounts of rMAP was carried out by fermentation in a BioFlo 3000 fermentor (New Brunswick Scientific, NJ) equipped with a 3.3 L capacity bioreactor using a SMD1168/pPIC9K:MTSP1 Sac SC1 clone. ZA001 complex media (10 g/L yeast extract, 20 g/L peptone, 40 g/L glycerol, 5 g/L ammonium sulfate, 0.2 g/L calcium sulfate dihydrate, 2 g/L magnesium sulfate heptahydrate, 2 g/L potassium sulfate, 25 g/L sodium hexametaposphate, 4.35 ml/L PTM1) was inoculated with 100 ml of an overnight culture of the *P. pastoris* transformant. The culture was supplemented with 50% glycerol by fed-batch phase and induced for 18-24 hours with methanol controlled at 0.025%.

### B. Purification of Recombinant Serine Protease Domain of Matriptase or MTSP1 (rMAP)

The rMAP was secreted into the culture medium, so the first step of the purification involved the removal of cells and cell debris by centrifugation at 5000 g for 30 minutes. The resulting supernatant was decanted, adjusted to pH 8.0 with 10 N NaOH, and filtered through a SartoBran 300 0.45 + 0.2µM capsule. This supernatant was concentrated to 1 L by ultrafiltration using a 10 kDa ultrafiltration cartridge (NC SRT UF system with AG/Technologies UFP-10-C-5A filter), and the buffer was exchanged by crossflow filtration into 50 mM tris-HCl, 50 mM NaCl, 0.05% tween-80, pH 8.0 (buffer A). The filtration unit was rinsed once with 1 L buffer A which was combined with the concentrate.

The concentrated rMAP-containing solution was passed over a 150 ml benzamidine column that had been equilibrated with buffer A, at a flow rate of 8 ml/min. The column was washed with 3 column volumes of 50 mM tris-HCl, 1.0 M NaCl, 0.05% tween-80, pH 8.0 (buffer B) and eluted with 3 column volumes of 50 mM tris-HCl, 1.0 M L-arginine, 0.05% tween-80, pH 8.0 (buffer C). Fractions containing rMAP were identified by activity assay and pooled. This pooled material was concentrated to 10 ml using a JumboSep concentrator (Pall Gelman) and a 10 kDa cutoff membrane. Once concentrated to 10 ml, the buffer was exchanged into 50 mM Na₂HPO₄, 125 mM NaCl, pH 5.5 (buffer D) and the volume adjusted to 5-10 ml. The retentate was removed and the concentrator washed with buffer D which was added to the concentrate. The total sample volume was adjusted 15 ml.

The partially purified rMAP was passed through a 5 ml Q-sepharose Fast Flow HiTrap column (Amersham-Pharmacia Biotech) pre-equilibrated with 15 ml of buffer D. The flow through was collected. The HiTrap column was washed with an additional 10 ml of buffer D. Both flow throughs were pooled, and the protein concentration was determined by measurement of OD₂₈₀ (using an extinction coefficient of 2.012 mg/00₂₈₀). Purified rMAP was then deglycosylated by the addition 0.1 µl of Endoglycosidase H (ProZyme, 5 U/ml) per mg of protein and incubating overnight at 4°C with gentle swirling.

The conductivity of the deglycosylated pool was adjusted to 2.0-3.0 mS/cm with Nanopure H₂O and the pH adjusted to 6.5 (-200-300 mL final volume). The rMAP was then further purified by anion exchange chromatography by loading directly onto a Pharmacia Akta Explorer system using a 7 mL Source 15Q anion exchange column (Amersham-Pharmacia Biotech). The protein was eluted in a buffer containing 50 mM HEPES, pH 6.5 with a 0-0.33 M NaCl gradient over 10 column volumes at a flow rate of 6 ml/min. Fractions containing protein were pooled, and benzamidine was added to a final concentration of 10 mM. Protein purity was examined by SDS-PAGE and protein concentration determined by measurement of OD₂₈₀ and use of a theoretical extinction coefficient of 2.012 mg/OD₂₈₀.

### EXAMPLE 8

### Assays

### Amidolytic Assay for Determining Inhibition of Serine Protease Activity of Matriptase or MTSP1

The ability of test compounds to act as inhibitors of rMAP catalytic activity was assessed by determining the inhibitor-induced inhibition of amidolytic activity by the MAP, as measured by IC₅₀ values. The assay buffer was HBSA (10 mM Hepes, 150mM sodium chloride, pH 7.4, 0.1 % bovine serum albumin). All reagents were from Sigma Chemical Co. (St. Louis, MO), unless otherwise indicated.

Two IC₅₀ assays (a) one at either 30-minutes or 60-minutes (a 30-minute or a 60-minute preincubation of test compound and enzyme) and (b) one at 0-minutes (no preincubation of test compound and enzyme) were conducted. For the IC₅₀ assay at either 30-minutes or 60-minutes, the following reagents were combined in appropriate wells of a Corning microtiter plate: 50 microliters of HBSA, 50 microliters of the test compound, diluted (covering a broad concentration range) in HBSA (or HBSA alone for uninhibited velocity measurement), and 50 microliters of the rMAP (Corvas International) diluted in buffer, yielding a final enzyme concentration of 250 pM as determined by active site filtration. Following either a 30-minute or a 60-minute incubation at ambient temperature, the assay was initiated by the addition of 50 microliters of the substrate S-2765 (N-α-Benzyloxycarbonyl-D-arginyl-L-glycyl-L-arginine-p-nitroaniline dihydrochloride; DiaPharma Group, Inc.; Franklin, OH) to each well, yielding a final assay volume of 200 microliters and a final substrate concentration of 100 µM (about 4-times *K*ₘ). Before addition to the assay mixture, S-2765 was reconstituted in deionized water and diluted in HBSA. For the IC₅₀ assay at 0 minutes; the same reagents were combined: 50 microliters of HBSA, 50 microliters of the test compound, diluted (covering the identical concentration range) in HBSA (or HBSA alone for uninhibited velocity measurement), and 50 microliters of the substrate S-2765. The assay was initiated by the addition of 50 microliters of rMAP. The final concentrations of all components were identical in both IC₅₀ assays (at 30- or 60- and 0-minute).

The initial velocity of chromogenic substrate hydrolysis was measured in both assays by the change of absorbance at 405 nM using a Thermo Max^{®} Kinetic Microplate Reader (Molecular Devices) over a 5 minute period, in which less than 5% of the added substrate was used. The concentration of added inhibitor, which caused a 50% decrease in the initial rate of hydrolysis was defined as the respective IC₅₀ value in each of the two assays (30- or 60-minutes and 0-minute).

### In vitro enzyme assays for specificity determination

The ability of compounds to act as a selective inhibitor of matriptase activity was assessed by determining the concentration of test compound that inhibits the activity of matriptase by 50%, (IC₅₀) as described in the above Example, and comparing IC₅₀ value for matriptase to that determined for all or some of the following serine proteases: thrombin, recombinant tissue plasminogen activator (rt-PA), plasmin, activated protein C, chymotrypsin, factor Xa and trypsin.

The buffer used for all assays was HBSA (10 mM HEPES, pH 7.5, 150 mM sodium chloride, 0.1 % bovine serum albumin). The assay for IC₅₀ determinations was conducted by combining in appropriate wells of a Corning microtiter plate, 50 microliters of HBSA, 50 microliters of the test compound at a specified concentration (covering a broad concentration range) diluted in HBSA (or HBSA alone for V₀ (uninhibited velocity) measurement), and 50 microliters of the enzyme diluted in HBSA. Following a 30 minute incubation at ambient temperature, 50 microliters of the substrate at the concentrations specified below were added to the wells, yielding a final total volume of 200 microliters. The initial velocity of chromogenic substrate hydrolysis was measured by the change in absorbance at 405 nm using a Thermo Max^{®} Kinetic Microplate Reader over a 5 minute period in which less than 5% of the added substrate was used. The concentration of added inhibitor which caused a 50% decrease in the initial rate of hydrolysis was defined as the IC₅₀ value.

### Thrombin (flla) Assay

Enzyme activity was determined using the chromogenic substrate, Pefachrome t-PA (CH₃SO₂-D-hexahydrotyrosine-glycyl-L-Arginine-p-nitroaniline, obtained from Pentapharm Ltd.). The substrate was reconstituted in deionized water prior to use. Purified human α-thrombin was obtained from Enzyme Research Laboratories, Inc. The buffer used for all assays was HBSA (10 mM HEPES, pH 7.5, 150 mM sodium chloride, 0.1 % bovine serum albumin).

IC₅₀ determinations were conducted where HBSA (50 µL), α-thrombin (50 µl) (the final enzyme concentration is 0.5 nM) and inhibitor (50 µl) (covering a broad concentration range), were combined in appropriate wells and incubated for 30 minutes at room temperature prior to the addition of substrate Pefachrome-t-PA (50 µl) (the final substrate concentration is 250 µM, about 5 times Km). The initial velocity of Pefachrome t-PA hydrolysis was measured by the change in absorbance at 405 nm using a Thermo Max^{®} Kinetic Microplate Reader over a 5 minute period in which less than 5% of the added substrate was used. The concentration of added inhibitor which caused a 50% decrease in the initial rate of hydrolysis was defined as the IC₅₀ value.

### Factor Xa

Factor Xa catalytic activity was determined using the chromogenic substrate S-2765 (N-benzyloxycarbonyl-D-arginine-L-glycine-L-arginine-p-nitroaniline), obtained from DiaPharma Group (Franklin, OH). All substrates were reconstituted in deionized water prior to use. The final concentration of S-2765 was 250 µM (about 5-times Km). Purified human Factor X was obtained from Enzyme Research Laboratories, Inc. (South Bend, IN) and Factor Xa (FXa) was activated and prepared from it as described [Bock, P.E., Craig, P.A., Olson, S.T., and Singh, P. Arch. Biochem. Biophys. 273:375-388 (1989)]. The enzyme was diluted into HBSA prior to assay in which the final concentration was 0.25 nM. Recombinant tissue plasminogen activator (rt-PA) Assay

rt-PA catalytic activity was determined using the substrate, Pefachrome t-PA (CH₃SO₂-D-hexahydrotyrosine-glycyl-L-arginine-p-nitroaniline, obtained from Pentapharm Ltd.). The substrate was made up in deionized water followed by dilution in HBSA prior to the assay in which the final concentration was 500 micromolar (about 3-times Km). Human rt-PA (Activase^{®}) was obtained from Genentech Inc. The enzyme was reconstituted in deionized water and diluted into HBSA prior to the assay in which the final concentration was 1.0 nM.

### Plasmin Assay

Plasmin catalytic activity was determined using the chromogenic substrate, S-2366 [L-pyroglutamyl-L-prolyl-L-arginine-p-nitroaniline hydrochloride], which was obtained from DiaPharma group. The substrate was made up in deionized water followed by dilution in HBSA prior to the assay in which the final concentration was 300 micromolar (about 2.5-times Km). Purified human plasmin was obtained from Enzyme Research Laboratories, Inc. The enzyme was diluted into HBSA prior to assay in which the final concentration was 1.0 nM.

### Activated Protein C (aPC) Assay

aPC catalytic activity was determined using the chromogenic substrate, Pefachrome PC (delta-carbobenzloxy-D-lysine-L-prolyl-L-arginine-p-nitroaniline dihydrochloride), obtained from Pentapharm Ltd.). The substrate was made up in deionized water followed by dilution in HBSA prior to the assay in which the final concentration was 400 micromolar (about 3-times Km). Purified human aPC was obtained from Hematologic Technologies, Inc. The enzyme was diluted into HBSA prior to assay in which the final concentration was 1.0 nM.

### Chymotrypsin Assay

Chymotrypsin catalytic activity was determined using the chromogenic substrate, S-2586 (methoxy-succinyl-L-arginine-L-prolyl-L-tyrosyl-p-nitroanilide), which was obtained from DiaPharma Group. The substrate was made up in deionized water followed by dilution in HBSA prior to the assay in which the final concentration was 100 micromolar (about 9-times Km). Purified (3X-crystallized; CDI) bovine pancreatic alpha-chymotrypsin was obtained from Worthington Biochemical Corp. The enzyme was reconstituted in deionized water and diluted into HBSA prior to assay in which the final concentration was 0.5 nM.

### Trypsin Assay

Trypsin catalytic activity was determined using the chromogenic substrate, S-2222 (benzoyl-L-isoleucine-L-glutamic acid-[gamma-methyl ester]-L-arginine-p-nitroanilide), which was obtained from DiaPharma Group. The substrate was made up in deionized water followed by dilution in HBSA prior to the assay in which the final concentration was 250 micromolar (about 4-times Km). Purified (3X-crystallized; TRL3) bovine pancreatic trypsin was obtained from Worthington Biochemical Corp. The enzyme was reconstituted in deionized water and diluted into HBSA prior to assay in which the final concentration was 0.5 nM.

Since modifications will be apparent to those of skill in this art, it is intended that this invention be limited only by the scope of the appended claims.

## Claims

1. An isolated, substantially purified single-chain polypeptide, comprising a protease domain of a type-II membrane-type serine protease (MTSP) or a catalytically active fragment thereof, wherein:
the MTSP protease domain or catalytically active fragment thereof is the only portion of the single-chain polypeptide from the MTSP;
the MTSP protease domain or catalytically active fragment thereof has serine protease activity as a single chain;
a free Cys in the protease domain is replaced with another amino acid, whereby the resulting polypeptide exhibits proteolytic activity; and
the MTSP is not an endotheliase.

2. The substantially purified polypeptide of claim 1, wherein the MTSP portion has an N-terminus that comprises IVNG, ILGG, VGLL or ILGG.

3. The substantially purified polypeptide of claim 1, wherein the MTSP is selected from among :
MTSP1, which is a polypeptide that comprises the sequence of amino acids set forth in SEQ ID No. 2 or 50, or comprises a protease domain that has at least 80%, 90%, or 95% sequence identity with amino acids 615-855 of SEQ ID No. 2;
MTSP3, which is a polypeptide that comprises a sequence of amino acids having at least about 85% or 90% sequence identity with the sequence of amino acids set forth in SEQ ID No. 4, or a polypeptide that comprises the sequence of amino acids set forth as amino acids 205-437 in SEQ ID No. 4;
MTSP4 which is a polypeptide that comprises a sequence of amino acids having at least about 85% or 90% or 95% sequence identity with the sequence of amino acids set forth in any of SEQ ID Nos. 6, 8 and 10, or a polypeptide that comprises the sequence of amino acids set forth in any of SEQ ID Nos. 6, 8 and 10; and
MTSP6, which is a polypeptide that comprises a sequence of amino acids having at least about 85% or 90% or 95% sequence identity with the sequence of amino acids set forth in SEQ ID No. 12, or a polypeptide that comprises the sequence of amino acids set forth in SEQ ID No. 12.

4. The substantially purified polypeptide of claim 1, wherein the MTSP protease domain has a sequence of amino residues set forth as amino acid residues 615-855 of SEQ ID No. 2, as amino acid residues 205-437 of SEQ ID NO. 4, as the amino acid residues in SEQ ID No. 6, or as amino acid residues 217-443 in SEQ ID No. 12.

5. The substantially purified polypeptide of claim 1 that has at least about 40%, 60%, 80%, 90% or 95% sequence identity with a protease domain that comprises the sequence of amino acid residues set forth as amino acid residues 615-855 of SEQ ID No. 2, as amino acid residues 205-437 of SEQ ID NO. 4, as the amino acid residues in SEQ ID No. 6, or as amino acid residues 217-443 in SEQ ID No. 12.

6. The polypeptide of claim 1, wherein the free Cys in the protease domain is replaced with a serine.

7. The polypeptide of claim 1, wherein the MTSP is selected from among corin (SEQ ID No. 62), enteropeptidase (SEQ ID No. 64), human airway trypsin-like protease (HAT; SEQ ID No. 66), TMPRSS2 (SEQ ID No. 70), and TMPRSS4 (SEQ ID No. 72).

8. A conjugate, comprising:
a) a polypeptide of any of claims 1 to 7, and
b) a targeting agent linked to the protein directly or via a linker.

9. The conjugate of claim 8, wherein the targeting agent permits
i) affinity isolation or purification of the conjugate;
ii) attachment of the conjugate to a surface;
iii) detection of the conjugate; or
iv) targeted delivery to a selected tissue or cell.

10. A solid support, comprising two or more polypeptides of any of claims 1 to 7 linked thereto either directly or via a linker.

11. The support of claim 10 , wherein the polypeptides comprise an array.

12. The support of claim 10, wherein the polypeptides comprise a plurality of different MTSP protease domains.

13. A method for identifying compounds that modulate the protease activity of an MTSP, comprising:
contacting a polypeptide of any of claims 1 to 7 with a substrate proteolytically cleaved by the MTSP, and, either simultaneously, before or after, adding a test compound or plurality thereof;
measuring the amount of substrate cleaved in the presence of the test compound;
and
selecting compounds that change the amount cleaved compared to a control, whereby compounds that modulate the activity of the MTSP are identified.

14. The method of claim 13, wherein the test compounds are small molecules, peptides, peptidomimetics, natural products, antibodies or fragments thereof.

15. The method of claim 13, wherein a plurality of the test substances are screened simultaneously.

16. The method of claim 13, wherein the change in the amount cleaved is assessed by comparing the amount cleaved in the presence of the test compound with the amount in the absence of the test compound.

17. The method of claim 13, wherein a plurality of the polypeptides are linked to a solid support, either directly or via a linker.

18. The method of claim 13, wherein the polypeptides comprise an array.

19. The method of claim 13, wherein the polypeptides comprise a plurality of different MTSP proteases.

20. A method of identifying a compound that specifically binds to a single chain protease domain of an MTSP, comprising:
contacting a polypeptide of any of claims 1 to 7 with a test compound or plurality thereof under conditions conducive to binding thereof; and
identifying compounds that specifically bind to the MTSP single chain protease domain or compounds that inhibit binding of a compound known to bind to the MTSP single chain protease domain, wherein the known compound is contacted with the polypeptide before, simultaneously with or after the test compound.

21. The method of claim 20, wherein the polypeptide is linked either directly or indirectly via a linker to a solid support.

22. The method of claim 20, wherein the test compounds are small molecules, peptides, peptidomimetics, natural products, antibodies or fragments thereof.

23. The method of claim 20, wherein a plurality of the test substances are screened for simultaneously.

24. The method of claim 20, wherein a plurality of the polypeptides are linked to a solid support.

25. Use of a polypeptide of claim 1 for identifying compounds that modulate the activity of a polypeptide of claim 1.

26. The use of claim 25, wherein the compounds inhibit the proteolytic activity thereof.

27. The use of claim 25, wherein the MTSP is selected from among MTSP1, MTSP3, MTSP4, and MTSP6 as claimed in claim 3, corin (SEQ ID No. 62), enteropeptidase (SEQ ID No. 64), human airway trypsin-like protease (HAT; SEQ ID No. 66), TMPRSS2 (SEQ ID No. 70), and TMPRSS4 (SEQ ID No. 72).

28. A polypeptide of any of claims 1 to 7 for use in the treatment of a neoplastic disease.

29. Use of a polypeptide of any of claims 1 to 7 for formulation of a medicament for treatment of a neoplastic disease.

## Patentansprüche

1. Isoliertes, im Wesentlichen gereinigtes einkettiges Polypeptid, das eine Proteasedomäne einer membranständigen Serinprotease (MTSP) vom Typ II oder ein katalytisch aktives Fragment davon umfasst, wobei:
die MTSP-Proteasedomäne oder das katalytisch aktive Fragment davon der einzige Teilbereich des einkettigen Polypeptids aus der MTSP ist;
die MTSP-Proteasedomäne oder das katalytisch aktive Fragment davon eine Serinproteaseaktivität als eine Einzelkette aufweist;
ein freies Cys in der Proteasedomäne durch eine andere Aminosäure ersetzt ist, wodurch das resultierende Polypeptid eine proteolytische Aktivität zeigt; und
die MTSP keine Endotheliase ist.

2. Im Wesentlichen gereinigtes Polypeptid nach Anspruch 1, wobei der MTSP-Teilbereich einen N-Terminus aufweist, der IVNG, ILGG, VGLL oder ILGG umfasst.

3. Im Wesentlichen gereinigtes Polypeptid nach Anspruch 1, wobei die MTSP ausgewählt ist unter:
der MTSP1, die ein Polypeptid ist, das die Aminosäuresequenz umfasst, die in SEQ ID No. 2 oder 50 dargestellt ist, oder das eine Proteasedomäne umfasst, die mindestens 80 %, 90 % oder 95 % Sequenzidentität mit den Aminosäuren 615-855 von SEQ ID No. 2 aufweist;
der MTSP3, die ein Polypeptid ist, das eine Aminosäuresequenz umfasst, die mindestens 85 % oder 90 % Sequenzidentität mit der Aminosäuresequenz hat, die in SEQ ID No. 4 dargestellt ist,
oder ein Polypeptid ist, das die Aminosäuresequenz umfasst, die als Aminosäuren 205-437 in SEQ ID No. dargestellt ist;
der MTSP4, die ein Polypeptid ist, das eine Aminosäuresequenz umfasst, die mindestens etwa 85 % oder 90 % oder 95 % Sequenzidentität mit der Aminosäuresequenz aufweist, die in einer der SEQ ID No. 6, 8 und 10 dargestellt ist, oder ein Polypeptid ist, das die Aminosäuresequenz umfasst, die in einer der SEQ ID No. 6, 8 und 10 dargestellt ist; und
der MTSP6, die ein Polypeptid ist, das eine Aminosäuresequenz umfasst, die mindestens etwa 85 % oder 90 % oder 95 % Sequenzidentität mit der Aminosäuresequenz aufweist, die in SEQ ID No. 12 dargestellt ist, oder ein Polypeptid ist, das die Aminosäuresequenz umfasst, die in SEQ ID No. 12 dargestellt ist.

4. Im Wesentlichen gereinigtes Polypeptid nach Anspruch 1, wobei die MTSP-Proteasedomäne eine Sequenz von Aminosäureresten aufweist, die als Aminosäurereste 615-855 der SEQ ID No. 2, als Aminosäurereste 205-437 der SEQ ID No. 4, als die Aminosäurereste in SEQ ID No. 6 oder als Aminosäurereste 217-443 in SEQ ID No. 12 dargestellt ist.

5. Im Wesentlichen gereinigtes Polypeptid nach Anspruch 1, das mindestens etwa 40 %, 60 %, 80 %, 90 % oder 95 % Sequenzidentität mit einer Proteasedomäne hat, die die Sequenz von Aminosäureresten umfasst, die als Aminosäurereste 615-855 der SEQ ID No. 2, als Aminosäurereste 205-437 der SEQ ID No. 4, als die Aminosäurereste in SEQ ID No. 6 oder als Aminosäurereste 217-443 in SEQ ID No. 12 dargestellt sind.

6. Polypeptid nach Anspruch 1, wobei das freie Cys in der Proteasedomäne durch ein Serin ersetzt ist.

7. Polypeptid nach Anspruch 1, wobei die MTSP unter Corin (SEQ ID No. 62), Enteropeptidase (SEQ ID No. 64), der trypsinartigen Protease der menschlichen Luftwege (HAT; SEQ ID No. 66), TMPRSS2 (SEQ ID No. 70) und TMPRSS4 (SEQ ID No. 72) ausgewählt ist.

8. Konjugat, das umfasst:
a) ein Polypeptid nach einem der Ansprüche 1 bis 7, und
b) ein Targeting-Mittel, das direkt oder über einen Linker mit dem Protein verbunden ist.

9. Konjugat nach Anspruch 8, wobei das Targeting-Mittel ermöglicht:
i) die Affinitätsisolierung oder -reinigung des Konjugats;
ii) die Befestigung des Konjugats an einer Oberfläche;
iii) den Nachweis des Konjugats; oder
iv) die adressierte Lieferung an ein ausgewähltes Gewebe oder eine ausgewählte Zelle.

10. Fester Träger, der zwei oder mehr Polypeptide nach einem der Ansprüche 1 bis 7 umfasst, die direkt oder über einen Linker mit dem Träger verbunden sind.

11. Träger nach Anspruch 10, wobei die Polypeptide ein Array umfassen.

12. Träger nach Anspruch 10, wobei die Polypeptide eine Vielzahl verschiedener MTSP-Proteasedomänen umfassen.

13. Verfahren zur Identifizierung von Verbindungen, die die Proteaseaktivität einer MTSP modulieren, das umfasst:
Inkontaktbringen eines Polypeptids nach einem der Ansprüche 1 bis 7 mit einem Substrat, das proteolytisch durch die MTSP gespalten wird, und entweder gleichzeitiges oder vorheriges oder nachträgliches Hinzugeben einer Testverbindung oder einer Vielzahl davon ;
Messen der Substratmenge, die in Gegenwart der Testverbindung gespalten wird;
und
Auswählen der Verbindungen, die die gespaltene Menge, verglichen mit einer Kontrolle, ändern, wodurch Verbindungen, die die Aktivität der MTSP modulieren, identifiziert werden.

14. Verfahren nach Anspruch 13, wobei die Testverbindungen kleine Moleküle, Peptide, Peptidomimetika, Naturstoffe, Antikörper oder Fragmente davon sind.

15. Verfahren nach Anspruch 13, wobei eine Vielzahl von Testverbindungen gleichzeitig durchgemustert werden.

16. Verfahren nach Anspruch 13, wobei die Änderung der gespaltenen Menge durch Vergleich der gespaltenen Menge in Gegenwart der Testverbindung mit der Menge in Abwesenheit der Testverbindung untersucht wird.

17. Verfahren nach Anspruch 13, wobei eine Vielzahl der Polypeptide entweder direkt oder über einen Linker mit einem festen Träger verbunden wird.

18. Verfahren nach Anspruch 13, wobei die Polypeptide ein Array umfassen.

19. Verfahren nach Anspruch 13, wobei die Polypeptide eine Vielzahl verschiedener MTSP-Proteasen umfassen.

20. Verfahren zum Identifizieren einer Verbindung, die spezifisch an eine einkettige Proteasedomäne einer MTSP bindet, das umfasst:
Inkontaktbringen eines Polypeptids nach einem der Ansprüche 1 bis 7 mit einer Testverbindung oder einer Vielzahl davon unter Bedingungen, die förderlich sind für das Anbinden; und
Identifizieren der Verbindungen, die spezifisch an die einkettige MTSP-Proeasedomäne binden, oder der Verbindungen, die das Anbinden einer Verbindung, von der bekannt ist, dass sie an die einkettige MTSP-Proteasedomäne bindet, hemmen, wobei die bekannte Verbindung vor, gleichzeitig mit oder nach der Testverbindung mit dem Polypeptid in Kontakt gebracht wird.

21. Verfahren nach Anspruch 20, wobei das Polypeptid entweder direkt oder indirekt über einen Linker mit einem festen Träger verbunden ist.

22. Verfahren nach Anspruch 20, wobei die Testverbindungen kleine Moleküle, Peptide, Peptidomimetika, Naturstoffe, Antikörper oder Fragmente davon sind.

23. Verfahren nach Anspruch 20, wobei eine Vielzahl der Testsubstanzen gleichzeitig durchgemustert wird.

24. Verfahren nach Anspruch 20, wobei eine Vielzahl der Polypeptide mit einem festen Träger verbunden ist.

25. Verwendung eines Polypeptids nach Anspruch 1 für die Identifizierung von Verbindungen, die die Aktivität eines Polypeptids nach Anspruch 1 modulieren.

26. Verwendung nach Anspruch 25, wobei die Verbindungen die proteolytische Aktivität davon hemmen.

27. Verwendung nach Anspruch 25, wobei die MTSP unter MTSP1, MTSP3, MTSP4 und MTSP6, wie sie in Anspruch 3 beansprucht werden, Corin (SEQ ID No. 62), Enteropeptidase (SEQ ID No. 64), der trypsinartigen Protease der menschlichen Luftwege (HAT; SEQ ID No. 66), TMPRSS2 (SEQ ID No. 70) und TMPRSS4 (SEQ ID No. 72) ausgewählt wird.

28. Polypeptid nach einem der Ansprüche 1 bis 7 zur Verwendung bei der Behandlung einer neoplastischen Erkrankung.

29. Verwendung eines Polypeptids nach einem der Ansprüche 1 bis 7 für die Formulierung eines Arzneimittels für die Behandlung einer neoplastischen Erkrankung.

## Revendications

1. Polypeptide monocaténaire isolé, substantiellement purifié, comprenant un domaine de protéase d'une sérine-protéase membranaire de type II (MTSP) ou un de ses fragments catalytiquement actifs, dans lequel :
le domaine de protéase de MTSP ou son fragment catalytiquement actif est la seule partie du polypeptide monocaténaire provenant de la MTSP ;
le domaine de protéase de MTSP ou son fragment catalytiquement actif possède une activité de sérine-protéase à l'état de chaîne unique ;
un résidu Cys libre dans le domaine de protéase est remplacé par un autre aminoacide, ce qui fait que le polypeptide résultant présente une activité protéolytique ; et
la MTSP n'est pas une endothéliase.

2. Polypeptide substantiellement purifié suivant la revendication 1, dans lequel la partie de MTSP a une extrémité N-terminale qui comprend IVNG, ILGG, VGLL ou ILGG.

3. Polypeptide substantiellement purifié suivant la revendication 1, dans lequel la MTSP est choisie entre :
la MTSP1, qui est un polypeptide qui comprend la séquence d'aminoacides représentée dans la SEQ ID N° 2 ou 50, ou qui comprend un domaine de protéase qui présente une identité de séquence d'au moins 80 %, 90 % ou 95 % avec les aminoacides 615-855 de la SEQ ID N° 2 ;
la MTSP3, qui est un polypeptide qui comprend une séquence d'aminoacides ayant une identité de séquence d'au moins environ 85 % ou 90 % avec la séquence d'aminoacides représentée dans la SEQ ID N° 4, ou un polypeptide qui comprend la séquence d'aminoacides représentée en tant qu'aminoacides 205-437 dans la SEQ ID N° 4 ;
la MTSP4, qui est un polypeptide qui comprend une séquence d'aminoacides présentant une identité de séquence d'au moins environ 85 % ou 90 % ou 95 % avec la séquence d'aminoacides représentée dans l'une quelconque des SEQ ID N° 6, 8 et 10, ou un polypeptide qui comprend la séquence d'aminoacides représentée dans l'une quelconque des SEQ ID N° 6, 8 et 10 ; et
la MTSP6, qui est un polypeptide qui comprend une séquence d'aminoacides présentant une identité de séquence d'au moins environ 85 % ou 90 % ou 95 % avec la séquence d'aminoacides représentée dans la SEQ ID N° 12, ou un polypeptide qui comprend la séquence d'aminoacides représentée dans la SEQ ID N° 12.

4. Polypeptide substantiellement purifié suivant la revendication 1, dans lequel le domaine de protéase de MTSP a une séquence de résidus d'aminoacides représentée en tant que résidus d'aminoacides 615-855 de la SEQ ID N° 2, en tant que résidus d'aminoacides 205-437 de la SEQ ID N° 4, en tant que résidus d'aminoacides dans la SEQ ID N° 6 ou en tant que résidus d'aminoacides 217-443 dans la SEQ ID N° 12.

5. Polypeptide substantiellement purifié suivant la revendication 1, qui présente une identité de séquence d'au moins environ 40 %, 60 %, 80 %, 90 % ou 95 % avec un domaine de protéase qui comprend la séquence de résidus d'aminoacides représentée en tant que résidus d'aminoacides 615-855 de la SEQ ID N° 2, en tant que résidus d'aminoacides 205-437 de la SEQ ID N° 4, en tant que résidus d'aminoacides dans la SEQ ID N° 6 ou en tant que résidus d'aminoacides 217-443 dans la SEQ ID N° 12.

6. Polypeptide suivant la revendication 1, dans lequel le résidu Cys libre dans le domaine de protéase est remplacé par un résidu sérine.

7. Polypeptide suivant la revendication 1, dans lequel la MTSP est choisie entre la corine (SEQ ID N° 62), l'entéropeptidase (SEQ ID N° 64), la protéase du type trypsine des voies aériennes humaines (HAT, SEQ ID N° 66), TMPRSS2 (SEQ ID N° 70) et TMPRSS4 (SEQ ID N° 72).

8. Conjugué, comprenant :
a) un polypeptide de l'une quelconque des revendications 1 à 7, et
b) un agent de ciblage lié à la protéine directement ou par un groupe de liaison.

9. Conjugué suivant la revendication 8, dans lequel l'agent de ciblage permet
i) l'isolement ou la purification par affinité du conjugué ;
ii) la fixation du conjugué à une surface ;
iii) la détection du conjugué ; ou
iv) l'administration ciblée à un tissu ou une cellule choisi.

10. Support solide, comprenant deux ou plus de deux polypeptides de l'une quelconque des revendications 1 à 7 liés les uns aux autres directement ou par un groupe de liaison.

11. Support suivant la revendication 10, dans lequel les polypeptides comprennent un réseau.

12. Support suivant la revendication 10, dans lequel les polypeptides comprennent une pluralité de domaines de protéase de MTSP différents.

13. Méthode pour identifier des composés qui modulent l'activité de protéase d'une MTSP, comprenant :
la mise en contact d'un polypeptide de l'une quelconque des revendications 1 à 7 avec un substrat clivé protéolytiquement par la MTSP et, simultanément, avant ou après, l'addition d'un composé d'essai ou d'une pluralité de tels composés ;
la mesure de la quantité de substrat clivé en présence du composé d'essai ; et
la sélection des composés qui modifient la quantité clivée comparativement à un témoin, ce qui permet d'identifier les composés qui modulent l'activité de la MTSP.

14. Méthode suivant la revendication 13, dans laquelle les composés d'essai sont de petites molécules, des peptides, des agents peptidomimétiques, des produits naturels, des anticorps ou leurs fragments.

15. Méthode suivant la revendication 13, dans laquelle une pluralité de substances d'essai sont testées simultanément.

16. Méthode suivant la revendication 13, dans laquelle la variation de la quantité clivée est déterminée en comparant la quantité clivée en présence du composé d'essai avec la quantité en l'absence du composé d'essai.

17. Méthode suivant la revendication 13, dans laquelle une pluralité de polypeptides est liée à un support solide, directement ou par un groupe de liaison.

18. Méthode suivant la revendication 13, dans laquelle les polypeptides comprennent un réseau.

19. Méthode suivant la revendication 13, dans laquelle les polypeptides comprennent une pluralité de protéases de MTSP différentes.

20. Méthode pour identifier un composé qui se lie spécifiquement à un domaine de protéase monocaténaire d'une MTSP, comprenant :
la mise en contact d'un polypeptide de l'une quelconque des revendications 1 à 7 avec un composé d'essai ou une pluralité de tels composés dans des conditions conduisant à leur liaison ; et
l'identification des composés qui se lient spécifiquement à un domaine de protéase monocaténaire de MTSP ou des composés qui inhibent la liaison d'un composé connu en tant que composé se liant au domaine de protéase monocaténaire de MTSP, le composé connu étant mis en contact avec le polypeptide avant, simultanément avec ou après le composé d'essai.

21. Méthode suivant la revendication 20, dans laquelle le polypeptide est lié directement ou indirectement par un groupe de liaison à un support solide.

22. Méthode suivant la revendication 20, dans laquelle les composés d'essai sont de petites molécules, des peptides, des agents peptidomimétiques, des produits naturels, des anticorps ou leurs fragments.

23. Méthode suivant la revendication 20, dans laquelle une pluralité de substances d'essai sont testées simultanément.

24. Méthode suivant la revendication 20, dans laquelle une pluralité de polypeptides est liée à un support solide.

25. Utilisation d'un polypeptide de la revendication 1 pour l'identification de composés qui modulent l'activité d'un polypeptide de la revendication 1.

26. Utilisation suivant la revendication 25, dans laquelle les composés inhibent l'activité protéolytique d'un tel polypeptide.

27. Utilisation suivant la revendication 25, dans laquelle la MTSP est choisie entre les MTSP1, MTSP3, MTSP4 et MTSP6 suivant la revendication 3, la corine (SEQ ID N° 62), l'entéropeptidase (SEQ ID N° 64), la protéase analogue à la trypsine des voies aériennes humaines (HAT, SEQ ID N° 66), TMPRSS2 (SEQ ID N° 70) et TMPRSS4 (SEQ ID N° 72).

28. Polypeptide suivant l'une quelconque des revendications 1 à 7, destiné à être utilisé dans le traitement d'une maladie néoplasique.

29. Utilisation d'un polypeptide de l'une quelconque des revendications 1 à 7 pour la formulation d'un médicament destiné au traitement d'une maladie néoplasique.
